(19) Europäisches Patentamt
European Patent Office
Office européen des brevets

(11) EP 2 123 652 A1

(12) **EUROPEAN PATENT APPLICATION**
published in accordance with Art. 153(4) EPC

(43) Date of publication:
**25.11.2009 Bulletin 2009/48**

(21) Application number: **08711532.5**

(22) Date of filing: **19.02.2008**

(51) Int Cl.:
***C07D 471/10*** (2006.01) ***A61K 31/438*** (2006.01)
***A61K 31/537*** (2006.01) ***A61P 1/00*** (2006.01)
***A61P 1/12*** (2006.01) ***A61P 1/16*** (2006.01)
***A61P 3/00*** (2006.01) ***A61P 3/04*** (2006.01)
***A61P 3/06*** (2006.01) ***A61P 3/10*** (2006.01)
***A61P 5/48*** (2006.01) ***A61P 7/00*** (2006.01)
***A61P 9/04*** (2006.01) ***A61P 9/10*** (2006.01)
***A61P 9/12*** (2006.01) ***A61P 13/12*** (2006.01)
***A61P 15/00*** (2006.01) ***A61P 19/10*** (2006.01)
***A61P 21/04*** (2006.01) ***A61P 25/16*** (2006.01)

(86) International application number:
**PCT/JP2008/052708**

(87) International publication number:
**WO 2008/102749 (28.08.2008 Gazette 2008/35)**

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **20.02.2007 JP 2007039946**

(71) Applicant: **Takeda Pharmaceutical Company Limited**
**Osaka 541-0045 (JP)**

(72) Inventors:
- **FUKATSU, Kohji**
  **Osaka-shi**
  **Osaka 532-8686 (JP)**
- **KAMATA, Makoto**
  **Osaka-shi**
  **Osaka 532-8686 (JP)**
- **YAMASHITA, Tohru**
  **Osaka-shi**
  **Osaka 532-8686 (JP)**

(74) Representative: **Jones, Nicholas Andrew et al**
**Withers & Rogers LLP**
**Goldings House**
**2 Hays Lane**
**GB-London SE1 2HW (GB)**

(54) **HETEROCYCLIC COMPOUND**

(57) The present invention provides a compound having an ACC inhibitory action, which is useful for the prophylaxis or treatment of obesity, diabetes, hypertension, hyperlipidemia, cardiac failure, diabetic complications, metabolic syndrome, sarcopenia, cancer and the like, and has superior efficacy.

The present invention provides a compound represented by the formula (I):

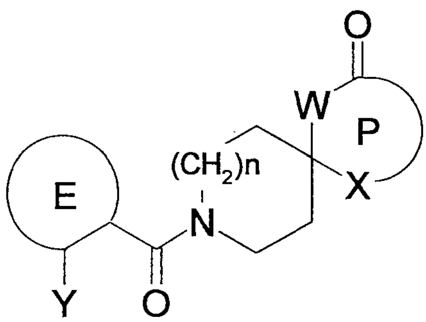

( I )

EP 2 123 652 A1

wherein each symbol is as in the specification, or a salt thereof.

SEQUENCE LISTING

<110> TAKEDA PHARMACEUTICAL COMPANY LIMITED

<120> Heterocyclic Compound

<130> 091178

<150> JP 2007-039946

<151> 2007-02-20

<160> 6

<170> PatentIn version 3.1

<210> 1
<211> 40
<212> DNA
<213> Artificial

<220>
<223> primer for cloning human ACC1 gene

<400> 1
aaaagtcgac ccaccatgga tgaaccttct cccttggccc 40

<210> 2
<211> 37
<212> DNA
<213> Artificial

<220>
<223> primer for cloning human ACC1 gene

<400> 2
aaaagcggcc gcctacgtag aaggggagtc catagtg 37

<210> 3
<211> 41
<212> DNA
<213> Artificial

<220>
<223> primer for cloning human ACC2 gene

<400> 3
aaaagtcgac ccaccatggt cttgcttctt tgtctatctt g 41

<210> 4
<211> 35
<212> DNA
<213> Artificial

<220>
<223> primer for cloning human ACC2 gene

<400> 4
tttttctaga tcaggtagag gccgggctgt ccatg 35

<210> 5
<211> 35
<212> DNA

<213> Artificial

<220>
<223> primer for cloning human ACC2 gene

<400> 5
ccaggtcgac ccgccaacgg gactgggaca caagg 35

<210> 6
<211> 25
<212> DNA
<213> Artificial

<220>
<223> primer for cloning human ACC2 gene

<400> 6
cgcactctca gtttcccgga ttccc 25

## Description

## Technical Field

The present invention relates to a heterocyclic compound having an acetyl-CoA carboxylase (in the present specification, sometimes to be abbreviated as ACC) inhibitory action, which is useful for the prophylaxis or treatment of obesity, diabetes, hypertension, hyperlipidemia, cardiac failure, diabetic complications, metabolic syndrome, sarcopenia, cancer and the like.

## Background Art

ACC is an enzyme that converts acetyl-CoA to malonyl-CoA, and catalyzes a rate determining reaction in fatty acid metabolism. Malonyl-CoA, which is produced by an ACC catalyst reaction, inhibits fatty acid oxidation in mitochondria based on the feedback inhibition of carnitine palmitoyl transferase-1 (CPT-1). Accordingly, ACC plays a key role in controlling the balance between use of carbohydrate and fatty acid in the liver and skeletal muscle, and further, controlling insulin sensitivity in the liver, skeletal muscle and adipose tissue.

A reduced level of malonyl-CoA by ACC inhibition can promote an increase in fatty acid oxidation, decreased secretion of triglyceride (TG)-rich lipoprotein (VLDL) in the liver, regulation of insulin secretion in the pancreas, and further, improvement in the insulin sensitivity in the liver, skeletal muscle and adipose tissue.

In addition, long-term administration of a compound having an ACC inhibitory action can strikingly decrease the TG content of the liver and adipose tissues and selectively decrease body fat in obese test subjects taking low fat diet, by promoting fatty acid oxidation and suppressing de novo synthesis of fatty acid.

Accordingly, a compound having an ACC inhibitory action is extremely useful for the prophylaxis or treatment of metabolic syndrome, obesity, hypertension, diabetes, cardiovascular diseases associated with atherosclerosis and the like.

As ACC inhibitors, the following compound has been reported.

(1) a compound represented by the formula:

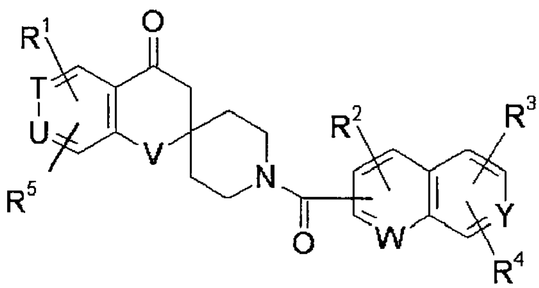

wherein

$R^1$ and $R^5$ are each independently a hydrogen atom, halogen, a lower alkoxy group, $-Q^1-N(R^a)-Q^2-R^b$, a lower alkyl group (optionally substituted by halogen and the like), an aryl or heterocyclic group (each optionally substituted by halogen and the like) or the like;

$R^2$, $R^3$ and $R^4$ are each independently a hydrogen atom, halogen, a lower alkoxy group (optionally substituted by halogen and the like), an aryl or aromatic heterocyclic group (each optionally substituted by halogen and the like) or the like;

$Q^1$ and $Q^2$ are each independently a single bond, -CO-, $-SO_2-$ or the like;

$R^a$ and $R^b$ are each independently a hydrogen atom, halogen, a lower alkoxy group, $-N(R^i)R^j$, a lower alkyl group (optionally substituted by halogen and the like), an aromatic heterocyclic group optionally substituted by a lower alkyl group (optionally substituted by halogen and the like), or the like;

$R^i$ and $R^j$ are each independently a hydrogen atom, a lower alkyl group or a lower haloalkyl group; and

T, U, W and Y are each independently a nitrogen atom or methine;

V is an oxygen atom or a sulfur atom

(see patent documents 1 and 2).

(2) a compound represented by the formula:

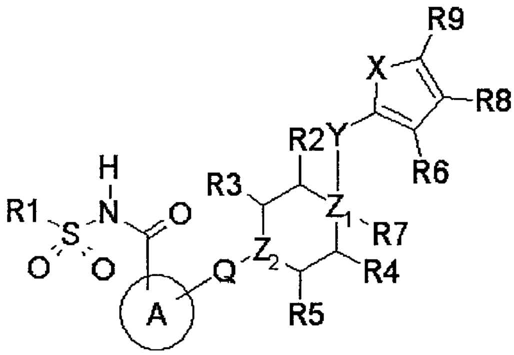

or

**[0013]**

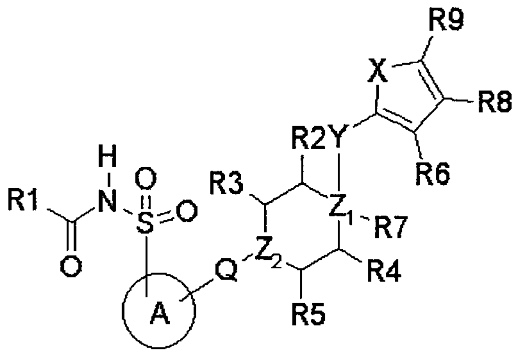

wherein

ring A is an optionally substituted aromatic hydrocarbon group, an optionally substituted aromatic heterocyclic group, an optionally substituted cycloalkenyl group or an optionally substituted cyclic alkyl group;

Q is $-CH_2-$, $-C_2H_4-$, $-C_3H_6-$, -CH=CH-, $-CH=CHCH_2-$, $-CH_2CH=CH-$, - $CH_2NHCO-$, $-NHCOCH_2-$, $-CONHCH_2-$, -NH-CO-, -CONH-, -NHCONH-, - $CH_2NHCS-$, $-NHCSCH_2-$, $-CSNHCH_2-$, -NHCS-, -CSNH-, -NHCSNH-, - $CH_2NHSO_2-$, $-NHSO_2CH_2-$, $-SO_2NHCH_2-$, $-NHSO_2-$, $-SO_2NH-$, $-NHSO_2NH-$, - S-, -O- or -NH-;

R1 is an optionally substituted $C_{1-12}$ alkyl group, an optionally substituted aromatic hydrocarbon group, an optionally substituted aromatic heterocyclic group, an optionally substituted $C_{1-12}$ alkoxy group or the like;

R2 to R9 are each independently an optionally substituted $C_{1-12}$ alkyl group, an optionally substituted aromatic hydrocarbon group, an optionally substituted aromatic heterocyclic group, a hydrogen atom, a hydroxy group, halogen or the like;

X is -CR10=CR11-, -N=CR10-, -CR10=N-, -S-, -O-, -NH- or - CH(R10)-;

R10 and R11 are each independently an optionally substituted $C_{1-12}$ alkyl group, an optionally substituted $C_{1-12}$ alkoxy group, an optionally substituted aromatic hydrocarbon group, an optionally substituted aromatic heterocyclic group, a hydrogen atom, a hydroxy group, halogen or the like;

Y is -C(O)-, -S-, -S(O)-, $-S(O)_2-$, -N(R13)-, -CH(R14)- or -O-; R13 is an optionally substituted $C_{1-12}$ alkyl group, an optionally substituted aromatic hydrocarbon group, an optionally substituted aromatic heterocyclic group, a hydrogen atom or the like;

R14 is an optionally substituted $C_{1-12}$ alkyl group, an optionally substituted $C_{1-12}$ alkoxy group, an optionally substituted aromatic hydrocarbon group, an optionally substituted aromatic heterocyclic group, a hydrogen atom, a hydroxy group, halogen or the like; and
$Z_1$ is a nitrogen atom or a carbon atom,

provided that when $Z_1$ is a nitrogen atom, then R7 should be unsubstituted, and when $Z_1$ is a carbon atom, then R6 and R7 in combination form the linkage represented by the following (a) to (x):
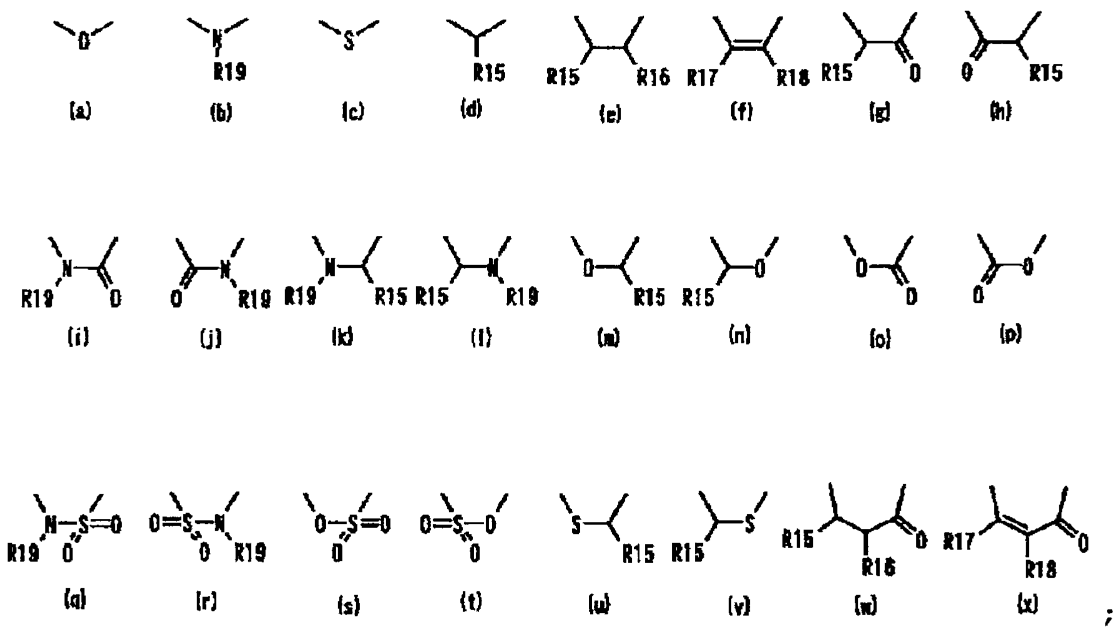

(a) (b) (c) (d) (e) (f) (g) (h) (i) (j) (k) (l) (m) (n) (o) (p) (q) (r) (s) (t) (u) (v) (w) (x) ;

R15 to R18 are each independently an optionally substituted $C_{1-12}$ alkyl group, an optionally substituted $C_{1-12}$ alkoxy group, an optionally substituted aromatic hydrocarbon group, an optionally substituted aromatic heterocyclic group, a hydrogen atom, a hydroxy group, halogen or the like;
R19 is an optionally substituted $C_{1-12}$ alkyl group, an optionally substituted aromatic hydrocarbon group, an optionally substituted aromatic heterocyclic group, a hydrogen atom or the like; and
$Z_2$ is -CH= or N

(see patent document 3).
(3) a compound represented by formula:

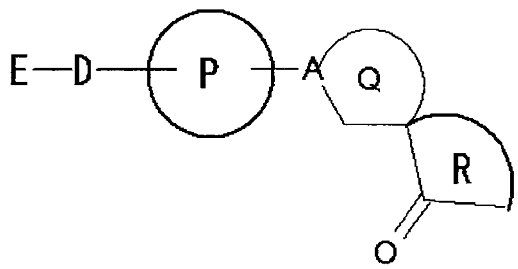

wherein

E is an optionally substituted cyclic group;
D is a carbonyl group or a sulfonyl group;
A is CH or N;
ring P is an optionally further substituted 5- to 7-membered ring;
ring Q is an optionally further substituted 5- to 7-membered non-aromatic ring; and
ring R is an optionally fused 5- to 7-membered non-aromatic ring, which is optionally further substituted

(see patent document 4).
However, the compound of the present invention is not reported.

patent document 1: WO 2007/011809
patent document 2: WO 2007/011811
patent document 3: JP-A-2005-119987
patent document 4: WO 2007/013691

**Disclosure of the Invention**

**Problems to be Solved by the Invention**

There is a demand for the development of a compound having an ACC inhibitory action, which is useful for the prophylaxis or treatment of obesity, diabetes, hypertension, hyperlipidemia, cardiac failure, diabetic complications, metabolic syndrome, sarcopenia, cancer and the like, and has superior efficacy.
The present inventors have found that a compound represented by the formula (I):
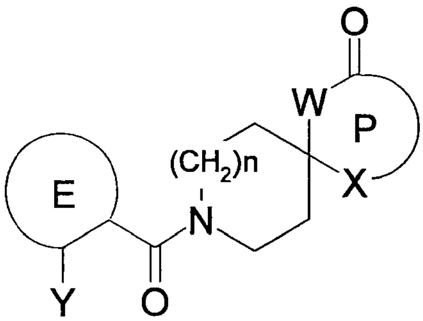

(I)

wherein

ring E is an optionally further substituted 6-membered aromatic ring, or an optionally fused 5-membered aromatic heterocycle which is optionally further substituted;
ring P is an optionally fused non-aromatic ring which is optionally further substituted;
W is O, S, a $C_{1-4}$ alkylene or $NR^{3a}$ wherein $R^{3a}$ is a hydrogen atom or a substituent;
X is O, S, SO, $SO_2$, CO, $CR^1R^2$ or $NR^{3b}$ wherein $R^1$, $R^2$ and $R^{3b}$ are the same or different and each is a hydrogen atom or a substituent;
Y is an optionally substituted amino group; and
n is 0, 1, 2 or 3,
or a salt thereof [hereinafter sometimes to be referred to as compound (I)] has a superior ACC inhibitory action, which is useful for the prophylaxis or treatment of obesity, diabetes, hypertension, hyperlipidemia, cardiac failure, diabetic complications, metabolic syndrome, sarcopenia, cancer and the like, and has superior efficacy. Based on this finding, the present inventors have conducted intensive studies and completed the present invention.

Accordingly, the present invention relates to

(1) compound (I);

(2) compound (I) wherein the optionally fused 5-membered aromatic heterocycle of the optionally fused 5-membered aromatic heterocycle which is optionally further substituted for ring E is an optionally fused thiophene ring;
(3) compound (I) wherein the 6-membered aromatic ring of the optionally further substituted 6-membered aromatic ring for ring E, or the optionally fused 5-membered aromatic heterocycle of the optionally fused 5-membered aromatic heterocycle which is optionally further substituted for ring E is a benzene ring, a pyridine ring, a thiophene ring, a benzothiophene ring or a thienopyridine ring;
(4) compound (I) wherein the optionally fused non-aromatic ring of the optionally fused non-aromatic ring which is optionally further substituted for ring P is a dihydrochromene ring, an optionally oxidized dihydrothiochromene ring or a piperidine ring, each of which is substituted by oxo group(s);
(5) a compound represented by the formula (IIa):

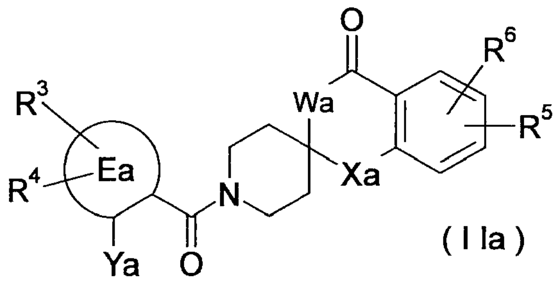

(IIa)

wherein

ring Ea is a thiophene ring optionally condensed with a benzene ring or a pyridine ring, or a pyridine ring;
Wa is O, S, $CH_2$ or NH;
Xa is O, S, SO, $SO_2$, CO, $CH_2$ or NH;
Ya is $-NH_2$, -NHCONHR, -NHCOOR or -NHCOR;
$R^3$ to $R^6$ are the same or different and each is a hydrogen atom, a halogen atom, a $C_{1-6}$ alkyl group, a $C_{1-6}$ alkoxy group, a cyano group, a nitro group, an amino group, a carboxy group, a hydroxy group, -COR, $-NHSO_2R$, -NHCONHR, $-C_{1-6}$ alkylene-COOH, $-C_{1-6}$ alkylene-COOR, $-O-C_{1-6}$ alkylene-$C_{6-14}$ arene, -CONH-(5- or 6-membered heterocycle), a 5- or 6-membered heterocyclic group or a $C_{6-14}$ aryl group; and
R is a hydrogen atom or a $C_{1-6}$ alkyl group,
or a salt thereof [hereinafter sometimes to be referred to as compound (IIa)];

(6) compound (IIa) wherein ring Ea is a thiophene ring optionally condensed with a benzene ring or a pyridine ring;
(7) compound (IIa) wherein ring Ea is a thiophene ring, a benzothiophene ring or a thienopyridine ring;
(8) 1'-({7-methoxy-2-[(methylcarbamoyl)amino]-1-benzothiophen-3-yl}carbonyl)-4-oxo-3,4-dihydrospiro[chromene-2,4'-piperidine]-6-carboxylic acid,
1-ethyl-3-(3-{[4'-oxo-6'-(5-oxo-4,5-dihydro-1,2,4-oxadiazol-3-yl)-3',4'-dihydro-1H-spiro[piperidine-4,2'-thiochromen]-1-yl]carbonyl}-1-benzothiophen-2-yl)urea,
4-{5-[(ethylcarbamoyl)amino]-4-[(4'-oxo-3',4'-dihydro-1H-spiro[piperidine-4,2'-thiochromen]-1-yl)carbonyl]thiophen-2-yl}benzoic acid,
N-{3-[(6-cyano-4-oxo-3,4-dihydro-1'H-spiro[chromene-2,4'-piperidin]-1'-yl)carbonyl]-6-methoxy-1-benzothiophen-2-yl}acetamide,
N-[({3-[(6-cyano-4-oxo-3,4-dihydro-1'H-spiro[chromene-2,4'-piperidin]-1'-yl)carbonyl]-7-methoxy-1-benzothiophen-2-yl}amino)carbonyl]methanesulfonamide,
1-{3-[(6'-cyano-4'-oxo-3',4'-dihydro-1H-spiro[piperidine-4,2'-thiochromen]-1-yl)carbonyl]-6-(trifluoromethyl)thieno[2,3-b]pyridin-2-yl}-3-methylurea,
1-{3-[(6-methyl-4-oxo-3,4-dihydro-1'H-spiro[chromene-2,4'-piperidin]-1'-yl)carbonyl]-6-(trifluoromethyl)thieno[2,3-b]pyridin-2-yl}-3-methylurea,
1-{3-[(6-cyano-4-oxo-3,4-dihydro-1'H-spiro[chromene-2,4'-piperidin]-1'-yl)carbonyl]-6-(trifluoromethyl)thieno[2,3-b]pyridin-2-yl}-3-methylurea,
1-{3-[(6-cyano-4-oxo-3,4-dihydro-1'H-spiro[chromene-2,4'-piperidin]-1'-yl)carbonyl]-6-(trifluoromethyl)thieno[2,3-b]pyridin-2-yl}-3-ethylurea,
1-ethyl-3-{3-[(6-methyl-4-oxo-3,4-dihydro-1'H-spiro[chromene-2,4'-piperidin]-1'-yl)carbonyl]-7-oxo-4,5,6,7-tetrahydro-1-benzothiophen-2-yl}urea, or
1-ethyl-3-(7-oxo-3-{[4-oxo-6-(trifluoromethyl)-3,4-dihydro-1'H-spiro[chromene-2,4'-piperidin]-1'-yl]carbonyl}-4,5,6,7-tetrahydro-1-benzothiophen-2-yl)urea
or a salt thereof;
(9) a prodrug of compound (I) or compound (IIa);
(10) a pharmaceutical agent comprising compound (I) or compound (IIa) or a prodrug thereof;
(11) the pharmaceutical agent of the above-mentioned (10), which is an acetyl-CoA carboxylase inhibitor;
(12) the pharmaceutical agent of the above-mentioned (10), which is an agent for the prophylaxis or treatment of obesity, diabetes, hypertension, hyperlipidemia, cardiac failure, diabetic complications, metabolic syndrome, sarcopenia or cancer;
(13) use of compound (I) or compound (IIa) or a prodrug thereof, for the production of an acetyl-CoA carboxylase inhibitor;
(14) use of compound (I) or compound (IIa) or a prodrug thereof, for the production of an agent for the prophylaxis or treatment of obesity, diabetes, hypertension, hyperlipidemia, cardiac failure, diabetic complications, metabolic syndrome, sarcopenia or cancer;
(15) a method of inhibiting acetyl-CoA carboxylase in a mammal, which comprises administering compound (I) or compound (IIa) or a prodrug thereof to the mammal;
(16) a method for the prophylaxis or treatment of obesity, diabetes, hypertension, hyperlipidemia, cardiac failure, diabetic complications, metabolic syndrome, sarcopenia or cancer in a mammal, which comprises administering compound (I) or compound (IIa) or a prodrug thereof to the mammal;

and the like.

**Effect of the Invention**

The compound of the present invention has an ACC inhibitory action, which is useful for the prophylaxis or treatment of obesity, diabetes, hypertension, hyperlipidemia, cardiac failure, diabetic complications, metabolic syndrome, sarcopenia, cancer and the like, and has superior efficacy.

[Detailed Description of the Invention]

The definition of each symbol in the formula (I) is described in detail in the following.
The "halogen atom" in the present specification means, unless otherwise specified, a fluorine atom, a chlorine atom, a bromine atom or an iodine atom.
The "$C_{1-3}$ alkylenedioxy group" in the present specification means, unless otherwise specified, methylenedioxy, ethylenedioxy or the like.
The "$C_{1-6}$ alkyl group" in the present specification means, unless otherwise specified, methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, 2-ethylbutyl or the like.
The "$C_{1-6}$ alkoxy group" in the present specification means, unless otherwise specified, methoxy, ethoxy, propoxy, isopropoxy, butoxy, isobutoxy, sec-butoxy, tert-butoxy or the like.
The "$C_{1-6}$ alkoxy-carbonyl group" in the present specification means, unless otherwise specified, methoxycarbonyl, ethoxycarbonyl, propoxycarbonyl, tert-butoxycarbonyl or the like.
The "$C_{1-6}$ alkyl-carbonyl group" in the present specification means, unless otherwise specified, acetyl, propanoyl, butanoyl, isobutanoyl, pentanoyl, isopentanoyl, hexanoyl or the like.
X is O, S, SO, $SO_2$, CO, $CR^1R^2$ or $NR^{3b}$ wherein $R^1$, $R^2$ and $R^{3b}$ are the same or different and each is a hydrogen atom or a substituent.
Examples of the "substituent" for $R^1$, $R^2$ or $R^{3b}$ include an "optionally substituted hydrocarbon group", an "optionally substituted heterocyclic group", an "optionally substituted hydroxy group", an "optionally substituted mercapto group", an "optionally substituted amino group", a "cyano group", a "nitro group", an "acyl group", a "halogen atom" and the like.
Examples of the "hydrocarbon group" of the above-mentioned "optionally substituted hydrocarbon group" include a $C_{1-10}$ alkyl group, a $C_{2-10}$ alkenyl group, a $C_{2-10}$ alkynyl group, a $C_{3-10}$ cycloalkyl group, a $C_{3-10}$ cycloalkenyl group, a $C_{4-10}$ cycloalkadienyl group, a $C_{6-14}$ aryl group, a $C_{7-13}$ aralkyl group, a $C_{8-13}$ arylalkenyl group and the like.
Examples of the $C_{1-10}$ alkyl group include methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, 2-ethylbutyl, heptyl, octyl, nonyl, decyl and the like.
Examples of the $C_{2-10}$ alkenyl group include ethenyl, 1-propenyl, 2-propenyl, 2-methyl-1-propenyl, 1-butenyl, 2-butenyl, 3-butenyl, 3-methyl-2-butenyl, 1-pentenyl, 2-pentenyl, 3-pentenyl, 4-pentenyl, 4-methyl-3-pentenyl, 1-hexenyl,

3-hexenyl, 5-hexenyl, 1-heptenyl, 1-octenyl and the like.

Examples of the $C_{2-10}$ alkynyl group include ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl, 1-heptynyl, 1-octynyl and the like.

Examples of the $C_{3-10}$ cycloalkyl group include cyclopropyl, cyclobutyl, cyclopentyl, cyclohexyl, cycloheptyl, cyclooctyl, bicyclo[2.2.1]heptyl, bicyclo[2.2.2]octyl, bicyclo[3.2.1]octyl, bicyclo[3.2.2]nonyl, bicyclo[3.3.1]nonyl, bicyclo [4.2.1]nonyl, bicyclo[4.3.1]decyl, adamantyl and the like.

Examples of the $C_{3-10}$ cycloalkenyl group include 2-cyclopenten-1-yl, 3-cyclopenten-1-yl, 2-cyclohexen-1-yl, 3-cyclohexen-1-yl and the like.

Examples of the $C_{4-10}$ cycloalkadienyl group include 2,4-cyclopentadien-1-yl, 2,4-cyclohexadien-1-yl, 2,5-cyclohexadien-1-yl and the like.

The above-mentioned $C_{3-10}$ cycloalkyl group, $C_{3-10}$ cycloalkenyl group and $C_{4-10}$ cycloalkadienyl group are each optionally condensed with a benzene ring to form a fused ring group. Examples of the fused ring group include indanyl, dihydronaphthyl, tetrahydronaphthyl, fluorenyl and the like.

Examples of the $C_{6-14}$ aryl group include phenyl, naphthyl, anthryl, phenanthryl, acenaphthyl, biphenylyl and the like.

Examples of the $C_{7-13}$ aralkyl group include benzyl, phenethyl, naphthylmethyl, biphenylylmethyl and the like. Examples of the $C_{8-13}$ arylalkenyl group include styryl and the like.

The $C_{1-10}$ alkyl group, $C_{2-10}$ alkenyl group and $C_{2-10}$ alkynyl group which are exemplified as the above-mentioned "hydrocarbon group" optionally have 1 to 3 substituents at substitutable positions.

Examples of the substituent include

(1) a $C_{3-10}$ cycloalkyl group (e.g., cyclopropyl, cyclohexyl);
(2) a $C_{6-14}$ aryl group (e.g., phenyl, naphthyl) optionally substituted by 1 to 3 substituents selected from

(a) a $C_{1-6}$ alkyl group optionally substituted by 1 to 3 halogen atoms,
(b) a hydroxy group,
(c) a $C_{1-6}$ alkoxy group optionally substituted by 1 to 3 substituents selected from a halogen atom and a $C_{6-14}$ aryl group (e.g., phenyl),
(d) a halogen atom,
(e) a $C_{1-6}$ alkoxy-carbonyl group optionally substituted by 1 to 3 $C_{6-14}$ aryl groups (e.g., phenyl), and
(f) a carboxy group;

(3) an aromatic heterocyclic group (e.g., thienyl, furyl, pyridyl, pyrazolyl, imidazolyl, tetrazolyl, oxazolyl, thiazolyl, oxadiazolyl, thiadiazolyl) optionally substituted by 1 to 3 substituents selected from

(a) a $C_{1-6}$ alkyl group optionally substituted by 1 to 3 halogen atoms,
(b) a hydroxy group,
(c) a $C_{1-6}$ alkoxy group optionally substituted by 1 to 3 halogen atoms, and
(d) a halogen atom;

(4) a non-aromatic heterocyclic group (e.g., tetrahydrofuryl, morpholinyl, thiomorpholinyl, piperidyl, pyrrolidinyl, piperazinyl, dihydrooxadiazolyl, dihydrothiadiazolyl) optionally substituted by 1 to 3 substituents selected from

(a) a $C_{1-6}$ alkyl group optionally substituted by 1 to 3 halogen atoms,
(b) a hydroxy group,
(c) a $C_{1-6}$ alkoxy group optionally substituted by 1 to 3 halogen atoms,
(d) a halogen atom,
(e) an oxo group, and
(f) an amino group;

(5) an amino group optionally mono- or di-substituted by substituent(s) selected from

(a) a $C_{1-6}$ alkyl group optionally substituted by 1 to 3 halogen atoms,
(b) a $C_{1-6}$ alkyl-carbonyl group optionally substituted by 1 to 3 halogen atoms,
(c) a $C_{1-6}$ alkoxy-carbonyl group optionally substituted by 1 to 3 halogen atoms,
(d) a $C_{1-6}$ alkylsulfonyl group (e.g., methylsulfonyl) optionally substituted by 1 to 3 halogen atoms,
(e) a carbamoyl group optionally mono- or di-substituted by $C_{1-6}$ alkyl group(s) optionally substituted by 1 to 3

halogen atoms, and
(f) an aromatic heterocyclic group (e.g., thienyl, furyl, pyridyl, pyrazolyl, imidazolyl, tetrazolyl, oxazolyl, thiazolyl, oxadiazolyl, thiadiazolyl);

(6) a $C_{1-6}$ alkyl-carbonyl group optionally substituted by 1 to 3 halogen atoms;
(7) a $C_{1-6}$ alkoxy-carbonyl group optionally substituted by 1 to 3 substituents selected from

(a) a halogen atom,
(b) a $C_{1-6}$ alkoxy group, and
(c) a $C_{6-14}$ aryl group (e.g., phenyl);

(8) a $C_{1-6}$ alkylsulfonyl group (e.g., methylsulfonyl, ethylsulfonyl, isopropylsulfonyl) optionally substituted by 1 to 3 halogen atoms;
(9) a carbamoyl group optionally mono- or di-substituted by substituent(s) selected from

(a) a $C_{1-6}$ alkyl-carbonyl group optionally substituted by 1 to 3 halogen atoms, and
(b) a heterocyclic group (e.g., triazolyl);

(10) a thiocarbamoyl group optionally mono- or di-substituted by $C_{1-6}$ alkyl group(s) optionally substituted by 1 to 3 halogen atoms;
(11) a sulfamoyl group optionally mono- or di-substituted by $C_{1-6}$ alkyl group(s) optionally substituted by 1 to 3 halogen atoms;
(12) a carboxy group;
(13) a hydroxy group;
(14) a $C_{1-6}$ alkoxy group optionally substituted by 1 to 3 substituents selected from

(a) a halogen atom,
(b) a carboxy group,
(c) a $C_{1-6}$ alkoxy group,
(d) a $C_{1-6}$ alkoxy-carbonyl group optionally substituted by 1 to 3 $C_{6-14}$ aryl groups (e.g., phenyl),
(e) an amino group optionally mono- or di-substituted by substituent(s) selected from a $C_{1-6}$ alkyl group and a $C_{1-6}$ alkoxy-carbonyl group, and
(f) a $C_{6-14}$ aryl group (e.g., phenyl);

(15) a $C_{2-6}$ alkenyloxy group (e.g., ethenyloxy) optionally substituted by 1 to 3 halogen atoms;
(16) a $C_{7-13}$ aralkyloxy group (e.g., benzyloxy);
(17) a $C_{6-14}$ aryloxy group (e.g., phenyloxy, naphthyloxy);
(18) a $C_{1-6}$ alkyl-carbonyloxy group (e.g., acetyloxy, tert-butylcarbonyloxy);
(19) a $C_{6-14}$ aryl-carbonyl group (e.g., benzoyl) optionally substituted by 1 to 3 substituents selected from

(a) a halogen atom, and
(b) a $C_{1-6}$ alkyl group optionally substituted by 1 to 3 halogen atoms;

(20) a non-aromatic heterocyclylcarbonyl group (e.g., pyrrolidinylcarbonyl, morpholinylcarbonyl) optionally substituted by 1 to 3 $C_{1-6}$ alkyl groups optionally substituted by 1 to 3 halogen atoms;
(21) a mercapto group;
(22) a $C_{1-6}$ alkylthio group (e.g., methylthio, ethylthio) optionally substituted by 1 to 3 substituents selected from

(a) a halogen atom, and
(b) a $C_{1-6}$ alkoxy-carbonyl group;

(23) a $C_{7-13}$ aralkylthio group (e.g., benzylthio);
(24) a $C_{6-14}$ arylthio group (e.g., phenylthio, naphthylthio);
(25) a cyano group;
(26) a nitro group;
(27) a halogen atom;
(28) a $C_{1-3}$ alkylenedioxy group;
(29) an aromatic heterocyclylcarbonyl group (e.g., pyrazolylcarbonyl, pyrazinylcarbonyl, isoxazolylcarbonyl, pyridylcarbonyl, thiazolylcarbonyl) optionally substituted by 1 to 3 $C_{1-6}$ alkyl groups optionally substituted by 1 to 3 halogen atoms;
(30) a formyl group;

and the like. When the number of the substituents is not less than 2, the respective substituents may be the same or different.
The $C_{3-10}$ cycloalkyl group, $C_{3-10}$ cycloalkenyl group, $C_{4-10}$ cycloalkadienyl group, $C_{6-14}$ aryl group, $C_{7-13}$ aralkyl group and $C_{8-13}$ arylalkenyl group which are exemplified as the above-mentioned "hydrocarbon group" optionally have 1 to 3 substituents at substitutable positions.
Examples of the substituent include

(1) the groups exemplified as the substituents for the above-mentioned $C_{1-10}$ alkyl group and the like;
(2) a $C_{1-6}$ alkyl group optionally substituted by 1 to 3 substituents selected from

(a) a halogen atom,
(b) a carboxy group,
(c) a hydroxy group,
(d) a $C_{1-6}$ alkoxy-carbonyl group optionally substituted by 1 to 3 $C_{6-14}$ aryl groups (e.g., phenyl),
(e) a $C_{1-6}$ alkoxy group,
(f) an amino group optionally mono- or di-substituted by $C_{1-6}$ alkyl group(s), and
(g) a 2,4-dioxo-1,3-thiazolidin-5-ylidene;

(3) a $C_{2-6}$ alkenyl group (e.g., ethenyl, 1-propenyl) optionally substituted by 1 to 3 substituents selected from

(a) a halogen atom,
(b) a carboxy group,
(c) a hydroxy group,
(d) a $C_{1-6}$ alkoxy-carbonyl group,
(e) a $C_{1-6}$ alkoxy group, and
(f) an amino group optionally mono- or di-substituted by $C_{1-6}$ alkyl group(s);

(4) a $C_{7-13}$ aralkyl group (e.g., benzyl) optionally substituted by 1 to 3 substituents selected from

(a) a $C_{1-6}$ alkyl group optionally substituted by 1 to 3 halogen atoms,
(b) a hydroxy group,
(c) a $C_{1-6}$ alkoxy group, and
(d) a halogen atom;

and the like. When the number of the substituents is not less than 2, the respective substituents may be the same or different.
Examples of the "heterocyclic group" of the above-mentioned "optionally substituted heterocyclic group" include an "aromatic heterocyclic group" and a "non-aromatic heterocyclic group".
Examples of the aromatic heterocyclic group include a 4-to 7-membered (preferably 5- or 6-menbered) monocyclic aromatic heterocyclic group containing, as a ring-constituting atom besides carbon atoms, 1 to 4 hetero atoms selected from an oxygen atom, a sulfur atom and a nitrogen atom, and a fused aromatic heterocyclic group. Examples of the fused aromatic heterocyclic group include a group derived from a fused ring wherein a ring corresponding to the 4- to 7-membered monocyclic aromatic heterocyclic group and 1 or 2 rings selected from a 5- or 6-membered aromatic heterocycle containing 1 or 2 nitrogen atoms (e.g., pyrrole, imidazole, pyrazole, pyrazine, pyridine, pyrimidine), a 5-membered aromatic heterocycle containing one sulfur atom (e.g., thiophene) and a benzene ring are condensed, and the like.
Preferable examples of the aromatic heterocyclic group include monocyclic aromatic heterocyclic groups such as furyl (e.g., 2-furyl, 3-furyl), thienyl (e.g., 2-thienyl, 3-thienyl), pyridyl (e.g., 2-pyridyl, 3-pyridyl, 4-pyridyl), pyrimidinyl (e.g., 2-pyrimidinyl, 4-pyrimidinyl, 5-pyrimidinyl), pyridazinyl (e.g., 3-pyridazinyl, 4-pyridazinyl), pyrazinyl (e.g., 2-pyrazinyl), pyrrolyl (e.g., 1-pyrrolyl, 2-pyrrolyl, 3-pyrrolyl), imidazolyl (e.g., 1-imidazolyl, 2-imidazolyl, 4-imidazolyl, 5-imidazolyl), pyrazolyl (e.g., 1-pyrazolyl, 3-pyrazolyl, 4-pyrazolyl), thiazolyl (e.g., 2-thiazolyl, 4-thiazolyl, 5-thiazolyl), isothiazolyl (e.g., 3-isothiazolyl, 4-isothiazolyl, 5-isothiazolyl), oxazolyl (e.g., 2-oxazolyl, 4-oxazolyl, 5-oxazolyl), isoxazolyl (e.g., 3-isoxazolyl, 4-isoxazolyl, 5-isoxazolyl), oxadiazolyl (e.g., 1,2,4-oxadiazol-5-yl, 1,3,4-oxadiazol-2-yl), thiadiazolyl (e.g., 1,3,4-thiadiazol-2-yl), triazolyl (e.g., 1,2,4-triazol-1-yl, 1,2,4-triazol-3-yl, 1,2,3-triazol-1-yl, 1,2,3-triazol-2-yl, 1,2,3-triazol-4-yl),

tetrazolyl (e.g., tetrazol-1-yl, tetrazol-5-yl), triazinyl (e.g., 1,2,4-triazin-1-yl, 1,2,4-triazin-3-yl) and the like;
fused aromatic heterocyclic groups such as quinolyl (e.g., 2-quinolyl, 3-quinolyl, 4-quinolyl, 6-quinolyl), isoquinolyl (e.g., 3-isoquinolyl), quinazolyl (e.g., 2-quinazolyl, 4-quinazolyl), quinoxalyl (e.g., 2-quinoxalyl, 6-quinoxalyl), benzofuranyl (e.g., 2-benzofuranyl, 3-benzofuranyl), benzothienyl (e.g., 2-benzothienyl, 3-benzothienyl), benzoxazolyl (e.g., 2-benzoxazolyl), benzisoxazolyl (e.g., 7-benzisoxazolyl), benzothiazolyl (e.g., 2-benzothiazolyl), benzimidazolyl (e.g., benzimidazol-1-yl, benzimidazol-2-yl, benzimidazol-5-yl), benzotriazolyl (e.g., 1H-1,2,3-benzotriazol-5-yl), indolyl (e.g., indol-1-yl, indol-2-yl, indol-3-yl, indol-5-yl), indazolyl (e.g., 1H-indazol-3-yl), pyrrolopyrazinyl (e.g., 1H-pyrrolo[2,3-b]pyrazin-2-yl, 1H-pyrrolo[2,3-b]pyrazin-6-yl), imidazopyridinyl (e.g., 1H-imidazo[4,5-b]pyridin-2-yl, 1H-imidazo[4,5-c]pyridin-2-yl, 2H-imidazo[1,2-a]pyridin-3-yl), thienopyridinyl (e.g., thieno[2,3-b]pyridin-3-yl), imidazopyrazinyl (e.g., 1H-imidazo[4,5-b]pyrazin-2-yl), pyrazolopyridinyl (e.g., 1H-pyrazolo[4,3-c]pyridin-3-yl), pyrazolothienyl (e.g., 2H-pyrazolo[3,4-b]thiophen-2-yl), pyrazolotriazinyl (e.g., pyrazolo[5,1-c][1,2,4]triazin-3-yl) and the like;
and the like.

Examples of the non-aromatic heterocyclic group include a 4- to 7-membered (preferably 5- or 6-menbered) monocyclic non-aromatic heterocyclic group containing, as a ring-constituting atom besides carbon atoms, 1 to 4 hetero atoms selected from an oxygen atom, a sulfur atom and a nitrogen atom, and a fused aromatic heterocyclic group. Examples of the fused aromatic heterocyclic group include a group derived from a fused ring wherein a ring corresponding to the 4- to 7-membered monocyclic non-aromatic heterocyclic group and 1 or 2 rings selected from a 5- or 6-membered aromatic heterocycle containing 1 or 2 nitrogen atoms (e.g., pyrrole, imidazole, pyrazole, pyrazine, pyridine, pyrimidine), a 5-membered aromatic heterocycle containing one sulfur atom (e.g., thiophene) and a benzene ring are condensed, a group wherein the above-mentioned group is partially saturated, and the like.

Preferable examples of the non-aromatic heterocyclic group include monocyclic non-aromatic heterocyclic groups such as pyrrolidinyl (e.g., 1-pyrrolidinyl, 2-pyrrolidinyl), piperidyl (e.g., piperidino, 2-piperidyl, 3-piperidyl, 4-piperidyl), morpholinyl (e.g., morpholino), thiomorpholinyl (e.g., thiomorpholino), piperazinyl (e.g., 1-piperazinyl, 2-piperazinyl, 3-piperazinyl), hexamethyleniminyl (e.g., hexamethylenimin-1-yl), oxazolidinyl (e.g., oxazolidin-2-yl), thiazolidinyl (e.g., thiazolidin-2-yl), imidazolidinyl (e.g., imidazolidin-2-yl, imidazolidin-3-yl), oxazolinyl (e.g., oxazolin-2-yl), thiazolinyl (e.g., thiazolin-2-yl), imidazolinyl (e.g., imidazolin-2-yl, imidazolin-3-yl), dioxolyl (e.g., 1,3-dioxol-4-yl), dioxolanyl (e.g., 1,3-dioxolan-4-yl), dihydrooxadiazolyl (e.g., 4,5-dihydro-1,2,4-oxadiazol-3-yl), pyranyl (e.g., 4-pyranyl), tetrahydropyranyl (e.g., 2-tetrahydropyranyl, 3-tetrahydropyranyl, 4-tetrahydropyranyl), thiopyranyl (e.g., 4-thiopyranyl), tetrahydrothiopyranyl (e.g., 2-tetrahydrothiopyranyl, 3-tetrahydrothiopyranyl, 4-tetrahydrothiopyranyl), tetrahydrofuryl (e.g., tetrahydrofuran-3-yl, tetrahydrofuran-2-yl), pyrazolidinyl (e.g., pyrazolidin-1-yl, pyrazolidin-3-yl), pyrazolinyl (e.g., pyrazolin-1-yl), tetrahydropyrimidinyl (e.g., tetrahydropyrimidin-1-yl), dihydrotriazolyl (e.g., 2,3-dihydro-1H-1,2,3-triazol-1-yl), tetrahydrotriazolyl (e.g., 2,3,4,5-tetrahydro-1H-1,2,3-triazol-1-yl), tetrahydrooxazinyl (e.g., 3,4,5,6-tetrahydro-1,3-oxazinyl) and the like;
fused non-aromatic heterocyclic groups such as dihydroindolyl (e.g., 2,3-dihydro-1H-indol-1-yl), dihydroisoindolyl (e.g., 1,3-dihydro-2H-isoindol-2-yl), dihydrobenzofuranyl (e.g., 2,3-dihydro-1-benzofuran-5-yl), dihydrobenzodioxinyl (e.g., 2,3-dihydro-1,4-benzodioxinyl), dihydrobenzodioxepinyl (e.g., 3,4-dihydro-2H-1,5-benzodioxepinyl), tetrahydrobenzofuranyl (e.g., 4,5,6,7-tetrahydro-1-benzofuran-3-yl), chromenyl (e.g., 4H-chromen-2-yl, 2H-chromen-3-yl), dihydrochromenyl (e.g., 3,4-dihydro-2H-chromen-2-yl), dihydroquinolinyl (e.g., 1,2-dihydroquinolin-4-yl), tetrahydroquinolinyl (e.g., 1,2,3,4-tetrahydroquinolin-4-yl), dihydroisoquinolinyl (e.g., 1,2-dihydroisoquinolin-4-yl), tetrahydroisoquinolinyl (e.g., 1,2,3,4-tetrahydroisoquinolin-4-yl), dihydrophthalazinyl (e.g., 1,4-dihydrophthalazin-4-yl), dihydrobenzooxazinyl (e.g., 2,3-dihydro-4H-benzo-1,3-oxazinyl), dihydropyranopyridyl (e.g., 2,3-dihydro-4H-pyrano [3,2-b]pyridyl), dihydrothiochromenyl (e.g., 2,3-dihydro-4H-thiochromenyl), 1-oxidodihydrothiochromenyl (e.g., 1-oxido-2,3-dihydro-4H-thiochromenyl), 1,1-dioxidodihydrothiochromenyl (e.g., 1,1-dioxido-2,3-dihydro-4H-thiochromenyl), tetrahydroquinazolinyl (e.g., 1,2,3,4-tetrahydroquinazolinyl) and the like;
and the like.

The "heterocyclic group" of the above-mentioned "optionally substituted heterocyclic group" optionally has 1 to 3 substituents at substitutable positions. Examples of the substituent include those similar to the substituents that the $C_{3-10}$ cycloalkyl group and the like exemplified as the "hydrocarbon group" of the above-mentioned "optionally substituted hydrocarbon group" optionally has. When the heterocyclic group is a "non-aromatic heterocyclic group", the substituent further includes an oxo group. When the number of the substituents is not less than 2, the respective substituents may be the same or different.

Examples of the "optionally substituted hydroxy group" include a hydroxy group optionally substituted by a substituent selected from a $C_{1-10}$ alkyl group, a $C_{2-10}$ alkenyl group, a $C_{3-10}$ cycloalkyl group, a $C_{3-10}$ cycloalkenyl group, a $C_{6-14}$ aryl group, a $C_{7-13}$ aralkyl group, a $C_{8-13}$ arylalkenyl group, a $C_{1-6}$ alkyl-carbonyl group, a heterocyclic group and the like, each of which is optionally substituted.

Examples of the $C_{1-10}$ alkyl group, $C_{2-10}$ alkenyl group, $C_{3-10}$ cycloalkyl group, $C_{3-10}$ cycloalkenyl group, $C_{6-14}$ aryl group, $C_{7-13}$ aralkyl group and $C_{8-13}$ arylalkenyl group include those exemplified as the "hydrocarbon group" of the above-mentioned "optionally substituted hydrocarbon group".

Examples of the heterocyclic group include those similar to the "heterocyclic group" of the above-mentioned "optionally substituted heterocyclic group".

The above-mentioned $C_{1-10}$ alkyl group, $C_{2-10}$ alkenyl group, $C_{3-10}$ cycloalkyl group, $C_{3-10}$ cycloalkenyl group, $C_{6-14}$ aryl group, $C_{7-13}$ aralkyl group, $C_{8-13}$ arylalkenyl group, $C_{1-6}$ alkyl-carbonyl group and heterocyclic group optionally have 1 to 3 substituents at substitutable positions. When the number of the substituents is not less than 2, the respective substituents may be the same or different.

Examples of the substituent for the $C_{1-10}$ alkyl group, $C_{2-10}$ alkenyl group and $C_{1-6}$ alkyl-carbonyl group include those similar to the substituents that the $C_{1-10}$ alkyl group and the like exemplified as the "hydrocarbon group" of the above-mentioned "optionally substituted hydrocarbon group" optionally has.

Examples of the substituent for the $C_{3-10}$ cycloalkyl group, $C_{3-10}$ cycloalkenyl group, $C_{6-14}$ aryl group, $C_{7-13}$ aralkyl group and $C_{8-13}$ arylalkenyl group include those similar to the substituents that the $C_{3-10}$ cycloalkyl group and the like exemplified as the "hydrocarbon group" of the above-mentioned "optionally substituted hydrocarbon group" optionally has. Examples of the substituent for the heterocyclic group include those similar to the substituents of the above-mentioned "optionally substituted heterocyclic group".

Examples of the "optionally substituted mercapto group" include a mercapto group optionally substituted by a substituent selected from a $C_{1-10}$ alkyl group, a $C_{2-10}$ alkenyl group, a $C_{3-10}$ cycloalkyl group, a $C_{3-10}$ cycloalkenyl group, a $C_{6-14}$ aryl group, a $C_{7-13}$ aralkyl group, a $C_{8-13}$ arylalkenyl group, a $C_{1-6}$ alkyl-carbonyl group, a heterocyclic group and the like, each of which is optionally substituted.

Examples of the substituent include those similar to the substituents of the above-mentioned "optionally substituted hydroxy group".

Examples of the "optionally substituted amino group" include an amino group optionally mono- or di-substituted by substituent (s) selected from a $C_{1-10}$ alkyl group, a $C_{2-10}$ alkenyl group, a $C_{3-10}$ cycloalkyl group, a $C_{3-10}$ cycloalkenyl group, a $C_{6-14}$ aryl group, a $C_{7-13}$ aralkyl group, a $C_{8-13}$ arylalkenyl group and a heterocyclic group, each of which is optionally substituted; an acyl group and the like.

Examples of the $C_{1-10}$ alkyl group, $C_{2-10}$ alkenyl group, $C_{3-10}$ cycloalkyl group, $C_{3-10}$ cycloalkenyl group, $C_{6-14}$ aryl group, $C_{7-13}$ aralkyl group and $C_{8-13}$ arylalkenyl group include those exemplified as the "hydrocarbon group" of the above-mentioned "optionally substituted hydrocarbon group".

Examples of the heterocyclic group include those similar to the "heterocyclic group" of the above-mentioned "optionally substituted heterocyclic group". Of these, a 5- to 7-membered monocyclic aromatic heterocyclic group is preferable.

The $C_{1-10}$ alkyl group, $C_{2-10}$ alkenyl group, $C_{3-10}$ cycloalkyl group, $C_{3-10}$ cycloalkenyl group, $C_{6-14}$ aryl group, $C_{7-13}$ aralkyl group, $C_{8-13}$ arylalkenyl group and heterocyclic group optionally have 1 to 3 substituents at substitutable positions. When the number of the substituents is not less than 2, the respective substituents may be the same or different.

Examples of the substituent for the $C_{1-10}$ alkyl group and $C_{2-10}$ alkenyl group include those similar to the substituents that the $C_{1-10}$ alkyl group and the like exemplified as the "hydrocarbon group" of the above-mentioned "optionally substituted hydrocarbon group" optionally has.

Examples of the substituent for the $C_{3-10}$ cycloalkyl group, $C_{3-10}$ cycloalkenyl group, $C_{6-14}$ aryl group, $C_{7-13}$ aralkyl group and $C_{8-13}$ arylalkenyl group include those similar to the substituents that the $C_{3-10}$ cycloalkyl group and the like exemplified as the "hydrocarbon group" of the above-mentioned "optionally substituted hydrocarbon group" optionally has. Examples of the substituent for the heterocyclic group include those similar to the substituents of the above-mentioned "optionally substituted heterocyclic group".

Examples of the "acyl group" exemplified as the substituent for the "optionally substituted amino group" include those similar to the "acyl group" below, which is exemplified as the "substituent" for $R^1$, $R^2$ or $R^{3b}$.

Examples of the "acyl group" which is exemplified as the "substituent" for $R^1$, $R^2$ or $R^{3b}$ include a group represented by the formula: $-COR^A$, $-CO-OR^A$, $-SO_3R^A$, $-SO_2R^A$, $-SOR^A$, $-CO-NR^{A'}R^{B'}$, $-CS-NR^{A'}R^{B'}$ or $-SO_2NR^{A'}R^{B'}$ wherein $R^A$ is a hydrogen atom, an optionally substituted hydrocarbon group or an optionally substituted heterocyclic group, and $R^{A'}$ and $R^{B'}$ are the same or different and each is a hydrogen atom, an optionally substituted hydrocarbon group or an optionally substituted heterocyclic group, or $R^{A'}$ and $R^{B'}$ optionally form, together with the adjacent nitrogen atom, an optionally substituted nitrogen-containing heterocycle, and the like.

Examples of the "optionally substituted hydrocarbon group" and "optionally substituted heterocyclic group" for $R^A$, $R^{A'}$ or $R^{B'}$ include those similar to the "optionally substituted hydrocarbon group" and "optionally substituted heterocyclic group", which are exemplified as the "substituent" for $R^1$ or the like.

Examples of the "nitrogen-containing heterocycle" of the "optionally substituted nitrogen-containing heterocycle" formed by $R^{a'}$ and $R^{b'}$ together with the adjacent nitrogen atom include a 5- to 7-membered nitrogen-containing heterocycle containing, as a ring-constituting atom besides carbon atoms, at least one nitrogen atom and optionally further containing one or two hetero atoms selected from an oxygen atom, a sulfur atom and a nitrogen atom. Preferable examples of the nitrogen-containing heterocycle include pyrrolidine, imidazolidine, pyrazolidine, piperidine, piperazine, morpholine, thiomorpholine and the like.

The nitrogen-containing heterocycle optionally has 1 to 3 substituents at substitutable positions. Examples of the substituent include those similar to the substituents of the above-mentioned "optionally substituted heterocyclic group". When the number of the substituents is not less than 2, the respective substituents may be the same or different.

Preferable examples of the "acyl group" include

(1) a formyl group;
(2) a carboxy group;
(3) a $C_{1-6}$ alkyl-carbonyl group optionally substituted by 1 to 3 substituents selected from

- (i) a halogen atom,
- (ii) a $C_{1-6}$ alkoxy-carbonyl group,
- (iii) a $C_{6-14}$ aryl group (e.g., phenyl),
- (iv) a $C_{1-6}$ alkoxy group, and
- (v) a carboxy group;

(4) a $C_{1-6}$ alkoxy-carbonyl group optionally substituted by 1 to 3 substituents selected from

- (i) a halogen atom,
- (ii) a $C_{6-14}$ aryl group (e.g., phenyl), and
- (iii) a $C_{1-6}$ alkoxy group;

(5) a $C_{3-10}$ cycloalkyl-carbonyl group (e.g., cyclopropylcarbonyl, cyclopentylcarbonyl, cyclohexylcarbonyl);
(6) a $C_{6-14}$ aryl-carbonyl group (e.g., benzoyl) optionally substituted by 1 to 3 halogen atoms;
(7) a carbamoyl group optionally mono- or di-substituted by substituent(s) selected from

- (i) a $C_{1-6}$ alkyl group optionally substituted by 1 to 3 substituents selected from
  - (a) a halogen atom,
  - (b) a $C_{1-6}$ alkoxy-carbonyl group,
  - (c) a $C_{6-14}$ aryl group (e.g., phenyl),
  - (d) a $C_{1-6}$ alkoxy group, and
  - (e) an aromatic heterocyclic group (e.g., furyl),
- (ii) a $C_{3-10}$ cycloalkyl group (e.g., cyclohexyl),
- (iii) a $C_{6-14}$ aryl group (e.g., phenyl) optionally substituted by 1 to 3 substituents selected from
  - (a) a halogen atom,
  - (b) a $C_{1-6}$ alkyl group optionally substituted by 1 to 3 halogen atoms, and
  - (c) a $C_{1-6}$ alkoxy group,
- (iv) an aromatic heterocyclic group (e.g., pyridyl), and
- (v) a $C_{1-6}$ alkylsulfonyl group (e.g., methylsulfonyl);

(8) a $C_{1-6}$ alkylsulfonyl group (e.g., methylsulfonyl, ethylsulfonyl, isopropylsulfonyl) optionally substituted by 1 to 3 substituents selected from

- (i) a halogen atom, and
- (ii) a $C_{6-14}$ aryl group (e.g., phenyl);

(9) a $C_{6-14}$ arylsulfonyl group (e.g., benzenesulfonyl) optionally substituted by 1 to 3 halogen atoms;
(10) a sulfamoyl group optionally mono- or di-substituted by substituent(s) selected from

- (i) a $C_{1-6}$ alkyl group optionally substituted by 1 to 3 substituents selected from
  - (a) a halogen atom, and
  - (b) a non-aromatic heterocyclic group (e.g., pyrrolidinyl) optionally substituted by oxo group(s);

(11) a thiocarbamoyl group optionally mono- or di-substituted by substituent(s) selected from a $C_{1-6}$ alkyl group

optionally substituted by 1 to 3 halogen atoms;
(12) an aromatic heterocyclylcarbonyl group (e.g., furylcarbonyl, thienylcarbonyl) optionally substituted by 1 to 3 substituents selected from a $C_{1-6}$ alkyl group optionally substituted by 1 to 3 halogen atoms;
(13) a non-aromatic heterocyclylcarbonyl group (e.g., tetrahydrofurylcarbonyl) optionally substituted by 1 to 3 substituents selected from a $C_{1-6}$ alkyl group optionally substituted by 1 to 3 halogen atoms;
(14) an oxalo group (-CO-COOH) optionally substituted by a $C_{1-6}$ alkyl group (e.g., ethyl);

and the like.
Preferably, $R^1$ and $R^2$ are both hydrogen atoms.

$R^{3b}$ is preferably a hydrogen atom.
X is preferably O, S, SO, $SO_2$, CO, $CH_2$ or NH, more preferably O, S, SO, $SO_2$ or NH, particularly preferably O or S.

W is O, S, a $C_{1-4}$ alkylene or $NR^{3a}$ wherein $R^{3a}$ is a hydrogen atom or a substituent.
Examples of the "substituent" for $R^{3a}$ include those similar to the above-mentioned "substituent" for $R^1$ or the like.
Examples of the "$C_{1-4}$ alkylene" for W include $-CH_2-$, $-(CH_2)_2-$, $-(CH_2)_3-$, $-(CH_2)_4-$, $-CH(CH_3)-$, $-CH(C_2H_5)-$, $-CH(C_3H_7)-$, $-CH(i\text{-}C_3H_7)-$, $-CH(CH_3)CH_2-$, $-CH_2CH(CH_3)-$, $-CH(CH_3)(CH_2)_2-$, $-(CH_2)_2CH(CH_3)-$, $-CH_2-CH(CH_3)-CH_2-$, $-C(CH_3)_2-$, $-(CH(CH_3))_2-$, $-CH_2-CH(CH_3)-$, $-CH_2-C(CH_3)_2-$ and the like. Of these, $-CH_2-$(methylene) is preferable.
W is preferably a $C_{1-4}$ alkylene or $NR^{3a}$ wherein $R^{3a}$ is as defined above, preferably a hydrogen atom, more preferably methylene or NH, particularly preferably methylene.
Y is an optionally substituted amino group. Examples of the "optionally substituted amino group" include those similar to the "optionally substituted amino group" exemplified as the above-mentioned "substituent" for $R^1$ or the like.
Y is preferably an amino group optionally mono- or di-substituted by substituent(s) selected from

(1) a $C_{1-6}$ alkoxy-carbonyl group (preferably ethoxycarbonyl, tert-butoxycarbonyl) optionally substituted by 1 to 3 halogen atoms (preferably a chlorine atom),
(2) a carbamoyl group optionally mono- or di-substituted by substituent(s) selected from

(a) a $C_{1-6}$ alkyl group (preferably methyl, ethyl, isopropyl) optionally substituted by 1 to 3 substituents selected from a carboxy group and a $C_{1-6}$ alkoxy-carbonyl group (preferably tert-butoxycarbonyl), and
(b) a $C_{1-6}$ alkylsulfonyl group (preferably methylsulfonyl),

(3) a $C_{1-6}$ alkyl-carbonyl group (preferably acetyl) optionally substituted by 1 to 3 substituents selected from

(a) a halogen atom (preferably a fluorine atom), and
(b) a carboxy group, and

(4) an oxalo group (-CO-COOH) optionally substituted by a $C_{1-6}$ alkyl group (preferably ethyl).

Y is more preferably an amino group optionally mono- or di-substituted by substituent(s) selected from

(1) a $C_{1-6}$ alkoxy-carbonyl group (preferably tert-butoxycarbonyl),
(2) a carbamoyl group optionally mono- or di-substituted by $C_{1-6}$ alkyl group(s) (preferably methyl, ethyl), and
(3) a $C_{1-6}$ alkyl-carbonyl group (preferably acetyl).

Ring E is an optionally further substituted 6-membered aromatic ring, or an optionally fused 5-membered aromatic heterocycle which is optionally further substituted.
Examples of the "6-membered aromatic ring" of the "optionally further substituted 6-membered aromatic ring" for ring E include benzene, and a 6-membered ring (e.g., pyridine, pyrazine, pyrimidine, pyridazine), from among the rings corresponding to the monocyclic aromatic heterocyclic group exemplified as the "heterocyclic group" of the "optionally substituted heterocyclic group", which is exemplified as the above-mentioned "substituent" for $R^1$ or the like. Of these, benzene and pyridine are preferable, and pyridine is particularly preferable.
Examples of the "optionally fused 5-membered aromatic heterocycle" of the "optionally fused 5-membered aromatic heterocycle which is optionally further substituted" for ring E include a 5-membered ring (e.g., thiophene, furan, pyrrole, imidazole, pyrazole), from among the rings corresponding to the monocyclic aromatic heterocyclic group exemplified as the "heterocyclic group" of the "optionally substituted heterocyclic group", which is exemplified as the above-mentioned "substituent" for $R^1$ or the like, and a ring wherein the 5-membered aromatic heterocycle and a ring selected from a 5- to 7-membered monocyclic aromatic heterocycle, benzene and partially saturated rings thereof are condensed.

Examples of the 5- to 7-membered monocyclic aromatic heterocycle include a 5- to 7-membered ring (e.g., pyridine, pyrazine, pyrimidine, pyridazine), from among the rings corresponding to the monocyclic aromatic heterocyclic group exemplified as the "heterocyclic group" of the "optionally substituted heterocyclic group", which is exemplified as the above-mentioned "substituent" for $R^1$ or the like.
Specific examples of the "optionally fused 5-membered aromatic heterocycle" include thiophene, furan, pyrrole, imidazole, pyrazole, benzothiophene, tetrahydrobenzothiophene, thienopyridine, benzofuran, indole, indazole, purine and the like. Of these, an optionally fused thiophene (preferably thiophene, benzothiophene, thienopyridine, tetrahydrobenzothiophene) is preferable, and thiophene optionally condensed with benzene or pyridine (thiophene, benzothiophene or thienopyridine) is particularly preferable.

The "6-membered aromatic ring" of the "optionally further substituted 6-membered aromatic ring" for ring E and the "optionally fused 5-membered aromatic heterocycle" of the "optionally fused 5-membered aromatic heterocycle which is optionally further substituted" for ring E optionally further have, besides group Y, 1 to 3 substituents at substitutable positions. Examples of the substituent include those similar to the substituents that the $C_{3-10}$ cycloalkyl group and the like exemplified as the above-mentioned "substituent" for $R^1$ or the like optionally has. When the number of the substituents is not less than 2, the respective substituents may be the same or different.
When ring E is a fused ring, i.e., a "fused 5-membered aromatic heterocycle", then the fused ring optionally has the above-mentioned substituent(s) at any position on the ring.

Preferable specific examples of the substituent for ring E include

(1) a $C_{6-14}$ aryl group (e.g., phenyl) optionally substituted by 1 to 3 substituents selected from

(a) a hydroxy group,
(b) a $C_{1-6}$ alkoxy group (preferably methoxy) optionally substituted by 1 to 3 $C_{6-14}$ aryl groups (preferably phenyl),
(c) a $C_{1-6}$ alkoxy-carbonyl group (preferably methoxycarbonyl) optionally substituted by 1 to 3 $C_{6-14}$ aryl groups (preferably phenyl), and
(d) a carboxy group,

(2) a halogen atom (preferably a chlorine atom, a bromine atom),
(3) a $C_{1-6}$ alkyl group (preferably methyl) optionally substituted by 1 to 3 halogen atoms (preferably a fluorine atom),
(4) a $C_{1-6}$ alkoxy group (preferably methoxy),
(5) a $C_{1-6}$ alkyl-carbonyl group (preferably acetyl),
(6) a $C_{1-6}$ alkoxy-carbonyl group (preferably methoxycarbonyl) optionally substituted by 1 to 3 $C_{6-14}$ aryl groups (preferably phenyl),
(7) a carboxy group,
(8) an aromatic heterocyclic group (preferably pyrazolyl) optionally substituted by 1 to 3 $C_{1-6}$ alkyl groups (preferably methyl),
(9) a non-aromatic heterocyclic group (preferably piperidyl) optionally substituted by 1 to 3 substituents selected from

(a) an amino group, and
(b) a hydroxy group,

(10) an oxo group

and the like. More preferable specific examples of the substituent for ring E are those recited above except an oxo group.

Ring E is preferably a 6-membered aromatic ring (preferably benzene or pyridine, more preferably pyridine), or an optionally fused 5-membered aromatic heterocycle (preferably an optionally fused thiophene (preferably thiophene, benzothiophene, thienopyridine, tetrahydrobenzothiophene), particularly preferably thiophene optionally condensed with benzene or pyridine (thiophene, benzothiophene or thienopyridine)), each of which is optionally further substituted by 1 to 3 (preferably 1 or 2) substituents (preferably excluding an oxo group) selected from

(1) a $C_{6-14}$ aryl group (e.g., phenyl) optionally substituted by 1 to 3 substituents selected from

(a) a hydroxy group,
(b) a $C_{1-6}$ alkoxy group (preferably methoxy) optionally substituted by 1 to 3 $C_{6-14}$ aryl groups (preferably phenyl),
(c) a $C_{1-6}$ alkoxy-carbonyl group (preferably methoxycarbonyl) optionally substituted by 1 to 3 $C_{6-14}$ aryl groups (preferably phenyl), and
(d) a carboxy group,

(2) a halogen atom (preferably a chlorine atom, a bromine atom),
(3) a $C_{1-6}$ alkyl group (preferably methyl) optionally substituted by 1 to 3 halogen atoms (preferably a fluorine atom),
(4) a $C_{1-6}$ alkoxy group (preferably methoxy),
(5) a $C_{1-6}$ alkyl-carbonyl group (preferably acetyl),
(6) a $C_{1-6}$ alkoxy-carbonyl group (preferably methoxycarbonyl) optionally substituted by 1 to 3 $C_{6-14}$ aryl groups (preferably phenyl),
(7) a carboxy group,
(8) an aromatic heterocyclic group (preferably pyrazolyl) optionally substituted by 1 to 3 $C_{1-6}$ alkyl groups (preferably methyl),
(9) a non-aromatic heterocyclic group (preferably piperidyl) optionally substituted by 1 to 3 substituents selected from

(a) an amino group, and
(b) a hydroxy group, and

(10) an oxo group.

Ring E is more preferably thiophene optionally condensed with benzene or pyridine (thiophene, benzothiophene or thienopyridine), or pyridine, each of which is optionally further substituted by 1 to 3 (preferably 1 or 2) substituents selected from

(1) a $C_{6-14}$ aryl group (e.g., phenyl) optionally substituted by 1 to 3 substituents selected from

(a) a hydroxy group,
(b) a $C_{1-6}$ alkoxy group (preferably methoxy) optionally substituted by 1 to 3 $C_{6-14}$ aryl groups (preferably phenyl),
(c) a $C_{1-6}$ alkoxy-carbonyl group (preferably methoxycarbonyl) optionally substituted by 1 to 3 $C_{6-14}$ aryl groups (preferably phenyl), and
(d) a carboxy group,

(2) a halogen atom (preferably a chlorine atom, a bromine atom),
(3) a $C_{1-6}$ alkyl group (preferably methyl) optionally substituted by 1 to 3 halogen atoms (preferably a fluorine atom),
(4) a $C_{1-6}$ alkoxy group (preferably methoxy),
(5) a $C_{1-6}$ alkyl-carbonyl group (preferably acetyl),
(6) a $C_{1-6}$ alkoxy-carbonyl group (preferably methoxycarbonyl) optionally substituted by 1 to 3 $C_{6-14}$ aryl groups (preferably phenyl),
(7) a carboxy group,
(8) an aromatic heterocyclic group (preferably pyrazolyl) optionally substituted by 1 to 3 $C_{1-6}$ alkyl groups (preferably methyl), and
(9) a non-aromatic heterocyclic group (preferably piperidyl) optionally substituted by 1 to 3 substituents selected from

(a) an amino group, and
(b) a hydroxy group.

Ring P is an optionally fused non-aromatic ring which is optionally further substituted.
Examples of the "optionally fused non-aromatic ring" of the "optionally fused non-aromatic ring which is optionally further substituted" for ring P include a ring having a structure containing -CO-W-C-X- wherein each symbol is as defined above, from among the rings corresponding to the "optionally substituted heterocyclic group" exemplified as the above-mentioned "substituent" for $R^1$ or the like.
Preferable examples thereof include

(1) a dihydrochromene ring substituted by an oxo group (preferably 2,3-dihydro-4H-chromen-4-one),
(2) a tetrahydropyran ring substituted by an oxo group (preferably tetrahydro-4H-pyran-4-one),
(3) a thiazolidine ring substituted by an oxo group (preferably 1,3-thiazolidin-2-one, 1,3-thiazolidin-5-one),
(4) an oxazolidine ring substituted by an oxo group (preferably 1,3-oxazolidin-2-one, 1,3-oxazolidin-5-one),
(5) a piperidine ring substituted by an oxo group (preferably piperidin-2-one),
(6) a tetrahydrooxazine ring substituted by an oxo group (preferably 3,4,5,6-tetrahydro-1,3-oxazin-4-one),
(7) a dihydrobenzooxazine ring substituted by an oxo group (preferably 2,3-dihydro-4H-benzo-1,3-oxazin-4-one),
(8) a dihydropyranopyridine ring substituted by an oxo group (preferably 2,3-dihydro-4H-pyrano[3,2-b]pyridin-4-one),
(9) an optionally oxidized dihydrothiochromene ring which is substituted by an oxo group (preferably 2,3-dihydro-4H-thiochromen-4-one, 1-oxido-2,3-dihydro-4H-thiochromen-4-one, 1,1-dioxido-2,3-dihydro-4H-thiochromen-4-one),

(10) a tetrahydroquinazoline ring substituted by an oxo group (preferably 1,2,3,4-tetrahydroquinazolin-4-one), and

(11) an imidazolidine ring substituted by an oxo group (preferably imidazolidin-2-one). Of these,

(1) a dihydrochromene ring substituted by an oxo group (preferably 2,3-dihydro-4H-chromen-4-one),

(5) a piperidine ring substituted by an oxo group (preferably piperidin-2-one), and

(9) an optionally oxidized dihydrothiochromene ring which is substituted by an oxo group (preferably 2,3-dihydro-4H-thiochromen-4-one, 1-oxido-2,3-dihydro-4H-thiochromen-4-one, 1,1-dioxido-2,3-dihydro-4H-thiochromen-4-one)

are preferable.

Another preferable embodiment is the following non-aromatic ring condensed with a benzene ring:

(1) a dihydrochromene ring substituted by an oxo group (preferably 2,3-dihydro-4H-chromen-4-one),

(7) a dihydrobenzooxazine ring substituted by an oxo group (preferably 2,3-dihydro-4H-benzo-1,3-oxazin-4-one),

(9) an optionally oxidized dihydrothiochromene ring which is substituted by an oxo group (preferably 2,3-dihydro-4H-thiochromen-4-one, 1-oxido-2,3-dihydro-4H-thiochromen-4-one, 1,1-dioxido-2,3-dihydro-4H-thiochromen-4-one), and (10) a tetrahydroquinazoline ring substituted by an oxo group (preferably 1,2,3,4-tetrahydroquinazolin-4-one).

The "non-aromatic ring" of the "non-aromatic ring which is optionally further substituted" for ring P optionally further has 1 to 3 substituents at substitutable positions in the moiety other than the -CO-W-C-X- moiety of the ring. Examples of the substituent include those similar to the substituents that the $C_{3-10}$ cycloalkyl group and the like exemplified as the above-mentioned "substituent" for $R^1$ or the like optionally has, and an oxo group. When the number of the substituents is not less than 2, the respective substituents may be the same or different.

Preferable specific examples of the substituent for ring P include

(1) a $C_{1-6}$ alkyl group (preferably methyl, ethyl, isopropyl, isobutyl) optionally substituted by 1 to 3 substituents selected from

(a) a $C_{1-6}$ alkoxy-carbonyl group (preferably methoxycarbonyl) optionally substituted by 1 to 3 $C_{6-14}$ aryl groups (preferably phenyl),

(b) a carboxy group, and

(c) a 2,4-dioxo-1,3-thiazolidin-5-ylidene group,

(2) a $C_{1-6}$ alkoxy group (preferably methoxy) optionally substituted by 1 to 3 $C_{6-14}$ aryl groups (preferably phenyl),

(3) a formyl group,

(4) an oxo group,

(5) a $C_{1-6}$ alkyl-carbonyl group (preferably acetyl),

(6) a cyano group,

(7) a hydroxy group,

(8) a carboxy group,

(9) a nitro group,

(10) a halogen atom (preferably a fluorine atom, a chlorine atom, a bromine atom),

(11) an aromatic heterocyclic group (preferably pyridyl, pyrazolyl, tetrazolyl) optionally substituted by 1 to 3 $C_{1-6}$ alkyl groups (preferably methyl),

(12) a non-aromatic heterocyclic group (preferably pyrrolidinyl, dihydrooxadiazolyl, dihydrothiadiazolyl) optionally substituted by 1 to 3 oxo groups,

(13) an amino group optionally mono- or di-substituted by substituent(s) selected from

(a) a $C_{1-6}$ alkylsulfonyl group (preferably methylsulfonyl), and

(b) a carbamoyl group optionally mono- or di-substituted by $C_{1-6}$ alkyl group(s) (preferably methyl),

(14) a carbamoyl group optionally mono- or di-substituted by heterocyclic group(s) (preferably triazolyl)

and the like.

More preferable specific examples of the substituent for ring P include

(1) a $C_{1-6}$ alkyl group (preferably methyl) optionally substituted by 1 to 3 substituents selected from

(a) a $C_{1-6}$ alkoxy-carbonyl group (preferably methoxycarbonyl),
(b) a carboxy group, and
(c) a 2,4-dioxo-1,3-thiazolidin-5-ylidene group,

(2) a $C_{1-6}$ alkoxy group (preferably methoxy) optionally substituted by) 1 to 3 $C_{6-14}$ aryl groups (preferably phenyl),
(3) a formyl group,
(5) a $C_{1-6}$ alkyl-carbonyl group (preferably acetyl),
(6) a cyano group,
(7) a hydroxy group,
(8) a carboxy group,
(9) a nitro group,
(10) a halogen atom (preferably a fluorine atom, a chlorine atom, a bromine atom),
(11) an aromatic heterocyclic group (preferably pyridyl, pyrazolyl, tetrazolyl) optionally substituted by 1 to 3 $C_{1-6}$ alkyl groups (preferably methyl),
(12) a non-aromatic heterocyclic group (preferably pyrrolidinyl, dihydrooxadiazolyl, dihydrothiadiazolyl) optionally substituted by 1 to 3 oxo groups,
(13) an amino group optionally mono- or di-substituted by substituent(s) selected from

(a) a $C_{1-6}$ alkylsulfonyl group (preferably methylsulfonyl), and
(b) a carbamoyl group optionally mono- or di-substituted by $C_{1-6}$ alkyl group (s) (preferably methyl),

(14) a carbamoyl group optionally mono- or di-substituted by heterocyclic group(s) (preferably triazolyl)

and the like.

Ring P is preferably an optionally fused non-aromatic ring which is optionally further substituted by 1 to 3 (preferably 1 or 2) substituents selected from

(1) a $C_{1-6}$ alkyl group (preferably methyl, ethyl, isopropyl, isobutyl) optionally substituted by 1 to 3 substituents selected from

(a) a $C_{1-6}$ alkoxy-carbonyl group (preferably methoxycarbonyl) optionally substituted by 1 to 3 $C_{6-14}$ aryl groups (preferably phenyl),
(b) a carboxy group, and
(c) a 2,4-dioxo-1,3-thiazolidin-5-ylidene group,

(2) a $C_{1-6}$ alkoxy group (preferably methoxy) optionally substituted by 1 to 3 $C_{6-14}$ aryl groups (preferably phenyl),
(3) a formyl group,
(4) an oxo group,
(5) a $C_{1-6}$ alkyl-carbonyl group (preferably acetyl),
(6) a cyano group,
(7) a hydroxy group,
(8) a carboxy group,
(9) a nitro group,
(10) a halogen atom (preferably a fluorine atom, a chlorine atom, a bromine atom),
(11) an aromatic heterocyclic group (preferably pyridyl, pyrazolyl, tetrazolyl) optionally substituted by 1 to 3 $C_{1-6}$ alkyl groups (preferably methyl),
(12) a non-aromatic heterocyclic group (preferably pyrrolidinyl, dihydrooxadiazolyl, dihydrothiadiazolyl) optionally substituted by 1 to 3 oxo groups,
(13) an amino group optionally mono- or di-substituted by substituent(s) selected from

(a) a $C_{1-6}$ alkylsulfonyl group (preferably methylsulfonyl), and

(b) a carbamoyl group optionally mono- or di-substituted by $C_{1-6}$ alkyl group(s) (preferably methyl), and

(14) a carbamoyl group optionally mono- or di-substituted by heterocyclic group(s) (preferably triazolyl)

(preferably

(1) a dihydrochromene ring substituted by an oxo group (preferably 2,3-dihydro-4H-chromen-4-one),
(5) a piperidine ring substituted by an oxo group (preferably piperidin-2-one),
(6) a tetrahydrooxazine ring substituted by an oxo group (preferably 3,4,5,6-tetrahydro-1,3-oxazin-4-one),
(7) a dihydrobenzooxazine ring substituted by an oxo group (preferably 2,3-dihydro-4H-benzo-1,3-oxazin-4-one),
(8) a dihydropyranopyridine ring substituted by an oxo group (preferably 2,3-dihydro-4H-pyrano[3,2-b]pyridin-4-one),
(9) an optionally oxidized dihydrothiochromene ring which is substituted by an oxo group (preferably 2,3-dihydro-4H-thiochromen-4-one, 1-oxido-2,3-dihydro-4H-thiochromen-4-one, 1,1-dioxido-2,3-dihydro-4H-thiochromen-4-one),
(10) a tetrahydroquinazoline ring substituted by an oxo group (preferably 1,2,3,4-tetrahydroquinazolin-4-one), or
(11) an imidazolidine ring substituted by an oxo group

(preferably imidazolidin-2-one)
more preferably

(1) a dihydrochromene ring substituted by an oxo group (preferably 2,3-dihydro-4H-chromen-4-one),
(5) a piperidine ring substituted by an oxo group (preferably piperidin-2-one), or
(9) an optionally oxidized dihydrothiochromene ring which is substituted by an oxo group (preferably 2,3-dihydro-4H-thiochromen-4-one, 1-oxido-2,3-dihydro-4H-thiochromen-4-one, 1,1-dioxido-2,3-dihydro-4H-thiochromen-4-one)).

Alternatively, ring P is preferably a non-aromatic ring condensed with a benzene ring, which is optionally further substituted by 1 to 3 (preferably 1 or 2) substituents selected from

(1) a $C_{1-6}$ alkyl group (preferably methyl) optionally substituted by 1 to 3 substituents selected from

(a) a $C_{1-6}$ alkoxy-carbonyl group (preferably methoxycarbonyl),
(b) a carboxy group, and
(c) a 2,4-dioxo-1,3-thiazolidin-5-ylidene group,

(2) a $C_{1-6}$ alkoxy group (preferably methoxy) optionally substituted by 1 to 3 $C_{6-14}$ aryl groups (preferably phenyl),
(3) a formyl group,
(5) a $C_{1-6}$ alkyl-carbonyl group (preferably acetyl),
(6) a cyano group,
(7) a hydroxy group,
(8) a carboxy group,
(9) a nitro group,
(10) a halogen atom (preferably a fluorine atom, a chlorine atom, a bromine atom),
(11) an aromatic heterocyclic group (preferably pyridyl, pyrazolyl, tetrazolyl) optionally substituted by 1 to 3 $C_{1-6}$ alkyl groups (preferably methyl),
(12) a non-aromatic heterocyclic group (preferably pyrrolidinyl, dihydrooxadiazolyl, dihydrothiadiazolyl) optionally substituted by 1 to 3 oxo groups,
(13) an amino group optionally mono- or di-substituted by substituent(s) selected from

(a) a $C_{1-6}$ alkylsulfonyl group (preferably methylsulfonyl),
and
(b) a carbamoyl group optionally mono- or di-substituted by $C_{1-6}$ alkyl group(s) (preferably methyl), and

(14) a carbamoyl group optionally mono- or di-substituted by heterocyclic group(s) (preferably triazolyl)

(preferably

(1) a dihydrochromene ring substituted by an oxo group (preferably 2,3-dihydro-4H-chromen-4-one),
(7) a dihydrobenzooxazine ring substituted by an oxo group (preferably 2,3-dihydro-4H-benzo-1,3-oxazin-4-one),
(9) an optionally oxidized dihydrothiochromene ring which is substituted by an oxo group (preferably 2,3-dihydro-4H-thiochromen-4-one, 1-oxido-2,3-dihydro-4H-thiochromen-4-one, 1,1-dioxido-2,3-dihydro-4H-thiochromen-4-one), or

(10) a tetrahydroquinazoline ring substituted by an oxo group (preferably 1,2,3,4-tetrahydroquinazolin-4-one)).

n is 0, 1, 2 or 3.
n is preferably 0, 1 or 2, more preferably 1.

Preferable examples of compound (I) include the following compound.

[Compound A]

Compound (I) wherein
ring E is benzene, or an optionally fused 5-membered aromatic heterocycle (preferably thiophene, benzothiophene or thienopyridine), each of which is optionally further substituted by 1 to 3 substituents selected from

(1) a $C_{6-14}$ aryl group (e.g., phenyl), and
(2) a halogen atom;

ring P is 2,3-dihydro-4H-chromen-4-one;
W is methylene;
X is O;
Y is an amino group optionally mono- or di-substituted by substituent(s) selected from

(1) a $C_{1-6}$ alkoxy-carbonyl group, and
(2) a carbamoyl group optionally mono- or di-substituted by $C_{1-6}$ alkyl group(s); and

n is 1.

[Compound B]

Compound (I) wherein
ring E is a 6-membered aromatic ring (preferably benzene or pyridine, more preferably pyridine), or an optionally fused 5-membered aromatic heterocycle (preferably an optionally fused thiophene (preferably thiophene, benzothiophene, thienopyridine, tetrahydrobenzothiophene), particularly preferably thiophene optionally condensed with benzene or pyridine (thiophene, benzothiophene or thienopyridine)), each of which is optionally further substituted by 1 to 3 (preferably 1 or 2) substituents (preferably excluding an oxo group) selected from

(1) a $C_{6-14}$ aryl group (e.g., phenyl) optionally substituted by 1 to 3 substituents selected from

(a) a hydroxy group,
(b) a $C_{1-6}$ alkoxy group (preferably methoxy) optionally substituted by 1 to 3 $C_{6-14}$ aryl groups (preferably phenyl),
(c) a $C_{1-6}$ alkoxy-carbonyl group (preferably methoxycarbonyl) optionally substituted by 1 to 3 $C_{6-14}$ aryl groups (preferably phenyl), and
(d) a carboxy group,

(2) a halogen atom (preferably a chlorine atom, a bromine atom),
(3) a $C_{1-6}$ alkyl group (preferably methyl) optionally substituted by 1 to 3 halogen atoms (preferably a fluorine atom),
(4) a $C_{1-6}$ alkoxy group (preferably methoxy),
(5) a $C_{1-6}$ alkyl-carbonyl group (preferably acetyl),
(6) a $C_{1-6}$ alkoxy-carbonyl group (preferably methoxycarbonyl) optionally substituted by 1 to 3 $C_{6-14}$ aryl groups (preferably phenyl),
(7) a carboxy group,
(8) an aromatic heterocyclic group (preferably pyrazolyl) optionally substituted by 1 to 3 $C_{1-6}$ alkyl groups (preferably methyl),
(9) a non-aromatic heterocyclic group (preferably piperidyl) optionally substituted by 1 to 3 substituents selected from

(a) an amino group, and
(b) a hydroxy group, and

(10) an oxo group;

ring P is an optionally fused non-aromatic ring which is optionally further substituted by 1 to 3 (preferably 1 or 2) substituents selected from

(1) a $C_{1-6}$ alkyl group (preferably methyl, ethyl, isopropyl, isobutyl) optionally substituted by 1 to 3 substituents selected from

(a) a $C_{1-6}$ alkoxy-carbonyl group (preferably methoxycarbonyl) optionally substituted by 1 to 3 $C_{6-14}$ aryl groups (preferably phenyl),
(b) a carboxy group, and
(c) a 2,4-dioxo-1,3-thiazolidin-5-ylidene group,

(2) a $C_{1-6}$ alkoxy group (preferably methoxy) optionally substituted by 1 to 3 $C_{6-14}$ aryl groups (preferably phenyl),
(3) a formyl group,
(4) an oxo group,
(5) a $C_{1-6}$ alkyl-carbonyl group (preferably acetyl),
(6) a cyano group,
(7) a hydroxy group,
(8) a carboxy group,
(9) a nitro group,
(10) a halogen atom (preferably a fluorine atom, a chlorine atom, a bromine atom),
(11) an aromatic heterocyclic group (preferably pyridyl, pyrazolyl, tetrazolyl) optionally substituted by 1 to 3 $C_{1-6}$ alkyl groups (preferably methyl),
(12) a non-aromatic heterocyclic group (preferably pyrrolidinyl, dihydrooxadiazolyl, dihydrothiadiazolyl) optionally substituted by 1 to 3 oxo groups,
(13) an amino group optionally mono- or di-substituted by substituent(s) selected from

(a) a $C_{1-6}$ alkylsulfonyl group (preferably methylsulfonyl), and
(b) a carbamoyl group optionally mono- or di-substituted by $C_{1-6}$ alkyl group (s) (preferably methyl), and

(14) a carbamoyl group optionally mono- or di-substituted by heterocyclic group(s) (preferably triazolyl)

(preferably

(1) a dihydrochromene ring substituted by an oxo group (preferably 2,3-dihydro-4H-chromen-4-one),
(5) a piperidine ring substituted by an oxo group (preferably piperidin-2-one),
(6) a tetrahydrooxazine ring substituted by an oxo group (preferably 3,4,5,6-tetrahydro-1,3-oxazin-4-one),
(7) a dihydrobenzooxazine ring substituted by an oxo group (preferably 2,3-dihydro-4H-benzo-1,3-oxazin-4-one),
(8) a dihydropyranopyridine ring substituted by an oxo group (preferably 2,3-dihydro-4H-pyrano[3,2-b]pyridin-4-one),
(9) an optionally oxidized dihydrothiochromene ring which is substituted by an oxo group (preferably 2,3-dihydro-4H-thiochromen-4-one, 1-oxido-2,3-dihydro-4H-thiochromen-4-one, 1,1-dioxido-2,3-dihydro-4H-thiochromen-4-one),
(10) a tetrahydroquinazoline ring substituted by an oxo group (preferably 1,2,3,4-tetrahydroquinazolin-4-one), or
(11) an imidazolidine ring substituted by an oxo group

(preferably imidazolidin-2-one)
more preferably

(1) a dihydrochromene ring substituted by an oxo group (preferably 2,3-dihydro-4H-chromen-4-one),
(5) a piperidine ring substituted by an oxo group (preferably piperidin-2-one), or
(9) an optionally oxidized dihydrothiochromene ring which is substituted by an oxo group (preferably 2,3-dihydro-4H-thiochromen-4-one, 1-oxido-2,3-dihydro-4H-thiochromen-4-one, 1,1-dioxido-2,3-dihydro-4H-thiochromen-4-one));

W is methylene or NH (preferably methylene);
X is O, S, SO, $SO_2$, CO, $CH_2$ or NH (preferably O or S);
Y is an amino group optionally mono- or di-substituted by substituent(s) selected from

(1) a $C_{1-6}$ alkoxy-carbonyl group (preferably ethoxycarbonyl, tert-butoxycarbonyl) optionally substituted by 1 to 3 halogen atoms (preferably a chlorine atom),
(2) a carbamoyl group optionally mono- or di-substituted by substituent(s) selected from

(a) a $C_{1-6}$ alkyl group (preferably methyl, ethyl, isopropyl) optionally substituted by 1 to 3 substituents selected from a carboxy group and a $C_{1-6}$ alkoxy-carbonyl group (preferably tert-butoxycarbonyl), and
(b) a $C_{1-6}$ alkylsulfonyl group (preferably methylsulfonyl),

(3) a $C_{1-6}$ alkyl-carbonyl group (preferably acetyl) optionally substituted by 1 to 3 substituents selected from

(a) a halogen atom (preferably a fluorine atom), and
(b) a carboxy group, and

(4) an oxalo group (-CO-COOH) optionally substituted by a $C_{1-6}$ alkyl group (preferably ethyl); and

n is 0, 1 or 2(preferably 1).

[Compound C]

Compound (I) wherein
ring E is thiophene optionally condensed with benzene or pyridine (thiophene, benzothiophene or thienopyridine), or pyridine, each of which is optionally further substituted by 1 to 3 (preferably 1 or 2) substituents selected from

(1) a $C_{6-14}$ aryl group (e.g., phenyl) optionally substituted by 1 to 3 substituents selected from

(a) a hydroxy group,
(b) a $C_{1-6}$ alkoxy group (preferably methoxy) optionally substituted by 1 to 3 $C_{6-14}$ aryl groups (preferably phenyl),
(c) a $C_{1-6}$ alkoxy-carbonyl group (preferably methoxycarbonyl) optionally substituted by 1 to 3 $C_{6-14}$ aryl groups (preferably phenyl), and
(d) a carboxy group,

(2) a halogen atom (preferably a chlorine atom, a bromine atom),
(3) a $C_{1-6}$ alkyl group (preferably methyl) optionally substituted by 1 to 3 halogen atoms (preferably a fluorine atom),
(4) a $C_{1-6}$ alkoxy group (preferably methoxy),
(5) a $C_{1-6}$ alkyl-carbonyl group (preferably acetyl),
(6) a $C_{1-6}$ alkoxy-carbonyl group (preferably methoxycarbonyl) optionally substituted by 1 to 3 $C_{6-14}$ aryl groups (preferably phenyl),
(7) a carboxy group,
(8) an aromatic heterocyclic group (preferably pyrazolyl) optionally substituted by 1 to 3 $C_{1-6}$ alkyl groups (preferably methyl), and
(9) a non-aromatic heterocyclic group (preferably piperidyl) optionally substituted by 1 to 3 substituents selected from

(a) an amino group, and
(b) a hydroxy group;

ring P is a non-aromatic ring condensed with a benzene ring, which is optionally further substituted by 1 to 3 (preferably 1 or 2) substituents selected from

(1) a $C_{1-6}$ alkyl group (preferably methyl) optionally substituted by 1 to 3 substituents selected from

(a) a $C_{1-6}$ alkoxy-carbonyl group (preferably methoxycarbonyl),
(b) a carboxy group, and
(c) a 2,4-dioxo-1,3-thiazolidin-5-ylidene group,

(2) a $C_{1-6}$ alkoxy group (preferably methoxy) optionally substituted by 1 to 3 $C_{6-14}$ aryl groups (preferably phenyl),
(3) a formyl group,
(5) a $C_{1-6}$ alkyl-carbonyl group (preferably acetyl),
(6) a cyano group,

(7) a hydroxy group,
(8) a carboxy group,
(9) a nitro group,
(10) a halogen atom (preferably a fluorine atom, a chlorine atom, a bromine atom),
(11) an aromatic heterocyclic group (preferably pyridyl, pyrazolyl, tetrazolyl) optionally substituted by 1 to 3 $C_{1-6}$ alkyl groups (preferably methyl),
(12) a non-aromatic heterocyclic group (preferably pyrrolidinyl, dihydrooxadiazolyl, dihydrothiadiazolyl) optionally substituted by 1 to 3 oxo groups,
(13) an amino group optionally mono- or di-substituted by substituent(s) selected from

(a) a $C_{1-6}$ alkylsulfonyl group (preferably methylsulfonyl), and
(b) a carbamoyl group optionally mono- or di-substituted by $C_{1-6}$ alkyl group (s) (preferably methyl), and

(14) a carbamoyl group optionally mono- or di-substituted by heterocyclic group(s) (preferably triazolyl)

(preferably

(1) a dihydrochromene ring substituted by an oxo group (preferably 2,3-dihydro-4H-chromen-4-one),
(7) a dihydrobenzooxazine ring substituted by an oxo group (preferably 2,3-dihydro-4H-benzo-1,3-oxazin-4-one),
(9) an optionally oxidized dihydrothiochromene ring which is substituted by an oxo group (preferably 2,3-dihydro-4H-thiochromen-4-one, 1-oxido-2,3-dihydro-4H-thiochromen-4-one, 1,1-dioxido-2,3-dihydro-4H-thiochromen-4-one), or
(10) a tetrahydroquinazoline ring substituted by an oxo group (preferably 1,2,3,4-tetrahydroquinazolin-4-one));

W is methylene or NH (preferably methylene);
X is O, S, SO, $SO_2$, CO, $CH_2$ or NH (preferably O, S, SO, $SO_2$ or NH, particularly preferably O or S);
Y is an amino group optionally mono- or di-substituted by substituent(s) selected from

(1) a $C_{1-6}$ alkoxy-carbonyl group (preferably tert-butoxycarbonyl),
(2) a carbamoyl group optionally mono- or di-substituted by $C_{1-6}$ alkyl group(s) (preferably methyl, ethyl), and
(3) a $C_{1-6}$ alkyl-carbonyl group (preferably acetyl); and

n is 1.

Of compound (I), compound (IIa) is preferable.
The definition of each symbol in the formula (IIa) is described in detail in the following.
Ring Ea is a thiophene ring optionally condensed with a benzene ring or a pyridine ring, or a pyridine ring.
Ring Ea is preferably a thiophene ring optionally condensed with a benzene ring or a pyridine ring, specifically, a thiophene ring, a benzothiophene ring or a thienopyridine ring.

Wa is O, S, $CH_2$ or NH.
Wa is preferably $CH_2$ or NH, more preferably $CH_2$.

Xa is O, S, SO, $SO_2$, CO, $CH_2$ or NH.
Xa is preferably O, S, SO, $SO_2$ or NH, particularly preferably O or S.

Ya is $-NH_2$, -NHCONHR, -NHCOOR or -NHCOR wherein R is a hydrogen atom or a $C_{1-6}$ alkyl group.

$R^3$ to $R^6$ are the same or different and each is a hydrogen atom, a halogen atom, a $C_{1-6}$ alkyl group, a $C_{1-6}$ alkoxy group, a cyano group, a nitro group, an amino group, a carboxy group, a hydroxy group, -COR, $-NHSO_2R$ or -NHCONHR wherein R is a hydrogen atom or a $C_{1-6}$ alkyl group, $-C_{1-6}$ alkylene-COOH, $-C_{1-6}$ alkylene-COOR, $-O-C_{1-6}$ alkylene-$C_{6-14}$ arene, -CONH-(5- or 6-membered heterocycle), a 5- or 6-membered heterocyclic group or a $C_{6-14}$ aryl group.

Examples of the "$C_{1-6}$ alkylene" of the "$-C_{1-6}$ alkylene-COOH" and "$-C_{1-6}$ alkylene-COOR" for $R^3$ to $R^6$ include $-CH_2-$, $-(CH_2)_2-$, $-(CH_2)_3-$, $-(CH_2)_4-$, $-(CH_2)_5-$, $-(CH_2)_6-$, $-CH(CH_3)-$, $-CH(C_2H_5)-$, $-CH(C_3H_7)-$, $-CH(i-C_3H_7)-$, $-CH(CH_3)CH_2-$, $-CH_2CH(CH_3)-$, $-CH(CH_3)(CH_2)_2-$, $-(CH_2)_2CH(CH_3)-$, $-CH_2-CH(CH_3)-CH_2-$, -C $(CH_3)2$-, $-(CH(CH_3))_2-$, $-(CH_2)_2C(CH_3)_2-$, $-(CH_2)_3C(CH_3)_2-$, $-CH_2-CH(CH_3)-$, $-CH_2-C(CH_3)_2-$ and the like. Of these, $-CH_2-$ is preferable.

Examples of the "$C_{6-14}$ aryl group" for $R^3$ to $R^6$ include those similar to the $C_{6-14}$ aryl group exemplified as the "hydrocarbon group" of the "optionally substituted hydrocarbon group", which is exemplified as the "substituent" for $R^1$ or the like in the formula (I). Of these, phenyl is preferable.
Examples of the "$C_{6-14}$ arene" of the "-O-$C_{1-6}$ alkylene-$C_{6-14}$ arene" for $R^3$ to $R^6$ include a ring corresponding to the above-mentioned "$C_{6-14}$ aryl group". Of these, benzene is preferable.
Examples of the "5- or 6-membered heterocyclic group" for $R^3$ to $R^6$ include a 5- or 6-membered ring (preferably pyridyl, pyrazolyl, tetrazolyl, piperidyl, pyrrolidinyl, dihydrooxadiazolyl, dihydrothiadiazolyl), from among the rings corresponding to the monocyclic aromatic heterocyclic group exemplified as the "heterocyclic group" of the "optionally substituted heterocyclic group", which is exemplified as the above-mentioned "substituent" for $R^1$ or the like.
Examples of the "5- or 6-membered heterocycle" of the "-CONH-(5- or 6-membered heterocycle)" for $R^3$ to $R^6$ include a ring (preferably triazole) corresponding to the above-mentioned "5- or 6-membered heterocyclic group".
$R^5$ and $R^6$ are the same or different and each is a hydrogen atom, a halogen atom, a $C_{1-6}$ alkyl group, a $C_{1-6}$ alkoxy group, a cyano group, a nitro group, an amino group, a carboxy group, a hydroxy group, -COR, -$NHSO_2R$, -NHCONHR wherein R is as defined above, -$C_{1-6}$ alkylene-COOH, -$C_{1-6}$ alkylene-COOR, -O-$C_{1-6}$ alkylene-$C_{6-14}$ arene, -CONH-(5- or 6-membered heterocycle) or a 5- or 6-membered heterocyclic group.
$R^3$ and $R^4$ are the same or different and each is a hydrogen atom, a halogen atom, a $C_{1-6}$ alkyl group, a $C_{1-6}$ alkoxy group, a carboxy group, -COR, a 5- or 6-membered heterocyclic group or a $C_{6-14}$ aryl group.
Preferable examples of compound (IIa) include the following compound.

[Compound A-a]

Compound (IIa) wherein

ring Ea is a thiophene ring, a benzothiophene ring, a thienopyridine ring or a pyridine ring ;
Wa is $CH_2$ or NH (preferably $CH_2$);
Xa is O, S, SO, $SO_2$ or NH (preferably O or S);
Ya is -$NH_2$, -NHCONHR, -NHCOOR or -NHCOR;
$R^5$ and $R^6$ are the same or different and each is a hydrogen atom, a halogen atom, a $C_{1-6}$ alkyl group, a $C_{1-6}$ alkoxy group, a cyano group, a nitro group, an amino group, a carboxy group, a hydroxy group, -COR, -$NHSO_2R$, -NHCONHR, -$C_{1-6}$ alkylene-COOH, -$C_{1-6}$ alkylene-COOR, -O-$C_{1-6}$ alkylene-$C_{6-14}$ arene, -CONH-(5- or 6-membered heterocycle) or a 5- or 6-membered heterocyclic group;
$R^3$ and $R^4$ are the same or different and each is a hydrogen atom, a halogen atom, a $C_{1-6}$ alkyl group, a $C_{1-6}$ alkoxy group, a carboxy group, -COR, a 5- or 6-membered heterocyclic group or a $C_{6-14}$ aryl group; and
R is a hydrogen atom or a $C_{1-6}$ alkyl group.

As a salt of the compound represented by the formula (I) or the formula (IIa), a pharmacologically acceptable salt is preferable. Examples of such salt include salts with inorganic base, salts with organic base, salts with inorganic acid, salts with organic acid, salts with basic or acidic amino acid, and the like.
Preferable examples of the salt with inorganic base include alkali metal salts such as sodium salt, potassium salt and the like; alkaline earth metal salts such as calcium salt, magnesium salt and the like; aluminum salt: ammonium salt and the like.
Preferable examples of the salt with organic base include salts with trimethylamine, triethylamine, pyridine, picoline, ethanolamine, diethanolamine, triethanolamine, tromethamine[tris(hydroxymethyl)methylamine], tert-butylamine, cyclohexylamine, benzylamine, dicyclohexylamine, N,N-dibenzylethylenediamine and the like.
Preferable examples of the salt with inorganic acid include salts with hydrochloric acid, hydrobromic acid, nitric acid, sulfuric acid, phosphoric acid and the like.
Preferable examples of the salt with organic acid include salts with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, p-toluenesulfonic acid and the like.
Preferable examples of the salt with basic amino acid include salts with arginine, lysine, ornithine and the like.
Preferable examples of the salt with acidic amino acid include salts with aspartic acid, glutamic acid and the like.
A prodrug of compound (I) or compound (IIa) (hereinafter to be collectively referred to as compound (I)) means a compound which is converted to compound (I) with a reaction due to an enzyme, an gastric acid, etc. under the physiological condition in the living body, that is, a compound which is converted to compound (I) by oxidation, reduction, hydrolysis, etc. according to an enzyme; a compound which is converted to compound (I) by hydrolysis etc. due to gastric acid, etc.
Examples of the prodrug of compound (I) include a compound obtained by subjecting an amino group in

compound (I) to an acylation, alkylation or phosphorylation (e.g., a compound obtained by subjecting an amino group in compound (I) to an eicosanoylation, alanylation, pentylaminocarbonylation, (5-methyl-2-oxo-1,3-dioxolen-4-yl)methoxycarbonylation, tetrahydrofuranylation, pyrrolidylmethylation, pivaloyloxymethylation or tert-butylation); a compound obtained by subjecting a hydroxy group in compound (I) to an acylation, alkylation, phosphorylation or boration (e.g., a compound obtained by subjecting a hydroxy group in compound (I) to an acetylation, palmitoylation, propanoylation, pivaloylation, succinylation, fumarylation, alanylation or dimethylaminomethylcarbonylation); a compound obtained by subjecting a carboxyl group in compound (I) to an esterification or amidation (e.g., a compound obtained by subjecting a carboxyl group in compound (I) to an ethyl esterification, phenyl esterification, carboxymethyl esterification, dimethylaminomethyl esterification, pivaloyloxymethyl esterification, ethoxycarbonyloxyethyl esterification, phthalidyl esterification, (5-methyl-2-oxo-1,3-dioxolen-4-yl)methyl esterification, cyclohexyloxycarbonylethyl esterification or methyl amidation etc.) and the like. These compounds can be produced from compound (I) according to a method known *per se*.

A prodrug for compound (I) may also be one which is converted to compound (I) under a physiological condition, such as those described in IYAKUHIN no KAIHATSU, Development of Pharmaceuticals, Vol. 7, Design of Molecules, p. 163-198, Published by HIROKAWA SHOTEN, 1990.

In addition, compound (I) may be labeled with an isotope (e.g., $^{3}H$, $^{14}C$, $^{35}S$, $^{125}I$) and the like.
Moreover, compound (I) may be an anhydride or a hydrate.

Compound (I) or a prodrug thereof (hereinafter sometimes to be abbreviated simply as the compound of the present invention) has low toxicity, and can be used as an agent for the prophylaxis or treatment of various diseases mentioned below in a mammal (e.g., human, mouse, rat, rabbit, dog, cat, bovine, horse, swine, monkey) directly or in the form of a pharmaceutical composition by admixing with a pharmacologically acceptable carrier and the like.

Here, examples of the pharmacologically acceptable carrier include various organic or inorganic carrier substances conventionally used as preparation materials, which are added as excipient, lubricant, binder or disintegrant for solid dosage forms; as solvent, solubilizing agent, suspending agent, isotonicity agent, buffer or soothing agent for liquid preparation, and the like. Where necessary, preparation additives such as preservative, antioxidant, colorant, sweetener and the like can also be used.

Preferable examples of the excipient include lactose, sucrose, D-mannitol, D-sorbitol, starch, pregelatinized starch, dextrin, crystalline cellulose, low-substituted hydroxypropylcellulose, sodium carboxymethylcellulose, gum arabic, pullulan, light anhydrous silicic acid, synthetic aluminum silicate and magnesium aluminometasilicate.

Preferable examples of the lubricant include magnesium stearate, calcium stearate, talc and colloidal silica.

Preferable examples of the binder include pregelatinized starch, sucrose, gelatin, gum arabic, methylcellulose, carboxymethylcellulose, sodium carboxymethylcellulose, crystalline cellulose, sucrose, D-mannitol, trehalose, dextrin, pullulan, hydroxypropylcellulose, hydroxypropylmethylcellulose and polyvinylpyrrolidone.

Preferable examples of the disintegrant include lactose, sucrose, starch, carboxymethylcellulose, calcium carboxymethylcellulose, sodium croscarmellose, sodium carboxymethylstarch, light anhydrous silicic acid and low-substituted hydroxypropylcellulose.

Preferable examples of the solvent include water for injection, physiological brine, Ringer's solution, alcohol, propylene glycol, polyethylene glycol, sesame oil, corn oil, olive oil and cottonseed oil.

Preferable examples of the solubilizing agent include polyethylene glycol, propylene glycol, D-mannitol, trehalose, benzyl benzoate, ethanol, trisaminomethane, cholesterol, triethanolamine, sodium carbonate, sodium citrate, sodium salicylate and sodium acetate.

Preferable examples of the suspending agent include surfactants such as stearyltriethanolamine, sodium lauryl sulfate, lauryl aminopropionic acid, lecithin, benzalkonium chloride, benzethonium chloride, glyceryl monostearate and the like; hydrophilic polymers such as polyvinyl alcohol, polyvinylpyrrolidone, sodium carboxymethylcellulose, methylcellulose, hydroxymethylcellulose, hydroxyethylcellulose, hydroxypropylcellulose and the like; polysorbates and polyoxyethylene hydrogenated castor oil.

Preferable examples of the isotonicity agent include sodium chloride, glycerol, D-mannitol, D-sorbitol and glucose.

Preferable examples of the buffer include buffers such as phosphate, acetate, carbonate, citrate and the like.
Preferable examples of the soothing agent include benzyl alcohol.

Preferable examples of the preservative include paraoxybenzoates, chlorobutanol, benzyl alcohol, phenethyl alcohol, dehydroacetic acid and sorbic acid.
Preferable examples of the antioxidant include sulfite, ascorbate and the like.

Preferable examples of the colorant include aqueous food tar colors (e.g., food colors such as Food Red No. 2 and No. 3, Food Yellow No. 4 and No. 5, Food Blue No. 1 and No. 2, etc.), water insoluble lake dye (e.g., aluminum salt of the above-mentioned aqueous food tar color) and natural dye (e.g., β-carotene, chlorophyll, ferric oxide red).

Preferable examples of the sweetening agent include sodium saccharin, dipotassium glycyrrhizinate, aspartame and stevia.

Examples of the dosage form of the above-mentioned pharmaceutical composition include oral preparations

such as tablets (inclusive of sugar-coated tablets, film-coated tablets, sublingual tablets, orally disintegrating tablets), capsules (inclusive of soft capsules, microcapsules), granules, powders, troches, syrups, emulsions, suspensions, films (e.g., orally disintegrable films) and the like; and parenteral agents such as injections (e.g., subcutaneous injections, intravenous injections, intramuscular injections, intraperitoneal injections, drip infusions), external preparations (e.g., dermal preparations, ointments), suppository (e.g., rectal suppositories, vaginal suppositories), pellets, nasal preparations, pulmonary preparations (inhalants), eye drops and the like. These may be safely administered orally or parenterally (e.g., topically, rectally, intravenously administered).

These preparations may be release control preparations (e.g., sustained-release microcapsule) such as immediate-release preparation, sustained-release preparation and the like.

A pharmaceutical composition can be produced by a method conventionally used in the technical field of pharmaceutical preparation, for example, the method described in the Japanese Pharmacopoeia and the like.

While the content of the compound of the present invention in the pharmaceutical composition varies depending on the dosage form, dose of the compound of the present invention, and the like, it is, for example, about 0.1 to 100 wt%.

During production of an oral preparation, coating may be applied as necessary for the purpose of masking of taste, enteric property or durability.

Examples of the coating base to be used for coating include sugar coating base, aqueous film coating base, enteric film coating base and sustained-release film coating base.

As the sugar coating base, sucrose is used. Moreover, one or more kinds selected from talc, precipitated calcium carbonate, gelatin, gum arabic, pullulan, carnauba wax and the like may be used in combination.

Examples of the aqueous film coating base include cellulose polymers such as hydroxypropyl cellulose, hydroxypropylmethyl cellulose, hydroxyethyl cellulose, methylhydroxyethyl cellulose etc.; synthetic polymers such as polyvinylacetal diethylaminoacetate, aminoalkyl methacrylate copolymer E [Eudragit E (trade name)], polyvinylpyrrolidone etc.; and polysaccharides such as pullulan etc.

Examples of the enteric film coating base include cellulose polymers such as hydroxypropylmethyl cellulose phthalate, hydroxypropylmethyl cellulose acetate succinate, carboxymethylethyl cellulose, cellulose acetate phthalate etc.; acrylic polymers such as methacrylic acid copolymer L [Eudragit L (trade name)], methacrylic acid copolymer LD [Eudragit L-30D55 (trade name)], methacrylic acid copolymer S [Eudragit S (trade name)] etc.; and naturally occurring substances such as shellac etc.

Examples of the sustained-release film coating base include cellulose polymers such as ethyl cellulose etc.; and acrylic polymers such as aminoalkyl methacrylate copolymer RS [Eudragit RS (trade name)], ethyl acrylate-methyl methacrylate copolymer suspension [Eudragit NE (trade name)] etc.

The above-mentioned coating bases may be used after mixing with two or more kinds thereof at appropriate ratios. For coating, for example, a light shielding agent such as titanium oxide, diiron trioxide and the like can be used.

The compound of the present invention shows low toxicity (e.g., acute toxicity, chronic toxicity, genetic toxicity, reproductive toxicity, cardiotoxicity, carcinogenicity and the like) and a few side effects. Therefore, it can be used as an agent for the prophylaxis or treatment or a diagnostic of various diseases in a mammal (e.g., human, bovine, horse, dog, cat, monkey, mouse, rat).

The compound of the present invention has a superior ACC (acetyl-CoA carboxylase) inhibitory action. Examples of ACC include liver, adipose tissue or pancreas-specific isozyme (ACC1); and muscle specific isozyme (ACC2). The compound of the present invention particularly has a selective inhibitory action on ACC2.

The compound of the present invention can be used as an agent for the prophylaxis or treatment of obesity, diabetes (e.g., type 1 diabetes, type 2 diabetes, gestational diabetes, obese diabetes), hyperlipidemia (e.g., hypertriglyceridemia, hypercholesterolemia, hypoHDL-emia, postprandial hyperlipemia), hypertension, cardiac failure, diabetic complications [e.g., neuropathy, nephropathy, retinopathy, diabetic cardiomyopathy, cataract, macroangiopathy, osteopenia, hyperosmolar diabetic coma, infections (e.g., respiratory infection, urinary tract infection, gastrointestinal infection, dermal soft tissue infections, inferior limb infection), diabetic gangrene, xerostomia, hypacusis, cerebrovascular disorder, peripheral blood circulation disorder], metabolic syndrome (pathology having three or more selected from hypertriglyceridemia (TG), low HDL cholesterol (HDL-C), hypertension, abdomen obesity and impaired glucose tolerance), sarcopenia, cancer and the like.

For diagnostic criteria of diabetes, Japan Diabetes Society reported new diagnostic criteria in 1999.

According to this report, diabetes is a condition showing any of a fasting blood glucose level (glucose concentration of intravenous plasma) of not less than 126 mg/dl, a 75 g oral glucose tolerance test (75 g OGTT) 2 hr level (glucose concentration of intravenous plasma) of not less than 200 mg/dl, and a non-fasting blood glucose level (glucose concentration of intravenous plasma) of not less than 200 mg/dl. A condition not falling under the above-mentioned diabetes and different from "a condition showing a fasting blood glucose level (glucose concentration of intravenous plasma) of less than 110 mg/dl or a 75 g oral glucose tolerance test (75 g OGTT) 2 hr level (glucose concentration of intravenous plasma) of less than 140 mg/dl" (normal type) is called a "borderline type".

In addition, ADA (American Diabetes Association) in 1997 and WHO in 1998 reported new diagnostic criteria

of diabetes.

According to these reports, diabetes is a condition showing a fasting blood glucose level (glucose concentration of intravenous plasma) of not less than 126 mg/dl and a 75 g oral glucose tolerance test 2 hr level (glucose concentration of intravenous plasma) of not less than 200 mg/dl.

According to the above-mentioned reports, impaired glucose tolerance is a condition showing fasting blood sugar level (glucose concentration of intravenous plasma) of less than 126 mg/dl and a 75 g oral glucose tolerance test 2 hr level (glucose concentration of intravenous plasma) of not less than 140 mg/dl and less than 200 mg/dl. According to the report of ADA, a condition showing a fasting blood glucose level (glucose concentration of intravenous plasma) of not less than 110 mg/dl and less than 126 mg/dl is called IFG (Impaired Fasting Glucose). According to the report of WHO, among the IFG (Impaired Fasting Glucose), a condition showing a 75g oral glucose tolerance test 2 hr level (glucose concentration of intravenous plasma) of less than 140 mg/dl is called IFG (Impaired Fasting Glycemia).

The compound of the present invention can be also used as an agent for the prophylaxis or treatment of diabetes, borderline type, impaired glucose tolerance, IFG (Impaired Fasting Glucose) and IFG (Impaired Fasting Glycemia), as determined according to the above-mentioned new diagnostic criteria. Moreover, the compound of the present invention can prevent progress of borderline type, impaired glucose tolerance, IFG (Impaired Fasting Glucose) or IFG (Impaired Fasting Glycemia) into diabetes.

The compound of the present invention can also be used, for example, as an agent for the prophylaxis or treatment of osteoporosis, cachexia (e.g., carcinocachexia, tuberculous cachexia, diabetic cachexia, hemopathic cachexia, endocrinopathic cachexia, infectious cachexia or cachexia induced by acquired immunodeficiency syndrome), fatty liver, polycystic ovary syndrome, renal disease (e.g., diabetic nephropathy, glomerulonephritis, glomerulosclerosis, nephrosis syndrome, hypertensive nephrosclerosis, terminal renal disorder), muscular dystrophy, myocardial infarction, angina pectoris, cerebrovascular disorder (e.g., cerebral infarction, cerebral apoplexy), Alzheimer's disease, Parkinson's disease, anxiety, dementia, insulin resistance syndrome, syndrome X, hyperinsulinemia, sensory abnormality in hyperinsulinemia, irritable bowel syndrome, acute or chronic diarrhea, inflammatory disease (e.g., chronic rheumatoid arthritis, spondylitis deformans, osteoarthritis, lumbago, gout, postoperative or posttraumatic inflammation, swelling, neuralgia, pharyngolaryngitis, cystitis, hepatitis (including nonalcoholic steatohepatitis), pneumonia, pancreatitis, enteritis, inflammatory bowel disease (including inflammatory colitis), ulcerative colitis, stomach mucous membrane injury (including stomach mucous membrane injury caused by aspirin)), small intestine mucous membrane injury, malabsorption, testis dysfunction, visceral obesity syndrome or sarcopenia.

In addition, the compound of the present invention can also be used as an agent for the prophylaxis or treatment of various cancers (particularly breast cancer (e.g., invasive ductal carcinoma, non-invasive ductal carcinoma, inflammatory breast cancer and the like), prostate cancer (e.g., hormone-dependent prostate cancer, hormone-independent prostate cancer and the like), pancreatic cancer (e.g., pancreatic duct cancer and the like), gastric cancer (e.g., papillary adenocarcinoma, mucinous adenocarcinoma, adenosquamous carcinoma and the like), lung cancer (e.g., non-small cell lung cancer, small cell lung cancer, malignant mesothelioma and the like), colon cancer (e.g., gastrointestinal stromal tumor and the like), rectal cancer (e.g., gastrointestinal stromal tumor and the like), colorectal cancer (e.g., familial colorectal cancer, hereditary nonpolyposis colorectal cancer, gastrointestinal stromal tumor and the like), small intestinal cancer (e.g., non-Hodgkin lymphoma, gastrointestinal stromal tumor and the like), esophagus cancer, duodenal cancer, cancer of the tongue, pharyngeal cancer (e.g., nasopharyngeal cancer, mesopharyngeal cancer, hypopharyngeal cancer and the like), salivary gland cancer, brain tumor (e.g., pineal astrocytoma, pilocytic astrocytoma, diffuse astrocytoma, anaplastic astrocytoma and the like), schwannoma, liver cancer (e.g., primary liver cancer, Extrahepatic Bile Duct Cancer and the like), kidney cancer (e.g., renal cell carcinoma, transitional carcinoma of kidney pelvis and urinary duct, and the like), biliary tract cancer, endometrial carcinoma, uterine cervical cancer, ovary cancer (e.g., ovarian epithelial carcinoma, extragonadal germ cell tumor, ovarian germ cell tumor, ovarian low malignant potential tumor and the like), urinary bladder cancer, urinary tract cancer, skin cancer (e.g., intraocular (ocular) melanoma, Merkel cell carcinoma and the like), hemangioma, malignant lymphoma, malignant melanoma, thyroid cancer (e.g., medullary thyroid carcinoma and the like), parathyroid cancer, nasal cavity cancer, paranasal sinus cancer, bone tumor (e.g., osteosarcoma, Ewing's tumor, uterus sarcoma, soft tissue sarcoma and the like), vascular fibroma, retinoblastoma, penile cancer, testis tumor, solid cancer in childhood (e.g., Wilms' tumor, childhood kidney tumor and the like), Kaposi's sarcoma, Kaposi's sarcoma derived from AIDS, maxillary tumor, fibrous histiocytoma, leiomyosarcoma, rhabdomyosarcoma, leukemia (e.g., acute myelocytic leukemia, acute lymphoblastic leukemia and the like) etc.).

The compound of the present invention can also be used for secondary prevention or suppression of progression of the above-mentioned various diseases (e.g., cardiovascular events such as myocardial infarction and the like).

While the dose of the compound of the present invention varies depending on the subject of administration, administration route, target disease, symptom and the like, for example, for oral administration to an adult diabetic patient, it is generally about 0.01 to 100 mg/kg body weight, preferably 0.05 to 30 mg/kg body weight, more preferably 0.1 to 10 mg/kg body weight for one dose, which is desirably administered once to 3 times a day.

With the aim of enhancing the action of the compound of the present invention or decreasing the dose of the

compound and the like, the compound can be used in combination with pharmaceutical agents such as therapeutic agents for diabetes, therapeutic agents for diabetic complications, therapeutic agents for hyperlipidemia, antihypertensive agents, antiobesity agents, diuretics, antithrombotic agents, anticancer agents and the like (hereinafter to be abbreviated as concomitant drug). The time of administration of the compound of the present invention and that of the concomitant drug are not limited, and they may be administered simultaneously or in a staggered manner to the administration subject. In addition, the compound of the present invention and the concomitant drug may be administered as two kinds of preparations containing respective active ingredients or a single preparation containing both active ingredients.

The dose of the concomitant drug can be appropriately determined based on the dose employed clinically. In addition, the mixing ratio of the compound of the present invention and the concomitant drug can be appropriately determined according to the administration subject, administration route, target disease, condition, combination, and the like. For example, when the administration subject is a human, the concomitant drug may be used in an amount of 0.01 to 100 parts by weight per 1 part by weight of the compound of the present invention.

Examples of the therapeutic agents for diabetes include insulin preparations (e.g., animal insulin preparations extracted from pancreas of bovine or swine; human insulin preparations genetically synthesized using *Escherichia coli* or yeast; zinc insulin; protamine zinc insulin; fragment or derivative of insulin (e.g., INS-1), oral insulin preparation), insulin sensitizers (e.g., pioglitazone or a salt thereof (preferably hydrochloride), rosiglitazone or a salt thereof (preferably maleate), Tesaglitazar, Ragaglitazar, Muraglitazar, Edaglitazone, Metaglidasen, Naveglitazar, AMG-131, THR-0921), α-glucosidase inhibitors (e.g., voglibose, acarbose, miglitol, emiglitate), biguanides (e.g., metformin, buformin or a salt thereof (e.g., hydrochloride, fumarate, succinate)), insulin secretagogues [e.g., sulfonylurea (e.g., tolbutamide, glibenclamide, gliclazide, chlorpropamide, tolazamide, acetohexamide, glyclopyramide, glimepiride, glipizide, glybuzole), repaglinide, nateglinide, mitiglinide or a calcium salt hydrate thereof], dipeptidyl peptidase IV inhibitors (e.g., Vildagliptin, Sitagliptin, Saxagliptin, T-6666, TS-021), β3 agonists (e.g., AJ-9677), GPR40 agonists, GLP-1 receptor agonists [e.g., GLP-1, GLP-LMR agent, NN-2211, AC-2993 (exendin-4), BIM-51077, Aib(8,35)hGLP-1(7,37)$NH_2$, CJC-1131], amylin agonists (e.g., pramlintide), phosphotyrosine phosphatase inhibitors (e.g., sodium vanadate), gluconeogenesis inhibitors (e.g., glycogen phosphorylase inhibitors, glucose-6-phosphatase inhibitors, glucagon antagonists), SGLUT (sodium-glucose cotransporter) inhibitors (e.g., T-1095), 11β-hydroxysteroid dehydrogenase inhibitors (e.g., BVT-3498), adiponectin or agonist thereof, IKK inhibitors (e.g., AS-2868), leptin resistance improving drugs, somatostatin receptor agonists, glucokinase activators (e.g., Ro-28-1675), GIP (Glucose-dependent insulinotropic peptide) and the like.

Examples of the therapeutic agent for diabetic complications include aldose reductase inhibitors (e.g., tolrestat, epalrestat, zenarestat, zopolrestat, minalrestat, fidarestat, CT-112), neurotrophic factors and increasing drugs thereof (e.g., NGF, NT-3, BDNF, neurotrophin production/secretion promoting agents (e.g., 4-(4-chlorophenyl)-2-(2-methyl-1-imidazolyl)-5-[3-(2-methylphenoxy)propyl]oxazole) described in WO01/14372), nerve regeneration promoters (e.g., Y-128), PKC inhibitors (e.g., ruboxistaurin mesylate), AGE inhibitors (e.g., ALT946, pimagedine, pyratoxanthine, N-phenacylthiazolium bromide (ALT766), ALT-711, EXO-226, pyridorin, pyridoxamine), active oxygen scavengers (e.g., thioctic acid), cerebral vasodilators (e.g., tiapuride, mexiletine), somatostatin receptor agonists (e.g., BIM23190), apoptosis signal regulating kinase-1 (ASK-1) inhibitor and the like.

Examples of the therapeutic agent for hyperlipidemia include statin compounds (e.g., pravastatin, simvastatin, lovastatin, atorvastatin, fluvastatin, rosuvastatin, pitavastatin or a salt thereof (e.g., sodium salt, calcium salt)), squalene synthase inhibitors (e.g., compounds described in WO97/10224, for example, N-[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]piperidine-4-acetic acid), fibrate compounds (e.g., bezafibrate, clofibrate, simfibrate, clinofibrate), ACAT inhibitors (e.g., Avasimibe, Eflucimibe), anion exchange resins (e.g., colestyramine), probucol, nicotinic acid drugs (e.g., nicomol, niceritrol), ethyl icosapentate, phytosterols (e.g., soysterol, γ-oryzanol) and the like.

Examples of the antihypertensive agent include angiotensin converting enzyme inhibitors (e.g., captopril, enalapril, delapril), angiotensin II antagonists (e.g., candesartan cilexetil, losartan, eprosartan, valsartan, telmisartan, irbesartan, tasosartan, 1-[[2'-(2,5-dihydro-5-oxo-4H-1,2,4-oxadiazol-3-yl)biphenyl-4-yl]methyl]-2-ethoxy-1H-benzimidazole-7-carboxylic acid), calcium antagonists (e.g., manidipine, nifedipine, amlodipine, efonidipine, nicardipine), potassium channel openers (e.g., levcromakalim, L-27152, AL 0671, NIP-121), clonidine and the like.

Examples of the antiobesity agent include central nervous system antiobesity drugs (e.g., dexfenfluramine, fenfluramine, phentermine, sibutramine, amfepramone, dexamphetamine, mazindol, phenylpropanolamine, clobenzorex; MCH receptor antagonists (e.g., SB-568849; SNAP-7941; compounds described in WO01/82925 and WO01/87834); neuropeptide Y antagonists (e.g., CP-422935); cannabinoid receptor antagonists (e.g., SR-141716, SR-147778); ghrelin antagonist; 11β-hydroxysteroid dehydrogenase inhibitors (e.g., BVT-3498)), pancreatic lipase inhibitors (e.g., orlistat, cetilistat), β3 agonists (e.g., AJ-9677, AZ40140), anorectic peptides (e.g., leptin, CNTF (ciliary neurotrophic factor)), cholecystokinin agonists (e.g., lintitript, FPL-15849), feeding deterrents (e.g., P-57) and the like.

Examples of the diuretics include xanthine derivatives (e.g., theobromine sodium salicylate, theobromine calcium salicylate), thiazide preparations (e.g., ethiazide, cyclopenthiazide, trichloromethiazide, hydrochlorothiazide, hydroflumethiazide, benzylhydrochlorothiazide, penflutizide, polythiazide, methyclothiazide), antialdosterone preparations (e.g., spironolactone, triamterene), carbonic anhydrase inhibitors (e.g., acetazolamide), chlorobenzenesulfonamide agents (e.g., chlortalidone, mefruside, indapamide), azosemide, isosorbide, ethacrynic acid, piretanide, bumetanide, furosemide and the like.

Examples of the antithrombotic agent include heparin (e.g., heparin sodium, heparin calcium, dalteparin sodium), warfarin (e.g., warfarin potassium), anti-thrombin drugs (e.g., aragatroban), thrombolytic agents (e.g., urokinase, tisokinase, alteplase, nateplase, monteplase, pamiteplase), platelet aggregation inhibitors (e.g., ticlopidine hydrochloride, cilostazol, ethyl icosapentate, beraprost sodium, sarpogrelate hydrochloride) and the like.

The production method of compound (I) is explained in the following. Compound (I) can be produced by, for example, Reaction Scheme 1, 2, 3, 5, 7 or 8 to be described in detail in the following or a method according thereto.
In the following Reaction Schemes 1 to 8, the compound used as a starting compound may be each in the form of a salt. Examples of the salt include those exemplified as the salt of compound (I).

In each reaction of the following Reaction Schemes 1 to 8, the product can be used for the next reaction as the reaction mixture or as a crude product, or can also be isolated according to a conventional method from the reaction mixture, and can also be easily purified according to a conventional separation means (e.g., recrystallization, distillation, chromatography).

When alkylation reaction, hydrolysis, amination reaction, esterification reaction, amidation reaction, etherification reaction, oxidation reaction, reduction reaction and the like are to be performed in the following Reaction Schemes 1 to 8, these reactions are performed according to a method known per se. Examples of such method include the methods described in ORGANIC FUNCTIONAL GROUP PREPARATIONS, 2nd ed., ACADEMIC PRESS, INC., 1989; Comprehensive Organic Transformations, VCH Publishers Inc., 1989 and the like, and the like.

In the following Reaction Schemes 1 to 8, the deprotection can be carried out according to a method known per se, for example, the method described in Protective Groups in Organic Synthesis, John Wiley and Sons (1980), or the like.
Specific examples thereof include a method using acid, base, ultraviolet rays, hydrazine, phenylhydrazine, sodium N-methyldithiocarbamate, tetrabutylammonium fluoride, palladium acetate, trialkylsilyl halides (e.g., trimethylsilyl iodide, trimethylsilyl bromide) and the like, reduction method and the like.

The solvents and bases used for each reaction are explained in the following.
Examples of the "nitrile solvent" include acetonitrile, propionitrile and the like.
Examples of the "amide solvent" include N,N-dimethylformamide (DMF), N,N-dimethylacetamide, N-methylpyrrolidone and the like.
Examples of the "halogenated hydrocarbon solvent" include dichloromethane, chloroform, 1,2-dichloroethane, carbon tetrachloride and the like.
Examples of the "ether solvent" include diethyl ether, diisopropyl ether, tert-butyl methyl ether, tetrahydrofuran (THF), 1,4-dioxane, 1,2-dimethoxyethane and the like.
Examples of the "aromatic solvent" include benzene, toluene, xylene, pyridine and the like.
Examples of the "aliphatic hydrocarbon solvent" include hexane, pentane, cyclohexane and the like.
Examples of the "sulfoxide solvent" include dimethyl sulfoxide (DMSO) and the like.
Examples of the "alcohol solvents" include methanol, ethanol, propanol, 2-propanol, butanol, isobutanol, tert-butanol and the like.
Examples of the "ester solvent" include methyl acetate, ethyl acetate, n-butyl acetate, tert-butyl acetate and the like.
Examples of the "ketone solvent" include acetone, methyl ethyl ketone and the like.
Examples of the "organic acid solvent" include formic acid, acetic acid, propionic acid, trifluoroacetic acid, methanesulfonic acid and the like.

Examples of the "inorganic base" include sodium hydroxide, potassium hydroxide, lithium hydroxide, barium hydroxide and the like.
Examples of the "basic salt" include sodium carbonate, potassium carbonate, cesium carbonate, sodium hydrogen carbonate, potassium hydrogen carbonate and the like.
Examples of the "aromatic amine" include pyridine, imidazole, 2,6-lutidine and the like.
Examples of the "secondary amine" include pyrrolidine, piperidine, morpholine and the like.
Examples of the "tertiary amine" include triethylamine, diisopropylethylamine, N-methylmorpholine, DBU (1,8-diazabicyclo[5.4.0]undec-7-ene), DBN (1,5-diazabicyclo[4.3.0]non-5-ene) and the like.
Examples of the "hydride of alkali metal or alkaline earth metal" include lithium hydride, sodium hydride, potassium hydride, calcium hydride and the like.
Examples of the "metal amide" include lithium amide, sodium amide, lithium diisopropylamide, lithium dicyclohexylamide, lithium hexamethyldisilazide, sodium hexamethyldisilazide, potassium hexamethyldisilazide and the like.
Examples of the "alkyl metal" include n-butyllithium, sec-butyllithium, tert-butyllithium, methylmagnesium bromide and the like.

Examples of the "aryl metal" include phenyllithium, phenylmagnesium bromide and the like.
Examples of the "metal alkoxide" include sodium methoxide, sodium ethoxide, potassium tert-butoxide and the like.
The abbreviations in the reaction schemes are explained.

Ring E, ring P, W, X, Y, $R^{3a}$ and $R^A$ are as defined above.
Ra, Ra' and Rb are each independently a hydrogen atom or a substituent. Examples of the "substituent" include those similar to the above-mentioned "substituent" for $R^1$ or the like.
Rc is a substituent. Examples of the "substituent" include those similar to the above-mentioned "substituent" for $R^1$ or the like.
Hal is a halogen atom.
L is a mercapto-protecting group mentioned below generally used in peptide chemistry and the like.
La is an amino-protecting group mentioned below generally used in peptide chemistry and the like.
Ring Eb is benzene or a 5- to 7-membered monocyclic aromatic heterocycle, each of which is optionally substituted. Examples of the 5- to 7-membered monocyclic aromatic heterocycle include a 5- to 7-membered ring (e.g., pyridine, pyrazine, pyrimidine, pyridazine), from among the rings corresponding to the monocyclic aromatic heterocyclic group exemplified as the "heterocyclic group" of the "optionally substituted heterocyclic group", which is exemplified as the above-mentioned "substituent" for $R^1$ or the like.
The "benzene" and "5- to 7-membered monocyclic aromatic heterocycle" of the "benzene or 5- to 7-membered monocyclic aromatic heterocycle, each of which is optionally substituted" for ring Eb each optionally further has 1 to 3 substituents at substitutable positions. Examples of the substituent include those similar to the substituents that the $C_{3-10}$ cycloalkyl group and the like exemplified as the above-mentioned "optionally substituted hydrocarbon group" exemplified as the above-mentioned "substituent" for $R^1$ or the like optionally has. When the number of the substituents is not less than 2, the respective substituents may be the same or different.
Wc is O or S.
Wb is a $C_{1-4}$ alkylene. The $C_{1-4}$ alkylene is as defined for the above-mentioned "$C_{1-4}$ alkylene" for W.
Z and Za are each independently a leaving group. Examples of the leaving group include a halogen atom, a sulfonated hydroxy group (e.g., a toluenesulfonyloxy group, a methanesulfonyloxy group, a trifluoromethanesulfonyloxy group) and the like.

<Reaction Scheme 1>

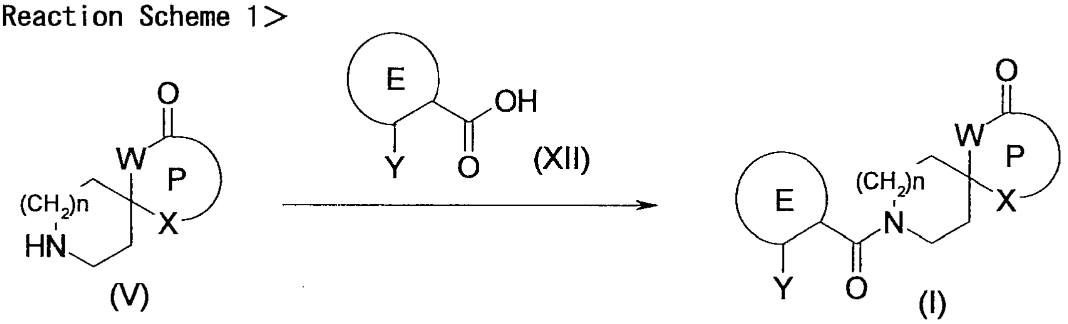

Compound (I) can be produced, for example, by subjecting compound (V) to an amidation reaction with compound (XII).
As compound (V), a commercially available product can be easily obtained, or it can be produced according to a method known per se, for example, the methods described in the below-mentioned Reaction Schemes 2 to 4 or 6 to 8 or a method analogous thereto.
As compound (XII), a commercially available product can be easily obtained, or it can be produced according to a method known per se, for example, the method described in the below-mentioned Reaction Scheme 5 or a method analogous thereto.
Examples of the "amidation reaction" include the following "method using a dehydration-condensation agent", "method using a reactive derivative of carboxylic acid" and the like.

i) Method using a dehydration-condensation agent

This reaction is carried out by reacting compound (V) with compound (XII) in the presence of a dehydration-condensation agent, in an inert solvent. Where necessary, the reaction may be carried out in the presence of a catalytic amount to 5 equivalents of 1-hydroxybenzotriazole (HOBt), a catalytic amount to 5 equivalents of a base or the like.
The amount of compound (V) to be used is generally 0.5 to 5 equivalents, preferably 0.8 to 1.5 equivalents, relative to compound (XII).
Examples of the dehydration-condensation agent include dicyclohexylcarbodiimide (DCC), 1-ethyl-3-(3-dimethylaminopropyl)carbodiimide hydrochloride (EDC·HCl) and the like. Of these, EDC·HCl is preferable. The amount of the dehydration-condensation agent to be used is generally 1 to 10 equivalents, preferably 1 to 5 equivalents, relative to compound (XII).
Examples of the inert solvent include nitrile solvents, amide solvents, halogenated hydrocarbon solvents, ether solvents and the like. These solvents may be used in a mixture of two or more kinds thereof at an appropriate ratio. Of those, amide solvents are preferable.
Examples of the base include aromatic amines, tertiary amines and the like.
The reaction temperature is generally -70 to 150°C, preferably -20 to 100°C.
The reaction time is generally 0.1 to 100 hr, preferably 1 to 48 hr.

ii) Method using a reactive derivative of carboxylic acid

This reaction is carried out by reacting a reactive derivative of compound (XII) with 0.5 to 5 equivalents (preferably 0.8 to 3 equivalents) of compound (V) in an inert solvent. Where necessary, the reaction may be carried out in the presence of 1 to 10 equivalents, preferably 1 to 3 equivalents of a base.
Examples of the reactive derivative of compound (XII) include acid halides (e.g., acid chloride, acid bromide), mixed acid anhydrides (e.g., acid anhydrides with a $C_{1-6}$ alkyl-carboxylic acid, a $C_{6-10}$ aryl-carboxylic acid or a $C_{1-6}$ alkylcarbonic acid), activated esters (e.g., esters with a phenol optionally having substituent(s), HOBt, N-hydroxysuccinimide or the like) and the like.
Examples of the "substituent" of the above-mentioned "phenol optionally having substituent(s)" include those similar to the substituents that the $C_{3-10}$ cycloalkyl group and the like exemplified as the "hydrocarbon group" of the above-mentioned "optionally substituted hydrocarbon group" exemplified as the above-mentioned "substituent" for $R^1$ or the like optionally has.
Specific examples of the "phenol optionally having substituent(s)" include phenol, pentachlorophenol, pentafluorophenol, p-nitrophenol and the like.
The reactive derivative is preferably an acid halide.
Examples of the inert solvent include ether solvents, halogenated hydrocarbon solvents, aromatic solvents, aliphatic hydrocarbon solvents, nitrile solvents, amide solvents, ketone solvents, sulfoxide solvents, water and the like. These solvents may be used in a mixture of two or more kinds thereof at an appropriate ratio. Of those, acetonitrile, THF, dichloromethane, chloroform and the like are preferable.
Examples of the base include aromatic amines, tertiary amines and the like.
The reaction temperature is generally -20°C to 100°C, preferably -20°C to 50°C.
The reaction time is generally 5 min to 40 hr, preferably 30 min to 18 hr.
In the following reaction schemes, the production methods of compound (I) and starting material compounds, in each of which n is 1, are explained. Compound (I) and starting material compounds, in each of which n is 0, 2 or 3, can be produced by a method similar or analogous to such methods.
<Reaction Scheme 2>

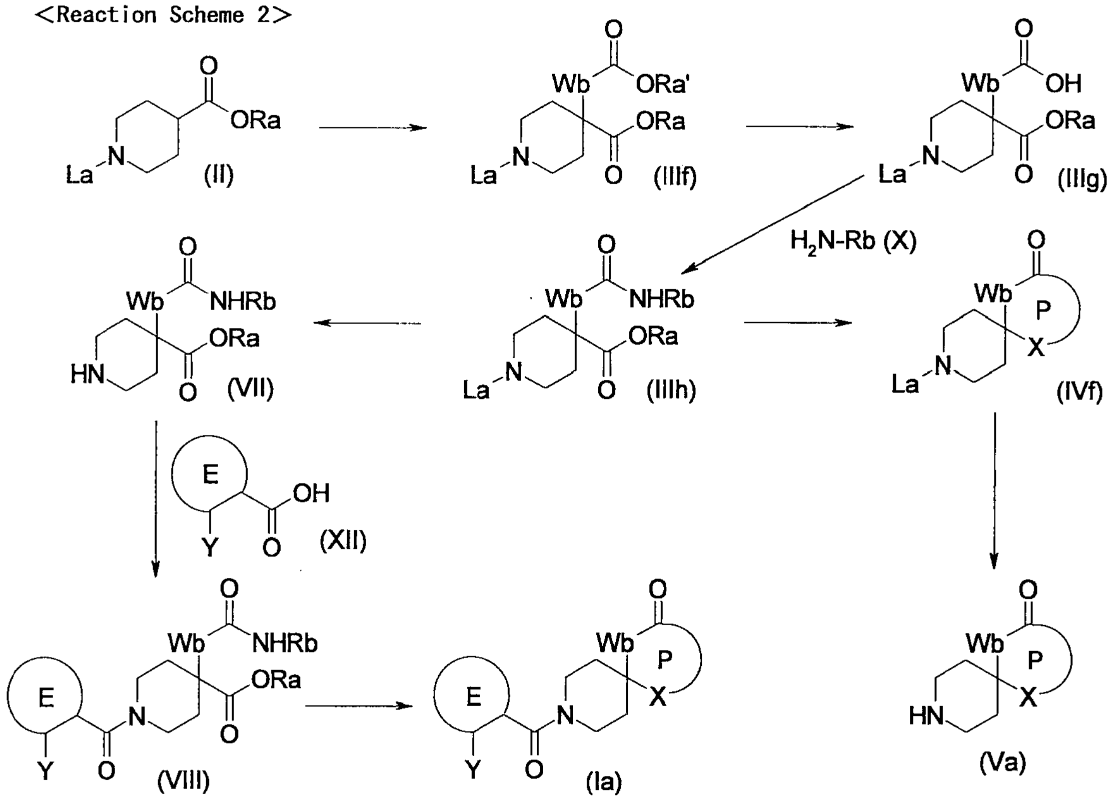


Compound (IIIf) can be produced, for example, by subjecting compound (II) to an alkylation reaction.
The alkylation reaction can be carried out, according to a method known per se, for example, the method described in Journal of Medicinal Chemistry (J. Med. Chem.) pages 2439-2441, 1998 or the like or a method analogous thereto.
As compound (II), a commercially available product can be easily obtained, or it can be produced according to a method known per se.
This reaction is carried out by reacting compound (II) with an alkylating agent in the presence of a base, in an inert solvent.
Examples of the alkylating agent include (alkoxycarbonyl)alkyl halides (e.g., ethyl bromoacetate), alkenyl esters (e.g., methyl acrylate) and the like. The amount of the alkylating agent to be used is generally 1 to 5 equivalents, preferably 1 to 1.5 equivalents, relative to compound (II).
Examples of the inert solvent include ether solvents, aromatic solvents, aliphatic hydrocarbon solvents and the like. These solvents may be used in a mixture of two or more kinds thereof at an appropriate ratio. Of those, ether solvents and the like are preferable.
Examples of the base include hydrides of alkali metal or alkaline earth metal, metal amides, alkyl metals, aryl metals and the like. The amount of the base to be used is generally 1 to 10 equivalents, preferably 1 to 1.5 equivalents, relative to compound (II).
The reaction temperature is generally -100°C to 150°C, preferably -78°C to 100°C.
The reaction time is generally 5 min to 48 hr, preferably 30 min to 24 hr.

Compound (IIIg) can be produced, for example, by subjecting compound (IIIf) to hydrolysis.
This reaction carried out by reacting compound (IIIf) with a base in an inert solvent.
Examples of the base include inorganic bases and the like. The amount of the base to be used is generally 1 to 10 equivalents, preferably 1 to 1.5 equivalents, relative to compound (IIIf).
Examples of the inert solvent include alcohol solvents, nitrile solvents, aromatic solvents, aliphatic hydrocarbon solvents, ether solvents, amide solvents, halogenated hydrocarbon solvents and the like. These solvents are preferably used in a mixture with water at an appropriate ratio. Of those, alcohol solvents containing water are preferable.
The reaction temperature is generally -78°C to 150°C, preferably -20°C to 100°C.
The reaction time is generally 5 min to 100 hr, preferably 30 min to 24 hr.

Compound (IIIg) can also be produced, for example, by reacting compound (IIIf) in the presence of a metal catalyst and a hydrogen source, in an inert solvent.
Examples of the metal catalyst include palladium-carbon, palladium black, palladium chloride, platinum oxide, platinum black, platinum-palladium, Raney-nickel, Raney-cobalt and the like. The amount of the metal catalyst to be used is

generally 0.001 to 1000 equivalents, preferably 0.01 to 100 equivalents, relative to compound (IIIf).
Examples of the hydrogen source include hydrogen gas, formic acid, amine salts of formic acid, phosphinates, hydrazine and the like.
Examples of the inert solvent include alcohol solvents, nitrile solvents, aromatic solvents, aliphatic hydrocarbon solvents, ether solvents, amide solvents, halogenated hydrocarbon solvents and the like. These solvents are preferably used in a mixture with water at an appropriate ratio. Of those, alcohol solvents are preferable.
The reaction temperature is generally -70 to 150°C, preferably -20 to 100°C.
The reaction time is generally 0.1 to 100 hr, preferably 0.1 to 40 hr.

Compound (IIIg) can also be produced, for example, according to the method described in Protective Groups in Organic Synthesis, John Wiley and Sons (1980).

Compound (IIIh) can be produced, for example, using compound (IIIg) and compound (X), according to the same method as in the amidation reaction of compound (V) and compound (XII) in the above-mentioned Reaction Scheme 1.

Compound (IVf) can be produced, for example, by subjecting compound (IIIh) to cyclization.
The cyclization can be carried out according to a method known per se, for example, the method described in Journal of Medicinal Chemistry (J. Med. Chem.) pages 4118-4129, 1998 or and the like or a method analogous thereto.
This reaction is carried out by reacting compound (IIIh) with a base in an inert solvent.
Examples of the base include hydrides of alkali metal or alkaline earth metal, and the like. The amount of the base to be used is generally 1 to 10 equivalents, preferably 1 to 1.5 equivalents, relative to compound (IIIh).
Examples of the inert solvent include nitrile solvents, aromatic solvents, aliphatic hydrocarbon solvents, ether solvents, amide solvents, halogenated hydrocarbon solvents and the like. These solvents may be used in a mixture of two or more kinds thereof at an appropriate ratio. Of those, amide solvents are preferable.
The reaction temperature is generally -100°C to 150°C, preferably 0°C to 100°C.
The reaction time is generally 5 min to 48 hr, preferably 30 min to 24 hr.

Compound (Va) can be produced, for example, by subjecting compound (IVf) to deprotection.

Compound (VII) can be produced, for example, by subjecting compound (IIIh) to deprotection.

Compound (VIII) can be produced, for example, using compound (VII) and compound (XII), according to the same method as in the amidation reaction of compound (V) and compound (XII) in the above-mentioned Reaction Scheme 1.

Compound (Ia) can be produced, for example, using compound (VIII), according to the same method as in the above-mentioned production reaction of compound (Va) from compound (IIIh).

<Reaction Scheme 3>

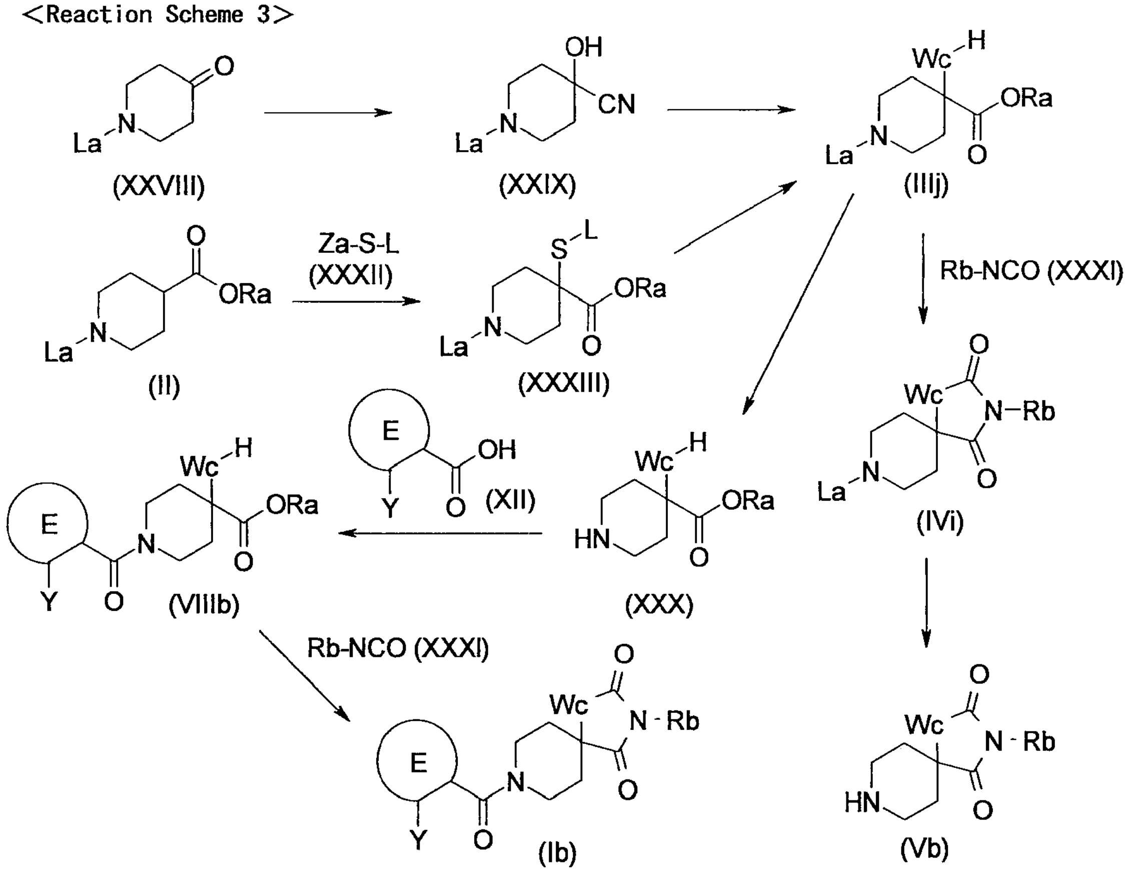


Compound (XXIX) can be produced, for example, by subjecting compound (XXVIII) to a cyanation reaction. As compound (XXVIII), a commercially available product can be easily obtained, or it can be produced according to a method known per se.
The cyanation reaction can be carried out according to a method known per se, for example, the method described in Journal of Medicinal Chemistry (J. Med. Chem.) pages 486-491, 1988 or the like or a method analogous thereto.
This reaction is carried out by reacting compound (XXVIII) with a cyanating agent in an inert solvent. Where necessary, the reaction may be carried out in the presence of 1 to 10 equivalents of an acid.
Examples of cyanating agent include sodium cyanide, potassium cyanide, trimethylsilyl cyanide and the like. The amount of the cyanating agent to be used is generally 1 to 10 equivalents, preferably 1 to 1.5 equivalents, relative to compound (XXVIII).
Examples of the acid include organic acids (e.g., formic acid, acetic acid), Lewis acids (e.g., aluminum chloride, boron trifluoride-diethyl etherate, zinc iodide) and the like.
Examples of the inert solvent include nitrile solvents, amide solvents, halogenated hydrocarbon solvents, ether solvents and the like. These solvents may be used in a mixture of two or more kinds thereof at an appropriate ratio. Of those, halogenated hydrocarbon solvents are preferable.
The reaction temperature is generally -100°C to 150°C, preferably 0°C to 100°C.
The reaction time is generally 5 min to 48 hr, preferably 30 min to 24 hr.

Compound (XXXIII) can be produced, for example, by reacting compound (II) with compound (XXXII).
As compound (XXXII), a commercially available product can be easily obtained, or it can be produced according to a method known per se.
This reaction is carried out by reacting compound (II) with compound (XXXII) in the presence of a base, in an inert solvent. The amount of compound (XXXII) to be used is generally 1 to 5 equivalents, preferably 1 to 1.5 equivalents, relative to compound (II).
Examples of the inert solvent include ether solvents, aromatic solvents, aliphatic hydrocarbon solvents and the like. These solvents may be used in a mixture of two or more kinds thereof at an appropriate ratio. Of those, ether solvents and the like are preferable.
Examples of the base include hydrides of alkali metal or alkaline earth metal, metal amides, alkyl metals, aryl metals

and the like. The amount of the base to be used is generally 1 to 10 equivalents, preferably 1 to 1.5 equivalents, relative to compound (II).
The reaction temperature is generally -100°C to 150°C, preferably -78°C to 100°C.
The reaction time is generally 5 min to 48 hr, preferably 30 min to 24 hr.
Compound (IIIj) can be produced, for example, by subjecting compound (XXIX) to hydrolysis.
This reaction is carried out by reacting compound (XXIX) with a base or an acid in an inert solvent.
Examples of the base include inorganic bases and the like. The amount of the base to be used is generally 1 to 10 equivalents, preferably 1 to 1.5 equivalents, relative to compound (XXIX).
Examples of the acid include organic acids (e.g., formic acid, acetic acid), hydrochloric acid, sulfuric acid and the like. The amount of the acid to be used is generally 1 to 50 equivalents, preferably 1 to 10 equivalents, relative to compound (XXIX). These acids may be used as a solvent.
Examples of the inert solvent include alcohol solvents, nitrile solvents, aromatic solvents, aliphatic hydrocarbon solvents, ether solvents, amide solvents, halogenated hydrocarbon solvents and the like. These solvents are preferably used in a mixture with water at an appropriate ratio. Of those, alcohol solvents containing water are preferable.
The reaction temperature is generally -78°C to 150°C, preferably -20°C to 100°C.
The reaction time is generally 5 min to 100 hr, preferably 30 min to 24 hr.
When a solvent other than an alcohol solvent is used as an inert solvent, compound (IIIj) can be obtained according to a method known per se (e.g., esterification).
Compound (IIIj) can also be produced, for example, by subjecting compound (XXXIII) to deprotection.
Compound (IVi) can be produced, for example, by subjecting compound (IIIj) to cyclization.
This reaction is carried out by reacting compound (IIIj) with compound (XXXI) (hereinafter sometimes to be referred to as an ester of isocyanic acid; e.g., ethylisocyanate, isopropylisocyanate) in the presence of a base, in an inert solvent. The amount of compound (XXXI) to be used is generally 1 to 5 equivalents, preferably 1 to 2 equivalents, relative to compound (IIIj).
Examples of the base include hydrides of alkali metal or alkaline earth metal, and the like. The amount of the base to be used is generally 0.5 to 10 equivalents, preferably 0.5 to 1.5 equivalents, relative to compound (IIIj).
Examples of the inert solvent include nitrile solvents, amide solvents, halogenated hydrocarbon solvents, ether solvents and the like. These solvents may be used in a mixture of two or more kinds thereof at an appropriate ratio. Of those, THF is preferable.
The reaction temperature is generally -78°C to 50°C, preferably room temperature to 50°C.
The reaction time is generally 5 min to 48 hr, preferably 30 min to 24 hr.
As compound (XXXI), a commercially available product can be easily obtained, or it can be produced according to a method known per se.
Compound (Vb) can also be produced, for example, by subjecting compound (IVi) to deprotection.
Compound (XXX) can be produced, for example, by subjecting compound (IIIj) to deprotection.
Compound (VIIIb) can be produced, for example, using compound (XXX) and compound (XII), according to the same method as in the amidation reaction of compound (V) and compound (XII) in the above-mentioned Reaction Scheme 1.
Compound (Ib) can also be produced, for example, using compound (VIIIb), according to the same method as in the above-mentioned cyclization of compound (IIIj).
<Reaction Scheme 4>

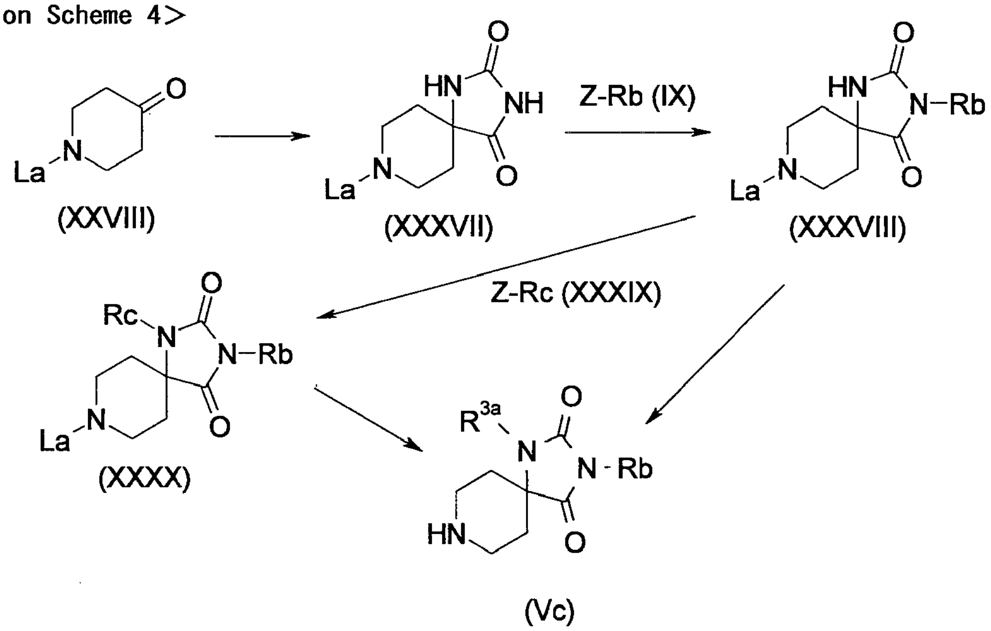


Compound (XXXVII) can be produced, for example, from compound (XXVIII) according to the method described in WO2006/053024 A2 or a method analogous thereto.

Compound (XXXVIII) can be produced, for example, by subjecting compound (XXXVII) to an alkylation reaction. The alkylation reaction can be carried out according to a method known per se, for example, the method described in Journal of Organic Chemistry (J. Org. Chem.) pages 2441-2450, 2004 or the like or a method analogous thereto.

This reaction is carried out by reacting compound (XXXVII) with compound (IX) in the presence of a base, in an inert solvent.

As compound (IX), a commercially available product can be easily obtained, or it can be produced according to a method known per se.

The amount of compound (IX) to be used is generally 1 to 5 equivalents, preferably 1 to 1.5 equivalents, relative to compound (XXXVII).

Examples of the inert solvent include nitrile solvents, amide solvents, halogenated hydrocarbon solvents, ether solvents and the like. These solvents may be used in a mixture of two or more kinds thereof at an appropriate ratio. Of those, THF, DMF and the like are preferable.

Examples of the base include hydrides of alkali metal or alkaline earth metal, and the like. The amount of the base to be used is generally 1 to 10 equivalents, preferably 1 to 1.5 equivalents, relative to compound (XXXVII).

The reaction temperature is generally -100°C to 150°C, preferably 0°C to 100°C.

The reaction time is generally 5 min to 48 hr, preferably 30 min to 24 hr.

Compound (XXXX) can be produced, for example, using compound (XXXIX) and compound (XXXVIII), according to the same method as in the above-mentioned alkylation reaction of compound (XXXVII).

As compound (XXXIX), a commercially available product can be easily obtained, or it can be produced according to a method known per se.

Compound (Vc) can be produced, for example, by subjecting compound (XXXVIII) or compound (XXXX) to deprotection.

<Reaction Scheme 5>

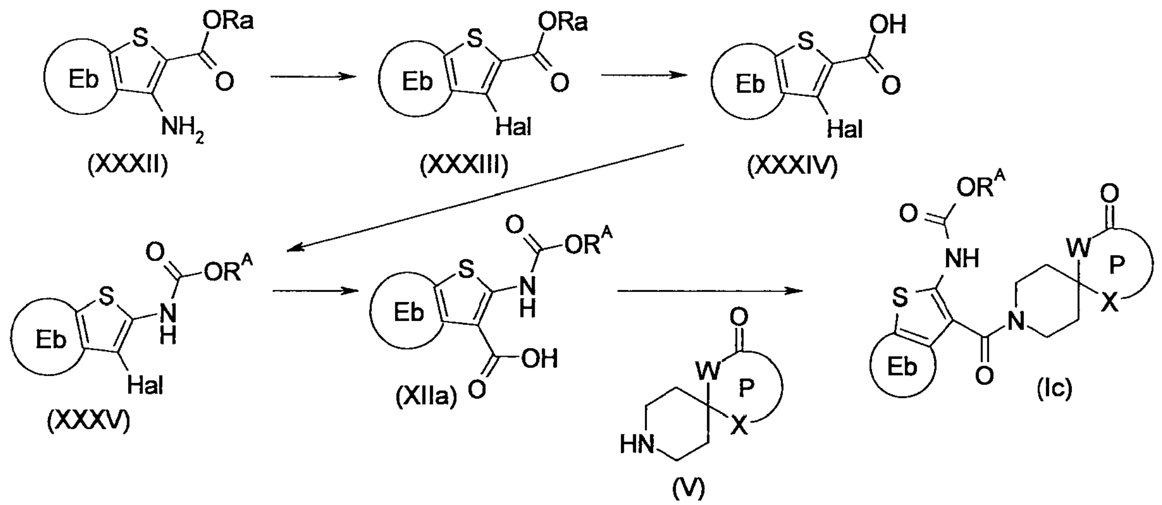

Compound (XXXIII) can be produced, for example, by subjecting compound (XXXII) to halogenation. This reaction is carried out by reacting compound (XXXII) in the presence of a nitrite and a copper halide, in an inert solvent. Compound (XXXII) used for this reaction can be produced, for example, according to the method described in W02004/000846 or a method analogous thereto.
Examples of the nitrite include sodium nitrite, potassium nitrite, tert-butyl nitrite, isoamyl nitrite and the like. The amount of the nitrite to be used is generally 1 to 3 equivalents, preferably 1 to 2 equivalents, relative to compound (XXXII).
Examples of the copper halide include copper(I) bromide, copper(II) bromide, copper(I) chloride and the like. The amount of the copper halide to be used is generally 0.5 to 3 equivalents, preferably 0.5 to 1.5 equivalents, relative to compound (XXXII).
Examples of the inert solvent include nitrile solvents, ether solvents, amide solvents, water, a mixture of two or more kinds thereof, and the like.
The reaction temperature is generally -78°C to 50°C, preferably -20°C to 10°C.
The reaction time is generally 5 min to 100 hr, preferably 30 min to 10 hr.
Compound (XXXIV) can be produced, for example, from compound (XXXIII), according to the same method as in the hydrolysis of compound (IIIf) in the above-mentioned Reaction Scheme 2.
Compound (XXXV) can be produced, for example, by directly subjecting compound (XXXIV), or a reactive derivative thereof (e.g., an acid halide, an acid amide, an acid anhydride, an ester etc.) and the like, which are obtained by converting compound (XXXIV), to a rearrangement reaction.
Examples of the above-mentioned rearrangement reaction include Curtius rearrangement, Hofmann rearrangement, Schmidt rearrangement and the like.
The rearrangement reaction using diphenylphosphoryl azide is exemplified below.
The amount of the diphenylphosphoryl azide to be used is generally 1 to 3 equivalents, preferably 1 to 1.5 equivalents, relative to compound (XXXIV).
Where necessary, the reaction may be carried out in the presence of a base.
Preferable examples of the base include aromatic amines, tertiary amines and the like.
This reaction is advantageously carried out in an inert solvent. While the solvent is not particularly limited as long as the reaction proceeds, for example, alcohol solvents are preferable.
The reaction time is generally about 10 min to about 48 hr, preferably about 15 min to about 24 hr.
The reaction temperature is generally -20°C to 200°C, preferably 0°C to 150°C.
The method described in "Jikken Kagaku Kouza (The Chemical Society of Japan ed.), 4th Edition, Vol. 20, pages 304 and 477-479, or a method analogous thereto, or the like is used as conditions of the other reaction.
Compound (XIIa) can be produced, for example, by reacting compound (XXXV) with an alkyl metals or an aryl metals, and reacting the obtained activated compound with a carbon dioxide.
The amount of the alkyl metal or aryl metal to be used is generally 1 to 2 equivalents, preferably 1 to 1.5 equivalents, relative to compound (XXXV).
The carbon dioxide is generally used in an excess amount.
This reaction is advantageously carried out in an inert solvent. While the solvent is not particularly limited as long as the

reaction proceeds, for example, aromatic solvents, aliphatic hydrocarbon solvents, ether solvents, a mixture thereof and the like are preferable.
The reaction time is generally 10 min to 48 hr, preferably 15 min to 24 hr.
The reaction temperature is generally -78°C to 100°C, preferably -78°C to 50°C.

Compound (XIIa) can also be produced by reacting compound (XXXV) with a transition metal catalyst A in an inert solvent under carbon monooxide atmosphere.
Examples of the transition metal catalyst A include palladium catalysts, nickel catalysts, iron catalysts, cobalt catalysts and the like. Examples of the palladium catalyst include dichlorobis(benzonitrile)palladium and the like. Examples of the nickel catalyst include tetracarbonylnickel (0) and the like. Examples of the iron catalyst include disodium tetracarbonylferrate and the like. Examples of the cobalt catalyst include dicobalt octacarbonyl and the like.
The amount of the transition metal catalyst A to be used is generally about 0.01 to 1 equivalents, preferably about 0.01 to 0.5 equivalents, relative to compound (XXXV).
Examples of the inert solvent include aromatic solvents, aliphatic hydrocarbon solvents, amide solvents, sulfoxide solvents and the like. These solvents may be used in a mixture of two or more kinds thereof at an appropriate ratio.
The reaction temperature is generally 0°C to 200°C, preferably 0°C to 150°C.
The reaction time is generally 1 hr to 48 hr, preferably 2 hr to 24 hr.

Compound (Ic) can be produced, for example, using compound (XIIa) and compound (V), according to the same method as in the amidation reaction of compound (V) and compound (XII) in the above-mentioned Reaction Scheme 1.

<Reaction Scheme 6>

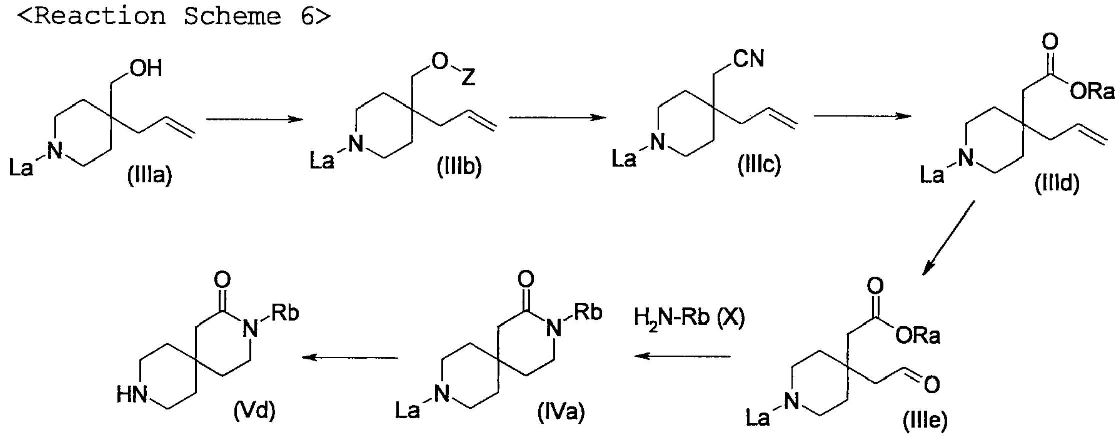


Compound (IIIb) can be produced, for example, by activating the hydroxyl group of compound (IIIa).
This reaction is carried out by reacting compound (IIIa) with a hydroxyl group-activator in the presence of a base, in an inert solvent.
Examples of the hydroxyl group-activator include methanesulfonyl chloride, p-toluenesulfonyl chloride and the like. The amount of the hydroxyl group-activator to be used is generally 1 to 10 equivalents, preferably 1 to 1.5 equivalents, relative to compound (IIIa).
Examples of the base include aromatic amines, tertiary amines and the like. The amount of the base to be used is generally 1 to 10 equivalents, preferably 1 to 1.5 equivalents, relative to compound (IIIa).
Examples of the inert solvent include aromatic solvents, aliphatic hydrocarbon solvents, ether solvents, ester solvents, amide solvents and the like. These solvents may be used in a mixture of two or more kinds thereof at an appropriate ratio.
The reaction temperature is generally -70 to 150°C, preferably -20 to 100°C.
The reaction time is generally 0.1 to 100 hr, preferably 0.1 to 40 hr.

Compound (IIIc) can be produced, for example, by subjecting compound (IIIb) to a cyanation reaction.
This reaction is carried out by reacting compound (IIIb) with a cyanating agent in an inert solvent.
Examples of the cyanating agent include sodium cyanide, potassium cyanide and the like.
Examples of the inert solvent include aromatic solvents, aliphatic hydrocarbon solvents, ether solvents, ester solvents, amide solvents, sulfoxide solvents, nitrile solvents and the like. These solvents may be used in a mixture of two or more kinds thereof at an appropriate ratio.
The reaction temperature is generally -70 to 150°C, preferably -20 to 100°C.
The reaction time is generally 0.1 to 100 hr, preferably 0.1 to 40 hr.

Compound (IIId) can be produced, for example, using compound (IIIc), according to the same method as in

the hydrolysis of compound (XXIX) in the above-mentioned Reaction Scheme 3.
When a solvent other than an alcohol solvent is used as an inert solvent, compound (IIId) can be produced according to a method known per se (e.g., esterification).

Compound (IIIe) can also be produced, for example, by subjecting compound (IIId) to an oxidization reaction. The oxidation reaction can be carried out according to a method known per se, for example, the method described in Heterocycles, pages 2263-2267, 1992 or the like or a method analogous thereto.
This reaction is carried out by reacting compound (IIId) with an oxidant in an inert solvent.
Examples of the oxidant include ozone, potassium permanganate, sodium periodate, osmium tetraoxide and the like. The amount of the oxidant to be used is generally 1 to 5 equivalents, preferably 1 to 1.5 equivalents, relative to compound (IIId).
Examples of the inert solvent include alcohol solvents, nitrile solvents, amide solvents, halogenated hydrocarbon solvents, ether solvents and the like. These solvents may be used in a mixture of two or more kinds thereof at an appropriate ratio. Of those, halogenated hydrocarbon solvents and the like are preferable.
The reaction temperature is generally -100°C to 50°C, preferably -78°C to 0°C.
The reaction time is generally 5 min to 48 hr, preferably 30 min to 24 hr.

Compound (IVa) can be produced, for example, by subjecting compound (IIIe) to a reductive amination reaction. The reductive amination reaction can be carried out according to a method known per se, for example, the method described in Tetrahedron Letters (Tetrahedron Lett.) pages 8345-8349, 2001 or the like or a method analogous thereto.
This reaction is carried out by reacting compound (IIIe) with compound (X) in the presence of a reducing agent, in an inert solvent. Where necessary, the reaction may be carried out in the presence of 1 to 50 equivalents of an organic acid.
The amount of compound (X) to be used is generally 1 to 5 equivalents, preferably 2 to 4 equivalents, relative to compound (IIIe).
Examples of the reducing agent include metal hydrogen compounds (e.g., sodium bis(2-methoxyethoxy)aluminum hydride, diisobutylaluminum hydride), metal hydrogen complex compounds (e.g., sodium borohydride, sodium cyanoborohydride, sodium triacetoxyborohydride, lithium aluminum hydride, sodium aluminum hydride) and the like. The amount of the reducing agent to be used is generally 0.1 to 20 equivalents, preferably 1 to 5 equivalents, relative to compound (IIIe).
Examples of the inert solvent include alcohol solvents, nitrile solvents, amide solvents, halogenated hydrocarbon solvents, ether solvents and the like. These solvents may be used in a mixture of two or more kinds thereof at an appropriate ratio. Of those, THF, dichloromethane and the like are preferable.
Examples of the organic acid include acetic acid and the like.
The reaction temperature is generally -78°C to 100°C, preferably 0°C to 50°C.
The reaction time is generally 5 min to 48 hr, preferably 30 min to 24 hr.

Compound (Vd) can be produced, for example, by subjecting compound (IVa) to deprotection.

<Reaction Scheme 7>

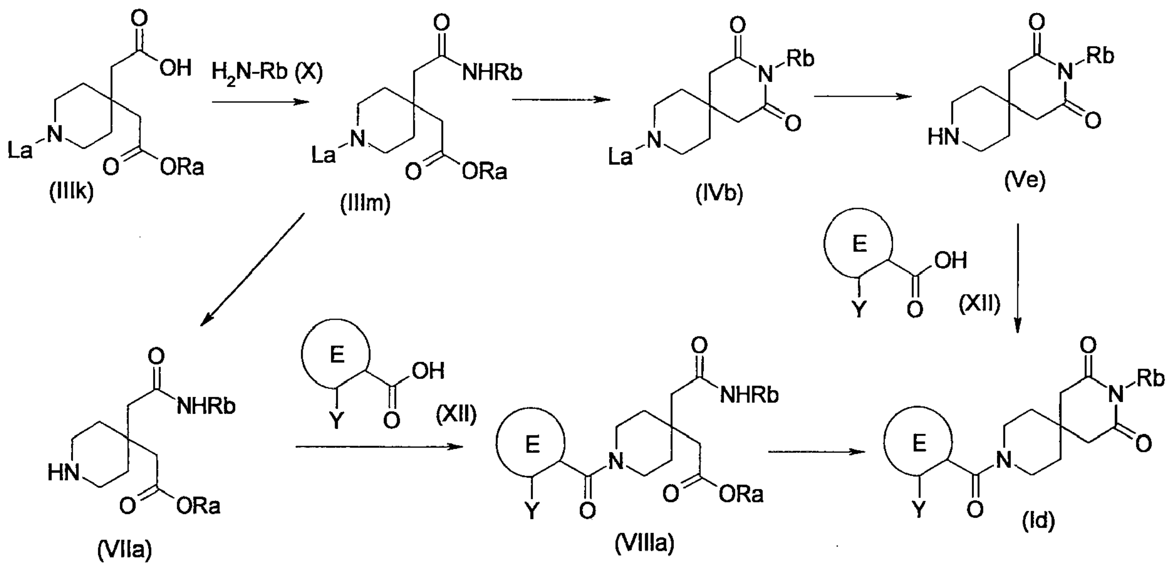


Compound (IIIm) can be produced, for example, using compound (IIIk) and compound (X), according to the

same method as in the amidation reaction of compound (V) and compound (XII) in the above-mentioned Reaction Scheme 1.

Compound (IVb) can be produced, for example, using compound (IIIm), according to the same method as in the production reaction of compound (IVf) from compound (IIIh) in the above-mentioned Reaction Scheme 2.

Compound (Ve) can be produced, for example, by subjecting compound (IVb) to deprotection.

Compound (VIIa) can be produced, for example, by subjecting compound (IIIm) to deprotection.

Compound (VIIIa) can be produced, for example, using compound (VIIa) and compound (XII), according to the same method as in the amidation reaction of compound (V) and compound (XII) in the above-mentioned Reaction Scheme 1.

Compound (Id) can be produced, for example, using compound (VIIIa), according to the same method as in the production reaction of compound (IVf) from compound (IIIh) in the above-mentioned Reaction Scheme 2. Compound (Id) can also be produced using compound (Ve) and compound (XII), according to the same method as in the amidation reaction of compound (V) and compound (XII) in the above-mentioned Reaction Scheme 1.

<Reaction Scheme 8>

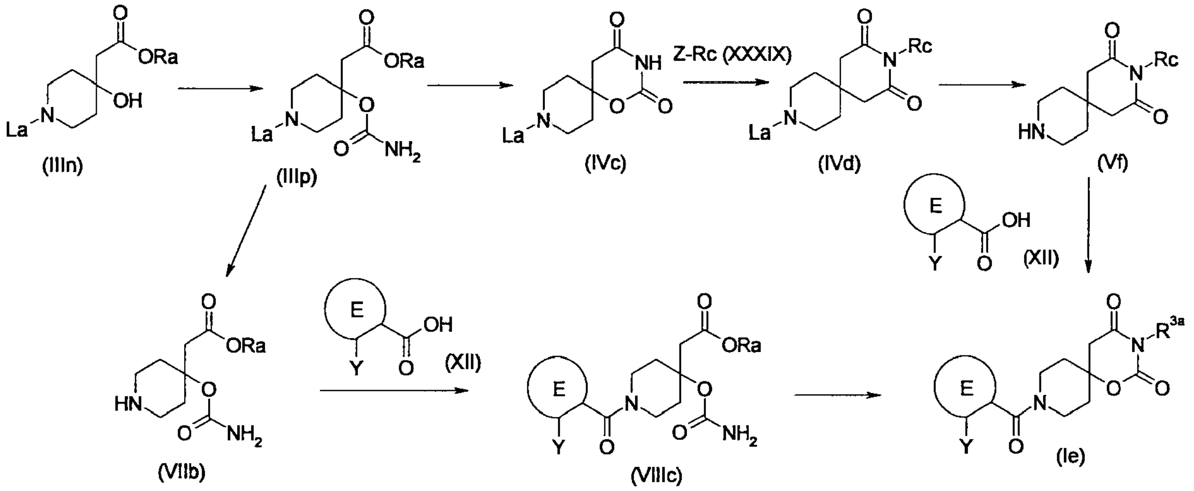


Compound (IIIp) can be produced, for example, by subjecting compound (IIIn) to a carbamoylation reaction. This reaction is carried out by reacting compound (IIIn) with trichloroacetyl isocyanate in an inert solvent, and then subjecting the resulting compound to decomposition with an ammonia solution and the like. The amount of the trichloroacetyl isocyanate to be used is generally 1 to 5 equivalents, preferably 1 to 2 equivalents, relative to compound (IIIn).
Alternatively, the reaction is carried out by reacting compound (IIIn) with trichloroethyl chlorocarbonate in the presence of a base, in an inert solvent, and then subjecting the activated compound to decomposition with an ammonia solution.
Examples of the base include hydrides of alkali metal or alkaline earth metal, and the like. The amount of the base to be used is generally 0.5 to 10 equivalents, preferably 0.5 to 1.5 equivalents, relative to compound (IIIn).
Examples of the inert solvent include nitrile solvents, amide solvents, halogenated hydrocarbon solvents, ether solvents and the like. These solvents may be used in a mixture of two or more kinds thereof at an appropriate ratio. Of those, THF is preferable.
The reaction temperature is generally -78°C to 50°C, preferably room temperature to 50°C.
The reaction time is generally 5 min to 48 hr, preferably 30 min to 24 hr.

Compound (IVc) can be produced, for example, using compound (IIIp), according to the same method as in the production reaction of compound (IVf) from compound (IIIh) in the above-mentioned Reaction Scheme 2.

Compound (IVd) can be produced, for example, using compound (IVc) and compound (XXXIX), according to the same method as in the alkylation reaction of compound (XXXVII) in the above-mentioned Reaction Scheme 4.

Compound (Vf) can be produced, for example, by subjecting compound (IVd) to deprotection.

Compound (VIIb) can be produced, for example, by subjecting compound (IIIp) to deprotection.

Compound (VIIIc) can be produced, for example, using compound (VIIb) and compound (XII), according to the same method as in the amidation reaction of compound (V) and compound (XII) in the above-mentioned Reaction Scheme 1.

Compound (Ie) can be produced, for example, using compound (VIIIc), according to the same method as in the production reaction of compound (IVf) from compound (IIIh) in the above-mentioned Reaction Scheme 2. Compound

(Ie) can also be produced using compound (Vf) and compound (XII), according to the same method as in the amidation reaction of compound (V) and compound (XII) in the above-mentioned Reaction Scheme 1.

In compound (I) thus obtained, a functional group within a molecule can also be converted to a desired functional group by a combination of chemical reactions known *per se*. Examples of the chemical reaction here include oxidation reaction, reduction reaction, alkylation reaction, acylation reaction, ureation reaction, hydrolysis reaction, amination reaction, esterification reaction, aryl coupling reaction, deprotection reaction and the like.

In the above-mentioned production methods, when the starting compound has an amino group, a carboxyl group, a hydroxy group, a carbonyl group or a mercapto group as a substituent, a protecting group generally used in peptide chemistry and the like may be introduced into these groups. By removing the protecting group as necessary after the reaction, the object compound can be obtained.

Examples of the amino-protecting group include a formyl group, a $C_{1-6}$ alkyl-carbonyl group, a $C_{1-6}$ alkoxy-carbonyl group, a benzoyl group, a $C_{7-10}$ aralkyl-carbonyl group (e.g., benzylcarbonyl), a $C_{7-14}$ aralkyloxy-carbonyl group (e.g., benzyloxycarbonyl, 9-fluorenylmethoxycarbonyl), a trityl group, a phthaloyl group, a N,N-dimethylaminomethylene group, a substituted silyl group (e.g., trimethylsilyl, triethylsilyl, dimethylphenylsilyl, tert-butyldimethylsilyl, tert-butyldiethylsilyl), a $C_{2-6}$ alkenyl group (e.g., 1-allyl) and the like. These groups are optionally substituted by 1 to 3 substituents selected from a halogen atom, a $C_{1-6}$ alkoxy group and a nitro group.

Examples of the carboxyl-protecting group include a $C_{1-6}$ alkyl group, a $C_{7-10}$ aralkyl group (e.g., benzyl), a phenyl group, a trityl group, a substituted silyl group (e.g., trimethylsilyl, triethylsilyl, dimethylphenylsilyl, tert-butyldimethylsilyl, tert-butyldiethylsilyl), a $C_{2-6}$ alkenyl group (e.g., 1-allyl) and the like. These groups are optionally substituted by 1 to 3 substituents selected from a halogen atom, a $C_{1-6}$ alkoxy group and a nitro group.

Examples of the hydroxy-protecting group include a $C_{1-6}$ alkyl group, a phenyl group, a trityl group, a $C_{7-10}$ aralkyl group (e.g., benzyl), a formyl group, a $C_{1-6}$ alkyl-carbonyl group, a benzoyl group, a $C_{7-10}$ aralkyl-carbonyl group (e.g., benzylcarbonyl), a 2-tetrahydropyranyl group, a 2-tetrahydrofuranyl group, a substituted silyl group (e.g., trimethylsilyl, triethylsilyl, dimethylphenylsilyl, tert-butyldimethylsilyl, tert-butyldiethylsilyl), a $C_{2-6}$ alkenyl group (e.g., 1-allyl) and the like. These groups are optionally substituted by 1 to 3 substituents selected from a halogen atom, a $C_{1-6}$ alkyl group, a $C_{1-6}$ alkoxy group and a nitro group.

Examples of the carbonyl-protecting group include a cyclic acetal (e.g., 1,3-dioxane), a non-cyclic acetal (e.g., a di-$C_{1-6}$ alkylacetal) and the like.

Examples of the mercapto-protecting group include a $C_{1-6}$ alkyl group, a phenyl group, a trityl group, a $C_{7-10}$ aralkyl group (e.g., benzyl), a $C_{1-6}$ alkyl-carbonyl group, a benzoyl group, a $C_{7-10}$ aralkyl-carbonyl group (e.g., benzylcarbonyl), a $C_{1-6}$ alkoxy-carbonyl group, a $C_{6-14}$ aryloxy-carbonyl group (e.g., phenyloxycarbonyl), a $C_{7-14}$ aralkyloxy-carbonyl group (e.g., benzyloxycarbonyl, 9-fluorenylmethoxycarbonyl), a 2-tetrahydropyranyl group, a $C_{1-6}$ alkylamino-carbonyl group (e.g., methylaminocarbonyl, ethylaminocarbonyl), and the like. These groups are optionally substituted by 1 to 3 substituents selected from a halogen atom, a $C_{1-6}$ alkyl group, a $C_{1-6}$ alkoxy group and a nitro group.

The above-mentioned protecting group can be removed according to deprotection known per se.

Compound (I) obtained by the above-mentioned production methods can be isolated and purified by a known means, for example, solvent extraction, liquid conversion, phase transfer, crystallization, recrystallization, chromatography and the like.

When compound (I) contains an optical isomer, a stereoisomer, a regioisomer or a rotamer, these are also encompassed in compound (I), and can be obtained as a single product according to synthesis and separation methods known *per se*. For example, when compound (I) has an optical isomer, an optical isomer resolved from this compound is also encompassed in compound (I).
The optical isomer can be produced by a method known *per se*.

Compound (I) may be a crystal.
Crystals of compound (I) (hereinafter sometimes to be abbreviated as the crystals of the present invention) can be produced by crystallization according to crystallization methods known *per se*.
In the present specification, the melting point means that measured using, for example, a micromelting point apparatus (Yanako, MP-500D or Buchi, B-545), a DSC (differential scanning calorimetry) device (SEIKO, EXSTAR6000) or the like. In general, the melting points vary depending on the measurement apparatuses, the measurement conditions and the like. The crystal in the present specification may show different values from the melting point described in the present specification, as long as they are within each of a general error range.
The crystal of the present invention is superior in physicochemical properties (e.g., melting point, solubility, stability) and biological properties (e.g., pharmacokinetics (absorption, distribution, metabolism, excretion), efficacy expression), and thus it is extremely useful as a medicament.

## Examples

The present invention is explained in detail in the following by referring to Reference Examples, Examples,

Experimental Examples and Preparation Examples, which are not to be construed as limitative. In addition, the present invention may be modified without departing from the scope of the invention.

Reference Example 1

ethyl 3-aminothieno[2,3-b]pyridine-2-carboxylate

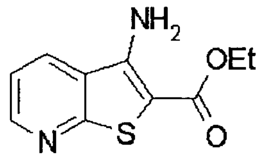

A suspension of 2-chloronicotinonitrile (25.7 g, 185 mmol), ethyl mercaptoacetate (27.0 g, 225 mmol) and sodium carbonate (19.9 g, 187 mmol) in ethanol (70 mL) was heated under reflux for 3 hr. The reaction mixture was cooled to room temperature, water was added, and the resulting solid was collected by filtration. This was suspended in methanol, collected by filtration, and dried to give the title compound (32.3 g, yield 83%).
$^1H$ NMR ($CDCl_3$) δ1.40 (3H, t, J = 7.2 Hz), 4.38 (2H, q, J = 7.2 Hz), 5.92 (2H, br s), 7.31 (1H, dd, J = 4.7 Hz, 8.0 Hz), 7.93 (1H, dd, J = 1.7 Hz, 8.0 Hz), 8.69 (1H, dd, J = 1.7 Hz, 4.7 Hz).

Reference Example 2

ethyl 3-bromothieno[2,3-b]pyridine-2-carboxylate

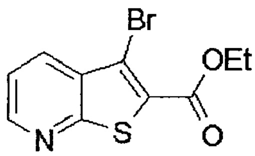

To a solution of copper bromide (36.1 g, 162 mmol) and tert-butyl nitrite (19.8 g, 192 mmol) in acetonitrile (360 mL) was added ethyl 3-aminothieno[2,3-b]pyridine-2-carboxylate (33.2 g, 148 mmol) obtained in Reference Example 1 at room temperature over 2 hr, and the mixture was stirred at room temperature for 30 min. 1N Hydrochloric acid (500 mL) was added to the reaction mixture, and the mixture was stirred for 5 min. The resulting solid was collected by filtration. This was dissolved in THF, and the solution was dried over anhydrous magnesium sulfate and passed through a basic silica gel. The eluate was concentrated under reduced pressure, and the obtained solid was suspended in hexane. The solid was collected by filtration, and dried to give the title compound (30.6 g, yield 72%).
$^1H$ NMR ($CDCl_3$) δ1.45 (3H, t, J = 7.2 Hz), 4.46 (2H, q, J = 7.2 Hz), 7.46 (1H, dd, J = 4.5 Hz, 8.3 Hz), 8.23 (1H, dd, J = 1.7 Hz, 8.3 Hz), 8.73 (1H, dd, J = 1.7 Hz, 4.5 Hz).

Reference Example 3

3-bromothieno[2,3-b]pyridine-2-carboxylic acid

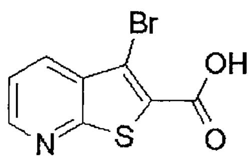

A mixture of ethyl 3-bromothieno[2,3-b]pyridine-2-carboxylate (5.00 g, 17.5 mmol) obtained in Reference Example 2, 2N aqueous sodium hydroxide solution (17.5 mL, 35.0 mmol) and ethanol (15 mL) was stirred at room

temperature for 1 hr. 1N Hydrochloric acid (35.0 mL) was added to the reaction mixture, and the resulting solid was collected by filtration. This was suspended in water and collected by filtration, the obtained solid was suspended in 2-propanol and collected by filtration, and the obtained solid was suspended in diisopropyl ether and collected by filtration to give the title compound (4.20 g, yield 93%).
$^1H$ NMR (DMSO-$d_6$) δ7.66 (1H, dd, J = 4.5 Hz, 8.1 Hz), 8.32 (1H, dd, J = 1.5 Hz, 8.1 Hz), 8.81 (1H, dd, J = 1.5 Hz, 4.5 Hz), 14.19 (1H, s).

Reference Example 4

tert-butyl (3-bromothieno[2,3-b]pyridin-2-yl)carbamate
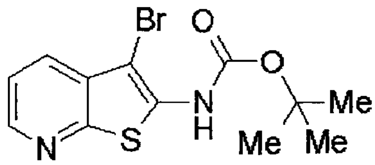

A solution of 3-bromothieno[2,3-b]pyridine-2-carboxylic acid (4.50 g, 17.4 mmol) obtained in Reference Example 3, diphenylphosphoryl azide (4.13 mL, 19.2 mmol) and diisopropylethylamine (4.56 mL, 26.2 mmol) in tert-butyl alcohol (90 mL) was stirred at room temperature for 15 min, and further stirred at 90°C for 16 hr. The solvent was evaporated under reduced pressure, water was added to the residue, and the mixture was extracted with ethyl acetate. The extract was dried over anhydrous magnesium sulfate, and passed through a basic silica gel. The obtained eluate was concentrated under reduced pressure, and the residue was purified by silica gel column chromatography (hexane:ethyl acetate=4:1 to 7:3) to give the title compound (4.58 g, yield 80%) as a powder.
$^1H$ NMR (DMSO-$d_6$) δ1.52 (9H, s), 7.47 (1H, dd, J = 4.8 Hz, 8.1 Hz), 7.90 (1H, dd, J = 1.5 Hz, 8.1 Hz), 8.47 (1H, dd, J = 1.5 Hz, 4.8 Hz), 10.28 (1H, s).

Reference Example 5

2-[(tert-butoxycarbonyl)amino]thieno[2,3-b]pyridine-3-carboxylic acid
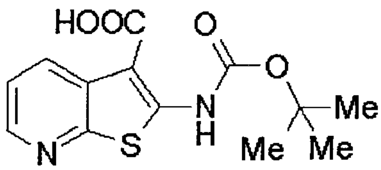

A solution of tert-butyl (3-bromothieno[2,3-b]pyridin-2-yl)carbamate (1.12 g, 3.40 mmol) obtained in Reference Example 4 in THF (11 mL) was cooled to -78°C, 1.6 M n-butyllithium hexane solution (4.7 mL, 7.48 mmol) was added dropwise, and the mixture was stirred at the same temperature for 1 hr. Dry carbon dioxide was blown into the obtained suspension until the suspended solid was disappeared. 1N Hydrochloric acid (7.5 mL) was added to the reaction mixture and the resulting solid was collected by filtration. The obtained solid was suspended in water and collected by filtration, the obtained solid was suspended in acetonitrile and collected by filtration, and the obtained solid was suspended in diethyl ether and collected by filtration to give the title compound (815 mg, yield 82%).
melting point 199-201°C
EI(pos) 295 $[M+]^+$
$^1H$ NMR (DMSO-$d_6$) δ1.54 (9H, s), 7.46 (1H, dd, J = 4.9 Hz, 8.1 Hz), 8.41-8.49 (2H, m), 10.96 (1H, s), 13.86 (1H, br s).

Reference Example 6

ethyl 3-amino-6-methylthieno[2,3-b]pyridine-2-carboxylate
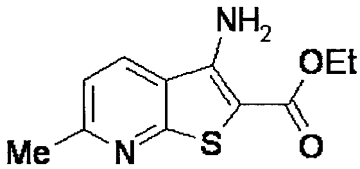

To a solution of 2-chloro-6-methylnicotinonitrile (44.0 g, 0.289 mol) and ethyl thioglycolate (35.0 mL, 0.318 mol) in DMF (500 mL) was added sodium ethoxide (21.7 g, 0.318 mol), and the mixture was stirred at room temperature for 30 min. Further, sodium ethoxide (5.00 g, 73.5 mmol) was added and the mixture was stirred at room temperature for 30 min. Water was added to the reaction mixture and the precipitated solid was collected by filtration, washed with water, and dried to give the title compound (66.4 g, yield 97%) as a yellow solid.
$^1$H NMR ($CDCl_3$) δ1.39 (3H, t, J = 7.2 Hz), 2.68 (3H, s), 4.36 (2H, q, J = 7.2 Hz), 5.89 (2H, br), 7.16 (1H, d, J = 8.3 Hz), 7.81 (1H, d, J = 8.3 Hz).

Reference Example 7

ethyl 3-bromo-6-methylthieno[2,3-b]pyridine-2-carboxylate
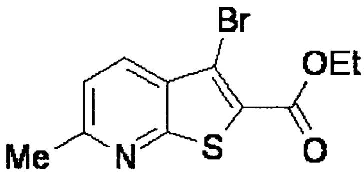

Ethyl 3-amino-6-methylthieno[2,3-b]pyridine-2-carboxylate (36.1 g, 0.153 mol) obtained in Reference Example 6 was added to a mixture of copper(II) bromide (37.5 g, 0.168 mol) and tert-butyl nitrite (23.6 mL, 0.199 mol) in acetonitrile (350 mL) under water-cooling over 2 hr, and the mixture was stirred for 1 hr. 1N Hydrochloric acid (700 mL) was slowly added to the reaction mixture, and the resulting precipitate was collected by filtration and washed with water. The solid was dissolved in THF, and the solution was diluted with ethyl acetate. This solution was washed with 1N hydrochloric acid and saturated brine, and dried over anhydrous sodium sulfate (solution A). The earlier filtrate was extracted with ethyl acetate, and the extract was washed with saturated brine, and dried over anhydrous sodium sulfate. The residue was subjected to basic silica gel column chromatography (ethyl acetate) to give a crude product (about 5 g). This was combined with solution A and the mixture was subjected to basic silica gel column chromatography (ethyl acetate) again and crystallized from hexane to give the title compound (30.5 g, yield 66%) as a pale-yellow solid.
$^1$H NMR ($CDCl_3$) δ1.35 (3H, t, J = 7.1 Hz), 2.67 (3H, s), 4.39 (2H, q, J = 7.1 Hz), 7.54 (1H, d, J = 8.3 Hz), 8.20 (1H, d, J = 8.3 Hz).

Reference Example 8

3-bromo-6-methylthieno[2,3-b]pyridine-2-carboxylic acid
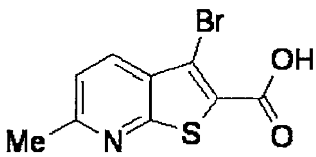

To a solution of ethyl 3-bromo-6-methylthieno[2,3-b]pyridine-2-carboxylate (79.2 g, 0.264 mol) obtained in Reference Example 7 in ethanol (250 mL) was added 2N aqueous sodium hydroxide solution (264 mL, 0.527 mol), and the mixture was stirred at room temperature for 16 hr. The reaction mixture was diluted with water (1 L) and adjusted to pH 5 - 6 with 1N hydrochloric acid (530 mL). The precipitated solid was collected, and washed with water. The obtained solid was suspended in acetone, collected by filtration, and washed successively with acetone and diisopropyl ether to

give the title compound (68.5 g, yield 95%).
$^1$H NMR ($CDCl_3$) δ2.66 (3H, s), 7.52 (1H, d, J = 8.5 Hz), 8.18 (1H, d, J = 8.3 Hz), 14.06 (1H, br s).

Reference Example 9

tert-butyl (3-bromo-6-methylthieno[2,3-b]pyridin-2-yl)carbamate

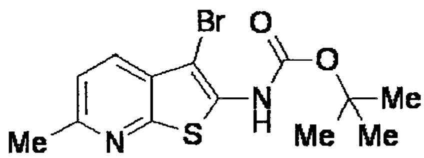

A solution of 3-bromo-6-methylthieno[2,3-b]pyridine-2-carboxylic acid (15.0 g, 55.2 mmol) obtained in Reference Example 8, diphenylphosphoryl azide (13.1 mL, 60.6 mmol) and triethylamine (11.6 mL, 82.8 mmol) in tert-butanol (100 mL) was heated to 90°C for 15 hr. The reaction mixture was diluted with ethyl acetate. This solution was washed successively with saturated aqueous sodium hydrogen carbonate solution and saturated brine, and dried over anhydrous magnesium sulfate. The solvent was evaporated under reduced pressure, and the obtained residue was purified by silica gel column chromatography (ethyl acetate:hexane=1:19 to 3:7) to give the title compound (16.3 g, yield 86%) as a white solid.
1H NMR (DMSO-d6) δ1.51 (9H, s), 2.58 (3H, s), 7.32 (1H, d, J = 8.4 Hz), 7.78 (1H, d, J = 8.4 Hz), 10.12 (1H, s).

Reference Example 10

2-[(tert-butoxycarbonyl)amino]-6-methylthieno[2,3-b]pyridine-3-carboxylic acid

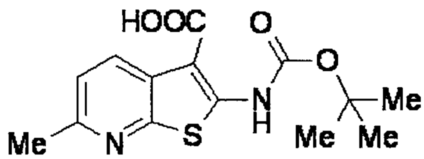

To a solution of tert-butyl (3-bromo-6-methylthieno[2,3-b]pyridin-2-yl)carbamate (9.07 g, 26.4 mmol) obtained in Reference Example 9 in dry THF (88 mL) was added dropwise 1.6 M n-butyllithium hexane solution (38 mL, 60.7 mmol) under a nitrogen atmosphere at -78°C, and the mixture was stirred at the same temperature for 1 hr. Dry carbon dioxide gas vaporized from dry ice was bubbled in at 0-10°C. The reaction mixture was diluted with water and ethyl acetate, 1N hydrochloric acid was added and the precipitated solid was collected by filtration. The obtained solid was suspended in water and collecting by filtration, and the obtained solid was suspended in acetonitrile/diethyl ether (1:1) and collecting by filtration to give the title compound (6.51 g, yield 80%) as white crystals.
melting point 162-163°C
EI(pos) 309 $[M+H]^+$
$^1$H NMR (DMSO-$d_6$) δ1.53 (9H, s), 2.50 (3H, s), 7.29 (1H, d, J = 8.3 Hz), 8.37 (1H, d, J = 8.3 Hz), 11.26 (1H, br s).

Reference Example 11

2-amino-6-methylthieno[2,3-b]pyridine-3-carboxylic acid trifluoroacetate

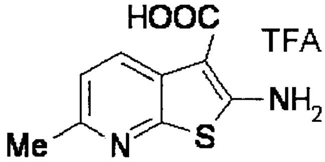

Trifluoroacetic acid was added to 2-[(tert-butoxycarbonyl)amino]-6-methylthieno[2,3-b]pyridine-3-carboxylic acid (10.2 g, 33.1 mmol) obtained in Reference Example 10, and the mixture was stirred for 1 hr and concentrated under reduced pressure. Ethyl acetate was added to the residue, and the resulting crystals were collected to give the title compound (6.76 g, yield 63%).
$^1$H NMR (DMSO-$d_6$) δ2.46 (3H, s), 7.14 (1H, d, J = 8.1 Hz), 8.07 (1H, d, J = 8.1 Hz).
Calculated:C; 41.00, H; 2.81, N; 8.69
Found:C; 41.29, H; 2.82, N; 8.75.

Reference Example 12

2-chloro-3-cyano-5,6-dimethylpyridine
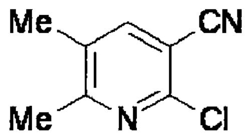

To a mixture of 28% sodium methoxide methanol solution (59 mL) and diethyl ether (380 mL) was added dropwise a mixture of 2-butanone (20.9 g, 290 mmol) and ethyl formate (23.0 g, 310 mmol) over 45 min while maintaining an inside temperature of 4-5°C. The mixture was stirred at room temperature for 6 hr, and the resulting precipitate was collected by filtration. A solution of the solid, 2-cyanoacetamide (14.1 g, 168 mmol), piperidine (12.3 mL, 124 mmol) and acetic acid (7.50 g, 124 mmol) in water (336 mL) was heated under reflux for 17 hr. Thereafter, acetic acid (26 mL) was added dropwise while maintaining an inside temperature of 65°C, and the mixture was cooled to room temperature, and the resulting precipitate was collected by filtration. The collected precipitate was suspended in a mixed solvent of acetonitrile and diisopropyl ether, collected by filtration, and dried under reduced pressure. The solid was added to phosphorus oxychloride (80 mL), and the mixture was stirred at 100°C for 6 hr. The reaction mixture was added to ice water, ethyl acetate was added, and the mixture was neutralized with potassium carbonate. The aqueous layer was extracted with ethyl acetate. The combined organic layers were washed with saturated brine, and dried over magnesium sulfate. The solvent was evaporated under reduced pressure, and the residue was washed with diethyl ether and dried to give the title compound (16.1 g, yield 33%).
$^1$H NMR (DMSO-$d_6$) δ2.28 (3H, s), 2.50 (3H, s), 8.22 (1H, s).

Reference Example 13

ethyl 3-amino-5,6-dimethylthieno[2,3-b]pyridine-2-carboxylate
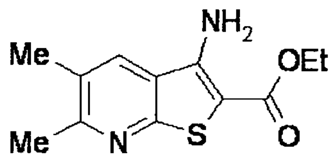

The title compound was obtained in the same manner as in Reference Example 1 and from 2-chloro-3-cyano-5,6-dimethylpyridine obtained in Reference Example 12. yield 83%. $^1$H NMR (DMSO-$d_6$) δ1.29 (3H, t, J = 7.2 Hz), 2.34 (3H, s), 2.53 (3H, s), 4.25 (2H, q, J = 7.2 Hz), 7.17 (2H, br), 8.24 (1H, s).

Reference Example 14

ethyl 3-bromo-5,6-dimethylthieno[2,3-b]pyridine-2-carboxylate
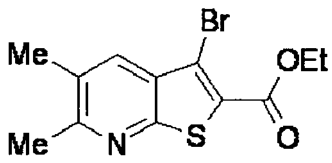

The title compound was obtained in the same manner as in Reference Example 2 and from ethyl 3-amino-5,6-dimethylthieno[2,3-b]pyridine-2-carboxylate obtained in Reference Example 13. yield 48%.
$^1$H NMR (DMSO-$d_6$) δ1.35 (3H, t, J = 7.0 Hz), 2.44 (3H, s), 2.61 (3H, s), 4.38 (2H, q, J = 7.0 Hz), 8.03 (1H, s).

Reference Example 15

3-bromo-5,6-dimethylthieno[2,3-b]pyridine-2-carboxylic acid
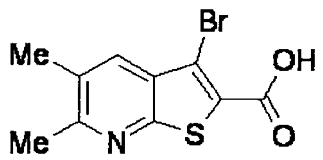

The title compound was obtained in the same manner as in Reference Example 3 and from ethyl 3-bromo-5,6-dimethylthieno[2,3-b]pyridine-2-carboxylate obtained in Reference Example 14. yield 99%.
$^1$H NMR (DMSO-$d_6$) δ2.42 (3H, s), 2.57 (3H, s), 7.95 (1H, s), 14.08 (1H, br).

Reference Example 16

tert-butyl (3-bromo-5,6-dimethylthieno[2,3-b]pyridin-2-yl)carbamate
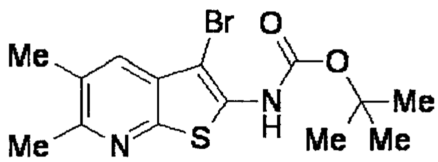

The title compound was obtained in the same manner as in Reference Example 4 and from 3-bromo-5,6-dimethylthieno[2,3-b]pyridine-2-carboxylic acid obtained in Reference Example 15. yield 47%.
$^1$H NMR ($CDCl_3$) δ1.57 (9H, s), 2.39 (3H, s), 2.59 (3H, s), 7.54 (1H, s).

Reference Example 17

2-[(tert-butoxycarbonyl)amino]-5,6-dimethylthieno[2,3-b]pyridine-3-carboxylic acid
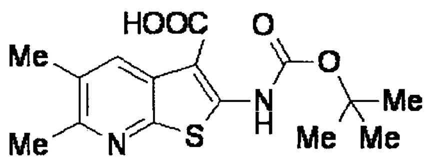

The title compound was obtained in the same manner as in Reference Example 5 and from tert-butyl (3-bromo-5,6-dimethylthieno[2,3-b]pyridin-2-yl)carbamate obtained in Reference Example 16. yield 80%.
$^1$H NMR (DMSO-$d_6$) δ1.57 (9H, s), 2.34 (3H, s), 2.50 (3H, s), 8.20 (1H, s), 11.10 (1H, br), 13.80 (1H, br).

Reference Example 18

tert-butyl 6-nitro-4-oxo-3,4-dihydro-1'H-spiro[chromene-2,4'-piperidine]-1'-carboxylate

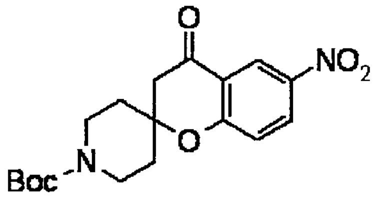

A solution of 1-(2-hydroxy-5-nitrophenyl)ethanone (5.00 g, 27.6 mmol), tert-butyl 4-oxopiperidine-1-carboxylate (5.77 g, 28.9 mmol) and pyrrolidine (1.96 g, 27.6 mmol) in methanol (70 mL) was stirred at 80°C for 16 hr, and the reaction mixture was concentrated under reduced pressure. The obtained residue was dissolved in ethyl acetate, and the solution was washed with saturated brine, and dried over anhydrous magnesium sulfate. The solution was subjected to basic silica gel column chromatography. The solvent was evaporated under reduced pressure. The obtained residue was dissolved in ethyl acetate, and the solution was left standing at room temperature. The resulting crystals were collected by filtration to give the title compound (5.17 g, yield 52%) as a powder.

EI(pos) 307.0 $[M+H]^+$

$^1$H NMR (DMSO-$d_6$) δ1.47 (9H, s), 1.68 (2H, m), 2.11 (2H, m), 2.81 (2H, s), 3.23 (2H, m), 3.95 (2H, m), 7.12 (1H, d, J = 9.09 Hz), 8.36 (1H, dd, J = 9.09, 3.03 Hz), 8.76 (1H, d, J = 3.03 Hz).

Reference Example 19

6-nitrospiro[chromene-2,4'-piperidin]-4(3H)-one hydrochloride

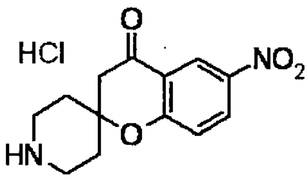

To tert-butyl 6-nitro-4-oxo-3,4-dihydro-1'H-spiro[chromene-2,4'-piperidine]-1'-carboxylate (1.50 g, 4.14 mmol) obtained in Reference Example 18 was added 4N hydrogen chloride-ethyl acetate (10 mL), and the resulting solid was collected by filtration 30 min later to give the title compound (1.10 g, yield 89%) as a powder. The compound was used for the next step without purification.

Reference Example 20

6-benzyl 1'-tert-butyl 4-oxo-3,4-dihydro-1'H-spiro[chromene-2,4'-piperidine]-1',6-dicarboxylate

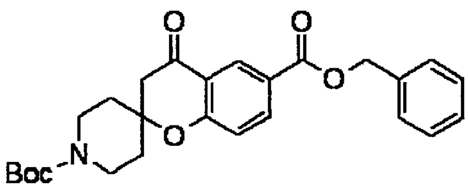

A solution of tert-butyl 6-bromo-4-oxo-3,4-dihydro-1'H spiro[chromene-2,4'-piperidine]-1'-carboxylate (1.00 mg, 2.52 mmol), benzyl alcohol (3.0 mL, 25.2 mmol) and palladium acetate (28.4 mg, 0.126 mmol) in DMF (3 mL) was stirred at 120°C under a carbon monoxide atmosphere for 16 hr. After completion of the reaction, the reaction mixture was

diluted with ethyl acetate, and the mixture was washed with saturated brine, and dried over anhydrous magnesium sulfate. The solvent was evaporated under reduced pressure, and the obtained residue was purified by silica gel column chromatography (ethyl acetate:hexane=0:1 to 3:7) to give the title compound (469 g, yield 41%) as a powder.
EI(pos) 352.1 $[M+H]^+$
$^1$H NMR (DMSO-$d_6$) δ1.46 (9H, s), 1.54-1.68 (2H, m), 1.98-2.05 (2H, m), 2,75 (2H, s), 3.21-3.60 (2H, m), 3.80-3.96 (2H, m), 5.35 (2H, s), 7.04 (1H, d, J = 8.71 Hz), 7.26-7.47 (5H, m), 8.19 (1H, dd, J = 8.71, 2.27 Hz), 8.59 (1H, d, J = 2.27 Hz).

Reference Example 21

benzyl 4-oxo-3,4-dihydrospiro[chromene-2,4'-piperidine]-6-carboxylate hydrochloride
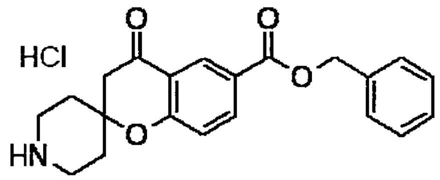

The title compound (324 mg, yield 81%) was obtained as a powder in the same manner as in Reference Example 19 and from 6-benzyl 1'-tert-butyl 4-oxo-3,4-dihydro-1'H-spiro[chromene-2,4'-piperidine]-1',6-dicarboxylate (469 mg, 1.03 mmol) obtained in Reference Example 20 and 4N hydrogen chloride-ethyl acetate (2.5 mL). The compound was used for the next step without purification.

Reference Example 22

tert-butyl 7-hydroxy-4-oxo-3,4-dihydro-1'H-spiro[chromene-2,4'-piperidine]-1'-carboxylate
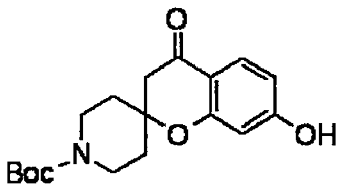

The title compound (14.98 g, yield 90%) was obtained as a powder in the same manner as in Reference Example 18 and from 1-(2,4-dihydroxyphenyl)ethanone (10.0 g, 50.2 mmol) and tert-butyl 4-oxopiperidine-1-carboxylate (7.64 g, 50.2 mmol).
$^1$H NMR (DMSO-$d_6$) δ1.48 (9H, s), 1.55-1.65 (4H, m), 2.66 (2H, s), 3.12-3.31 (2H, m), 3.82-3.96 (2H, m), 6.36 (1H, d, J = 2.3 Hz), 6.52 (1H, dd, J = 8.7, 2.3 Hz), 7.46-7.54 (1H, m), 7.78 (1H, d, J = 8.7 Hz).

Reference Example 23

tert-butyl 4-oxo-7-{[(trifluoromethyl)sulfonyl]oxy}-3,4-dihydro-1'H-spiro[chromene-2,4'-piperidine]-1'-carboxylate
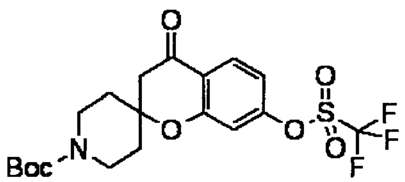

To a solution of tert-butyl 7-hydroxy-4-oxo-3,4-dihydro-1'H-spiro[chromene-2,4'-piperidine]-1'-carboxylate (2.00 g, 6.00 mmol) obtained in Reference Example 22 and triethylamine (1.68 mL, 12.0 mmol) in THF (20 mL) was added 1,1,1-trifluoro-N-phenyl-N-[(trifluoromethyl)sulfonyl]methanesulfonamide (2.58 g, 7.20 mmol), and the mixture was stirred with heating at room temperature for 1.5 hr. After completion of the reaction, the reaction mixture was diluted with ethyl acetate, and the mixture was washed with saturated brine, and dried over anhydrous magnesium sulfate. The solvent was evaporated under reduced pressure, and the obtained residue was purified by silica gel column chromatography (ethyl acetate:hexane=3:17 to 1:1) to give the title compound (2.79 g, quantitative) as an oil.
EI(pos) 409 $[M-tBu]^+$

Reference Example 24

tert-butyl 7-cyano-4-oxo-3,4-dihydro-1'H-spiro[chromene-2,4' piperidine]-1'-carboxylate
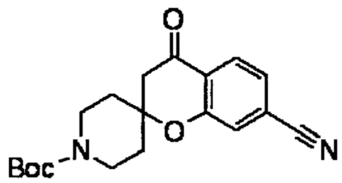

To a solution of tert-butyl 4-oxo-7-{[(trifluoromethyl)sulfonyl]oxy}-3,4-dihydro-1'H-spiro[chromene-2,4'-piperidine]-1'-carboxylate (2.79 g, 6.00 mmol) obtained in Reference Example 23 and zinc cyanide (2.12 g, 18.0 mmol) in DMF (20 mL) was added tetrakistriphenylphosphine palladium (694 mg, 0.600 mmol), and the mixture was stirred under a nitrogen atmosphere by heating at 100°C for 15 hr. After completion of the reaction, the reaction mixture was diluted with ethyl acetate, and the mixture was washed with saturated brine, and dried over anhydrous magnesium sulfate. The solvent was evaporated under reduced pressure, and the obtained residue was purified by silica gel column chromatography (ethyl acetate:hexane=1:17 to 1:1) to give the title compound (2.00 g, yield 97%) as an oil. $^1$H NMR (DMSO-$d_6$) δ1.46 (9H, s), 1.59-1.69 (2H, m), 1.98-2.03 (2H, m), 2.78 (2H, s), 3.17-3.25 (2H, m), 3.81-3.97 (2H, m), 7.26-7.29 (1H, m), 7.33 (1H, d, J = 1.1 Hz), 7.95 (1H, d, J = 8.0 Hz).

Reference Example 25

4-oxo-3,4-dihydrospiro[chromene-2,4'-piperidine]-7-carbonitrile hydrochloride
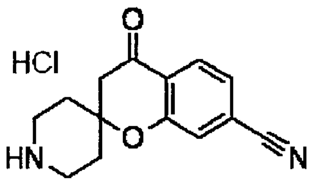

The title compound (706 mg, yield 87%) was obtained as a powder in the same manner as in Reference Example 19 and from tert-butyl 7-cyano-4-oxo-3,4-dihydro-1'H-spiro[chromene-2,4'-piperidine]-1'-carboxylate (1.00 g, 2.92 mmol) obtained in Reference Example 24 and 4N hydrogen chloride-ethyl acetate (10 mL). The compound was used for the next step without purification.

Reference Example 26

tert-butyl 7-(benzyloxy)-4-oxo-3,4-dihydro-1'H-spiro[chromene-2,4'-piperidine]-1'-carboxylate
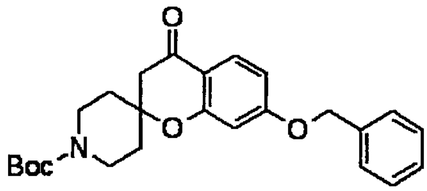

A solution of tert-butyl 7-hydroxy-4-oxo-3,4-dihydro-1'H-spiro[chromene-2,4'-piperidine]-1'-carboxylate (2.00 g, 6.00 mmol) obtained in Reference Example 22, benzyl bromide (0.856 mL, 7.20 mmol) and potassium carbonate (1.58 g, 12.0 mmol) in DMF (20 mL) was stirred at room temperature for 0.5 hr. After completion of the reaction, the reaction mixture was diluted with ethyl acetate, and the mixture was washed with saturated brine, and dried over anhydrous magnesium sulfate. The solvent was evaporated under reduced pressure, and the obtained residue was purified by silica gel column chromatography (ethyl acetate:hexane=3:17 to 3:2) to give the title compound (2.37 g, yield 93%) as a powder.
EI(pos) 424 $[M+H]^+$

Reference Example 27

7-(benzyloxy)spiro[chromene-2,4'-piperidin]-4(3H)-one hydrochloride
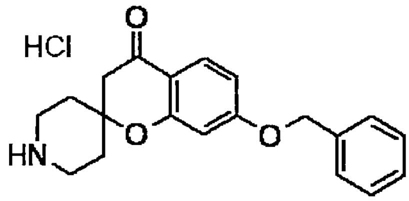

The title compound (1.74 g, yield 86%) was obtained as a powder in the same manner as in Reference Example 19 and from tert-butyl 7-(benzyloxy)-4-oxo-3,4-dihydro-1'H-spiro[chromene-2,4'-piperidine]-1'-carboxylate (2.37 g, 5.60 mmol) obtained in Reference Example 26 and 4N hydrogen chloride-ethyl acetate (20 mL). The compound was used for the next step without purification.

Reference Example 28

tert-butyl 7-(1-methylethenyl)-4-oxo-3,4-dihydro-1'H-spiro[chromene-2,4'-piperidine]-1'-carboxylate
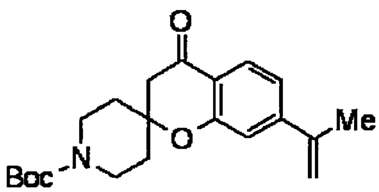

To a solution of tert-butyl 4-oxo-7-{[(trifluoromethyl)sulfonyl]oxy}-3,4-dihydro-1'H-spiro[chromene-2,4'-piperidine]-1'-carboxylate (2.14 g, 6.00 mmol) obtained in Reference Example 23 and 4,4,5,5-tetramethyl-2-(1-methylethenyl)-1,3,2-dioxaborolane (2.26 mL, 12.0 mmol) in a mixed solvent of THF (20 mL)-2M aqueous sodium carbonate solution (6 mL) was added tetrakistriphenylphosphine palladium (347 mg, 0.300 mmol) and the mixture was stirred with heating under a nitrogen atmosphere at 80°C for 15 hr. After completion of the reaction, the reaction mixture was diluted with ethyl acetate, and the mixture was washed with saturated brine, and dried over anhydrous magnesium sulfate. The solvent was evaporated under reduced pressure, and the obtained residue was purified by silica gel column chromatography (ethyl acetate:hexane=1:9 to 1:1) to give the title compound (2.02 g, yield 94%) as an oil.
$^1$H NMR ($CDCl_3$) δ1.46 (9H, s), 1.56-1.66 (2H, m), 1.97-2.08 (2H, m), 2.14 (3H, s), 2.71 (2H, s), 3.20-3.28 (2H, m),

3.81-3.95 (2H, m), 5.22 (1H, m), 5.49 (1H, s), 7.04 (1H, d, J = 1.5 Hz), 7.15 (1H, dd, J = 8.3, 1.7 Hz), 7.81 (1H, d, J = 8.3 Hz).

Reference Example 29

tert-butyl 7-acetyl-4-oxo-3,4-dihydro-1'H-spiro[chromene-2,4'-piperidine]-1'-carboxylate

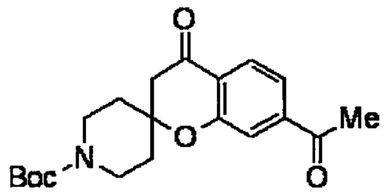

Ozone gas was passed through a solution of tert-butyl 7-(1-methylethenyl)-4-oxo-3,4-dihydro-1'H-spiro [chromene-2,4'-piperidine]-1'-carboxylate (2.02 g, 5.65 mmol) obtained in Reference Example 28 in a mixed solvent of dichloromethane (20 mL) and methanol (10 mL) at -78°C for 10 min. Dimethyl sulfide (1.25 mL) was added, and the mixture was warmed to room temperature and stirred at room temperature for 10 min. The solvent was evaporated under reduced pressure and the obtained residue was purified by silica gel column chromatography (ethyl acetate: hexane=3:17 to 1:1) to give the title compound (2.03 g, quantitative) as an oil.
EI(pos) 260 $[M-Boc]^+$

Reference Example 30

7-acetylspiro[chromene-2,4'-piperidin]-4(3H)-one hydrochloride

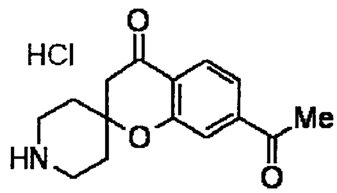

The title compound (1.06 g, yield 63%) was obtained as a powder in the same manner as in Reference Example 19 and from tert-butyl 7-acetyl-4-oxo-3,4-dihydro-1'H-spiro[chromene-2,4'-piperidine]-1'-carboxylate (2.03 g, 5.65 mmol) obtained in Reference Example 29 and 4N hydrogen chloride-ethyl acetate (10 mL). The compound was used for the next step without purification.

Reference Example 31

tert-butyl 5-cyano-4-oxo-3,4-dihydro-1'H-spiro[chromene-2,4'-piperidine]-1'-carboxylate

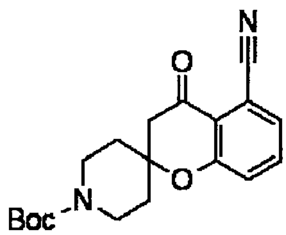

The title compound (1.63 g, yield 83%) was obtained as an oil in the same manner as in Reference Example 24 and from tert-butyl 4-oxo-5-{[(trifluoromethyl)sulfonyl]oxy}-3,4-dihydro-1'H-spiro[chromene-2,4'-piperidine]-1'-carboxylate (2.65 g, 5.69 mmol) and zinc cyanide (2.11 g, 18.0 mmol).
$^1H$ NMR ($CDCl_3$) δ1.46 (9H, s), 1.59-1.71 (2H, m), 1.95-2.07 (2H, m), 2.81 (2H, s), 3.17-3.25 (2H, m), 3.80-3.95 (2H, m), 7.24-7.26 (1H, m), 7.41 (1H, dd, J = 7.4, 1.0 Hz), 7.57 (1H, dd, J = 8.4, 7.4 Hz).

Reference Example 32

4-oxo-3,4-dihydrospiro[chromene-2,4'-piperidine]-5-carbonitrile hydrochloride
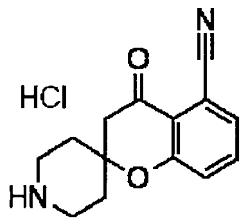

The title compound (1.32 g, quantitative) was obtained as a powder in the same manner as in Reference Example 19 and from tert-butyl 5-cyano-4-oxo-3,4-dihydro-1'H-spiro[chromene-2,4'-piperidine]-1'-carboxylate (1.63 g, 4.76 mmol) obtained in Reference Example 31 and 4N hydrogen chloride-ethyl acetate (10 mL). The compound was used for the next step without purification.

Reference Example 33

tert-butyl 4-oxo-7-(pyrrolidin-1-yl)-3,4-dihydro-1'H-spiro[chromene-2,4'-piperidine]-1'-carboxylate (I) and tert-butyl 7-fluoro-4-oxo-3,4-dihydro-1'H-spiro[chromene-2,4'-piperidine]-1'-carboxylate (II)
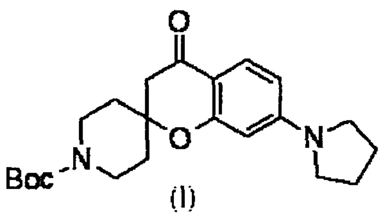
(I)

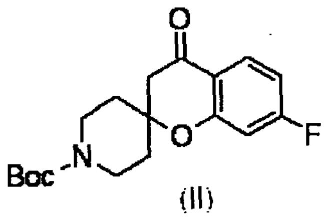
(II)

The title compound (1) (2.59 g, yield 20%) and the title compound (II) (8.69 g, yield 80%) were each obtained as a powder in the same manner as in Reference Example 18 and from 1-(4-fluoro-2-hydroxyphenyl)ethanone (5.00 g, 32.4 mmol) and tert-butyl 4-oxopiperidine-1-carboxylate (6.47 g, 32.4 mmol). the title compound (I)
EI(pos) 387 $[M+H]^+$
the title compound (II)
EI(pos) 280 $[M-tBu]^+$

Reference Example 34

7-(pyrrolidin-1-yl)spiro[chromene-2,4'-piperidin]-4(3H)-one dihydrochloride
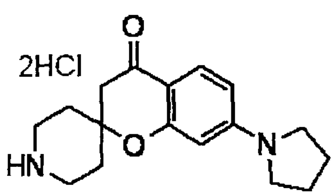

The title compound (1.48 g, quantitative) was obtained as a powder in the same manner as in Reference Example 19 and from tert-butyl 4-oxo-7-(pyrrolidin-1-yl)-3,4-dihydro-1'H-spiro[chromene-2,4'-piperidine]-1'-carboxylate (1.60 g, 4.13 mmol) obtained in Reference Example 33 and 4N hydrogen chloride-ethyl acetate (10 mL). The compound was used for the next step without purification.

Reference Example 35

2-{4-[(tert-butoxycarbonyl)amino]piperidin-1-yl}-5-nitropyridine-4-carboxylic acid
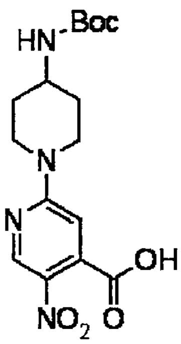

A solution of 2-chloro-5-nitropyridine-4-carboxylic acid (1.50 g, 7.41 mmol), tert-butyl piperidin-4-ylcarbamate (1.48 g, 7.41 mmol) and triethylamine (3.1 mL, 22.2 mmol) in THF (19 mL) was stirred with heating at 50°C for 2 hr. After completion of the reaction, the mixture was neutralized with 1N hydrochloric acid, and extracted with ethyl acetate. The extract was dried over anhydrous sodium sulfate. The solvent was evaporated under reduced pressure, and the obtained residue was purified by silica gel column chromatography (ethyl acetate:hexane=4:1 to 1:0) to give the title compound (1.57 g, yield 58%) as a powder.
EI(pos) 311.1 $[M+H-tBu]^+$

Reference Example 36

2-[4-(benzyloxy)piperidin-1-yl]-5-nitropyridine-4-carboxylic acid
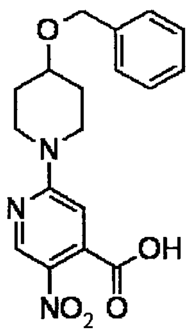

The title compound (568 mg, yield 18%) was obtained as a powder in the same manner as in Reference Example 35 and from 2-chloro-5-nitropyridine-4-carboxylic acid (1.80 g, 8.95 mmol), 4-(benzyloxy)piperidine (1.71 g, 8.95 mmol) and triethylamine (3.7 mL, 26.9 mmol).
EI(pos) 358.1 $[M+H]^+$

Reference Example 37

tert-butyl 4-[2-(benzyloxy)-2-oxoethyl]-4-hydroxypiperidine-1-carboxylate
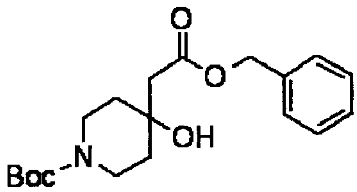

To a solution of zinc powder (7.39 g, 113 mmol) in THF (180 mL) was added dropwise benzyl bromoacetate (17.9 mL, 113 mmol) at room temperature, and the mixture was stirred at 40°C for 10 min. After allowing to return to room temperature, a solution of tert-butyl 4-oxopiperidine-1-carboxylate (15.0 g, 75.3 mmol) in THF (45 mL) was added dropwise, and the mixture was stirred with heating at 60°C for 30 min. After completion of the reaction, water (45 mL) was slowly added dropwise at room temperature, ethyl acetate and water were added, and the insoluble material was filtered off through celite. The extract was separated, washed with saturated brine, and dried over anhydrous sodium sulfate. The residue was subjected to basic silica gel column chromatography (ethyl acetate) and the solvent was evaporated under reduced pressure. The obtained residue was purified by silica gel column chromatography (ethyl acetate:hexane=1:9 to 3:7) to give the title compound (21.9 g, yield 83%) as an oil.
$^1$H NMR ($CDCl_3$) δ1.45 (9H, s), 1.46-1.52 (2H, m), 1.63-1.67 (2H, m), 2.51 (2H, s), 3.14-3.22 (2H, m), 3.49 (1H, br), 3.78-3.82 (2H, m), 5.15 (2H, s), 7.30-7.39 (5H, m).

Reference Example 38

tert-butyl 4-[2-(benzyloxy)-2-oxoethyl]-4-(carbamoyloxy)piperidine-1-carboxylate
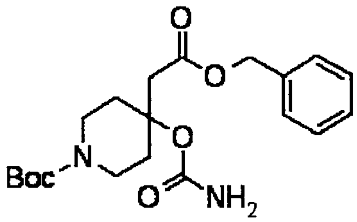

To a solution of tert-butyl 4-[2-(benzyloxy)-2-oxoethyl]-4-hydroxypiperidine-1-carboxylate (3.00 g, 8.59 mmol) obtained in Reference Example 37 in THF (20 mL) was added dropwise trichloroacetyl isocyanate (2.05 mL, 17.2 mmol) under ice-cooling, and the mixture was stirred at room temperature for 2 hr. 7M Ammonia-methanol (20 mL) was added and the mixture was stirred for 1 hr, and concentrated under reduced pressure. Ethyl acetate was added to the residue, and the mixture was washed with saturated brine, and dried over anhydrous sodium sulfate. The residue was subjected to basic silica gel column chromatography (ethyl acetate), and triturated with diisopropyl ether and hexane to give the title compound (3.37 g, quantitative).
$^1$H NMR ($CDCl_3$) δ1.45 (9H, s), 1.58-1.68 (2H, m), 2.28-2.32 (2H, m), 2.99-3.07 (4H, m), 3.79-3.83 (2H, m), 4.66 (2H, br), 5.12 (2H, s), 7.33-7.35 (5H, m).

Reference Example 39

benzyl [4-(carbamoyloxy)piperidin-4-yl]acetate hydrochloride
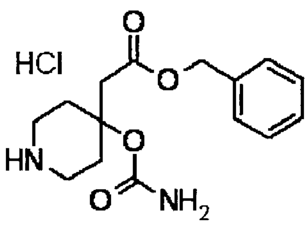

The title compound (460 mg, yield 55%) was obtained as a powder in the same manner as in Reference Example 19 and from tert-butyl 4-[2-(benzyloxy)-2-oxoethyl]-4-(carbamoyloxy)piperidine-1-carboxylate (1.00 g, 2.54 mmol) obtained in Reference Example 38 and 4N hydrogen chloride-ethyl acetate (10 mL).
$^1$H NMR ($CD_3OD$) δ1.92-2.02 (4H, m), 2.55-2.61 (2H, m), 3.10 (2H, s), 3.13-3.22 (2H, m), 5.12 (2H, s), 7.29-7.36 (5H, m).

Reference Example 40

benzyl {1-[(2-amino-1-benzothiophen-3-yl)carbonyl]-4-(carbamoyloxy)piperidin-4-yl}acetate
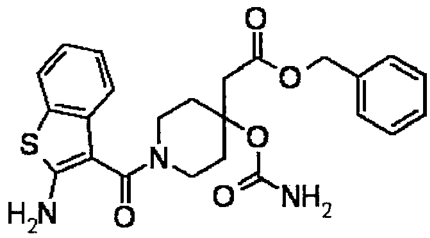

To a solution of benzyl [4-(carbamoyloxy)piperidin-4-yl]acetate hydrochloride (460 mg, 1.40 mmol) obtained in Reference Example 39, 2-amino-1-benzothiophene-3-carboxylic acid (270 mg, 1.40 mmol, produced by the method described in WO07/013691) and 1-hydroxybenzotriazole (189 mg, 1.40 mmol) in DMF (3.5 mL) were added 1-ethyl-3-(3'-dimethylaminopropyl)carbodiimide hydrochloride (268 mg, 1.40 mmol) and triethylamine (0.195 mL, 1.40 mmol), and the mixture was stirred at room temperature for 16 hr. The reaction mixture was diluted with ethyl acetate, and the mixture was washed with 10% aqueous potassium carbonate solution and saturated brine, and dried over anhydrous sodium sulfate. The residue was subjected to basic silica gel column chromatography (ethyl acetate), and the solvent was evaporated under reduced pressure. The residue was purified by silica gel column chromatography (hexane:ethyl acetate=3:1 to 0:1) to give the title compound (257 mg, yield 39%) as a powder.
EI(pos) 468.5 $[M+H]^+$

Reference Example 41

benzyl [4-(carbamoyloxy)-1-({2-[(ethylcarbamoyl)amino]-1-benzothiophen-3-yl}carbonyl)piperidin-4-yl]acetate
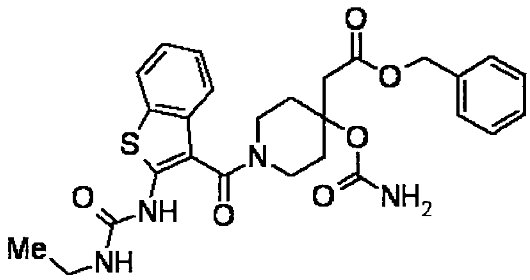

To a solution of benzyl {1-[(2-amino-1-benzothiophen-3-yl)carbonyl]-4-(carbamoyloxy)piperidin-4-yl}acetate (255 mg, 0.545 mmol) obtained in Reference Example 40 in pyridine (2 mL) was added ethyl isocyanate (0.173 mL, 2.18 mmol), and the mixture was stirred at 70°C for 16 hr. The solvent was evaporated under reduced pressure, and the residue was purified by basic silica gel column chromatography (hexane:ethyl acetate=3:1 to 0:1) to give the title compound (252 mg, yield 86%) as an oil.
EI(pos) 539.1 $[M+H]^+$

Reference Example 42

tert-butyl 2,4-dioxo-1-oxa-3,9-diazaspiro[5.5]undecane-9-carboxylate
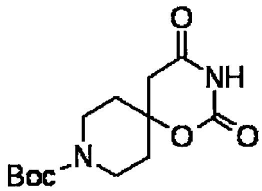

To a solution of tert-butyl 4-[2-(benzyloxy)-2-oxoethyl]-4-(carbamoyloxy)piperidine-1-carboxylate (5.00 g, 12.7 mmol) obtained in Reference Example 38 in THF (25 mL) was added potassium tert-butoxide (1.43 g, 12.7 mmol) under ice-cooling, and the mixture was stirred at room temperature for 30 min. The reaction mixture was diluted with ethyl acetate, and the mixture was washed with 0.5N hydrochloric acid and saturated brine, and dried over anhydrous sodium sulfate. The solvent was evaporated under reduced pressure, and the residue was purified by silica gel column chromatography (hexane:ethyl acetate=1:1 to 1:3) to give the title compound (1.64 g, yield 45%) as a powder.
$^1$H NMR ($CDCl_3$) δ1.46 (9H, m), 1.63-1.73 (2H, m), 1.94-1.98 (2H, m), 2.67 (2H, s), 3.23-3.31 (2H, m), 3.90 (2H, m), 8.08 (1H, br).

Reference Example 43

tert-butyl 3-[2-(benzyloxy)-2-oxoethyl]-2,4-dioxo-1-oxa-3,9-diazaspiro[5.5]undecane-9-carboxylate
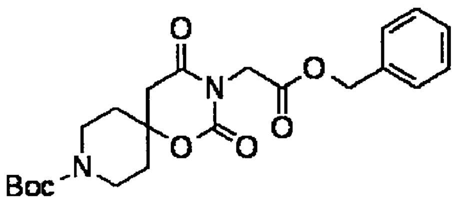

The title compound (304 mg, quantitative) was obtained as an oil in the same manner as in Reference Example 26 and from tert-butyl 2,4-dioxo-1-oxa-3,9-diazaspiro[5.5]undecane-9-carboxylate (200 mg, 0.703 mmol) obtained in Reference Example 42 and benzyl bromoacetate (0.111 mL, 0.703 mmol).
$^1$H NMR ($CDCl_3$) δ1.46 (9H, m), 1.58-1.66 (2H, m), 1.98-2.05 (2H, m), 2.77 (2H, s), 3.17-3.25 (2H, m), 3.83-3.87 (2H, m), 4.61 (2H, s), 5.17 (2H, s), 7.31-7.40 (5H, m).

Reference Example 44

benzyl (2,4-dioxo-1-oxa-3,9-diazaspiro[5.5]undec-3-yl)acetate hydrochloride
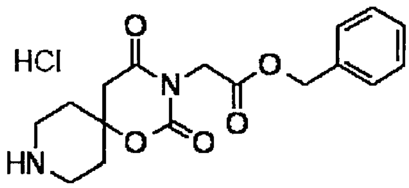

The title compound (242 mg, yield 93%) was obtained as a powder in the same manner as in Reference Example 19 and from tert-butyl 3-[2-(benzyloxy)-2-oxoethyl]-2,4-dioxo-1-oxa-3,9-diazaspiro[5.5]undecane-9-carboxylate (304 mg, 0.703 mmol) obtained in Reference Example 43 and 4N hydrogen chloride-ethyl acetate (4 mL). The compound was used for the next step without purification.

Reference Example 45

tert-butyl 3-[3-(benzyloxy)-3-oxopropyl]-2,4-dioxo-1-oxa-3,9-diazaspiro[5.5]undecane-9-carboxylate

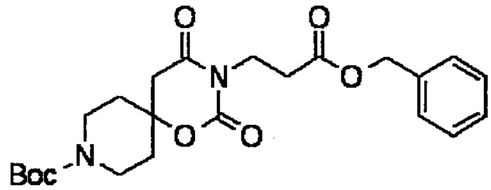

The title compound (245 mg, yield 62%) was obtained as an oil in the same manner as in Reference Example 26 and from tert-butyl 2,4-dioxo-1-oxa-3,9-diazaspiro[5.5]undecane-9-carboxylate (250 mg, 0.879 mmol) obtained in Reference Example 42 and benzyl 3-bromopropanoate (214 mg, 0.879 mmol).
EI(pos) 447.5 $[M+H]^+$

Reference Example 46

benzyl 3-(2,4-dioxo-1-oxa-3,9-diazaspiro[5.5]undec-3-yl)propanoate hydrochloride

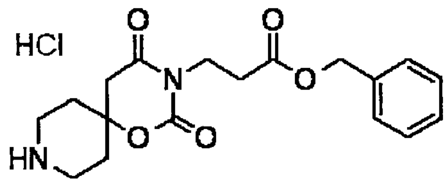

The title compound (190 mg, yield 91%) was obtained as a powder in the same manner as in Reference Example 19 and from tert-butyl 3-[3-(benzyloxy)-3-oxopropyl]-2,4-dioxo-1-oxa-3,9-diazaspiro[5.5]undecane-9-carboxylate (245 mg, 0.549 mmol) obtained in Reference Example 45 and 4N hydrogen chloride-ethyl acetate (3 mL). The compound was used for the next step without purification.

Reference Example 47

tert-butyl 3-ethyl-2,4-dioxo-1-oxa-3,9-diazaspiro[5.5]undecane-9-carboxylate

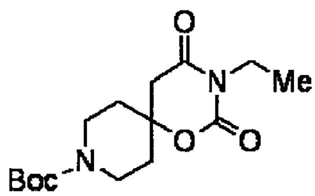

The title compound (715 mg, quantitative) was obtained as an oil in the same manner as in Reference Example 26 and from tert-butyl 2,4-dioxo-1-oxa-3,9-diazaspiro[5.5]undecane-9-carboxylate (650 mg, 2.29 mmol) obtained in Reference Example 42 and ethyl iodide (0.183 mL, 2.29 mmol).
EI(pos) 255 $[M-tBu]^+$

Reference Example 48

3-ethyl-1-oxa-3,9-diazaspiro[5.5]undecane-2,4-dione hydrochloride

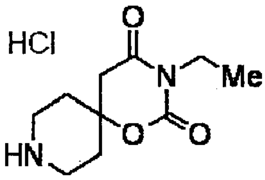

The title compound (517 mg, quantitative) was obtained as a powder in the same manner as in Reference Example 19 and from tert-butyl 3-ethyl-2,4-dioxo-1-oxa-3,9-diazaspiro[5.5]undecane-9-carboxylate (760 mg, 2.44 mmol) obtained in Reference Example 47 and 4N hydrogen chloride-ethyl acetate (5 mL). The compound was used for the next step without purification.

Reference Example 49

tert-butyl 6-ethenyl-4-oxo-3,4-dihydro-1'H-spiro[chromene-2,4'-piperidine]-1'-carboxylate

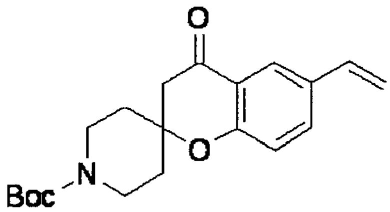

To a solution of tert-butyl 6-bromo-4-oxo-3,4-dihydro-1'H-spiro[chromene-2,4'-piperidine]-1'-carboxylate (2.00 g, 5.05 mmol) and tributyl(ethenyl)stannane (2.95 mL, 10.1 mmol) in 1,4-dioxane (20 mL) was added tetrakistriphenylphosphine palladium (292 mg, 0.253 mmol), and the mixture was stirred with heating under a nitrogen atmosphere at 100°C for 15 hr. After completion of the reaction, the reaction mixture was diluted with ethyl acetate, and the mixture was washed with saturated brine, and dried over anhydrous magnesium sulfate. The solvent was evaporated under reduced pressure, and the obtained residue was purified by silica gel column chromatography (ethyl acetate:hexane=1:19 to 2:3) to give the title compound (1.73 g, quantitative) as an oil.
EI(pos) 288 $[M-tBu]^+$

Reference Example 50

tert-butyl 6-formyl-4-oxo-3,4-dihydro-1'H-spiro[chromene-2,4'-piperidine]-1'-carboxylate

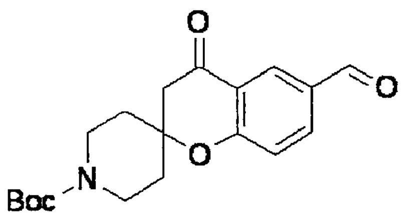

The title compound (588 mg, yield 32%) was obtained as a powder in the same manner as in Reference Example 29 and from tert-butyl 6-ethenyl-4-oxo-3,4-dihydro-1'H-spiro[chromene-2,4'-piperidine]-1'-carboxylate (1.82 g, 5.30 mmol) obtained in Reference Example 49.
EI(pos) 290 $[M-tBu]^+$

Reference Example 51

4-oxo-3,4-dihydrospiro[chromene-2,4'-piperidine]-6-carbaldehyde hydrochloride

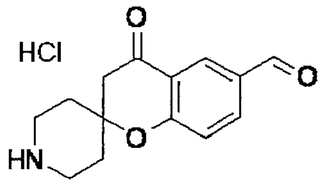

The title compound (478 mg, quantitative) was obtained as a powder in the same manner as in Reference Example 19 and from tert-butyl 6-formyl-4-oxo-3,4-dihydro-1'H-spiro[chromene-2,4'-piperidine]-1'-carboxylate (588 mg, 1.70 mmol) obtained in Reference Example 50 and 4N hydrogen chloride-ethyl acetate (5 mL). The compound was used for the next step without purification.

Reference Example 52

tert-butyl 8-chloro-4-oxo-3,4-dihydro-1'H-spiro[chromene-2,4'-piperidine]-1'-carboxylate

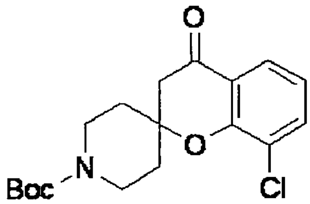

The title compound (3.84 g, yield 93%) was obtained as a powder in the same manner as in Reference Example 18 and from 1-(3-chloro-2-hydroxyphenyl)ethanone (2.00 g, 11.7 mmol) and tert-butyl 4-oxopiperidine-1-carboxylate (2.57 g, 12.9 mmol). $^1H$ NMR ($CDCl_3$) δ1.47 (9H, s), 1.55-1.67 (2H, m), 2.03-2.07 (2H, m), 2.75 (2H, s), 3.21-3.29 (2H, m), 3.88-4.05 (2H, m), 6.96 (1H, t, J = 7.8 Hz), 7.58 (1H, dd, J = 7.8, 1.7 Hz), 7.78 (1H, dd, J = 8.0, 1.5 Hz).

Reference Example 53

8-chlorospiro[chromene-2,4'-piperidin]-4(3H)-one hydrochloride

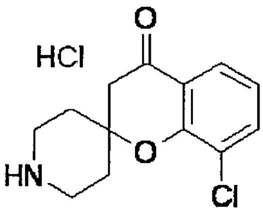

The title compound (1.21 g, yield 80%) was obtained as a powder in the same manner as in Reference Example 19 and from tert-butyl 8-chloro-4-oxo-3,4-dihydro-1'H-spiro[chromene-2,4'-piperidine]-1'-carboxylate (1.84 g, 5.23 mmol) obtained in Reference Example 52 and 4N hydrogen chloride-ethyl acetate (10 mL). The compound was used for the next step without purification.

Reference Example 54

tert-butyl 6-(benzyloxy)-4-oxo-3,4-dihydro-1'H-spiro[chromene-2,4'-piperidine]-1'-carboxylate

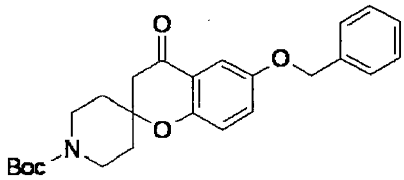

The title compound (2.65 g, yield 65%) was obtained as a powder in the same manner as in Reference Example 26 and from tert-butyl 6-hydroxy-4-oxo-3,4-dihydro-1'H-spiro[chromene-2,4'-piperidine]-1'-carboxylate (3.23 g, 9.69 mmol) and benzyl bromide (1.4 mL, 11.7 mmol).
$^1$H NMR ($CDCl_3$) δ1.46 (9H, s), 1.53-1.64 (2H, m), 1.98-2.04 (2H, m), 2.69 (2H, s), 3.16-3.24 (2H, m), 3.80-3.94 (2H, m), 5.04 (2H, s), 6.92 (1H, d, J = 9.1 Hz), 7.18 (1H, dd, J = 8.9, 3.2 Hz), 7.30-7.45 (6H, m).

Reference Example 55

6-(benzyloxy)spiro[chromene-2,4'-piperidin]-4(3H)-one hydrochloride
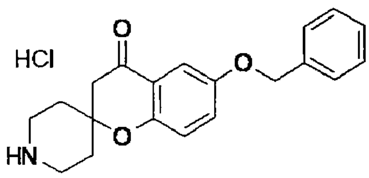

The title compound (2.25 g, quantitative) was obtained as a powder in the same manner as in Reference Example 19 and from tert-butyl 6-(benzyloxy)-4-oxo-3,4-dihydro-1'H-spiro[chromene-2,4'-piperidine]-1'-carboxylate (2.65 g, 6.26 mmol) obtained in Reference Example 54 and 4N hydrogen chloride-ethyl acetate (10 mL). The compound was used for the next step without purification.

Reference Example 56

tert-butyl 4-oxo-3,4-dihydro-1'H-spiro[chromene-2,3'-pyrrolidine]-1'-carboxylate
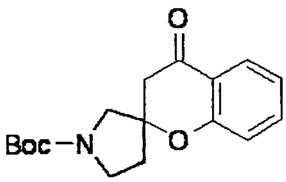

The title compound (11.5 g, yield 70%) was obtained as an oil in the same manner as in Reference Example 18 and from 1-(2-hydroxyphenyl)ethanone (7.35 g, 54 mmol) and tert-butyl 3-oxopyrrolidine-1-carboxylate (10.0 g, 54 mmol).
$^1$H NMR (DMSO-$d_6$) δ1.34-1.46 (9H, m), 1.91-2.08 (1H, m), 2.09-2.22 (1H, m), 2.94 (1H, d, J = 16.95 Hz), 3.11 (1H, d, J = 16.95 Hz), 3.31-3.42 (2H, m), 3.43-3.53 (1H, m), 3.59 (1H, dd, J = 12.06, 1.70 Hz), 6.98-7.14 (2H, m), 7.51-7.65 (1H, m), 7.76 (1H, dd, J = 7.82, 1.60 Hz).

Reference Example 57

spiro[chromene-2,3'-pyrrolidin]-4(3H)-one hydrochloride
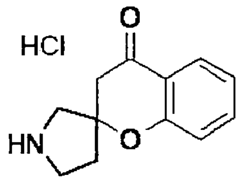

The title compound (360 mg, yield 46%) was obtained as a powder in the same manner as in Reference Example 19 and from tert-butyl 4-oxo-3,4-dihydro-1'H-spiro[chromene-2,3'-pyrrolidine]-1'-carboxylate (1.0 g, 3.3 mmol) obtained in Reference Example 56 and 4N hydrogen chloride-ethyl acetate (3 mL).
$^1$H NMR (DMSO-$d_6$) δ2.00-2.13 (1H, m), 2.24-2.35 (1H, m), 3.06 (1H, d, J = 16.95 Hz), 3.18 (1H, d, J = 16.95 Hz), 3.22-3.58 (4H, m), 7.05 (1H, d, J = 8.29 Hz), 7.09-7.17 (1H, m), 7.59-7.67 (1H, m), 7.78 (1H, dd, J = 7.91, 1.51 Hz), 9.64 (1H, br), 9.78 (1H, br).

Reference Example 58

1-(3-hydroxypyridin-2-yl)ethanone
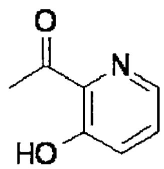

To a solution of 3-hydroxypyridine-2-carbonitrile (3.00 g, 25.0 mmol) in THF (50 mL) was added a 3M solution of methylmagnesium bromide in THF (25 mL) under ice-cooling, and the mixture was stirred at room temperature for 30 min. The reaction mixture was neutralized with 6N hydrochloric acid, and extracted twice with ethyl acetate. The combined organic layers were washed with saturated brine, and dried over anhydrous sodium sulfate. The solvent was evaporated under reduced pressure to give the title compound (3.43 g, quantitative) as a powder. The compound was used for the next step without purification.

Reference Example 59

tert-butyl 4'-oxo-3',4'-dihydro-1H-spiro[piperidine-4,2'-pyrano[3,2-b]pyridine]-1-carboxylate
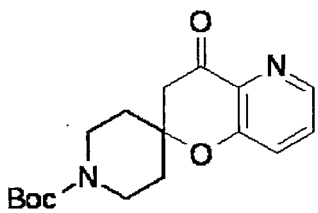

The title compound (5.21 g, yield 65%) was obtained as a powder in the same manner as in Reference Example 18 and from 1-(3-hydroxypyridin-2-yl)ethanone (3.42 g, 25.0 mmol) obtained in Reference Example 58 and tert-butyl 4-oxopiperidine-1-carboxylate (4.99 g, 25.0 mmol).
EI(pos) 319 $[M+H]^+$

Reference Example 60

spiro[piperidine-4,2'-pyrano[3,2-b]pyridin]-4'(3'H)-one dihydrochloride
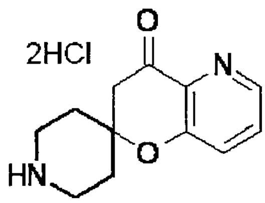

The title compound (880 mg, quantitative) was obtained as a powder in the same manner as in Reference Example 19 and from tert-butyl 4'-oxo-3',4'-dihydro-1H-spiro[piperidine-4,2'-pyrano[3,2-b]pyridine]-1-carboxylate (965 mg, 3.02 mmol) obtained in Reference Example 59 and 4N hydrogen chloride-ethyl acetate (10 mL). The compound was used for the next step without purification.

Reference Example 61

tert-butyl 4-{[tert-butyl(dimethyl)silyl]oxy}-6-(hydroxycarbamimidoyl)-1'H-spiro[chromene-2,4'-piperidine]-1'-carboxylate

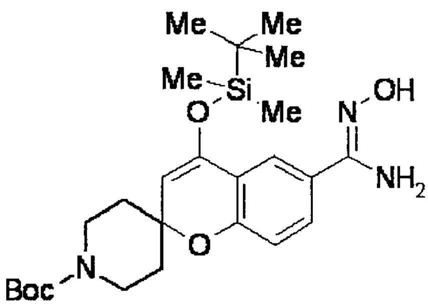

To a solution of tert-butyl 6-cyano-4-oxo-3,4-dihydro-1'H-spiro[chromene-2,4'-piperidine]-1'-carboxylate (3.24 g, 9.46 mmol) in THF (45 mL) was added dropwise a 1.1M solution (10.3 mL, 11.4 mmol) of LHDMS in THF at -78°C, and 30 min later, a solution of tert-butyl(dimethyl)silyl trifluoromethanesulfonate (2.61 mL, 11.4 mmol) in THF (1.5 mL) was added dropwise. The mixture was warmed to room temperature and stirred for 30 min. To the reaction mixture was added 10% aqueous potassium carbonate solution, and the mixture was extracted with ethyl acetate. The extract was washed with 10% aqueous potassium carbonate solution and saturated brine, and dried over anhydrous sodium sulfate. The solvent was evaporated under reduced pressure, and the obtained oil was crystallized from hexane to give tert-butyl 4-{[tert-butyl(dimethyl)silyl]oxy}-6-cyano-1'H-spiro[chromene-2,4'-piperidine]-1'-carboxylate (2.41 g, yield 56%). A solution of the obtained intermediate (2.40 g, 5.28 mmol), hydroxylamine hydrochloride (1.10 g, 15.8 mmol) and triethylamine (2.20 mL, 15.8 mmol) in ethanol (20 mL) was stirred at 50°C for 16 hr. After completion of the reaction, the solvent was evaporated under reduced pressure. Ethyl acetate was added to the residue, and the mixture was washed with saturated brine, and dried over anhydrous magnesium sulfate. The solvent was evaporated under reduced pressure, and the obtained residue was purified by silica gel column chromatography (ethyl acetate:hexane=1:3 to 1:1), and triturated with hexane to give the title compound (1.98 g, yield 77%).
$^1$H NMR ($CDCl_3$) δ0.22 (6H, s), 1.01 (9H, s), 1.47 (9H, s), 1.54-1.68 (2H, m), 1.97-2.01 (2H, m), 3.24-3.32 (2H, m), 3.79 (2H, m), 4.74 (1H, s), 4.77 (2H, br), 6.82 (1H, d, J = 8.1 Hz), 7.45 (1H, dd, J = 8.1, 2.1 Hz), 7.52 (1H, br), 7.60 (1H, d, J = 2.1 Hz).

Reference Example 62

tert-butyl 4-{[tert-butyl(dimethyl)silyl]oxy}-6-(5-oxo-4,5-dihydro-1,2,4-oxadiazol-3-yl)-1'H-spiro[chromene-2,4'-piperidine]-1'-carboxylate

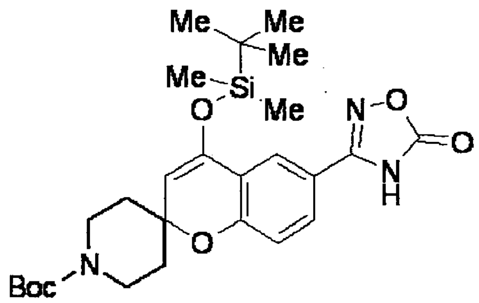

To a solution of tert-butyl 4-{[tert-butyl(dimethyl)silyl]oxy}-6-(hydroxycarbamimidoyl)-1'H-spiro[chromene-2,4'-piperidine]-1'-carboxylate (500 mg, 1.03 mmol) obtained in Reference Example 61 in THF (5 mL) was added carbonyldiimidazole (216 mg, 1.33 mmol) at room temperature, and the mixture was stirred for 1 hr and then stirred with heating at 80°C for 3 hr. The solvent was evaporated under reduced pressure, and the obtained residue was purified by silica gel column chromatography (ethyl acetate:hexane=1:3 to 1:1), and triturated with diisopropyl ether and hexane to give the title compound (360 mg, yield 68%).
$^1$H NMR ($CDCl_3$) δ0.25 (6H, s), 1.48 (9H, s), 1.57-1. 67 (2H, m), 1.97-2.01 (2H, m), 3.28 (2H, m), 3.80-3.84 (2H, m), 4.79 (1H, s), 6.92 (1H, d, J = 8.4 Hz), 7.58 (1H, dd, J = 8.4, 2.4 Hz), 7.79 (1H, d, J = 2.4 Hz).

Reference Example 63

tert-butyl 4-oxo-6-(5-oxo-4,5-dihydro-1,2,4-oxadiazol-3-yl)-3,4-dihydro-1'H-spiro[chromene-2,4'-piperidine]-1'-carboxylate
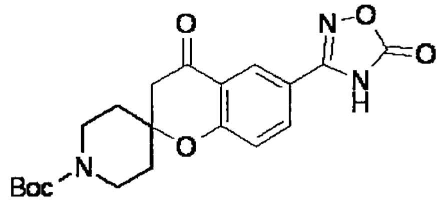

To a solution of tert-butyl 4-{[tert-butyl(dimethyl)silyl]oxy}-6-(5-oxo-4,5-dihydro-1,2,4-oxadiazol-3-yl)-1'H-spiro[chromene-2,4'-piperidine]-1'-carboxylate (357 mg, 0.692 mmol) obtained in Reference Example 62 in THF (3 mL) was added a 1M solution (2.08 mL) of tetrabutylammonium fluoride in THF and the mixture was stirred at room temperature for 30 min. The reaction mixture was diluted with ethyl acetate, and the mixture was washed with 0.5N hydrochloric acid and saturated brine, and dried over anhydrous sodium sulfate. The solvent was evaporated under reduced pressure and triturated with diisopropyl ether to give the title compound (278 mg, quantitative).
$^1$H NMR ($CDCl_3$) δ1.47 (9H, s), 1.71-1.78 (2H, m), 2.04-2.08 (2H, m), 3.01 (2H, s), 3.24 (2H, m), 3.88 (2H, m), 7.18 (1H, d, J = 8.7 Hz), 8.18 (1H, dd, J = 8.7, 2.4 Hz), 8.64 (1H, d, J = 2.4 Hz), 11.36 (1H, br).

Reference Example 64

6-(5-oxo-4,5-dihydro-1,2,4-oxadiazol-3-yl)spiro[chromene-2,4'-piperidin]-4(3H)-one hydrochloride
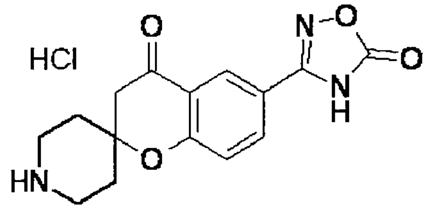

The title compound (230 mg, quantitative) was obtained as a powder in the same manner as in Reference Example 19 and from tert-butyl 4-oxo-6-(5-oxo-4,5-dihydro-1,2,4-oxadiazol-3-yl)-3,4-dihydro-1'H-spiro[chromene-2,4'-piperidine]-1'-carboxylate (273 mg, 0.680 mmol) obtained in Reference Example 63 and 4N hydrogen chloride-ethyl acetate (5 mL). The compound was used for the next step without purification.

Reference Example 65

tert-butyl 6-(2-methoxy-2-oxoethyl)-4-oxo-3,4-dihydro-1'H-spiro[chromene-2,4'-piperidine]-1'-carboxylate
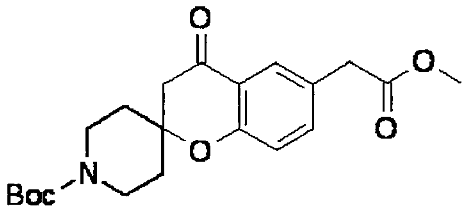

The title compound (6.63 g, yield 68%) was obtained as a powder in the same manner as in Reference Example 18 and from methyl (3-acetyl-4-hydroxyphenyl)acetate (5.18 g, 24.9 mmol) and tert-butyl 4-oxopiperidine-1-carboxylate (4.96 g, 24.9 mmol).
$^1H$ NMR ($CDCl_3$) δ1.46 (9H, s), 1.55-1.65 (2H, m), 2.00-2.04 (2H, m), 2.70 (2H, s), 3.16-3.24 (2H, m), 3.59 (2H, s), 3.70 (3H, s), 3.84-3.88 (2H, m), 6.96 (1H, d, J = 8.7 Hz), 7.43 (1H, dd, J = 8.7, 2.4 Hz), 7.74 (1H, d, J = 2.4 Hz).

Reference Example 66

methyl (4-oxo-3,4-dihydrospiro[chromene-2,4'-piperidin]-6-yl)acetate
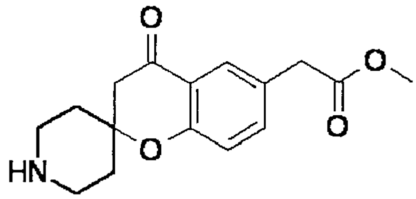

To tert-butyl 6-(2-methoxy-2-oxoethyl)-4-oxo-3,4-dihydro-1'H-spiro[chromene-2,4'-piperidine]-1'-carboxylate (3.00 g, 7.70 mmol) obtained in Reference Example 65 was added trifluoroacetic acid (40 mL), and 30 min later, the mixture was concentrated under reduced pressure. The residue was dissolved in ethyl acetate, and the solution was washed with 10% aqueous potassium carbonate solution and saturated brine, and dried over anhydrous sodium sulfate. The solvent was evaporated under reduced pressure to give the title compound (555 mg, yield 25%) as an oil. The compound was used for the next step without purification.

Reference Example 67

tert-butyl 4'-oxo-3',4'-dihydro-1H,1'H-spiro[piperidine-4,2'-quinazoline]-1-carboxylate
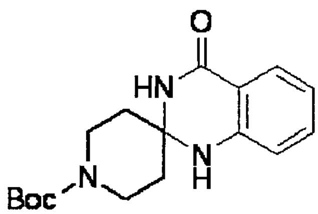

A solution of 2-aminobenzamide (2.93 g, 14.7 mmol), tert-butyl 4-oxopiperidine-1-carboxylate (2.00 g, 14.7 mmol), p-toluenesulfonic acid monohydrate (15.0 mg, 0.0789 mmol) and anhydrous magnesium sulfate (5.00 g) in dichloroethane (20 mL) was stirred with heating at 60°C for 13 hr. The reaction mixture was diluted with ethyl acetate, and the mixture was washed with saturated brine, and dried over anhydrous sodium sulfate. The obtained residue was washed with diethyl ether to give the title compound (3.77 g, yield 80%).
$^1$H NMR ($CDCl_3$) δ1.46 (9H, s), 1.80-1.88 (2H, m), 1.95-2.05 (2H, m), 3.54-3.58 (4H, m), 4.32 (1H, br), 6.68 (1H, d, J = 8.1 Hz), 6.86 (1H, t, J = 7.5 Hz), 7.15 (1H, br), 7.29-7.34 (1H, m), 7.87 (1H, d, J = 7.7 Hz).

Reference Example 68

1'H-spiro[piperidine-4,2'-quinazolin]-4'(3'H)-one hydrochloride
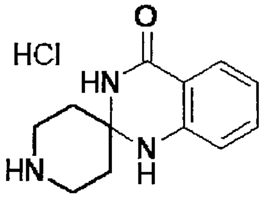

The title compound (1.16 g, quantitative) was obtained as a powder in the same manner as in Reference Example 19 and from tert-butyl 4'-oxo-3',4'-dihydro-1H,1'H-spiro[piperidine-4,2'-quinazoline]-1-carboxylate (1.27 g, 4.00 mmol) obtained in Reference Example 67 and 4N hydrogen chloride-ethyl acetate (10 mL). The compound was used for the next step without purification.

Reference Example 69

tert-butyl 4-oxo-6-(5-oxo-4,5-dihydro-1,2,4-thiadiazol-3-yl)-3,4-dihydro-1'H-spiro[chromene-2,4'-piperidine]-1'-carboxylate
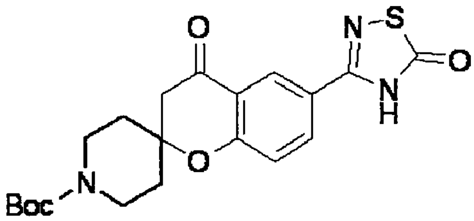

tert-Butyl 6-(hydroxycarbamimidoyl)-4-oxo-3,4-dihydro-1'H-spiro[chromene-2,4'-piperidine]-1'-carboxylate (2.58 g, yield 74%) was obtained in the same manner as in Reference Example 63 and from tert-butyl 4-{[tert-butyl(dimethyl)silyl]oxy}-6-(hydroxycarbamimidoyl)-1'H-spiro[chromene-2,4'-piperidine]-1'-carboxylate (4.53 g, 9.29 mmol) obtained in Reference Example 61 and a 1M solution (27.9 mL) of tetrabutylammonium fluoride in THF. To a solution of the obtained intermediate (500 mg, 1.33 mmol) in THF (10 mL) was added thiocarbonyldiimidazole (285 mg, 1.60 mmol), and the mixture was stirred at room temperature for 1.5 hr. The reaction mixture was diluted with ethyl acetate, and the mixture was washed with saturated brine, and dried over anhydrous sodium sulfate. The obtained residue was dissolved in chloroform (35 mL) and methanol (7 mL), silica gel (5 g) was added, and the mixture was stirred at room temperature for 2 days. The mixture was filtered, and silica gel was thoroughly washed with ethyl acetate. The filtrate was concentrated under reduced pressure. The residue was dissolved in ethyl acetate, and the solution was washed with 0.1N hydrochloric acid and saturated brine, and dried over anhydrous sodium sulfate. The residue was purified by silica gel column chromatography (ethyl acetate:hexane=1:3 to 1:1), and triturated with diisopropyl ether to give the title compound (109 mg, yield 20%).
$^1$H NMR ($CDCl_3$) δ1.47 (9H, s), 1.68-1.78 (2H, m), 2.02-2.07 (2H, m), 3.03 (2H, s), 3.28-3.31 (2H, m), 3.85 (2H, m), 7.15 (1H, d, J = 8.4 Hz), 8.30 (1H, dd, J = 8.4, 2.1 Hz), 8.70 (1H, d, J = 2.1 Hz), 11.82 (1H, br).

Reference Example 70

6-(5-oxo-4,5-dihydro-1,2,4-thiadiazol-3-yl)spiro[chromene-2,4'-piperidin]-4(3H)-one hydrochloride

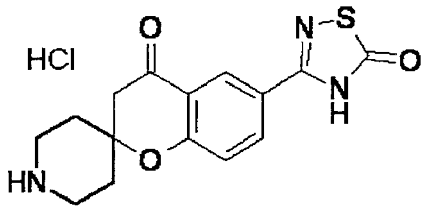

The title compound (91.2 mg, quantitative) was obtained as a powder in the same manner as in Reference Example 19 and from tert-butyl 4-oxo-6-(5-oxo-4,5-dihydro-1,2,4-thiadiazol-3-yl)-3,4-dihydro-1'H-spiro[chromene-2,4'-piperidine]-1'-carboxylate (106 mg, 0.254 mmol) obtained in Reference Example 69 and 4N hydrogen chloride-ethyl acetate (5 mL). The compound was used for the next step without purification.

Reference Example 71

2-benzyl 4-tert-butyl 3-methyl-5-{[(2,2,2-trichloroethoxy)carbonyl]amino}thiophene-2,4-dicarboxylate

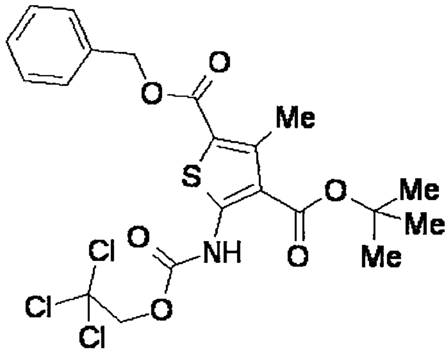

To a solution of 2-benzyl 4-tert-butyl 5-amino-3-methylthiophene-2,4-dicarboxylate (5.7 g, 16.4 mmol) in pyridine (30 mL) was added 2,2,2-trichloroethyl chlorocarbonate (2.4 mL, 17.3 mmol), and the mixture was stirred at room temperature for 16 hr. The reaction mixture was concentrated to dryness, the residue was diluted with ethyl acetate, and the mixture was washed with saturated brine, and dried over anhydrous magnesium sulfate. The residue was purified by silica gel column chromatography (ethyl acetate:hexane=0:1 to 7:3), and triturated with diisopropyl ether to give the title compound (2.62 g, yield 31%).

EI(pos) 524.4 $[M+H]^+$

$^1H$ NMR ($CDCl_3$) $\delta$1.61 (9H, s), 2.74 (3H, s), 4.88 (2H, s), 5.30 (2H, s), 7.26-7.50 (5H, m).

Reference Example 72

5-[(benzyloxy)carbonyl]-4-methyl-2-{[(2,2,2-trichloroethoxy)carbonyl]amino}thiophene-3-carboxylic acid

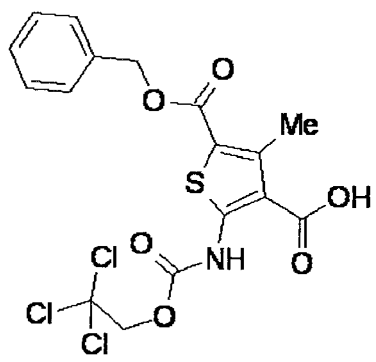

To 2-benzyl 4-tert-butyl 3-methyl-5-{[(2,2,2-trichloroethoxy)carbonyl]amino}thiophene-2,4-dicarboxylate (300 mg, 0.575 mmol) obtained in Reference Example 71 was added trifluoroacetic acid (3 mL), and the mixture was concentrated under reduced pressure 2 hr later. The residue was triturated with water to give the title compound (228 mg, yield 85%).
$^1$H NMR ($CDCl_3$) δ2.83 (3H, s), 4.90 (2H, s), 5.32 (2H, s), 7.26-7.50 (5H, m).

Reference Example 73

2-benzyl 4-tert-butyl 5-(acetylamino)-3-methylthiophene-2,4-dicarboxylate
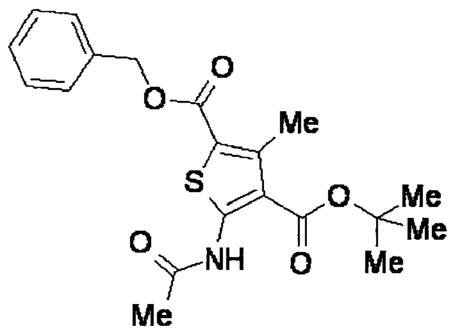

To a solution of 2-benzyl 4-tert-butyl 5-amino-3-methylthiophene-2,4-dicarboxylate (24.8 g, 71.5 mmol) in pyridine (150 mL) was added acetyl chloride (5.10 mL, 71.5 mmol), and the mixture was stirred at room temperature for 2.5 hr. The reaction mixture was concentrated to dryness, and the residue was subjected to basic silica gel column chromatography and triturated with diisopropyl ether-hexane to give the title compound (12.6 g, yield 45%).
EI(pos) 390.5 [M+H]$^+$

Reference Example 74

2-(acetylamino)-5-[(benzyloxy)carbonyl]-4-methylthiophene-3-carboxylic acid
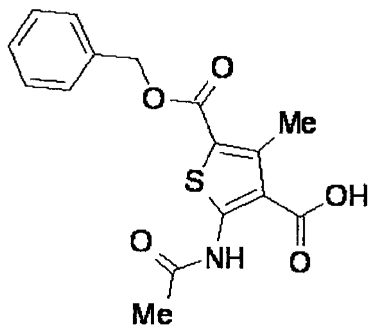

The title compound (2.72 g, quantitative) was obtained in the same manner as in Reference Example 72 and from 2-benzyl 4-tert-butyl 5-(acetylamino)-3-methylthiophene-2,4-dicarboxylate (3.00 g, 7.70 mmol) obtained in Reference Example 73 and trifluoroacetic acid (10 mL). The compound was used for the next step without purification.

Reference Example 75

tert-butyl 4-(cyanomethyl)-4-(prop-2-en-1-yl)piperidine-1-carboxylate

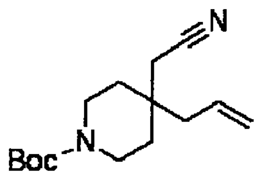

To a solution of tert-butyl 4-(hydroxymethyl)-4-(prop-2-en-1-yl)piperidine-1-carboxylate (2.00 g, 7.84 mmol) in THF (20 mL) were added triethylamine (2.2 mL, 15.7 mmol) and methanesulfonyl chloride (0.91 mL, 11.8 mmol) under ice-cooling, and the mixture was stirred at room temperature for 1 hr. The reaction mixture was diluted with ethyl acetate, and the mixture was washed with saturated aqueous sodium hydrogen carbonate solution, and dried over anhydrous sodium sulfate. The solvent was evaporated under reduced pressure, potassium cyanide (1.54 g, 23.5 mmol) and DMF (20 mL) were added to the obtained residue, and the mixture was stirred with heating at 110°C for 2 days. The reaction mixture was diluted with ethyl acetate, and the mixture was washed with saturated brine, and dried over anhydrous sodium sulfate. The solvent was evaporated under reduced pressure, and the obtained residue was purified by silica gel column chromatography (ethyl acetate:hexane=1:9 to 1:1) to give the title compound (1.22 g, yield 59%) as an oil.
$^1$H NMR ($CDCl_3$) δ1.46 (9H, s), 1.51-1.60 (4H, m), 2.27 (2H, d, J = 7.5 Hz), 2.34 (2H, s), 3.40-3.44 (4H, m), 5.18-5.23 (2H, m), 5.67-5.81 (1H, m).

Reference Example 76

tert-butyl 4-(2-methoxy-2-oxoethyl)-4-(prop-2-en-1-yl)piperidine-1-carboxylate

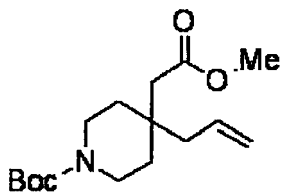

To a solution of tert-butyl 4-(cyanomethyl)-4-(prop-2-en-1-yl)piperidine-1-carboxylate (2.45 g, 9.27 mmol) obtained in Reference Example 75 in ethanol (30 mL) was added 8N aqueous sodium hydroxide solution (10 mL), and the mixture was refluxed by heating for 3 days. The reaction mixture was acidified with 1N hydrochloric acid, and extracted with ethyl acetate. The extract was washed with saturated brine, and dried over anhydrous sodium sulfate. The solvent was evaporated under reduced pressure. To a solution of the obtained residue in DMF (20 mL) were added potassium

carbonate (1.92 g, 13.9 mmol) and methyl iodide (0.70 mL, 11.2 mmol), and the mixture was stirred at room temperature for 15 min. The reaction mixture was diluted with ethyl acetate, and the mixture was washed with saturated brine, and dried over anhydrous sodium sulfate. The solvent was evaporated under reduced pressure and the obtained residue was purified by silica gel column chromatography (ethyl acetate:hexane=1:9 to 2:3) to give the title compound (1.01 g, yield 37%) as an oil. $^1H$ NMR ($CDCl_3$) δ1.45 (9H, s), 1.47-1.52 (4H, m), 2.22 (2H, d, J = 7.6 Hz), 2.33 (2H, s), 3.32-3.50 (4H, m), 3.66 (3H, s), 5.05-5.13 (2H, m), 5.74-5.88 (1H, m).

Reference Example 77

tert-butyl 9-ethyl-8-oxo-3,9-diazaspiro[5.5]undecane-3-carboxylate

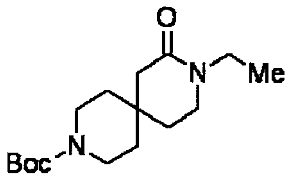

Ozone gas was passed through a solution of tert-butyl 4-(2-methoxy-2-oxoethyl)-4-(prop-2-en-1-yl)piperidine-1-carboxylate (1.06 g, 3.40 mmol) obtained in Reference Example 76 in a mixed solvent of dichloromethane (10 mL) and methanol (20 mL) at -78°C for 10 min. Dimethyl sulfide (0.75 mL) was added and the solvent was evaporated under reduced pressure. To a solution of the obtained residue in THF (5 mL) were added a 2M solution (3.4 mL, 6.80 mmol) of ethylamine in THF and sodium triacetoxyborohydride (1.45 g, 6.80 mmol), and the mixture was stirred at room temperature for 30 min. The reaction mixture was diluted with ethyl acetate, and the mixture was washed with 10% aqueous potassium carbonate solution and saturated brine, and dried over anhydrous sodium sulfate. The solvent was evaporated under reduced pressure and the obtained residue was purified by silica gel column chromatography (methanol:ethyl acetate=0:1 to 1:19) to give the title compound (800 mg, yield 79%) as an oil.
EI(pos) 297 $[M+H]^+$

Reference Example 78

3-ethyl-3,9-diazaspiro[5.5]undecan-2-one hydrochloride

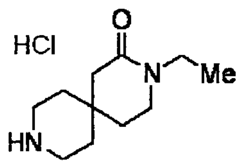

The title compound (628 mg, quantitative) was obtained as a powder in the same manner as in Reference Example 19 and from tert-butyl 9-ethyl-8-oxo-3,9-diazaspiro[5.5]undecane-3-carboxylate (800 mg, 2.70 mmol) obtained in Reference Example 77 and 4N hydrogen chloride-ethyl acetate (10 mL). The compound was used for the next step without purification.

Reference Example 79

tert-butyl 4-(2-ethoxy-2-oxoethyl)-4-[2-(ethylamino)-2-oxoethyl]piperidine-1-carboxylate

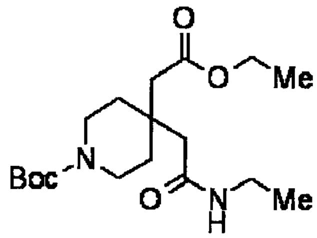

To a solution of tert-butyl 4,4-bis(2-ethoxy-2-oxoethyl)piperidine-1-carboxylate (6.69 g, 18.7 mmol) in THF (40 mL) and methanol (10 mL) was added 1N aqueous sodium hydroxide solution (20 mL, 20.0 mmol) and the mixture was stirred for 3 days. The reaction mixture was acidified with 1N hydrochloric acid, and extracted with ethyl acetate. The extract was washed with saturated brine, and dried over anhydrous sodium sulfate. The solvent was evaporated under reduced pressure to give 6.15 g of a crude product of [1-(tert-butoxycarbonyl)-4-(2-ethoxy-2-oxoethyl)piperidin-4-yl] acetic acid as an oil. To a solution of the crude product (2.00 g, 6.08 mmol), a 2M solution (9.2 mL, 18.2 mmol) of ethylamine in THF and 1-hydroxybenzotriazole (1.22 g, 9.12 mmol) in DMF (5 mL) was added 1-ethyl-3-(3'-dimethylaminopropyl)carbodiimide hydrochloride (1.95 g, 9.12 mmol), and the mixture was stirred at room temperature for 15 hr. The reaction mixture was diluted with ethyl acetate, and the mixture was washed 3 times with saturated brine, and dried over anhydrous sodium sulfate. The solvent was evaporated under reduced pressure and the obtained residue was purified by silica gel column chromatography (hexane:ethyl acetate=3:2 to 0:1) to give the title compound (650 mg, yield 30%) as an oil.
EI(pos) 301 $[M-tBu]^+$

Reference Example 80

ethyl {4-[2-(ethylamino)-2-oxoethyl]piperidin-4-yl}acetate hydrochloride
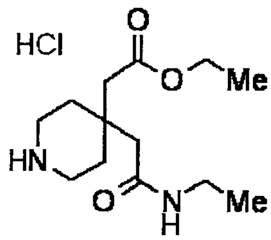

The title compound (644 mg, quantitative) was obtained as a powder in the same manner as in Reference Example 19 and from tert-butyl 4-(2-ethoxy-2-oxoethyl)-4-[2-(ethylamino)-2-oxoethyl]piperidine-1-carboxylate (784 mg, 2.20 mmol) obtained in Reference Example 79 and 4N hydrogen chloride-ethyl acetate (10 mL). The compound was used for the next step without purification.

Reference Example 81

ethyl {1-[(2-amino-1-benzothiophen-3-yl)carbonyl]-4-[2-(ethylamino)-2-oxoethyl]piperidin-4-yl}acetate
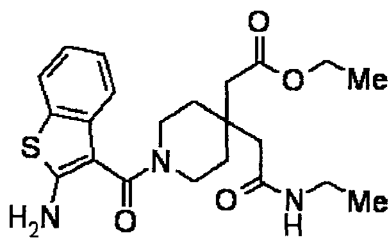

The title compound (700 mg, yield 73%) was obtained as an oil in the same manner as in Reference Example 40 and from ethyl {4-[2-(ethylamino)-2-oxoethyl]piperidin-4-yl}acetate hydrochloride (644 mg, 2.20 mmol) obtained in Reference Example 80 and 2-amino-1-benzothiophene-3-carboxylic acid (425 mg, 2.20 mmol, produced by the method described in WO07/013691).
EI(pos) 432 $[M+H]^+$

Reference Example 82

ethyl {4-[2-(ethylamino)-2-oxoethyl]-1-({2-[(ethylcarbamoyl)amino]-1-benzothiophen-3-yl}carbonyl)piperidin-4-yl}acetate
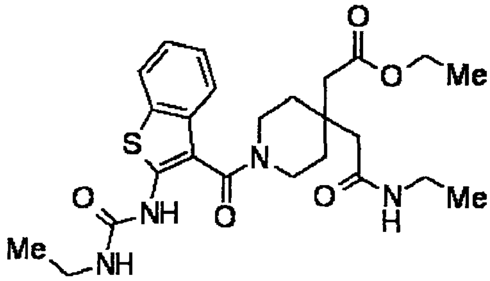

The title compound (673 mg, yield 82%) was obtained as an oil in the same manner as in Reference Example 41 and from ethyl {1-[(2-amino-1-benzothiophen-3-yl)carbonyl]-4-[2-(ethylamino)-2-oxoethyl]piperidin-4-yl}acetate (700 mg, 1.63 mmol) obtained in Reference Example 81 and ethyl isocyanate (0.385 mL, 4.87 mmol).
EI(pos) 503 $[M+H]^+$

Reference Example 83

tert-butyl 4-(2-ethoxy-2-oxoethyl)-4-{2-[(1-methylethyl)amino]-2-oxoethyl}piperidine-1-carboxylate
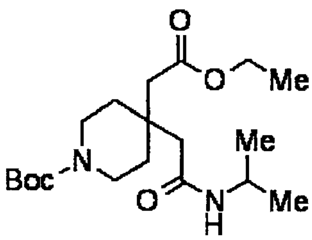

The title compound (3.13 g, yield 60%) was obtained as an oil in the same manner as in Reference Example 79 and from the crude product (4.16 g, 14.0 mmol) of [1-(tert-butoxycarbonyl)-4-(2-ethoxy-2-oxoethyl)piperidin-4-yl] acetic acid obtained in the step of Reference Example 79.
$^1H$ NMR ($CDCl_3$) δ1.14 (6H, d, J = 6.8 Hz), 1.28 (3H, t, J = 7.4 Hz), 1.45 (9H, s), 1.48-1.64 (4H, m), 2.31 (2H, s), 2.43 (2H, s), 3.39-3.50 (4H, m), 4.00-4.10 (1H, m), 4.13-4.20 (2H, m), 6.52 (1H, d, J = 8.0 Hz).
EI(pos) 371 $[M+H]^+$

Reference Example 84

ethyl (4-{2-[(1-methylethyl)amino]-2-oxoethyl}piperidin-4-yl)acetate hydrochloride
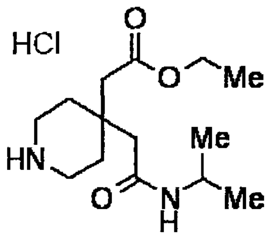

The title compound (697 mg, quantitative) was obtained as a powder in the same manner as in Reference Example 19 and from tert-butyl 4-(2-ethoxy-2-oxoethyl)-4-{2-[(1-methylethyl)amino]-2-oxoethyl}piperidine-1-carboxylate (840 mg, 2.27 mmol) obtained in Reference Example 83 and 4N hydrogen chloride-ethyl acetate (10 mL). The compound was used for the next step without purification.

Reference Example 85

ethyl (1-[(2-amino-1-benzothiophen-3-yl)carbonyl]-4-{2-[(1-methylethyl)amino]-2-oxoethyl}piperidin-4-yl)acetate

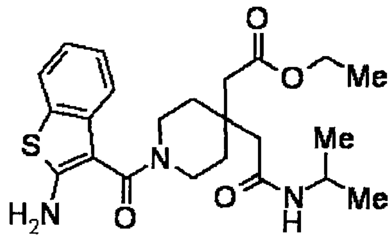

The title compound (510 mg, yield 50%) was obtained as a powder in the same manner as in Reference Example 40 and from ethyl (4-{2-[(1-methylethyl)amino]-2-oxoethyl}piperidin-4-yl)acetate hydrochloride (697 mg, 2.27 mmol) obtained in Reference Example 84 and 2-amino-1-benzothiophene-3-carboxylic acid (439 mg, 2.27 mmol, produced by the method described in WO07/013691)
EI(pos) 446 $[M+H]^+$

Reference Example 86

ethyl [1-({2-[(ethylcarbamoyl)amino]-1-benzothiophen-3-yl}carbonyl)-4-{2-[(1-methylethyl)amino]-2-oxoethyl}piperidin-4-yl]acetate

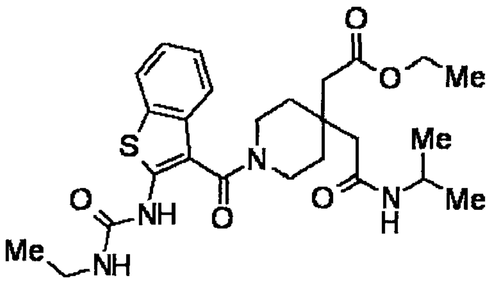

The title compound (550 mg, yield 92%) was obtained as a powder in the same manner as in Reference Example 41 and from ethyl (1-[(2-amino-1-benzothiophen-3-yl)carbonyl]-4-{2-[(1-methylethyl)amino]-2-oxoethyl}piperidin-4-yl)acetate (510 mg, 1.15 mmol) obtained in Reference Example 85 and ethyl isocyanate (0.181 mL, 2.29 mmol).

EI(pos) 517 [M+H]+

Reference Example 87

tert-butyl 4-(2-ethoxy-2-oxoethyl)-4-{2-[(2-methylpropyl)amino]-2-oxoethyl}piperidine-1-carboxylate

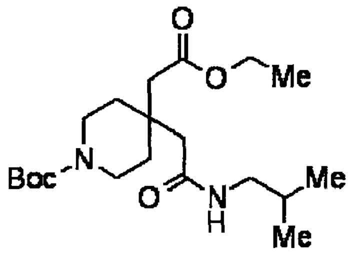

The title compound (700 mg, yield 38%) was obtained as an oil in the same manner as in Reference Example 79 and from the crude product (1.54 g, 4.68 mmol) of [1-(tert-butoxycarbonyl)-4-(2-ethoxy-2-oxoethyl)piperidin-4-yl] acetic acid obtained in the step of Reference Example 79.
EI(pos) 329 [M-tBu]+

Reference Example 88

ethyl (4-{2-[(2-methylpropyl)amino]-2-oxoethyl}piperidin-4-yl)acetate hydrochloride

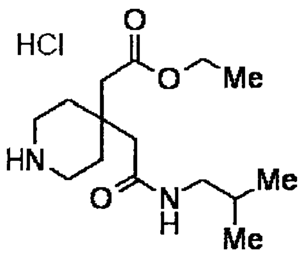

The title compound (584 mg, quantitative) was obtained as a powder in the same manner as in Reference Example 19 and from tert-butyl 4-(2-ethoxy-2-oxoethyl)-4-{2-[(2-methylpropyl)amino]-2-oxoethyl}piperidine-1-carboxylate (700 mg, 1.82 mmol) obtained in Reference Example 87 and 4N hydrogen chloride-dioxane (10 mL). The compound was used for the next step without purification.

Reference Example 89

ethyl (1-[(2-amino-1-benzothiophen-3-yl)carbonyl]-4-{2-[(2-methylpropyl)amino]-2-oxoethyl}piperidin-4-yl)acetate

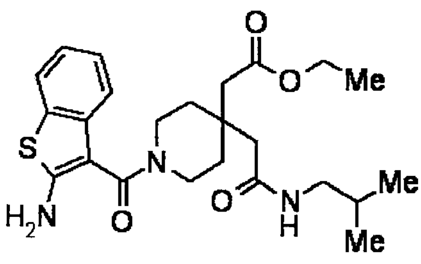

The title compound (746 mg, yield 89%) was obtained as an oil in the same manner as in Reference Example 40 and from ethyl (4-{2-[(2-methylpropyl)amino]-2-oxoethyl}piperidin-4-yl)acetate hydrochloride (584 mg, 1.82 mmol) obtained in Reference Example 88 and 2-amino-1-benzothiophene-3-carboxylic acid (352 mg, 1.82 mmol, produced by the method described in WO07/013691).
EI(pos) 460 $[M+H]^+$

Reference Example 90

ethyl [1-({2-[(ethylcarbamoyl)amino]-1-benzothiophen-3-yl}carbonyl)-4-{2-[(2-methylpropyl)amino]-2-oxoethyl}piperidin-4-yl]acetate
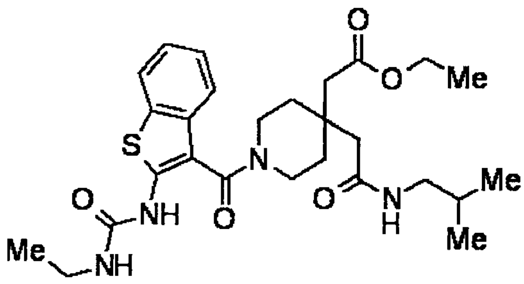

The title compound (765 mg, yield 88%) was obtained as an oil in the same manner as in Reference Example 41 and from ethyl (1-[(2-amino-1-benzothiophen-3-yl)carbonyl]-4-{2-[(2-methylpropyl)amino]-2-oxoethyl}piperidin-4-yl) acetate (746 mg, 1.63 mmol) obtained in Reference Example 89 and ethyl isocyanate (0.386 mL, 4.87 mmol).
EI(pos) 531 $[M+H]^+$

Reference Example 91

spiro[azepane-4,2'-chromen]-4'(3'H)-one hydrochloride
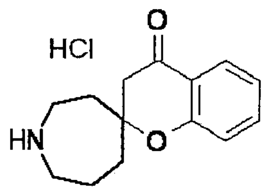

The title compound (1.04 g, yield 88%) was obtained as a powder in the same manner as in Reference Example 19 and from tert-butyl 4'-oxo-3',4'-dihydro-1H-spiro[azepane-4,2'-chromene]-1-carboxylate (1.46 g, 4.41 mmol) and 4N hydrogen chloride-ethyl acetate (20 mL). The compound was used for the next step without purification.

Reference Example 92

spiro[1,3-benzoxazin-2,4'-piperidin]-4(3H)-one
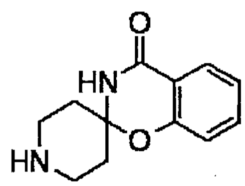

A solution of salicylamide (7.24 g, 52.8 mmol), benzyl 4-oxopiperidine-1-carboxylate (10.0 g, 52.8 mmol) and p-toluenesulfonic acid monohydrate (500 mg, 2.63 mmol) in toluene (100 mL) was heated under reflux for 16 hr while dehydrating using a Dean-Stark trap. The reaction mixture was cooled, and the resulting crystals were collected by filtration, and washed successively with water, ethanol and diethyl ether to give benzyl 4-oxo-3,4-dihydro-1'H-spiro[1,3-benzoxazin-2,4'-piperidine]-1'-carboxylate (9.70 g, yield 60%). THF (50 mL) and ethanol (50 mL) were added to the obtained intermediate (5.00 g, 16.2 mmol) and 10% palladium carbon (50% water-containing product, 1.0 g), and the mixture was stirred under a hydrogen atmosphere at 50°C for 5 hr. The reaction mixture was filtered, and the filtrate was concentrated under reduced pressure to give the title compound (2.71 g, yield 77%) as colorless crystals.
$^1$H NMR (DMSO-$d_6$) δ1.61-1.70 (2H, s), 1.87-1.1.91 (2H, m), 2.03 (1H, br), 2.68-2.74 (4H, m), 6.99-7.09 (2H, m), 7.46-7.52 (1H, m), 7.71-7.74 (1H, m), 8.68 (1H, s).

Reference Example 93

spiro[piperidine-4,2'-thiochromen]-4'(3'H)-one hydrochloride
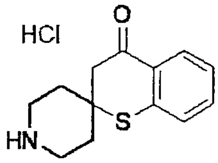

The title compound (477 mg, quantitative) was obtained as a powder in the same manner as in Reference Example 19 and from tert-butyl 4'-oxo-3',4'-dihydro-1H-spiro[piperidine-4,2'-thiochromene]-1-carboxylate (590 mg, 1.77 mmol) and 4N hydrogen chloride-ethyl acetate (10 mL). The compound was used for the next step without purification.

Reference Example 94

tert-butyl 4'-oxo-3',4'-dihydro-1H-spiro[piperidine-4,2'-thiochromene]-1-carboxylate 1'-oxide
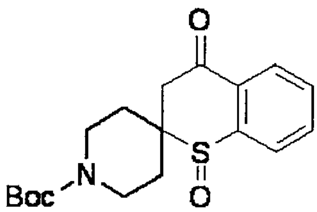

To a solution of tert-butyl 4'-oxo-3',4'-dihydro-1H-spiro[piperidine-4,2'-thiochromene]-1-carboxylate (1.00 g, 3.00 mmol) in dichloromethane (20 mL) was added 70% m-chloroperbenzoic acid (1.04 g, 4.21 mmol), and the mixture was stirred at room temperature for 30 min. The reaction mixture was evaporated under reduced pressure, and the obtained residue was purified by silica gel column chromatography (ethyl acetate:hexane=2:3 to 1:0) to give the title compound (1.00 g, yield 95%) as a powder.
$^1$H NMR ($CDCl_3$) δ1.45 (9H, s), 1.61-1.71 (2H, m), 1.95-2.04 (1H, m), 2.09-2.19 (1H, m), 2.73 (1H, d, J = 17.4 Hz), 3.21-3.31 (2H, m), 3.52 (1H, d, J = 17.4 Hz), 3.77-3.93 (2H, m), 7.65-7.71 (1H, m), 7.78-7.83 (1H, m), 7.89-7.92 (1H, m), 8.15-8.18 (1H, m).

Reference Example 95

spiro[piperidine-4,2'-thiochromen]-4'(3'H)-one 1'-oxide hydrochloride
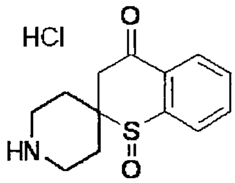

The title compound (411 mg, quantitative) was obtained as a powder in the same manner as in Reference Example 19 and from tert-butyl 4'-oxo-3',4'-dihydro-1H-spiro[piperidine-4,2'-thiochromene]-1-carboxylate 1'-oxide (500 mg, 1.44 mmol) obtained in Reference Example 94 and 4N hydrogen chloride-ethyl acetate (10 mL). The compound was used for the next step without purification.

Reference Example 96

spiro[piperidine-4,2'-thiochromen]-4'(3'H)-one 1',1'-dioxide hydrochloride
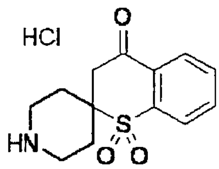

The title compound (800 mg, yield 88%) was obtained as a powder in the same manner as in Reference Example 19 and from tert-butyl 4'-oxo-3',4'-dihydro-1H-spiro[piperidine-4,2'-thiochromene]-1-carboxylate 1',1'-dioxide (1.09 g, 3.00 mmol) and 4N hydrogen chloride-ethyl acetate (10 mL). The compound was used for the next step without purification.

Reference Example 97

O-(2-acetyl-4-bromophenyl)dimethylthiocarbamate
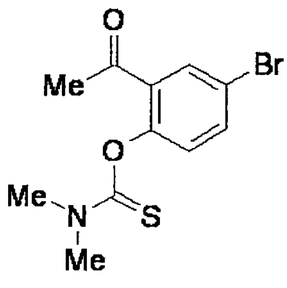

To a solution of 1-(5-bromo-2-hydroxyphenyl)ethanone (2.00 g, 9.30 mmol) and DABCO (2.09 g, 18.6 mmol) in DMF (23 mL) was added dimethylcarbamothioyl chloride (1.72 g, 14.0 mmol), and the mixture was stirred at room temperature for 16 hr. The reaction mixture was diluted with ethyl acetate, and the mixture was washed with 1N hydrochloric acid, 10% aqueous potassium carbonate solution and saturated brine, and dried over anhydrous sodium sulfate. The solvent was evaporated under reduced pressure and the obtained residue was purified by silica gel column chromatography (ethyl acetate:hexane=1:3 to 1:1) to give the title compound (61.3 mg, yield 67%) as an oil.
$^1H$ NMR ($CDCl_3$) δ2.53 (3H, s), 3.39 (3H, s), 3.45 (3H, s), 6.98 (1H, d, J = 9.0 Hz), 6.62 (1H, dd, J = 9.0, 2.7 Hz), 7.88 (1H, d, J = 2.7 Hz).

Reference Example 98

tert-butyl 6'-bromo-4'-oxo-3',4'-dihydro-1H-spiro[piperidine-4,2'-thiochromene]-1-carboxylate

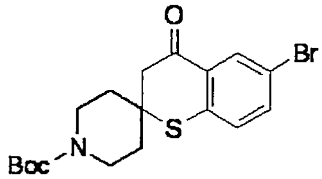

O-(2-Acetyl-4-bromophenyl) dimethylthiocarbamate (2.76 g, 9.13 mmol) obtained in Reference Example 97 was stirred with heating at 190°C for 6 hr, and the mixture was allowed to cool to room temperature. Methanol (5 mL) and 2N aqueous sodium hydroxide solution (5 mL) were added, and the mixture was stirred with heating at 90°C. Ethyl acetate was added to the reaction mixture, and the mixture was washed with 1N hydrochloric acid and saturated brine, and dried over anhydrous sodium sulfate. The solvent was evaporated under reduced pressure and the obtained residue was purified by silica gel column chromatography (ethyl acetate:hexane=1:3) to give 1-(5-bromo-2-sulfanylphenyl)ethanone (10.9 g, yield 52%) as an oil. A solution of the obtained intermediate (1.09 g, 4.71 mmol), tert-butyl 4-oxopiperidine-1-carboxylate (1.13 g, 5.65 mmol) and pyrrolidine (0.787 mL, 9.42 mmol) in methanol (10 mL) was stirred with heating at 90°C for 5 hr. Ethyl acetate was added to the reaction mixture, and the mixture was washed with 0.5N hydrochloric acid, 10% aqueous potassium carbonate solution and saturated brine, and dried over anhydrous sodium sulfate. The solvent was evaporated under reduced pressure and the obtained residue was purified by silica gel column chromatography (ethyl acetate:hexane=1:9 to 1:3) to give the title compound (901 mg, yield 46%) as a powder.
$^1$H NMR ($CDCl_3$) δ1.45 (9H, s), 1.63-1.73 (2H, m), 1.88-1.92 (2H, m), 2.92 (2H, s), 3.27-3.24 (2H, m), 3.84 (2H, m), 7.15 (1H, d, J = 8.1), 7.50 (1H, dd, J = 8.1, 2.7 Hz), 8.19 (1H, d, J = 2.7 Hz).

Reference Example 99

6'-bromospiro[piperidine-4,2'-thiochromen]-4'(3'H)-one hydrochloride

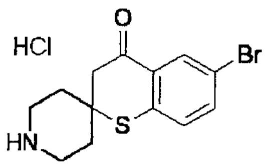

The title compound (606 mg, yield 80%) was obtained as a powder in the same manner as in Reference Example 19 and from tert-butyl 6'-bromo-4'-oxo-3',4'-dihydro-1H-spiro[piperidine-4,2'-thiochromene]-1-carboxylate (900 mg, 2.18 mmol) obtained in Reference Example 98 and 4N hydrogen chloride-ethyl acetate (10 mL). The compound was used for the next step without purification.

Reference Example 100

2-acetyl-4-cyanophenyl trifluoromethanesulfonate

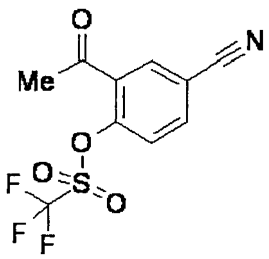

To a solution of 3-acetyl-4-hydroxybenzonitrile (1.00 g, 6.22 mmol) and pyridine (1.0 mL, 12.5 mmol) in dichloromethane (10 mL) was added dropwise trifluoromethanesulfonic anhydride (1.29 mL, 7.47 mmol), and the mixture was stirred at room temperature for 10 min. The solvent was evaporated under reduced pressure and the obtained residue was purified by silica gel column chromatography (ethyl acetate:hexane=1:19 to 2:3) to give the title compound (1.77 g, yield 97%) as a pale-yellow solid.
$^1$H NMR ($CDCl_3$) δ2.68 (3H, s), 7.51 (1H, d, J = 8.5), 7.90 (1H, dd, J = 8.6, 2.2 Hz), 8.11 (1H, d, J = 2.3 Hz).

Reference Example 101

2-ethylhexyl 3-[(2-acetyl-4-cyanophenyl)sulfanyl]propanoate
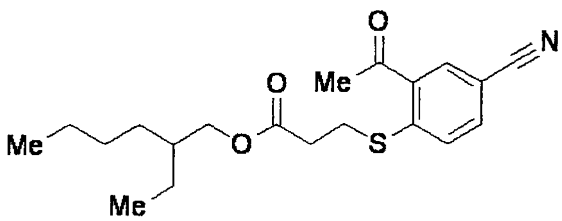

A solution of 2-acetyl-4-cyanophenyl trifluoromethanesulfonate (1.50 g, 5.12 mmol) obtained in Reference Example 100, tris(dibenzylideneacetone)dipalladium (235 mg, 0.256 mmol), 2-ethylhexyl 3-mercaptopropionate (2.36 mL, 10.3 mmol), 4,5-bis(diphenylphosphino)-9,9-dimethylxanthene (Xantphos) (297 mg, 0.512 mmol) and diisopropylethylamine (1.8 mL, 10.3 mmol) in 1,4-dioxane (15 mL) was refluxed by heating for 4.5 hr under an argon atmosphere. The reaction mixture was diluted with water and the mixture was filtered. The filtrate was extracted with ethyl acetate, and the extract was dried over anhydrous sodium sulfate. The solvent was evaporated under reduced pressure and the obtained residue was purified by silica gel column chromatography (ethyl acetate:hexane=1:19 to 3:7) to give the title compound (1.78 g, yield 96%) as an oil.
$^1$H NMR ($CDCl_3$) δ0.89 (3H, t, J = 7.4 Hz), 1.24-1.31 (7H, m), 2.64 (3H, 2), 2.72 (2H, t, J = 7.6 Hz), 3.22 (2H, d, J = 7.6 Hz), 4.04 (2H, dd, J = 5.8, 1.3 Hz), 7.47 (1H, d, J = 8.7), 7.69 (1H, dd, J = 8.5, 1.9 Hz), 8.08 (1H, d, J = 1.9 Hz).

Reference Example 102

tert-butyl 6'-cyano-4'-oxo-3',4'-dihydro-1H-spiro[piperidine-4,2'-thiochromene]-1-carboxylate
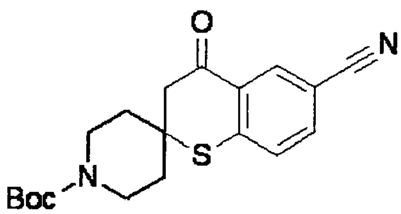

To a solution of 2-ethylhexyl 3-[(2-acetyl-4-cyanophenyl)sulfanyl]propanoate (1.78 g, 4.93 mmol) obtained in Reference Example 101 in THF-EtOH (20 mL-3.5 mL) was added sodium ethoxide (0.67 g, 9.85 mmol), and the mixture

was stirred at room temperature for 15 min. Saturated aqueous ammonium chloride solution was added to the reaction mixture, and the mixture was extracted with ethyl acetate. The extract was dried over anhydrous sodium sulfate, and the solvent was evaporated under reduced pressure. To a solution of the obtained intermediate in methanol (15 mL) were added tert-butyl 4-oxopiperidine-1-carboxylate (751 mg, 4.93 mmol) and pyrrolidine (0.82 mL, 9.86 mmol), and the mixture was stirred with heating at 50°C for 30 min. The solvent was evaporated under reduced pressure, and the residue was diluted with ethyl acetate, and the mixture was washed with 0.1N hydrochloric acid and saturated brine and dried over anhydrous sodium sulfate. The solvent was evaporated under reduced pressure, and the obtained residue was purified by basic silica gel column chromatography (ethyl acetate:hexane=1:9 to 3:7) to give the title compound (0.77 g, yield 44%) as a yellow solid.
$^1$H NMR ($CDCl_3$) δ1.45 (9H, s), 1.67-1.77 (2H, m), 1.86-1.96 (2H, m), 2.97 (2H, s), 3.16-3.28 (2H, m), 3.82-3.94 (2H, m), 7.38 (1H, d, J = 8.3), 7.61 (1H, dd, J = 8.3, 1.9 Hz), 8.34 (1H, d, J = 1.8 Hz).

Reference Example 103

4'-oxo-3',4'-dihydrospiro[piperidine-4,2'-thiochromene]-6'-carbonitrile hydrochloride
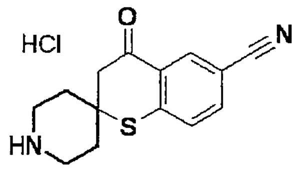

The title compound (2.47 g, quantitative) was obtained as a powder in the same manner as in Reference Example 19 and from tert-butyl 6'-cyano-4'-oxo-3',4'-dihydro-1H-spiro[piperidine-4,2'-thiochromene]-1-carboxylate (3.00 g, 8.37 mmol) obtained in Reference Example 102 and 4N hydrogen chloride-ethyl acetate (40 mL). The compound was used for the next step without purification.

Reference Example 104

tert-butyl 4'-{[tert-butyl(dimethyl)silyl]oxy}-6'-cyano-1H-spiro[piperidine-4,2'-thiochromene]-1-carboxylate
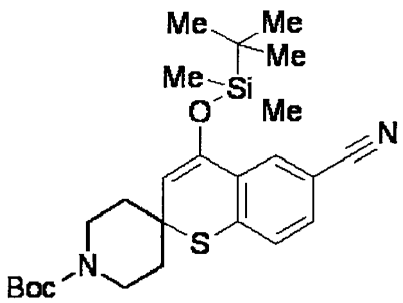

To a solution of tert-butyl 6'-cyano-4'-oxo-3',4'-dihydro-1H-spiro[piperidine-4,2'-thiochromene]-1-carboxylate (5.00 g, 13.9 mmol) obtained in Reference Example 102 in THF (60 mL) was added dropwise a 1.1M solution (15.2 mL, 16.7 mmol) of LHDMS in THF at -78°C, and 30 min later, a solution of tert-butyl(dimethyl)silyl trifluoromethanesulfonate (3.84 mL, 16.7 mmol) in THF (2 mL) was added dropwise, and the mixture was warmed to room temperature, and stirred for 30 min. 10% Aqueous potassium carbonate solution was added to the reaction mixture, and the mixture was extracted with ethyl acetate. The extract was washed with 10% aqueous potassium carbonate solution and saturated brine, and dried over anhydrous sodium sulfate. The solvent was evaporated under reduced pressure, and the obtained residue was purified by silica gel column chromatography (ethyl acetate:hexane=1:9 to 1:3) to give the title compound (6.59 g, quantitative) as an oil.
$^1$H NMR ($CDCl_3$) δ0.22 (6H, s), 1.01 (9H, s), 1.45 (9H, s), 1.70-1.79 (2H, m), 1.87-1.92 (2H, m), 3.29-3.38 (2H, m), 3.66-3.71 (2H, m), 5.11 (1H, s), 7.32 (1H, d, J = 8.1 Hz), 7.38 (1H, dd, J = 8.1, 1.8 Hz), 7.78 (1H, d, J = 1.8 Hz).

Reference Example 105

tert-butyl 4'-{[tert-butyl(dimethyl)silyl]oxy}-6'-(hydroxycarbamimidoyl)-1H-spiro[piperidine-4,2'-thiochromene]-1-carboxylate

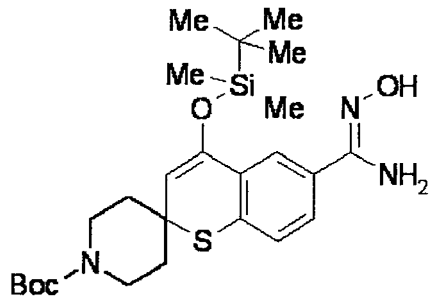

A solution of tert-butyl 4'-{[tert-butyl(dimethyl)silyl]oxy}-6'-cyano-1H-spiro[piperidine-4,2'-thiochromene]-1-carboxylate (6.59 g, 13.9 mmol) obtained in Reference Example 104, hydroxylamine hydrochloride (2.91 g, 41.8 mmol) and triethylamine (5.82 mL, 41.8 mmol) in ethanol (60 mL) was stirred at 50°C for 16 hr. After completion of the reaction, the solvent was evaporated under reduced pressure. Ethyl acetate was added to the residue, and the mixture was washed with saturated brine, and dried over anhydrous magnesium sulfate. The solvent was evaporated under reduced pressure, and the obtained residue was purified by silica gel column chromatography (ethyl acetate:hexane=1:3 to 1:1) to give the title compound (6.90 g, yield 98%).
$^1$H NMR ($CDCl_3$) δ0.21 (6H, s), 1.01 (9H, s), 1.45 (9H, s), 1.68-1.77 (2H, m), 1.87-1.92 (2H, m), 3.30-3.39 (2H, m), 3.65 (2H, m), 4.79 (2H, br), 5.08 (1H, s), 7.25 (1H, d, J = 7.8 Hz), 7.42 (1H, dd, J = 7.8, 2.1 Hz), 7.80 (1H, d, J = 2.1 Hz).

Reference Example 106

tert-butyl 4'-{[tert-butyl(dimethyl)silyl]oxy}-6'-(5-oxo-4,5-dihydro-1,2,4-oxadiazol-3-yl)-1H-spiro[piperidine-4,2'-thiochromene]-1-carboxylate

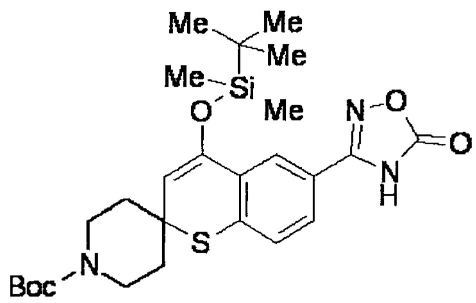

The title compound (4.60 g, yield 63%) was obtained as an oil in the same manner as in Reference Example 62 and from tert-butyl 4'-{[tert-butyl(dimethyl)silyl]oxy}-6'-(hydroxycarbamimidoyl)-1H-spiro[piperidine-4,2'-thiochromene]-1-carboxylate (6.90 g, 13.6 mmol) obtained in Reference Example 105 and carbonyldiimidazole (2.88 g, 17.7 mmol).
$^1$H NMR ($CDCl_3$) δ0.22 (6H, s), 1.01 (9H, s), 1.47 (9H, s), 1.68-1.77 (2H, m), 1.87-1.92 (2H, m), 3.30-3.37 (2H, m), 3.66-3.70 (2H, m), 5.13 (1H, s), 7.38 (1H, d, J = 8.1 Hz), 7.50 (1H, dd, J = 8.1, 2.1 Hz), 7.95 (1H, d, J = 2.1 Hz).

Reference Example 107

tert-butyl 4'-oxo-6'-(5-oxo-4,5-dihydro-1,2,4-oxadiazol-3-yl)-3',4'-dihydro-1H-spiro[piperidine-4,2'-thiochromene]-1-carboxylate

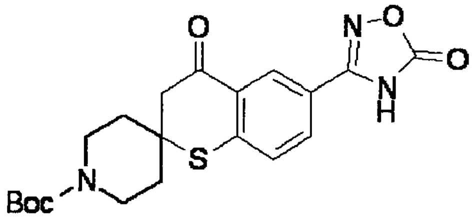

The title compound (2.84 g, yield 79%) was obtained as a powder in the same manner as in Reference Example 63 and from tert-butyl 4'-{[tert-butyl(dimethyl)silyl]oxy}-6'-(5-oxo-4,5-dihydro-1,2,4-oxadiazol-3-yl)-1H-spiro[piperidine-4,2'-thiochromene]-1-carboxylate (4.60 g, 8.65 mmol) obtained in Reference Example 106 and a 1M solution (26.0 mL) of tetrabutylammonium fluoride in THF.
$^1$H NMR ($CDCl_3$) δ1.46 (9H, s), 1.79-1.89 (2H, m), 1.93-1.98 (2H, m), 3.27-3.34 (4H, m), 3.83-3.88 (2H, m), 7.47 (1H, d, J = 8.4 Hz), 8.10 (1H, dd, J = 8.4, 1.8 Hz), 8.85 (1H, d, J = 1.8 Hz).

Reference Example 108

6'-(5-oxo-4,5-dihydro-1,2,4-oxadiazol-3-yl)spiro[piperidine-4,2'-thiochromen]-4'(3'H)-one hydrochloride
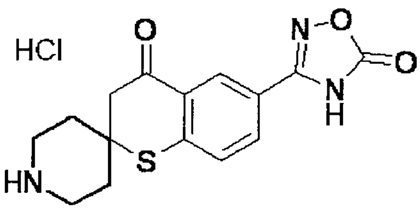

The title compound (2.40 g, quantitative) was obtained as a powder in the same manner as in Reference Example 19 and from tert-butyl 4'-oxo-6'-(5-oxo-4,5-dihydro-1,2,4-oxadiazol-3-yl)-3',4'-dihydro-1H-spiro[piperidine-4,2'-thiochromene]-1-carboxylate (2.83 g, 6.78 mmol) obtained in Reference Example 107 and 4N hydrogen chloride-ethyl acetate (60 mL). The compound was used for the next step without purification.

Reference Example 109

ethyl (1-[(2-amino-7-methoxy-1-benzothiophen-3-yl)carbonyl]-4-{2-[(1-methylethyl)amino]-2-oxoethyl}piperidin-4-yl)acetate
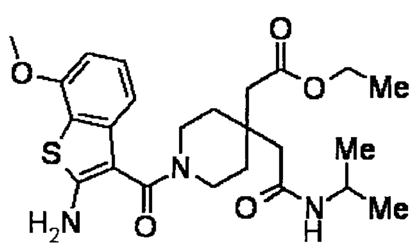

The title compound (710 mg, yield 86%) was obtained as a powder in the same manner as in Reference Example 40 and from ethyl (4-{2-[(1-methylethyl)amino]-2-oxoethyl}piperidin-4-yl)acetate hydrochloride (531 mg, 1.73 mmol) obtained in Reference Example 84 and 2-amino-7-methoxy-1-benzothiophene-3-carboxylic acid (386 mg, 1.73 mmol, produced by the method described in WO07/119833).
EI(pos) 476 [M+H]$^+$

Reference Example 110

ethyl [1-({2-[(ethylcarbamoyl)amino]-7-methoxy-1-benzothiophen-3-yl}carbonyl)-4-{2-[(1-methylethyl)amino]-2-oxoethyl}piperidin-4-yl]acetate

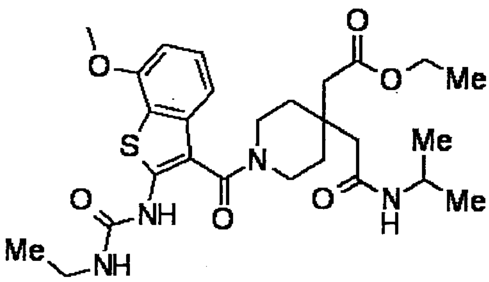

The title compound (814 mg, quantitative) was obtained as an oil in the same manner as in Reference Example 41 and from ethyl (1-[(2-amino-7-methoxy-1-benzothiophen-3-yl)carbonyl]-4-{2-[(1-methylethyl)amino]-2-oxoethyl}piperidin-4-yl)acetate (710 mg, 1.49 mmol) obtained in Reference Example 109 and ethyl isocyanate (0.355 mL, 4.48 mmol).
EI(pos) 547 $[M+H]^+$

Reference Example 111

tert-butyl 3-ethyl-2,4-dioxo-1,3,8-triazaspiro[4.5]decane-8-carboxylate

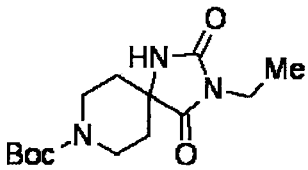

The title compound (2.52 g, yield 75%) was obtained as a powder in the same manner as in Reference Example 26 and from tert-butyl 2,4-dioxo-1,3,8-triazaspiro[4.5]decane-8-carboxylate (3.03 g, 11.3 mmol) and ethyl iodide (1.35 mL, 16.9 mmol).
$^1$H NMR ($CDCl_3$) δ1.07 (3H, t, J = 7.2 Hz), 1.41 (9H, s), 1.45-1.57 (2H, m), 1.66-1.75 (2H, m), 3.02-3.23 (2H, m), 3.37 (2H, q, J = 7.2 Hz), 8.79 (1H, br).

Reference Example 112

3-ethyl-1,3,8-triazaspiro[4.5]decane-2,4-dione hydrochloride

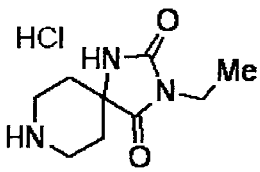

The title compound (1.94 g, quantitative) was obtained as a powder in the same manner as in Reference Example 19 and from tert-butyl 3-ethyl-2,4-dioxo-1,3,8-triazaspiro[4.5]decane-8-carboxylate (2.47 g, 8.31 mmol) obtained in Reference Example 111 and 4N hydrogen chloride-ethyl acetate (30 mL). The compound was used for the next step without purification.

Reference Example 113

tert-butyl 6-(1-methyl-1H-pyrazol-4-yl)-4-oxo-3,4-dihydro-1'H-spiro[chromene-2,4'-piperidine]-1'-carboxylate
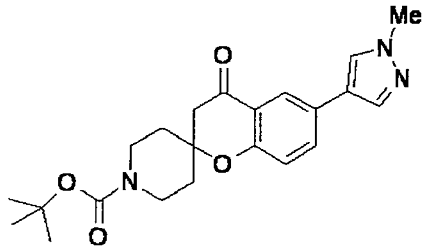

To a solution of tert-butyl 6-bromo-4-oxo-3,4-dihydro-1'H-spiro[chromene-2,4'-piperidine]-1'-carboxylate (800 mg, 2.02 mmol) and 1-methyl-4-(4,4,5,5-tetramethyl-1,3,2-dioxaborolan-2-yl)-1H-pyrazole (841 mg, 4.04 mmol) in a mixed solvent of THF (15 mL)-2N aqueous sodium carbonate solution (2 mL) was added tetrakistriphenylphosphine palladium (117 mg, 0.101 mmol) under a nitrogen atmosphere and the mixture was stirred with heating at 80°C for 15 hr. After completion of the reaction, the reaction mixture was diluted with ethyl acetate, and the mixture was washed with saturated brine, and dried over anhydrous magnesium sulfate. The solvent was evaporated under reduced pressure, and the obtained residue was purified by silica gel column chromatography (ethyl acetate:hexane=1:1 to 1:0) to give the title compound (802 mg, quantitative) as an oil.
EI(pos) 398 $[M+H]^+$

Reference Example 114

6-(1-methyl-1H-pyrazol-4-yl)spiro[chromene-2,4'-piperidin]-4(3H)-one hydrochloride
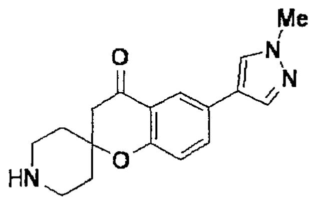

HCl

The title compound (720 mg, yield 96%) was obtained as a powder in the same manner as in Reference Example 19 and from tert-butyl 6-(1-methyl-1H-pyrazol-4-yl)-4-oxo-3,4-dihydro-1'H-spiro[chromene-2,4'-piperidine]-1'-carboxylate (893 mg, 2.25 mmol) obtained in Reference Example 113 and 4N hydrogen chloride-ethyl acetate (10 mL). The compound was used for the next step without purification.

Reference Example 115

dimethyl 6-methoxy-1-benzothiophene-2,3-dicarboxylate
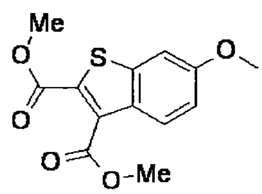

A mixture of 6-methoxy-1-benzothiophene-2,3-dione (7.62 g, 39.3 mmol) and chloroacetic acid (4.09 g, 43.2 mmol) and 2N aqueous sodium hydrogen carbonate solution (71 mL) was heated at 65°C for 3 hr. Then, 8N aqueous sodium hydroxide solution (40 mL) was added to the reaction mixture, and the mixture was heated at 100°C for 3.5 hr. The reaction mixture was acidified with 6N hydrochloric acid and the precipitated solid was collected by filtration and washed with water. A solution of the solid and sulfuric acid (8 mL) in methanol (150 mL) was heated under reflux for 4.5 hr. Water was added to the reaction mixture, and the mixture was extracted with ethyl acetate. The extract was washed with saturated brine, and dried over anhydrous magnesium sulfate. The solvent was evaporated under reduced pressure, and the obtained solid was washed with diethyl ether to give the title compound (8.19 g, yield 62%).
$^1$H NMR ($CDCl_3$) δ3.90 (3H, s), 3.93 (3H, s), 4.01 (3H, s), 7.07 (1H, dd, J = 9.0, 2.3 Hz), 7.26 (1H, s), 7.77 (1H, d, J = 8.9 Hz).

Reference Example 116

6-methoxy-3-(methoxycarbonyl)-1-benzothiophene-2-carboxylic acid
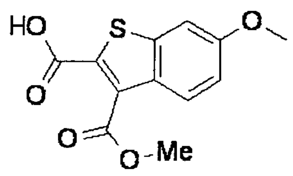

To a solution of dimethyl 6-methoxy-1-benzothiophene-2,3-dicarboxylate (8.19 g, 29.3 mmol) obtained in Reference Example 115 in a mixed solvent of THF (80 mL) and methanol (10 mL) was added 1N aqueous sodium hydroxide solution (40 mL) at room temperature, and the mixture was stirred for 1 hr. After completion of the reaction, the reaction mixture was acidified with 6N hydrochloric acid, and extracted with ethyl acetate. The extract was washed with saturated brine, and dried over anhydrous magnesium sulfate. The solvent was evaporated under reduced pressure to give the title compound (7.80 g, quantitative) as a powder. The compound was used for the next step without purification.

Reference Example 117

methyl 2-[(tert-butoxycarbonyl)amino]-6-methoxy-1-benzothiophene-3-carboxylate
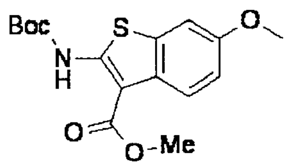

The title compound (7.19 g, yield 73%) was obtained as a powder in the same manner as in Reference Example 4 and from 6-methoxy-3-(methoxycarbonyl)-1-benzothiophene-2-carboxylic acid (7.80 g, 29.3 mmol) obtained in Reference Example 116 and diphenylphosphoryl azide (5.7 mL, 26.7 mmol).
$^1$H NMR ($CDCl_3$) δ1.56 (9H, s), 3.83 (3H, s), 3.99 (3H, s), 7.00 (1H, dd, J = 9.0, 2.5 Hz), 7.18 (1H, d, J = 2.5 Hz), 8.10 (1H, dd, J = 9.0 Hz), 10.65 (1H, br).

Reference Example 118

2-[(tert-butoxycarbonyl)amino]-6-methoxy-1-benzothiophene-3-carboxylic acid
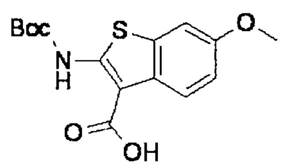

The title compound (1.22 g, yield 18%) was obtained as a powder in the same manner as in Reference Example 3 and from methyl 2-[(tert-butoxycarbonyl)amino]-6-methoxy-1-benzothiophene-3-carboxylate (7.19 g, 21.3 mmol) obtained in Reference Example 117. The compound was used for the next step without purification.

Reference Example 119

2-acetyl-4-chlorophenyl trifluoromethanesulfonate

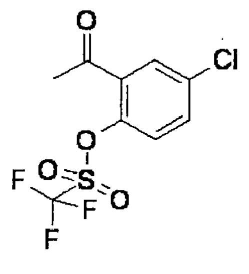

The title compound (11.1 g, quantitative) was obtained as an oil in the same manner as in Reference Example 100 and from 1-(5-chloro-2-hydroxyphenyl)ethanone (6.00 g, 35.2 mmol) and trifluoromethanesulfonic anhydride (7.1 mL, 42.2 mmol). EI(pos) 302.9 $[M+H]^+$

Reference Example 120

tert-butyl 6'-chloro-4'-oxo-3',4'-dihydro-1H-spiro[piperidine-4,2'-thiochromene]-1-carboxylate

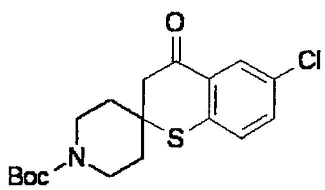

A solution of 2-acetyl-4-chlorophenyl trifluoromethanesulfonate (11.1 g, 35.2 mmol) obtained in Reference Example 119, tris(dibenzylideneacetone)dipalladium (805 mg, 0.880 mmol), (4-methoxyphenyl)methanethiol (5.42 g, 35.2 mmol), 4,5-bis(diphenylphosphino)-9,9-dimethylxanthene (Xantphos) (1.02 g, 1.76 mmol) and diisopropylethylamine (9.08 g, 70.4 mmol) in 1,4-dioxane (70 mL) was refluxed by heating for 2 hr under an argon atmosphere. The reaction mixture was diluted with water, and filtered. The filtrate was extracted with ethyl acetate, and the extract was dried over anhydrous sodium sulfate. The solvent was evaporated under reduced pressure, and the obtained residue was subjected to silica gel column chromatography, and treated with diisopropyl ether to give 1-{5-chloro-2-[(4-methoxybenzyl)sulfanyl]phenyl}ethanone (10.9 g, quantitative) as a powder. To the obtained powder (10.9 g, 35.2 mmol) was added trifluoroacetic acid (30 mL), and the mixture was stirred with heating at 50°C for 2 hr. The solvent was concentrated under reduced pressure. To a solution of the obtained residue in methanol (77 mL) were added tert-butyl 4-oxopiperidine-1-carboxylate (7.13 g, 35.2 mmol) and pyrrolidine (3.0 mL, 35.2 mmol), and the mixture was stirred with heating at 50°C for 2 hr. The solvent was evaporated under reduced pressure, and the residue was diluted with ethyl acetate. The mixture was washed with 0.1N hydrochloric acid and saturated brine, and dried over anhydrous sodium sulfate. The solvent was evaporated under reduced pressure, and the obtained residue was purified by silica gel column chromatography (ethyl acetate:hexane=0:1 to 1:4) and recrystallized from hexane to give the title compound (1.68 g, yield 13%) as a white solid.
$^1H$ NMR ($CDCl_3$) δ1.45 (9H, s), 1.67-1.77 (2H, m), 1.86-1.96 (2H, m), 2.93 (2H, s), 3.16-3.28 (2H, m), 3.82-3.94 (2H, m), 7.21 (1H, d, J = 8.5 Hz), 7.38 (1H, dd, J = 8.5, 2.6 Hz), 8.05 (1H, d, J = 2.6 Hz).

Reference Example 121

6'-chlorospiro[piperidine-4,2'-thiochromen]-4'(3'H)-one hydrochloride

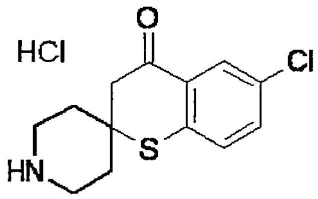

The title compound (729 mg, yield 88%) was obtained as a powder in the same manner as in Reference Example 19 and from tert-butyl 6'-chloro-4'-oxo-3',4'-dihydro-1H-spiro[piperidine-4,2'-thiochromene]-1-carboxylate (1.00 g, 2.72 mmol) obtained in Reference Example 120 and 4N hydrogen chloride-ethyl acetate (8 mL). The compound was used for the next step without purification.

Reference Example 122

2-acetyl-5-chlorophenyl trifluoromethanesulfonate

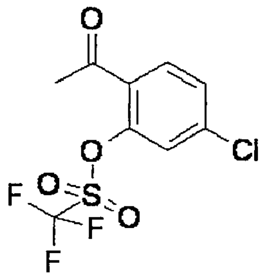

The title compound (8.89 mg, quantitative) was obtained as an oil in the same manner as in Reference Example 100 and from 1-(4-chloro-2-hydroxyphenyl)ethanone (5.00 g, 29.3 mmol) and trifluoromethanesulfonic anhydride (6.0 mL, 35.2 mmol). EI(pos) 302.9 $[M+H]^+$

Reference Example 123

tert-butyl 7'-chloro-4'-oxo-3',4'-dihydro-1H-spiro[piperidine-4,2'-thiochromene]-1-carboxylate

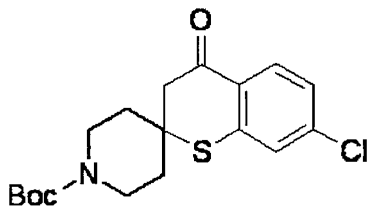

The title compound (4.34 g, yield 41%) was obtained as a powder in the same manner as in Reference Example 120 and from 2-acetyl-5-chlorophenyl trifluoromethanesulfonate (8.89 g, 29.3 mmol) obtained in Reference Example 122. $^1$H NMR ($CDCl_3$) δ1.45 (9H, s), 1.67-1.77 (2H, m), 1.86-1.96 (2H, m), 2.92 (2H, s), 3.16-3.28 (2H, m), 3.82-3.94 (2H, m), 7.15 (1H, dd, J = 8.4, 2.0 Hz), 7.28 (1H, d, J = 2.0 Hz), 8.01 (1H, d, J = 8.4 Hz).

Reference Example 124

7'-chlorospiro[piperidine-4,2'-thiochromen]-4'(3'H)-one hydrochloride

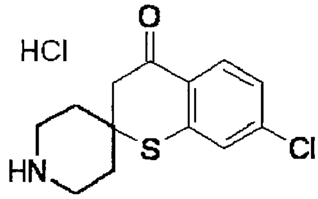

The title compound (1.47 g, yield 89%) was obtained as a powder in the same manner as in Reference Example 19 and from tert-butyl 7'-chloro-4'-oxo-3',4'-dihydro-1H-spiro[piperidine-4,2'-thiochromene]-1-carboxylate (2.00 g, 5.44 mmol) obtained in Reference Example 123 and 4N hydrogen chloride-ethyl acetate (8 mL). The compound was used for the next step without purification.

Reference Example 125

2-acetyl-4-methylphenyl trifluoromethanesulfonate

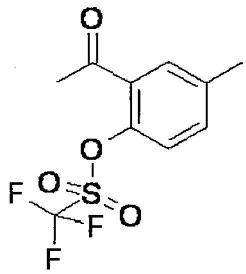

The title compound (8.65 g, yield 92%) was obtained as an oil in the same manner as in Reference Example 100 and from 1-(2-hydroxy-5-methylphenyl)ethanone (5.00 g, 33.3 mmol) and trifluoromethanesulfonic anhydride (6.7 mL, 39.9 mmol). EI(pos) 282.9 $[M+H]^+$

Reference Example 126

tert-butyl 6'-methyl-4'-oxo-3',4'-dihydro-1H-spiro[piperidine-4,2'-thiochromene]-1-carboxylate

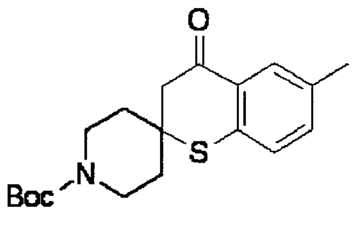

The title compound (587 mg, yield 5%) was obtained as a powder in the same manner as in Reference Example 120 and from 2-acetyl-4-methylphenyl trifluoromethanesulfonate (8.65 g, 30.6 mmol) obtained in Reference Example 125. $^1H$ NMR ($CDCl_3$) $\delta$1.45 (9H, s), 1.67-1.73 (2H, m), 1.86-1.96 (2H, m), 2.33 (3H, s), 2.91 (2H, s), 3.16-3.28 (2H, m), 3.82-3.94 (2H, m), 7.14-34 (1H, m), 7.20-7.26 (1H, m), 7.90 (1H, s).

Reference Example 127

6'-methylspiro[piperidine-4,2'-thiochromen]-4'(3'H)-one hydrochloride

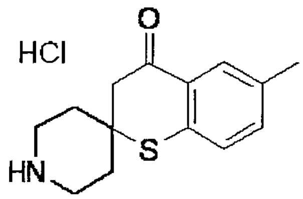

The title compound (337 mg, yield 93%) was obtained as a powder in the same manner as in Reference Example 19 and from tert-butyl 6'-methyl-4'-oxo-3',4'-dihydro-1H-spiro[piperidine-4,2'-thiochromene]-1-carboxylate (587 mg, 1.69 mmol) obtained in Reference Example 126 and 4N hydrogen chloride-ethyl acetate (3 mL). The compound was used for the next step without purification.

Reference Example 128

2-acetyl-4-methoxyphenyl trifluoromethanesulfonate
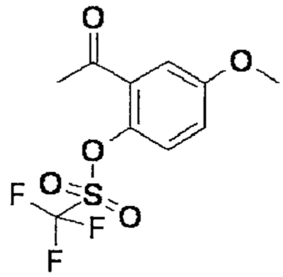

The title compound (9.02 g, quantitative) was obtained as an oil in the same manner as in Reference Example 100 and from 1-(2-hydroxy-5-methoxyphenyl)ethanone (5.00 g, 30.1 mmol) and trifluoromethanesulfonic anhydride (6.1 mL, 36.1 mmol). EI(pos) 298.9 $[M+H]^+$

Reference Example 129

tert-butyl 6'-methoxy-4'-oxo-3',4'-dihydro-1H-spiro[piperidine-4,2'-thiochromene]-1-carboxylate
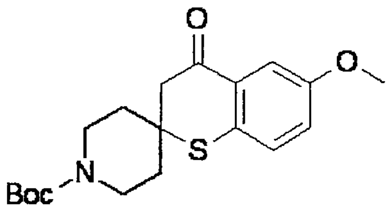

The title compound (193 mg, yield 2%) was obtained as a powder in the same manner as in Reference Example 120 and from 2-acetyl-4-methoxyphenyl trifluoromethanesulfonate (9.02 g, 30.1 mmol) obtained in Reference Example 128.
$^1$H NMR ($CDCl_3$) δ1.45 (9H, s), 1.67-1.77 (2H, m), 1.86-1.96 (2H, m), 2.92 (2H, s), 3.35-3.40 (2H, m), 3.67-3.95 (5H, m), 7.04 (1H, dd, J = 8.8, 2.9 Hz)), 7.18 (1H, d, J = 8.67 Hz), 7.59 (1H, d, J = 2.9 Hz).

Reference Example 130

6'-methoxyspiro[piperidine-4,2'-thiochromen]-4'(3'H)-one hydrochloride
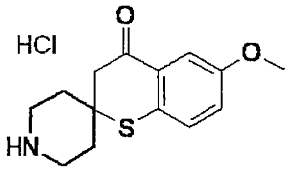

The title compound (81.0 mg, yield 51%) was obtained as a powder in the same manner as in Reference Example 19 and from tert-butyl 6'-methoxy-4'-oxo-3',4'-dihydro-1H-spiro[piperidine-4,2'-thiochromene]-1-carboxylate (81 mg, 0.532 mmol) obtained in Reference Example 129 and 4N hydrogen chloride-ethyl acetate (2 mL). The compound was used for the next step without purification.

Reference Example 131

tert-butyl 3-methyl-4-oxo-3,4-dihydro-1'H-spiro[chromene-2,4'-piperidine]-1'-carboxylate
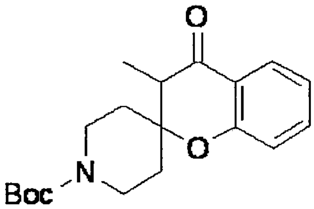

The title compound (4.22 g, yield 48%) was obtained as an oil in the same manner as in Reference Example 18 and from 1-(2-hydroxyphenyl)propan-1-one (4.00 g, 26.6 mmol) and tert-butyl 4-oxopiperidine-1-carboxylate (5.31 g, 26.6 mmol).
$^1H$ NMR ($CDCl_3$) δ1.19 (3H, d, J = 7.2 Hz), 1.46 (9H, s), 1.39-1.74 (2H, m), 1.91-1.99 (2H, m), 2.62 (1H, q, J = 7.2 Hz), 3.10-3.18 (2H, m), 3.94 (2H, m), 6.95-7.03 (2H, m), 7.45-7.51 (1H, m), 7.83-7.86 (1H, m).

Reference Example 132

3-methylspiro[chromene-2,4'-piperidin]-4(3H)-one hydrochloride
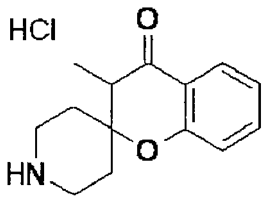

The title compound (1.34 g, yield 86%) was obtained as an oil in the same manner as in Reference Example 19 and from tert-butyl 3-methyl-4-oxo-3,4-dihydro-1'H-spiro[chromene-2,4'-piperidine]-1'-carboxylate (1.93 g, 5.82 mmol) obtained in Reference Example 131 and 4N hydrogen chloride-ethyl acetate (15 mL). The compound was used for the next step without purification.

Reference Example 133

tert-butyl 3,3-dimethyl-4-oxo-3,4-dihydro-1'H-spiro[chromene-2,4'-piperidine]-1'-carboxylate
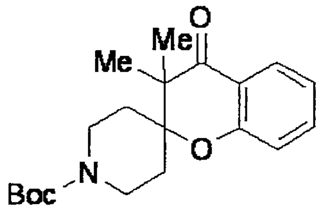

To a solution of tert-butyl 3-methyl-4-oxo-3,4-dihydro-1'H-spiro[chromene-2,4'-piperidine]-1'-carboxylate (2.27 g, 6.85 mmol) obtained in Reference Example 131 in THF (10 mL) was added dropwise a 1.1M solution (9.34 mL, 10.3 mmol) of LHDMS in THF at -78°C and, after 30 min, a solution of methyl iodide (0.64 mL, 10.3 mmol) in THF (1 mL) was added dropwise. The mixture was warmed to room temperature and stirred for 2 hr. Water was added to the reaction mixture, and the mixture was extracted with ethyl acetate. The extract was washed with 0.5N hydrochloric acid, 10% aqueous potassium carbonate solution and saturated brine, and dried over anhydrous sodium sulfate. The extract was subjected to basic silica gel column chromatography (ethyl acetate), and the solvent was evaporated under reduced pressure. The obtained residue was purified by silica gel column chromatography (ethyl acetate:hexane=1:9 to 1:3) to give the title compound (2.06 g, yield 87%) as an oil. $^1$H NMR ($CDCl_3$) δ1.18 (6H, s), 1.46 (9H, s), 1.59-1.74 (2H, s), 1.89-1.94 (2H, m), 3.09 (2H, m), 3.99 (2H, m), 6.94-7.03 (2H, m), 7.45-7.50 (1H, m), 7.84-7.87 (1H, m).

Reference Example 134

3,3-dimethylspiro[chromene-2,4'-piperidin]-4(3H)-one hydrochloride

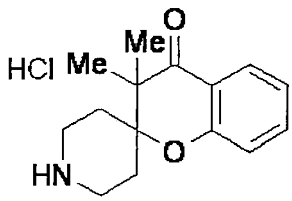

The title compound (1.68 g, quantitative) was obtained as a powder in the same manner as in Reference Example 19 and from tert-butyl 3,3-dimethyl-4-oxo-3,4-dihydro-1'H-spiro[chromene-2,4'-piperidine]-1'-carboxylate (2.06 g, 5.96 mmol) obtained in Reference Example 133 and 4N hydrogen chloride-ethyl acetate (50 mL). The compound was used for the next step without purification.

Reference Example 135

ethyl 3-amino-6-(trifluoromethyl)thieno[2,3-b]pyridine-2-carboxylate

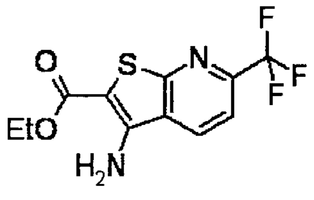

The title compound (6.29 g, yield 85%) was obtained as a yellow solid in the same manner as in Reference Example 1 and from 2-chloro-6-(trifluoromethyl)nicotinonitrile (5.25 g, 25.5 mmol).
$^1$H NMR (DMSO-$d_6$) δ 1.32 (3H, t, J = 7.1 Hz), 4.32 (2H, q, J = 7.1 Hz), 7.44 (2H, br), 7.99 (1H, d, J = 8.5 Hz), 8.84 (1H, d, J = 8.5 Hz).

Reference Example 136

ethyl 3-bromo-6-(trifluoromethyl)thieno[2,3-b]pyridine-2-carboxylate

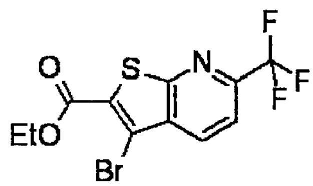

The title compound (5.75 g, yield 75%) was obtained as a yellow solid in the same manner as in Reference Example 2 and from ethyl 3-amino-6-(trifluoromethyl)thieno[2,3-b]pyridine-2-carboxylate (6.28 g, 21.7 mmol) obtained in Reference Example 135.

$^1$H NMR ($CDCl_3$) δ 1.46 (3H, t, J = 7.2 Hz), 4.49 (2H, q, J = 6.91 Hz), 7.81 (1H, d, J = 8.3 Hz), 8.40 (1H, d, J = 8.3 Hz).

Reference Example 137

tert-butyl [3-bromo-6-(trifluoromethyl)thieno[2,3-b]pyridin-2-yl]carbamate

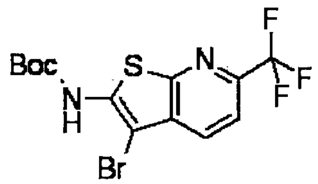

The title compound (5.38 g, yield 91%) was obtained as a yellow solid in the same manner as in Reference Examples 3 and 4 and from ethyl 3-bromo-6-(trifluoromethyl)thieno[2,3-b]pyridine-2-carboxylate (6.40 g, 18.2 mmol) obtained in Reference Example 136.

$^1$H NMR (DMSO-$d_6$) δ 7.96 (1H, d, J = 8.3 Hz), 8.36 (1H, d, J = 8.3 Hz).

Reference Example 138

2-[(tert-butoxycarbonyl)amino]-6-(trifluoromethyl)thieno[2,3-b]pyridine-3-carboxylic acid

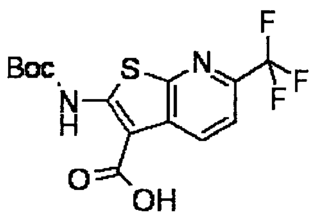

The title compound (1.11 g, yield 67%) was obtained as a yellow solid in the same manner as in Reference Example 5 and from tert-butyl [3-bromo-6-(trifluoromethyl)thieno[2,3-b]pyridin-2-yl]carbamate (1.80 g, 4.55 mmol) obtained in Reference Example 137.

$^1$H NMR (DMSO-$d_6$) δ 1.56 (9H, s), 7.92 (1H, d, J = 8.7 Hz), 8.66 (1H, d, J = 8.5 Hz), 11.06 (1H, br).

Reference Example 139

tert-butyl 6'-methyl-4'-oxo-3',4'-dihydro-1H-spiro[piperidine-4,2'-thiochromene]-1-carboxylate 1',1'-dioxide

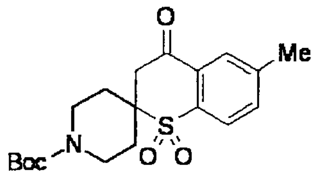

To a solution of tert-butyl 6'-methyl-4'-oxo-3',4'-dihydro-1H-spiro[piperidine-4,2'-thiochromene]-1-carboxylate (1.00 g, 2.88 mmol) obtained in Reference Example 126 in dichloromethane (10 mL) was added 70% m-chloroperbenzoic acid (1.84 g, 7.20 mmol), and the mixture was stirred at 0°C for 3 hr. The reaction mixture was evaporated under reduced pressure and the obtained residue was purified by silica gel column chromatography (ethyl acetate:hexane=0:1 to 2:3) to give the title compound (898 mg, yield 82%) as a powder.
EI(pos) 280 $[M\text{-}Boc]^+$

Reference Example 140

6'-methylspiro[piperidine-4,2'-thiochromen]-4'(3'H)-one 1',1'-dioxide hydrochloride
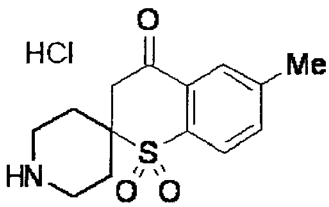

The title compound (788 mg, quantitative) was obtained as a powder in the same manner as in Reference Example 19 and from tert-butyl 6'-methyl-4'-oxo-3',4'-dihydro-1H-spiro[piperidine-4,2'-thiochromene]-1-carboxylate 1', 1'-dioxide (898 mg, 2.37 mmol) obtained in Reference Example 139 and 4N hydrogen chloride-ethyl acetate (6 mL). The compound was used for the next step without purification.

Reference Example 141

2-acetyl-4-fluorophenyl trifluoromethanesulfonate
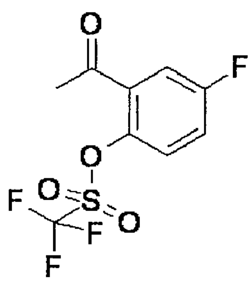

The title compound (9.30 g, quantitative) was obtained as an oil in the same manner as in Reference Example 100 and from 1-(5-fluoro-2-hydroxyphenyl)ethanone (5.00 g, 32.5 mmol) and trifluoromethanesulfonic anhydride (6.6 mL, 38.9 mmol). The compound was used for the next step without purification.

Reference Example 142

1-{5-fluoro-2-[(4-methoxybenzyl)thio]phenyl}ethanone

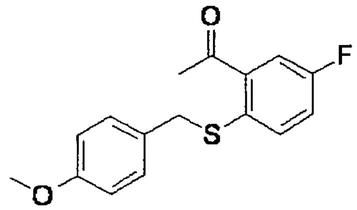

A solution of 2-acetyl-4-fluorophenyl trifluoromethanesulfonate (9.27 g, 32.4 mmol) obtained in Reference Example 141, tris(dibenzylideneacetone)dipalladium (743 mg, 1.63 mmol), (4-methoxyphenyl)methanethiol (5.00 g, 32.5 mmol), 4,5-bis(diphenylphosphino)-9,9-dimethylxanthene (Xantphos) (942 mg, 1.63 mmol) and diisopropylethylamine (8.38 g, 65.0 mmol) in 1,4-dioxane (65 mL) was refluxed by heating for 2 hr under an argon atmosphere. The reaction mixture was diluted with ethyl acetate and water, and filtered. The filtrate was extracted with ethyl acetate, and the extract was dried over anhydrous sodium sulfate. The solvent was evaporated under reduced pressure, and the obtained residue was purified by silica gel column chromatography (ethyl acetate:hexane=0:1 to 3:7) and treated with diisopropyl ether to give the title compound (6.36 g, yield 67%) as a powder.
$^1$H NMR ($CDCl_3$) δ2.56 (3H, s), 3.78 (3H, s), 4.04 (2H, s), 6.79-6.85 (2H, m), 7.07-7.14 (1H, m), 7.20-7.27 (2H, m), 7.30-7.40 (2H, m).

Reference Example 143

1-(5-fluoro-2-mercaptophenyl)ethanone
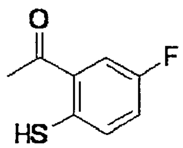

To a solution of 1-15-fluoro-2-[(4-methoxybenzyl)thio]phenyl}ethanone (4.70 g, 16.2 mmol) obtained in Reference Example 142 in toluene (30 mL) was added aluminum chloride (2.91 g, 21.9 mmol) under ice-cooling, and the mixture was stirred for 15 min. The reaction mixture was diluted with ethyl acetate, and extracted with 2N aqueous sodium hydroxide solution. The extract was acidified with 6N hydrochloric acid, and the mixture was extracted with ethyl acetate. The extract was washed with saturated brine, and dried over anhydrous sodium sulfate. The solvent was evaporated under reduced pressure to give the title compound (2.76 g, quantitative) as an oil. The compound was used for the next step without purification.
$^1$H NMR ($CDCl_3$) δ2.62 (3H, s), 4.45 (1H, s), 7.05-7.16 (1H, m), 7.25-7.34 (1H, m), 7.56 (1H, dd, J = 9.28, 2.84 Hz).

Reference Example 144

tert-butyl 6'-fluoro-4'-oxo-3',4'-dihydro-1H-spiro[piperidine-4,2'-thiochromene]-1-carboxylate
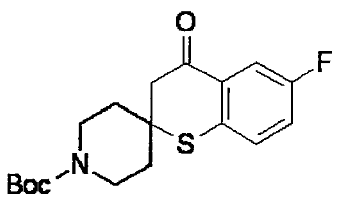

To a solution of 1-(5-fluoro-2-mercaptophenyl)ethanone (2.61 g, 15.3 mmol) obtained in Reference Example 143 in methanol (50 mL) were added tert-butyl 4-oxopiperidine-1-carboxylate (3.06 g, 15.3 mmol) and pyrrolidine (1.3

mL, 15.3 mmol), and the mixture was stirred with heating at 50°C for 1 hr. The solvent was evaporated under reduced pressure, and the residue was diluted with ethyl acetate. The mixture was washed with 0.1N hydrochloric acid and saturated brine, and dried over anhydrous sodium sulfate. The solvent was evaporated under reduced pressure, and the obtained residue was purified by silica gel column chromatography (ethyl acetate:hexane=0:1 to 3:7) and recrystallized from hexane to give the title compound (1.56 g, yield 29%) as a powder.
$^1$H NMR ($CDCl_3$) δ1.45 (9H, s), 1.67-1.77 (2H, m), 1.86-1.96 (2H, m), 2.93 (2H, s), 3.16-3.28 (2H, m), 3.82-3.94 (2H, m), 7.13-28 (2H, m), 7.77 (1H, dd, J = 9.23, 2.83 Hz).

Reference Example 145

6'-fluorospiro[piperidine-4,2'-thiochromen]-4'(3'H)-one hydrochloride
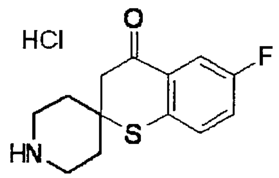

The title compound (1.30 g, quantitative) was obtained as a powder in the same manner as in Reference Example 19 and from tert-butyl 6'-fluoro-4'-oxo-3',4'-dihydro-1H-spiro[piperidine-4,2'-thiochromene]-1-carboxylate (1.56 g, 4.44 mmol) obtained in Reference Example 144 and 4N hydrogen chloride-ethyl acetate (10 mL). The compound was used for the next step without purification.

Reference Example 146

tert-butyl 6-methyl-4-oxo-3,4-dihydro-1'H-spiro[chromene-2,4'-piperidine]-1'-carboxylate
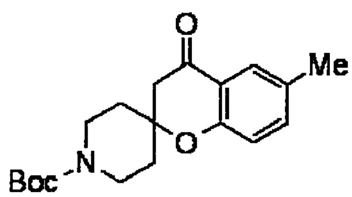

The title compound (9.92 g, yield 90%) was obtained as a powder in the same manner as in Reference Example 18 and from 1-(2-hydroxy-5-methylphenyl)ethanone (4.98 g, 33.2 mmol) and tert-butyl 4--oxopiperidine-1-carboxylate (6.61 g, 33.2 mmol). $^1$H NMR ($CDCl_3$) δ1.46 (9H, s), 1.52-1.64 (2H, m), 1.99-2.03 (2H, m), 2.30 (3H, s), 2.69 (2H, s), 3.16-3.24 (2H, m), 3.85 (2H, m), 6.87 (1H, d, J = 8.4 Hz), 7.30 (1H, dd, J = 8.4, 1.5 Hz), 7.64 (1H, d, J = 1.5 Hz).

Reference Example 147

6-methylspiro[chromene-2,4'-piperidin]-4(3H)-one hydrochloride
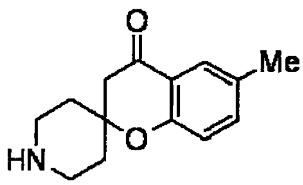

The titled compound (7.92 g, quantitative) was obtained as a powder in the same manner as in Reference Example 19 and from tert-butyl 6-methyl-4-oxo-3,4-dihydro-1'H-spiro[chromene-2,4'-piperidine]-1'-carboxylate (9.80 g, 29.6 mmol) obtained in Reference Example 146 and 4N hydrogen chloride-ethyl acetate (150 mL). The compound was used for the next step without purification.

Reference Example 148

tert-butyl 4-oxo-6-(trifluoromethyl)-3,4-dihydro-1'H-spiro[chromene-2,4'-piperidine]-1'-carboxylate
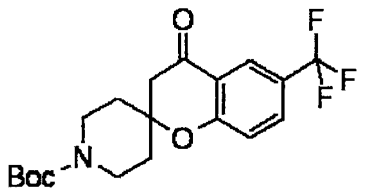

The title compound (1.80 g, yield 41%) was obtained as a powder in the same manner as in Reference Example 18 and from 1-[2-hydroxy-5-(trifluoromethyl)phenyl]ethanone (2.34 g, 11.5 mmol) and tert-butyl 4-oxopiperidine-1-carboxylate (2.28 g, 11.5 mmol).
$^1$H NMR ($CDCl_3$) δ1.46 (9H, s), 1.59-1.70 (2H, m), 2.00-2.04 (2H, m), 2.76 (2H, s), 3.17-3.25 (2H, m), 3.87 (2H, m), 7.09 (1H, d, J = 8.7 Hz), 7.71 (1H, dd, J = 8.7, 2.1 Hz), 8.15 (1H, d, J = 2.1 Hz).

Reference Example 149

6-(trifluoromethyl)spiro[chromene-2,4'-piperidin]-4(3H)-one hydrochloride
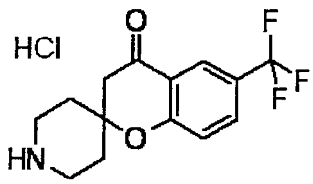

The title compound (1.49 g, quantitative) was obtained as a powder in the same manner as in Reference Example 19 and from tert-butyl 4-oxo-6-(trifluoromethyl)-3,4-dihydro-1'H-spiro[chromene-2,4'-piperidine]-1'-carboxylate (1.79 g, 4.64 mmol) obtained in Reference Example 148 and 4N hydrogen chloride-ethyl acetate (20 mL). The compound was used for the next step without purification.

Example 1

1'-[(2-amino-1-benzothiophen-3-yl)carbonyl]spiro[chromene-2,4'-piperidin]-4(3H)-one
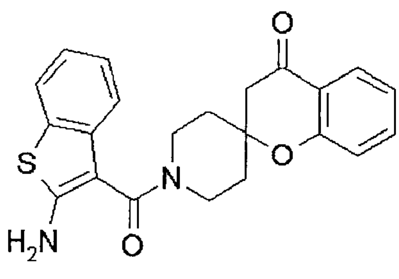

To a solution of spiro[chromene-2,4'-piperidin]-4(3H)-one hydrochloride (300 mg, 1.18 mmol), 2-amino-1-benzothiophene-3-carboxylic acid (228 mg, 1.18 mmol) (produced by the method described in WO07/013691) and 1-hydroxybenzotriazole (160 mg, 1.18 mmol) in DMF (10 mL) were added 1-ethyl-3-(3'-dimethylaminopropyl)carbodiimide hydrochloride (295 mg, 1.53 mmol) and triethylamine (0.494 mL, 3.54 mmol), and the mixture was stirred at room temperature for 18 hr. Water was added to the reaction mixture, and the mixture was extracted with ethyl acetate. The extract was washed with water, and concentrated under reduced pressure. The residue was purified by silica gel column chromatography (hexane:ethyl acetate=3:7 to ethyl acetate) to give the title compound (423 mg, yield 91%) as an oil.
$^1$H NMR ($CDCl_3$) δ1.61-1.88 (2H, m), 2.04-2.16 (2H, m), 2.71 (1.2H, s), 2.82 (0.8H, s), 3.35-3.54 (2H, m), 3.89-3.98 (0.8H, m), 4.02-4.08 (1.2H, m), 5.21 (0.8H, br s), 5.37 (1.2H, br s), 6.97-7.16 (3H, m), 7.26-7.42 (2H, m), 7.47-7.55 (2H, m), 7.85-7.88 (1H, m).

Example 2

N-ethyl-N'-{3-[(4-oxo-3,4-dihydro-1'H-spiro[chromene-2,4'-piperidin]-1'-yl)carbonyl]-1-benzothiophen-2-yl} urea

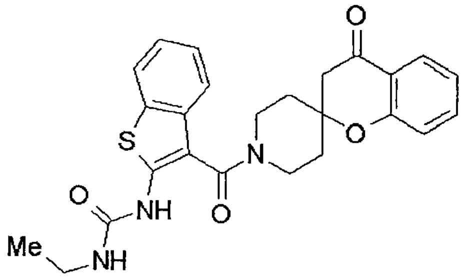

To a solution of 1'-[(2-aminobenzothiophen-3-yl)carbonyl]spiro[chromene-2,4'-piperidin]-4(3H)-one (410 mg, 1.05 mmol) obtained in Example 1 in pyridine (5 mL) was added ethyl isocyanate (165 mL, 2.10 mmol), and the mixture was stirred at 70°C for 15 hr. Water was added to the reaction mixture, and the mixture was extracted with ethyl acetate. The extract was washed with water, and concentrated under reduced pressure. The residue was purified by silica gel column chromatography (hexane:ethyl acetate=1:1 to ethyl acetate), and recrystallized from ethyl acetate to give the title compound (292 mg, yield 60%).
melting point 213-214°C
$^1$H NMR ($CDCl_3$) δ0.82-0.91 (3H, m), 1.68-1.87 (2H, m), 2.07-2.17 (2H, m), 2.70 (1.2H, s), 2.83 (0.8H, s), 3.02-3.11 (2H, m), 3.36-3.61 (2H, m), 3.85-4.20 (2H, m), 5.50-5.59 (1H, m), 6.89-6.92 (0.4H, m), 6.96-7.08 (2H, m), 7.18-7.27 (1H, m), 7.31-7.45 (2H, m), 7.51-7.56 (0.6H, m), 7.72 (1H, d, J = 7.7 Hz), 7.84-7.87 (1H, m), 9.40 (1H, br d, J = 13.6 Hz).

Example 3

tert-butyl {3-[(4-oxo-3,4-dihydro-1'H-spiro[chromene-2,4'-piperidin]-1'-yl)carbonyl]thieno[2,3-b]pyridin-2-yl} carbamate

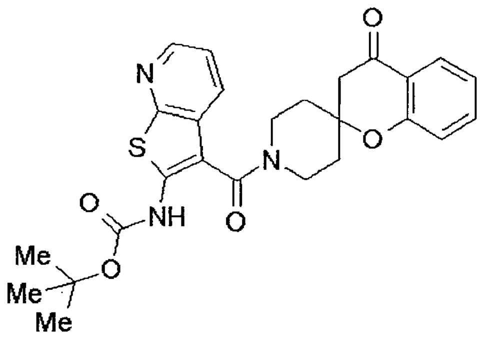

The title compound (369 mg, yield 63%) was obtained as an oil in the same manner as in Example 1 and from

spiro[chromene-2,4'-piperidin]-4(3H)-one hydrochloride (300 mg, 1.18 mmol) and 2-[(tert-butoxycarbonyl)amino]thieno [2,3-b]pyridine-3-carboxylic acid (348 mg, 1.18 mmol) obtained in Reference Example 5.
$^1$H NMR ($CDCl_3$) δ1.57 (9H, s), 1.60-1.86 (2H, m), 2.07-2.23 (2H, m), 2.72 (0.8H, s), 3.06 (1.2H, s), 3.36-3.54 (2H, m), 3.82-3.88 (0.8H, m), 4.01-4.05 (1.2H, m), 6.93-7.07 (2H, m), 7.27-7.34 (1H, m), 7.48-7.57 (1H, m), 7.66-7.77 (1H, m), 7.87 (1H, d, J = 7.8 Hz), 8.42-8.45 (1H, m), 9.16 (0.4H, br s), 9.36 (0.6H, br s).

Example 4

1'-[(2-aminothieno[2,3-b]pyridin-3-yl)carbonyl]spiro[chromene-2,4'-piperidin]-4(3H)-one
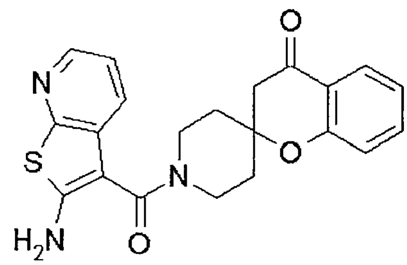

tert-Butyl {3-[(4-oxo-3,4-dihydro-1'H-spiro[chromene-2,4'-piperidin]-1'-yl)carbonyl]thieno[2,3-b]pyridin-2-yl} carbamate (350 mg, 0.709 mmol) obtained in Example 3 was dissolved in trifluoroacetic acid (3 mL), and the solution was stirred at room temperature for 30 min. The reaction mixture was basified with saturated aqueous sodium hydrogen carbonate solution, and extracted with ethyl acetate. The extract was washed with water, and concentrated under reduced pressure. The residue was purified by silica gel column chromatography (hexane:ethyl acetate=3:7 to ethyl acetate) to give the title compound (216 mg, yield 77%) as an oil.
$^1$H NMR ($CDCl_3$) δ1.59-1.82 (2H, m), 2.05-2.17 (2H, m), 2.71 (1.2H, s), 2.82 (0.8H, s), 3.38-3.50 (2H, m), 3.86 (0.8H, br d, J = 13.6 Hz), 3.99 (1.2H, br d, 13.3 Hz), 5.54 (0.8H, br s), 5.73 (1.2H, br s), 6.96-7.07 (2H, m), 7.17-7.24 (1H, m), 7.47-7.62 (2H, m), 7.87 (1H, dd, J = 1.9 Hz, 8.0 Hz), 8.22-8.24 (1H, m).

Example 5

N-ethyl-N'-{3-[(4-oxo-3,4-dihydro-1'H-spiro[chromene-2,4'-piperidin]-1'-yl)carbonyl]thieno[2,3-b]pyridin-2-yl} urea
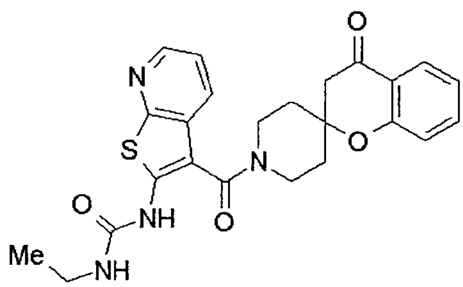

The title compound (120 mg, yield 51%) was obtained as crystals in the same manner as in Example 2 and from 1'-[(2-aminothieno[2,3-b]pyridin-3-yl)carbonyl]spiro[chromene-2,4'-piperidin]-4(3H)-one (200 mg, 0.508 mmol) obtained in Example 4.
EI(pos) 465 [M+]$^+$

Example 6

tert-butyl {3-[(4-oxo-3,4-dihydro-1'H-spiro[chromene-2,4'-piperidin]-1'-yl)carbonyl]-5-phenyl-2-thienyl}carbamate
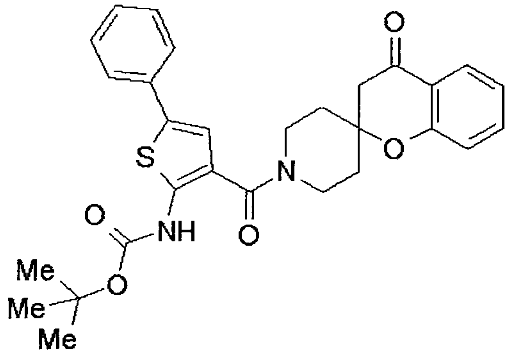

The title compound (350 mg, yield 85%) was obtained as an oil in the same manner as in Example 1 and from spiro[chromene-2,4'-piperidin]-4(3H)-one hydrochloride (200 mg, 0.791 mmol) and 2-[(tert-butoxycarbonyl)amino]-5-phenylthiophene-3-carboxylic acid (252 mg, 0.791 mmol)
(produced by the method described in WO07/013691).
EI(pos) 519 [M+]$^+$

Example 7

1'-[(2-amino-5-phenyl-3-thienyl)carbonyl]spiro[chromene-2,4'-piperidin]-4(3H)-one
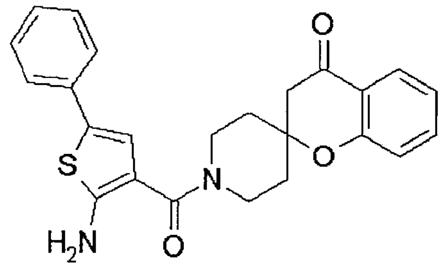

The title compound (215 mg, yield 76%) was obtained as an amorphous solid in the same manner as in Example 4 and from trifluoroacetic acid (5 mL) and tert-butyl {3-[(4-oxo-3,4-dihydro-1'H-spiro[chromene-2,4'-piperidin]-1'-yl)carbonyl]-5-phenyl-2-thienyl}carbamate (350 mg, 0.674 mmol) obtained in Example 6.
EI(pos) 418 [M+]$^+$

Example 8

N-methyl-N'-{3-[(4-oxo-3,4-dihydro-1'H-spiro[chromene-2,4'-piperidin]-1'-yl)carbonyl]-5-phenyl-2-thienyl}urea
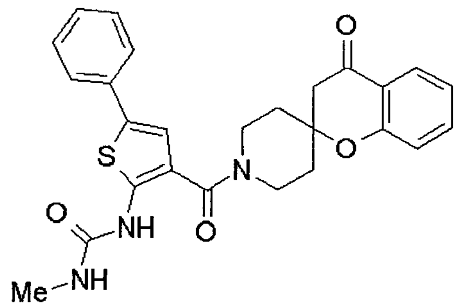

The title compound (20.1 mg, yield 8%) was obtained as an amorphous solid in the same manner as in Example

2 and from 1'-[(2-amino-5-phenyl-3-thienyl)carbonyl]spiro[chromene-2,4'-piperidin]-4(3H)-one (215 mg, 0.514 mmol) obtained in Example 7 and methyl isocyanate.
EI(pos) 476 [M+]$^+$

Example 9

1'-(2-amino-5-bromobenzoyl)spiro[chromene-2,4'-piperidin]-4(3H)-one

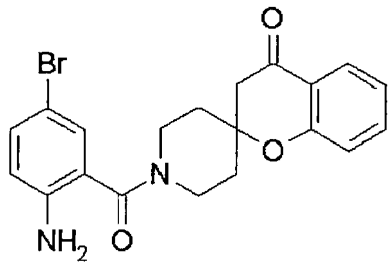

The title compound (484 mg, yield 92%) was obtained as an oil in the same manner as in Example 1 and from spiro[chromene-2,4'-piperidin]-4(3H)-one hydrochloride (320 mg, 1.26 mmol) and 5-bromoanthranic acid (272 mg, 1.26 mmol). EI(pos) 415 [M+]$^+$

Example 10

1'-[(4-aminobiphenyl-3-yl)carbonyl]spiro[chromene-2,4'-piperidin]-4(3H)-one

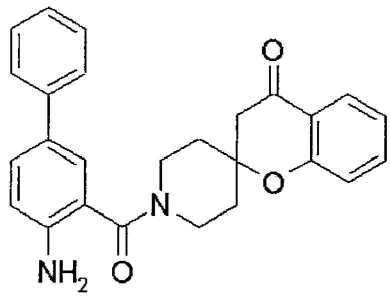

To a solution of 1'-(2-amino-5-bromobenzoyl)spiro[chromene-2,4'-piperidin]-4(3H)-one (400 mg, 0.963 mmol) obtained in Example 9, phenylboric acid (235 mg, 1.93 mmol) and 2N aqueous sodium carbonate solution (0.963 mL, 1.93 mmol) in THF (10 mL) was added tetrakistriphenylphosphine palladium (33.4 mg, 0.0289 mmol) under a nitrogen atmosphere, and the mixture was stirred with heating at 100°C for 16 hr. After completion of the reaction, the reaction mixture was diluted with ethyl acetate, and the mixture was washed with saturated brine, and dried over anhydrous sodium sulfate. The residue was purified by silica gel column chromatography (hexane:ethyl acetate=3:1 to ethyl acetate), and triturated with diisopropyl ether to give the title compound (174 mg, yield 44%).
EI(pos) 413 [M+]$^+$

Example 11

N-ethyl-N'-{3-[(4-oxo-3,4-dihydro-1'H-spiro[chromene-2,4'-piperidin]-1'-yl)carbonyl]biphenyl-4-yl}urea

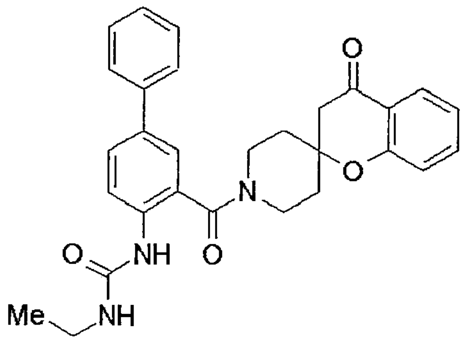

The title compound (104 mg, yield 59%) was obtained as a powder in the same manner as in Example 2 and from 1'-[(4-aminobiphenyl-3-yl)carbonyl]spiro[chromene-2,4'-piperidin]-4(3H)-one (150 mg, 0.364 mmol) obtained in Example 10. EI(pos) 484 [M+]$^+$

Example 12

1'-[(2-amino-7-methoxy-1-benzothiophen-3-yl)carbonyl]spiro[chromene-2,4'-piperidin]-4(3H)-one

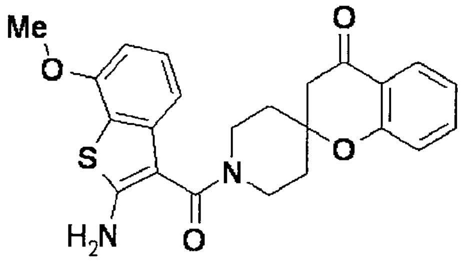

The title compound (251 mg, yield 63%) was obtained as a powder in the same manner as in Example 1 and from spiro[chromene-2,4'-piperidin]-4(3H)-one hydrochloride (200 mg, 0.788 mmol) and 2-amino-7-methoxy-1-benzothiophene-3-carboxylic acid (176 mg, 0.788 mmol) (produced by the method described in WO07/119833)
EI(pos) 423.4 [M+H]$^+$

Example 13

1-{7-methoxy-3-[(4-oxo-3,4-dihydro-1'H-spiro[chromene-2,4'-piperidin]-1'-yl)carbonyl]-1-benzothiophen-2-yl}-3-methylurea

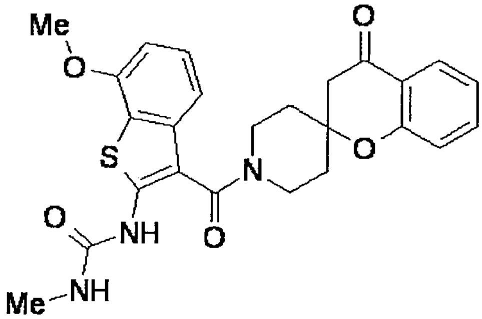

The title compound (144 mg, yield 57%) was obtained as a powder in the same manner as in Example 2 and from 1'-[(2-amino-7-methoxy-1-benzothiophen-3-yl)carbonyl]spiro[chromene-2,4'-piperidin]-4(3H)-one (222 mg, 0.525

mmol) obtained in Example 12 and methyl isocyanate (0.073 mL, 1.58 mmol). EI(pos) 480.4 [M+H]+

Example 14

1'-[(2-amino-7-methoxy-1-benzothiophen-3-yl)carbonyl]-4-oxo-3,4-dihydrospiro[chromene-2,4'-piperidine]-6-carbonitrile

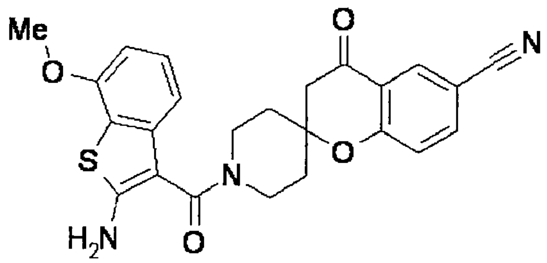

The title compound (1.27 g, yield 74%) was obtained as a powder in the same manner as in Example 1 and from 4-oxo-3,4-dihydrospiro[chromene-2,4'-piperidine]-6-carbonitrile hydrochloride (1.06 g, 4.03 mmol) and 2-amino-7-methoxy-1-benzothiophene-3-carboxylic acid (900 mg, 4.03 mmol) (produced by the method described in WO07/119833).
EI(pos) 447.9 [M+H]+

Example 15

1-{3-[(6-cyano-4-oxo-3,4-dihydro-1'H-spiro[chromene-2,4'-piperidin]-1'-yl)carbonyl]-7-methoxy-1-benzothiophen-2-yl}-3-methylurea

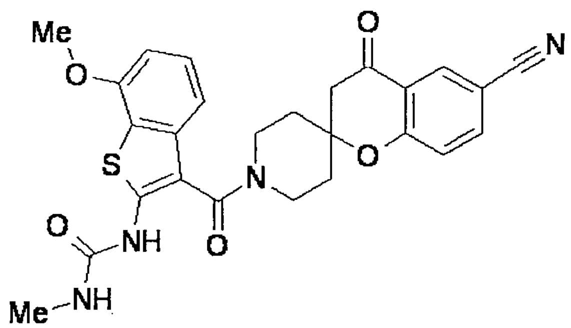

The title compound (528 mg, yield 58%) was obtained as a powder in the same manner as in Example 2 and from 1'-[(2-amino-7-methoxy-1-benzothiophen-3-yl)carbonyl]-4-oxo-3,4-dihydrospiro[chromene-2,4'-piperidine]-6-carbonitrile (800 mg, 1.80 mmol) obtained in Example 14 and methyl isocyanate (0.267 mL, 4.50 mmol).
EI(pos) 505.2 [M+H]+

Example 16

1-(7-methoxy-3-{[4-oxo-6-(1H-tetrazol-5-yl)-3,4-dihydro-1'H-spiro[chromene-2,4'-piperidin]-1'-yl]carbonyl}-1-benzothiophen-2-yl)-3-methylurea

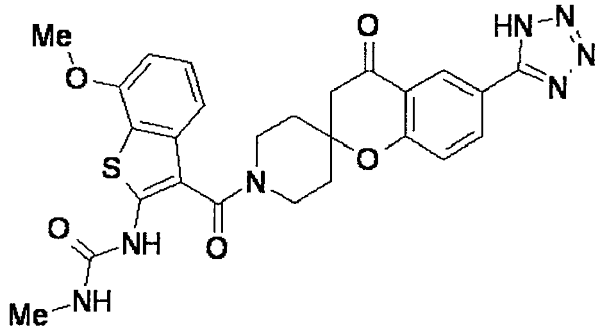

A solution of 1-{3-[(6-cyano-4-oxo-3,4-dihydro-1'H-spiro[chromene-2,4'-piperidin]-1'-yl)carbonyl]-7-methoxy-1-benzothiophen-2-yl}-3-methylurea (200 mg, 0.398 mmol) obtained in Example 15, sodium azide (77.6 mg, 1.19 mmol) and triethylammonium chloride (163 mg,1.19 mmol) in DMF (2.8 mL) was stirred at 120°C for 5 hr. The reaction mixture was acidified with 1N hydrochloric acid, and extracted with ethyl acetate. The extract was washed with saturated brine and dried over anhydrous magnesium sulfate. The solvent was evaporated under reduced pressure. The obtained residue was purified by silica gel column chromatography (ethyl acetate:methanol=1:0 to 8:2), and further purified by preparative liquid chromatography to give the title compound (46.7 mg, yield 22%) as a white solid.
EI(pos) 548.1 $[M+H]^+$

Example 17

1'-[(2-amino-7-methoxy-1-benzothiophen-3-yl)carbonyl]-6-nitrospiro[chromene-2,4'-piperidin]-4(3H)-one
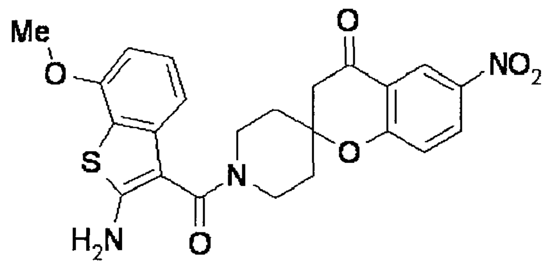

The title compound (1.02 g, yield 60%) was obtained as a powder in the same manner as in Example 1 and from 6-nitrospiro[chromene-2,4'-piperidin]-4(3H)-one hydrochloride (1.10 g, 3.68 mmol) obtained in Reference Example 19 and 2-amino-7-methoxy-1-benzothiophene-3-carboxylic acid (857 mg, 3.68 mmol) (produced by the method described in WO07/119833). EI(pos) 468.0 $[M+H]^+$

Example 18

1-{7-methoxy-3-[(6-nitro-4-oxo-3,4-dihydro-1'H-spiro[chromene-2,4'-piperidin]-1'-yl)carbonyl]-1-benzothiophen-2-yl}-3-methylurea
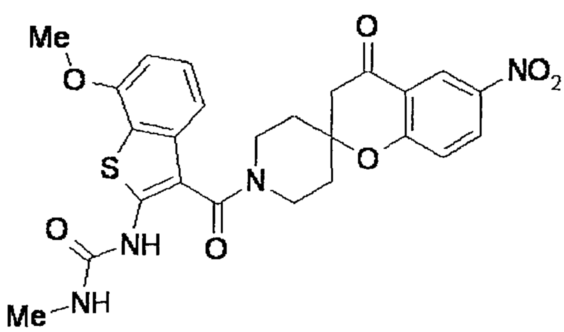

The title compound (642 mg, yield 56%) was obtained as a powder in the same manner as in Example 2 and from 1'-[(2-amino-7-methoxy-1-benzothiophen-3-yl)carbonyl]-6-nitrospiro[chromene-2,4'-piperidin]-4(3H)-one (1.02 g, 2.18 mmol) obtained in Example 17 and methyl isocyanate (0.324 mL, 5.45 mmol).
EI(pos) 525.0 $[M+H]^+$

Example 19

1-{3-[(6-amino-4-oxo-3,4-dihydro-1'H-spiro[chromene-2,4'-piperidin]-1'-yl)carbonyl]-7-methoxy-1-benzothiophen-2-yl}-3-methylurea

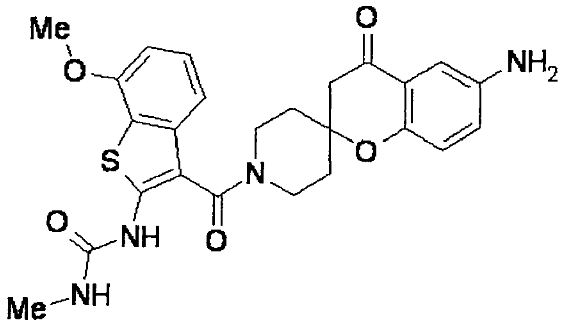

To 1-{7-methoxy-3-[(6-nitro-4-oxo-3,4-dihydro-1'H-spiro[chromene-2,4'-piperidin]-1'-yl)carbonyl]-1-benzothiophen-2-yl}-3-methylurea (500 mg, 0.953 mmol) obtained in Example 18 and 10% palladium carbon (50% water-containing product, 20.6 mg) was added THF (3.2 mL), and the mixture was stirred under a hydrogen atmosphere for 8 hr. The reaction mixture was filtered, and the filtrate was concentrated under reduced pressure. The obtained residue was subjected to silica gel column chromatography, the solution was concentrated to dryness, and the residue was suspended in diethyl ether. The resulting solid was collected by filtration to give the title compound (443 mg, yield 93%) as a powder.
EI(pos) 495.1 $[M+H]^+$

Example 20

N-[1'-({7- methoxy- 2-[(methylcarbamoyl) amino]- 1- benzothiophen- 3- yl} carbonyl)- 4- oxo- 3,4- dihydrospiro [chromene-2,4'-piperidin]-6-yl]methanesulfonamide

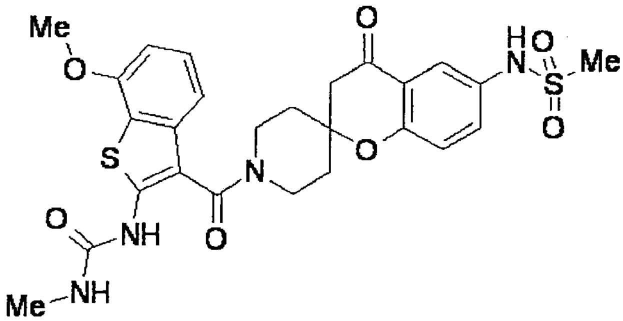

To a solution of 1-{3-[(6-amino-4-oxo-3,4-dihydro-1'H-spiro[chromene-2,4'-piperidin]-1'-yl)carbonyl]-7-methoxy-1-benzothiophen-2-yl}-3-methylurea (200 mg, 0.404 mmol) obtained in Example 19 in pyridine (1 mL) was added methanesulfonyl chloride (0.033 mL, 0.425 mmol) under ice-cooling and the mixture was stirred for 1 hr. The reaction mixture was concentrated, and the obtained residue was purified by silica gel column chromatography (ethyl acetate: methanol=1:0 to 19:1) to give the title compound (125 mg, yield 54%) as a white solid.
EI(pos) 573.0 $[M+H]^+$

Example 21

1-[1'-({7-methoxy-2-[(methylcarbamoyl)amino]-1-benzothiophen-3-yl}carbonyl)-4-oxo-3,4-dihydro-1'H-spiro [chromene-2,4'-piperidin]-6-yl]-3-methylurea

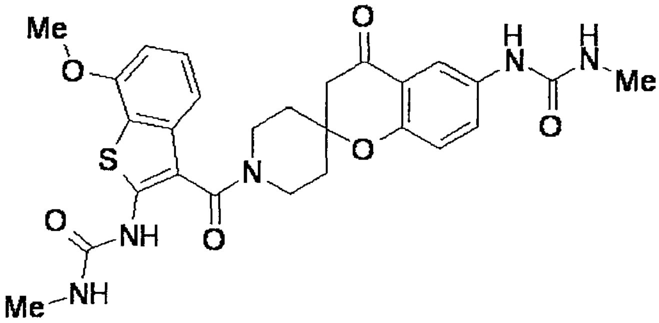

The title compound (2.2 mg, yield 1.5%) was obtained as a powder in the same manner as in Example 2 and from 1-{3-[(6-amino-4-oxo-3,4-dihydro-1'H-spiro[chromene-2,4'-piperidin]-1'-yl)carbonyl]-7-methoxy-1-benzothiophen-2-yl}-3-methylurea (130 mg, 0.263 mmol) obtained in Example 19 and methyl isocyanate (0.034 mL, 0.567 mmol).
EI(pos) 552.1 $[M+H]^+$

Example 22

benzyl 1'-[(2-amino-7-methoxy-1-benzothiophen-3-yl)carbonyl]-4-oxo-3,4-dihydrospiro[chromene-2,4'-piperidine]-6-carboxylate

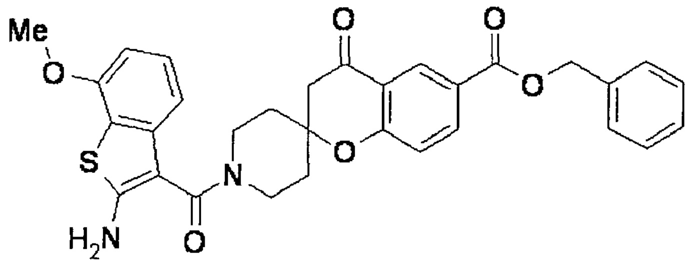

The title compound (404 mg, yield 87%) was obtained as a powder in the same manner as in Example 1 and from benzyl 4-oxo-3,4-dihydrospiro[chromene-2,4'-piperidine]-6-carboxylate hydrochloride (324 mg, 0.834 mmol) obtained in Reference Example 21 and 2-amino-7-methoxy-1-benzothiophene-3-carboxylic acid (195 mg, 0.834 mmol) (produced by the method described in WO07/119833).
EI(pos) 557.1 $[M+H]^+$

Example 23

benzyl 1'-({7-methoxy-2-[(methylcarbamoyl)amino]-1-benzothiophen-3-yl}carbonyl)-4-oxo-3,4-dihydrospiro [chromene-2,4'-piperidine]-6-carboxylate

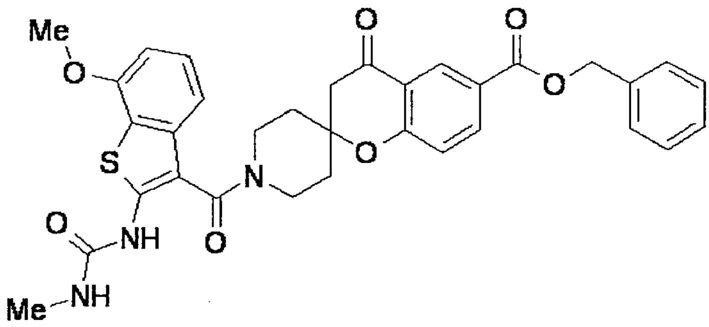

The title compound (303 mg, yield 68%) was obtained as a powder in the same manner as in Example 2 and from benzyl 1'-[(2-amino-7-methoxy-1-benzothiophen-3-yl)carbonyl]-4-oxo-3,4-dihydrospiro[chromene-2,4'-piperidine]-6-carboxylate (404 mg, 0.725 mmol) obtained in Example 22 and methyl isocyanate (0.107 mL, 1.82 mmol).
EI(pos) 614.1 $[M+H]^+$

Example 24

1'-( {7- methoxy- 2-[(methylcarbamoyl) amino]- 1- benzothiophen- 3- yl} carbonyl)- 4- oxo- 3,4- dihydrospiro [chromene-2,4'-piperidine]-6-carboxylic acid
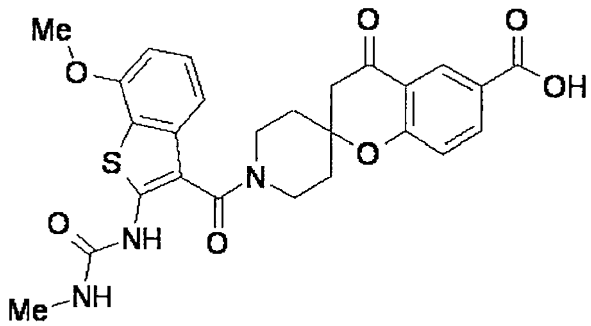

To benzyl 1'-({7-methoxy-2-[(methylcarbamoyl)amino]-1-benzothiophen-3-yl}carbonyl)-4-oxo-3,4-dihydrospiro[chromene-2,4'-piperidine]-6-carboxylate (303 mg, 0.494 mmol) obtained in Example 23 and 10% palladium carbon (50% water-containing product, 200 mg) were added MeOH (2 mL), AcOEt (1 mL) and THF (2 mL), and the mixture was stirred under a hydrogen atmosphere for 30 min. The reaction mixture was filtered, and the filtrate was concentrated under reduced pressure to give the title compound (221 mg, yield 85%) as a powder.
EI(pos) 524.2 $[M+H]^+$

Example 25

1'-[(2-amino-1-benzothiophen-3-yl)carbonyl]-6-bromospiro[chromene-2,4'-piperidin]-4(3H)-one
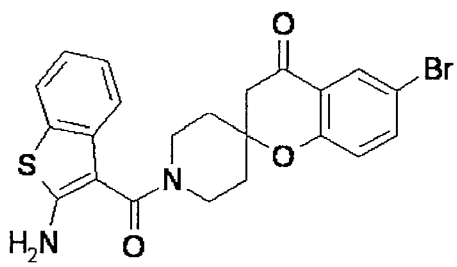

The title compound (1.41 g, quantitative) was obtained as an oil in the same manner as in Example 1 and from

6-bromospiro[chromene-2,4'-piperidin]-4(3H)-one hydrochloride (1.00 g, 3.01 mmol) and 2-amino-1-benzothiophene-3-carboxylic acid (581 mg, 3.01 mmol) (produced by the method described in WO07/013691).
EI(pos) 472 $[M+H]^+$

Example 26

1-{3-[(6-bromo-4-oxo-3,4-dihydro-1'H-spiro[chromene-2,4'-piperidin]-1'-yl)carbonyl]-1-benzothiophen-2-yl}-3-ethylurea
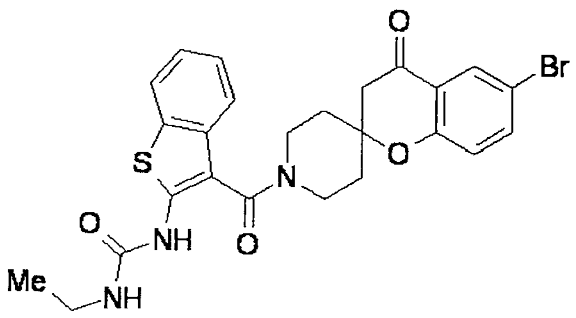

The title compound (1.32 g, yield 65%) was obtained as an amorphous solid in the same manner as in Example 2 and from 1'-[(2-amino-1-benzothiophen-3-yl)carbonyl]-6-bromospiro[chromene-2,4'-piperidin]-4(3H)-one (1.78 g, 3.75 mmol) obtained in Example 25 and ethyl isocyanate (0.735 mL, 7.50 mmol).
EI(pos) 544 $[M+H]^+$

Example 27

1-ethyl-3-{3-[(4-oxo-6-pyridin-2-yl-3,4-dihydro-1'H-spiro[chromene-2,4'-piperidin]-1'-yl)carbonyl]-1-benzothiophen-2-yl}urea
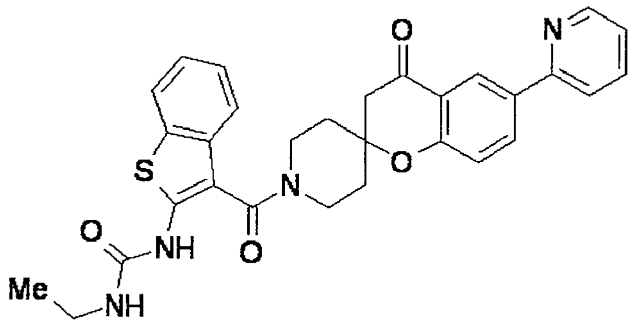

To a solution of 1-{3-[(6-bromo-4-oxo-3,4-dihydro-1'H-spiro[chromene-2,4'-piperidin]-1'-yl)carbonyl]-1-benzothiophen-2-yl}-3-ethylurea (500 mg, 0.922 mmol) obtained in Example 26 and 2-(tributylstannyl)pyridine (750 mg, 1.84 mmol) in 1,4-dioxane (10 mL) was added tetrakistriphenylphosphine palladium (53.3 mg, 0.0461 mmol) under a nitrogen atmosphere and the mixture was stirred with heating at 110°C for 15 hr. After completion of the reaction, the reaction mixture was diluted with ethyl acetate, and the mixture was washed with saturated brine, and dried over anhydrous magnesium sulfate. The solvent was evaporated under reduced pressure, and the obtained residue was purified by basic silica gel column chromatography (ethyl acetate:hexane=7:3 to 1:0) to give the title compound (96 mg, yield 19%) as a powder.
EI(pos) 541 $[M+H]^+$

Example 28

1'-[(2-amino-1-benzothiophen-3-yl)carbonyl]-6-(1-methyl-1H-pyrazol-4-yl)spiro[chromene-2,4'-piperidin]-4

(3H)-one
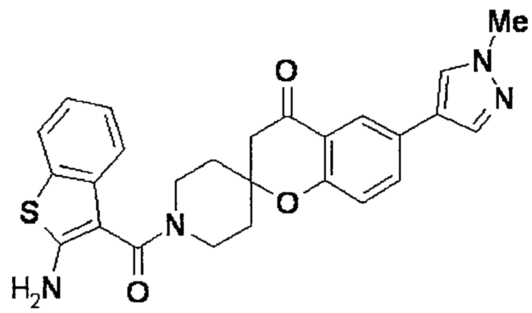

The title compound (440 mg, yield 78%) was obtained as an oil in the same manner as in Example 1 and from 6-(1-methyl-1H-pyrazol-4-yl)spiro[chromene-2,4'-piperidin]-4(3H)-one hydrochloride (400 mg, 1.20 mmol) obtained in Reference Example 114 and 2-amino-1-benzothiophene-3-carboxylic acid (232 mg, 1.20 mmol) (produced by the method described in WO07/013691).
EI(pos) 473 $[M+H]^+$

Example 29

1'-[(2-amino-1-benzothiophen-3-yl)carbonyl]-4-oxo-3,4-dihydrospiro[chromene-2,4'-piperidine]-7-carbonitrile
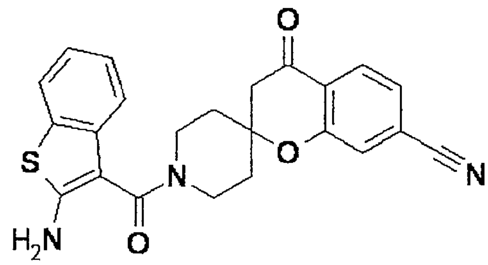

The title compound (580 mg, yield 96%) was obtained as an oil in the same manner as in Example 1 and from 4-oxo-3,4-dihydrospiro[chromene-2,4'-piperidine]-7-carbonitrile hydrochloride (400 mg, 1.44 mmol) obtained in Reference Example 25 and 2-amino-1-benzothiophene-3-carboxylic acid (278 mg, 1.44 mmol) (produced by the method described in WO07/013691).
EI(pos) 417 $[M+H]^+$

Example 30

1-{3-[(7-cyano-4-oxo-3,4-dihydro-1'H-spiro[chromene-2,4'-piperidin]-1'-yl)carbonyl]-1-benzothiophen-2-yl}-3-ethylurea
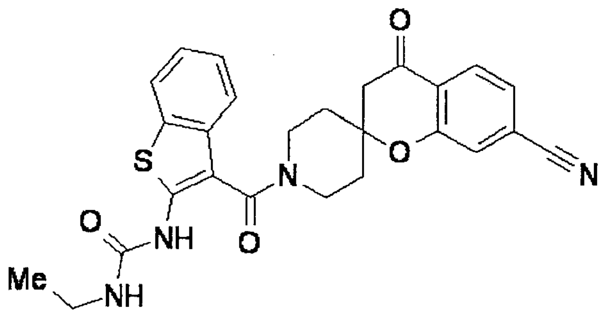

The title compound (480 mg, yield 70%) was obtained as an amorphous solid in the same manner as in Example 2 and from 1'-[(2-amino-1-benzothiophen-3-yl)carbonyl]-4-oxo-3,4-dihydrospiro[chromene-2,4'-piperidine]-7-carbonitrile (580 mg, 1.39 mmol) obtained in Example 29 and ethyl isocyanate (0.22 mL, 2.78 mmol).
EI(pos) 489 $[M+H]^+$

Example 31

1-ethyl-3-(3-{[4-oxo-7-(2H-tetrazol-5-yl)-3,4-dihydro-1'H-spiro[chromene-2,4'-piperidin]-1'-yl]carbonyl}-1-benzothiophen-2-yl)urea

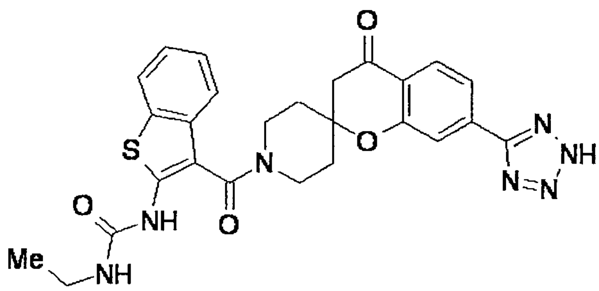

The title compound (411 mg, yield 84%) was obtained as a powder in the same manner as in Example 16 and from 1-{3-[(7-cyano-4-oxo-3,4-dihydro-1'H-spiro[chromene-2,4'-piperidin]-1'-yl)carbonyl]-1-benzothiophen-2-yl}-3-ethylurea (450 mg, 0.921 mmol) obtained in Example 30 and sodium azide (180 mg, 2.77 mmol).
EI(pos) 532 $[M+H]^+$

Example 32

1'-[(2-amino-1-benzothiophen-3-yl)carbonyl]-7-(benzyloxy)spiro[chromene-2,4'-piperidin]-4(3H)-one

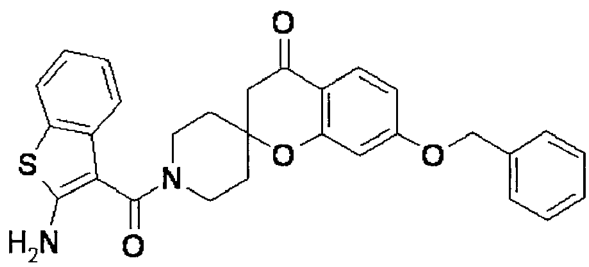

The title compound (693 mg, quantitative) was obtained as an oil in the same manner as in Example 1 and from 7-(benzyloxy)spiro[chromene-2,4'-piperidin]-4(3H)-one hydrochloride (500 mg, 1.39 mmol) obtained in Reference

Example 27 and 2-amino-1-benzothiophene-3-carboxylic acid (269 mg, 1.39 mmol) (produced by the method described in WO07/013691).
EI(pos) 499 $[M+H]^+$

Example 33

1-(3-{[7-(benzyloxy)-4-oxo-3,4-dihydro-1'H-spiro[chromene-2,4'-piperidin]-1'-yl]carbonyl}-1-benzothiophen-2-yl)-3-ethylurea

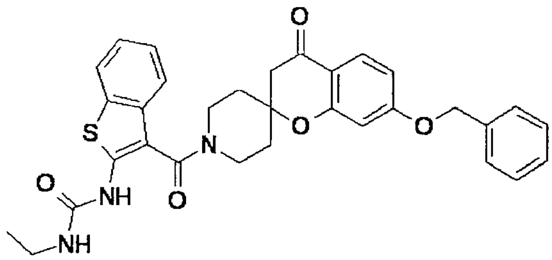

The title compound (654 mg, yield 82%) was obtained as an amorphous solid in the same manner as in Example 2 and from 1'-[(2-amino-1-benzothiophen-3-yl)carbonyl]-7-(benzyloxy)spiro[chromene-2,4'-piperidin]-4(3H)-one (693 mg, 1.39 mmol) obtained in Example 32 and ethyl isocyanate (0.345 mL, 3.53 mmol).
EI(pos) 570 $[M+H]^+$

Example 34

1-ethyl-3-{3-[(7-hydroxy-4-oxo-3,4-dihydro-1'H-spiro[chromene-2,4'-piperidin]-1'-yl)carbonyl]-1-benzothiophen-2-yl}urea

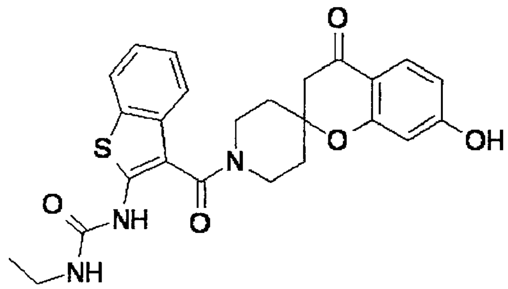

The title compound (106 mg, yield 50%) as a white solid was obtained in the same manner as in Example 24 and from 1-(3-{[7-(benzyloxy)-4-oxo-3,4-dihydro-1'H-spiro[chromene-2,4'-piperidin]-1'-yl]carbonyl}-1-benzothiophen-2-yl)-3-ethylurea (250 mg, 0.439 mmol) obtained in Example 33.
EI(pos) 480 $[M+H]^+$

Example 35

7-acetyl-1'-[(2-amino-1-benzothiophen-3-yl)carbonyl]spiro[chromene-2,4'-piperidin]-4(3H)-one

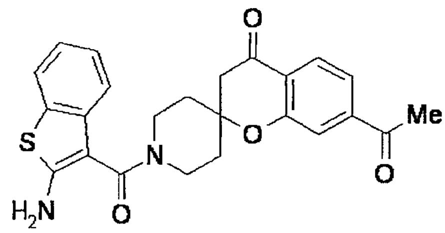

The title compound (640 mg, yield 97%) was obtained as an oil in the same manner as in Example 1 and from 7-acetylspiro[chromene-2,4'-piperidin]-4(3H)-one hydrochloride (450 mg, 1.52 mmol) obtained in Reference Example 30 and 2-amino-1-benzothiophene-3-carboxylic acid (294 mg, 1.52 mmol) (produced by the method described in WO07/013691).
EI(pos) 435 $[M+H]^+$

Example 36

1-{3-[(7-acetyl-4-oxo-3,4-dihydro-1'H-spiro[chromene-2,4'-piperidin]-1'-yl)carbonyl]-1-benzothiophen-2-yl}-3-ethylurea
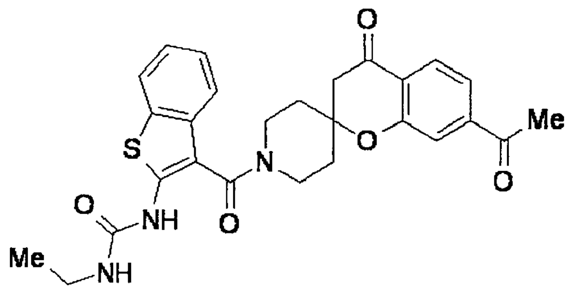

The title compound (442 mg, yield 59%) was obtained as an amorphous solid in the same manner as in Example 2 and from 7-acetyl-1'-[(2-amino-1-benzothiophen-3-yl)carbonyl]spiro[chromene-2,4'-piperidin]-4(3H)-one (640 mg, 1.48 mmol) obtained in Example 35 and ethyl isocyanate (0.234 mL, 2.95 mmol).
EI(pos) 506 $[M+H]^+$

Example 37

1'-({7-methoxy-2-[(methylcarbamoyl)amino]-1-benzothiophen-3-yl}carbonyl)-4-oxo-N-4H-1,2,4-triazol-3-yl-3,4-dihydrospiro[chromene-2,4'-piperidine]-6-carboxamide
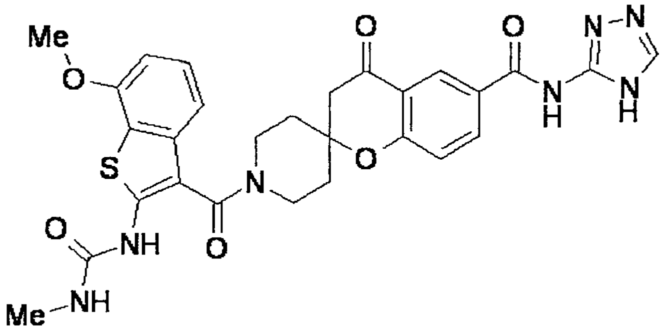

The title compound (62.4 mg, yield 34%) was obtained as a powder in the same manner as in Example 1 and from 1'-({7-methoxy-2-[(methylcarbamoyl)amino]-1-benzothiophen-3-yl}carbonyl)-4-oxo-3,4-dihydrospiro[chromene-2,4'-piperidine]-6-carboxylic acid (161 mg, 0.308 mmol) obtained in Example 24 and 4H-1,2,4-triazol-3-amine (25.9 mg, 0.308 mmol).
EI(pos) 590.1 $[M+H]^+$

Example 38

1'-[(2-amino-1-benzothiophen-3-yl)carbonyl]-4-oxo-3,4-dihydrospiro[chromene-2,4'-piperidine]-5-carbonitrile
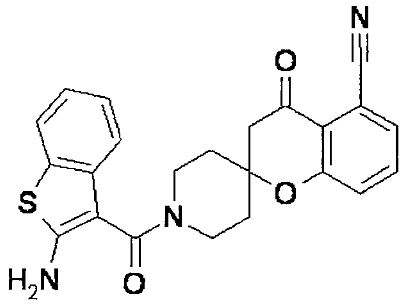

The title compound (720 mg, yield 96%) was obtained as an oil in the same manner as in Example 1 and from 4-oxo-3,4-dihydrospiro[chromene-2,4'-piperidine]-5-carbonitrile hydrochloride (500 mg, 1.80 mmol) obtained in Reference Example 32 and 2-amino-1-benzothiophene-3-carboxylic acid (347 mg, 1.80 mmol) (produced by the method described in WO07/013691).
EI(pos) 418 $[M+H]^+$

Example 39

1-{3-[(5-cyano-4-oxo-3,4-dihydro-1'H-spiro[chromene-2,4'-piperidin]-1'-yl)carbonyl]-1-benzothiophen-2-yl}-3-ethylurea
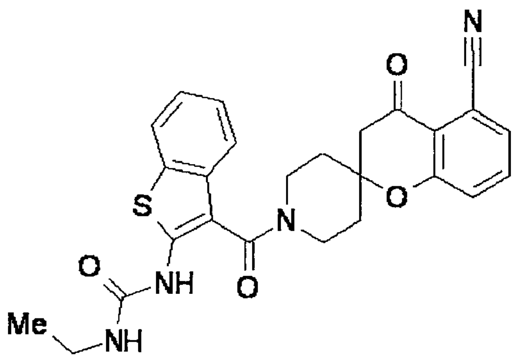

The title compound (742 mg, yield 84%) was obtained as an amorphous solid in the same manner as in Example 2 and from 1'-[(2-amino-1-benzothiophen-3-yl)carbonyl]-4-oxo-3,4-dihydrospiro[chromene-2,4'-piperidine]-5-carbonitrile (750 mg, 1.80 mmol) obtained in Example 38 and ethyl isocyanate (0.285 mL, 3.59 mmol).
EI(pos) 489 $[M+H]^+$

Example 40

1-ethyl-3-(3-{[4-oxo-5-(2H-tetrazol-5-yl)-3,4-dihydro-1'H-spiro[chromene-2,4'-piperidin]-1'-yl]carbonyl}-1-benzothiophen-2-yl)urea

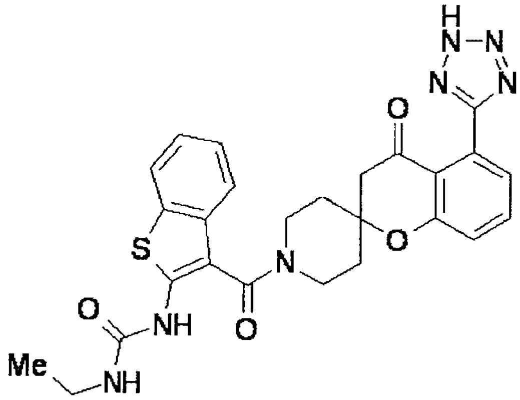

The title compound (35.7 mg, yield 4.7%) was obtained as a powder in the same manner as in Example 16 and from 1-{3-[(5-cyano-4-oxo-3,4-dihydro-1'H-spiro[chromene-2,4'-piperidin]-1'-yl)carbonyl]-1-benzothiophen-2-yl}-3-ethylurea (700 mg, 1.43 mmol) obtained in Example 39 and sodium azide (280 mg, 4.30 mmol).
EI(pos) 532 $[M+H]^+$

Example 41

1'-[(2-amino-1-benzothiophen-3-yl)carbonyl]-4-oxo-3,4-dihydrospiro[chromene-2,4'-piperidine]-6-carbonitrile
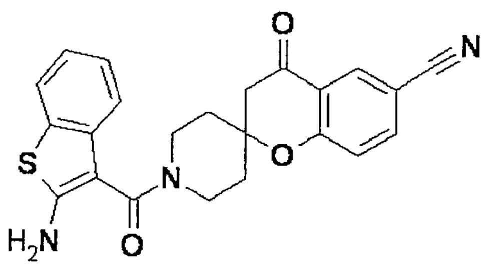

The title compound (570 mg, yield 95%) was obtained as an oil in the same manner as in Example 1 and from 4-oxo-3,4-dihydrospiro[chromene-2,4'-piperidine]-6-carbonitrile hydrochloride (400 mg, 1.44 mmol) and 2-amino-1-benzothiophene-3-carboxylic acid (278 mg, 1.44 mmol) (produced by the method described in WO07/013691).
EI(pos) 418 $[M+H]^+$

Example 42

1-{3-[(6-cyano-4-oxo-3,4-dihydro-1'H-spiro[chromene-2,4'-piperidin]-1'-yl)carbonyl]-1-benzothiophen-2-yl}-3-ethylurea
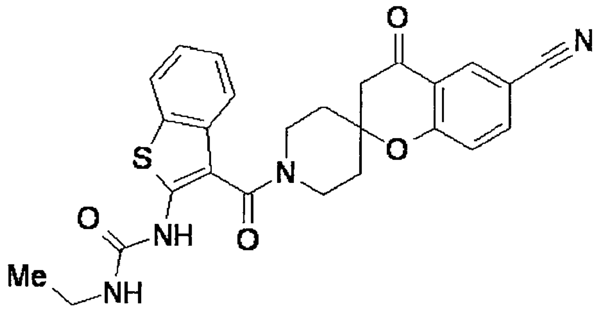

The title compound (570 mg, yield 85%) was obtained as an amorphous solid in the same manner as in Example 2 and from 1'-[(2-amino-1-benzothiophen-3-yl)carbonyl]-4-oxo-3,4-dihydrospiro[chromene-2,4'-piperidine]-6-carbonitrile (570 mg, 1.37 mmol) obtained in Example 41 and ethyl isocyanate (0.217 mL, 2.73 mmol).
EI(pos) 489 $[M+H]^+$

Example 43

1-ethyl-3-(3-{[4-oxo-6-(2H-tetrazol-5-yl)-3,4-dihydro-1'H-spiro[chromene-2,4'-piperidin]-1'-yl]carbonyl}-1-benzothiophen-2-yl)urea
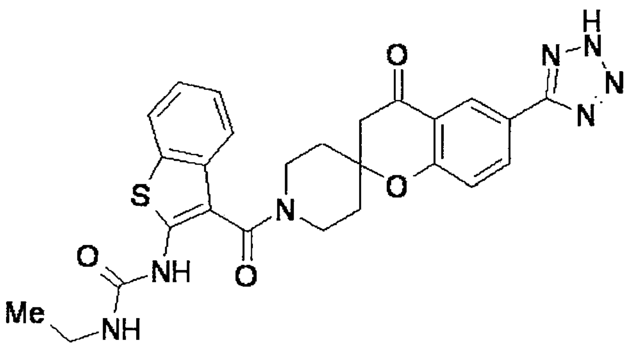

The title compound (190 mg, yield 32%) was obtained as a powder in the same manner as in Example 16 and from 1-{3-[(6-cyano-4-oxo-3,4-dihydro-1'H-spiro[chromene-2,4'-piperidin]-1'-yl)carbonyl]-1-benzothiophen-2-yl}-3-ethylurea (540 mg, 1.11 mmol) obtained in Example 42 and sodium azide (216 mg, 3.32 mmol).
EI(pos) 532 $[M+H]^+$

Example 44

1'-[(2-amino-1-benzothiophen-3-yl)carbonyl]-7-(pyrrolidin-1-yl)spiro[chromene-2,4'-piperidin]-4(3H)-one
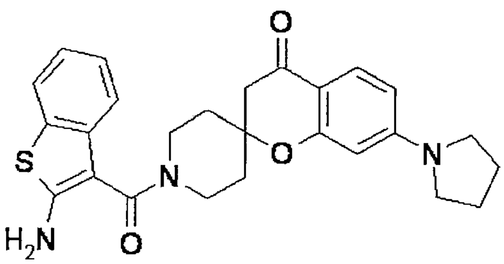

The title compound (641 mg, quantitative) was obtained as an oil in the same manner as in Example 1 and

from 7-(pyrrolidin-1-yl)spiro[chromene-2,4'-piperidin]-4(3H)-one dihydrochloride (500 mg, 1.39 mmol) obtained in Reference Example 34 and 2-amino-1-benzothiophene-3-carboxylic acid (269 mg, 1.39 mmol) (produced by the method described in WO07/013691).
EI(pos) 462 $[M+H]^+$

Example 45

1-ethyl-3-{3-[(4-oxo-7-(pyrrolidin-1-yl)-3,4-dihydro-1'H-spiro[chromene-2,4'-piperidin]-1'-yl)carbonyl]-1-benzothiophen-2-yl}urea
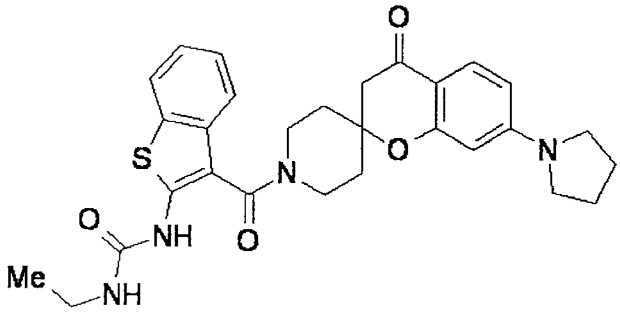

The title compound (568 mg, yield 76%) was obtained as an amorphous solid in the same manner as in Example 2 and from 1'-[(2-amino-1-benzothiophen-3-yl)carbonyl]-7-(pyrrolidin-1-yl)spiro[chromene-2,4'-piperidin]-4(3H)-one (641 mg, 1.39 mmol) obtained in Example 44 and ethyl isocyanate (0.237 mL, 2.99 mmol).
EI(pos) 533 $[M+H]^+$

Example 46

tert-butyl (1-{4-[(6-cyano-4-oxo-3,4-dihydro-1'H-spiro[chromene-2,4'-piperidin]-1'-yl)carbonyl]-5-nitropyridin-2-yl}piperidin-4-yl)carbamate
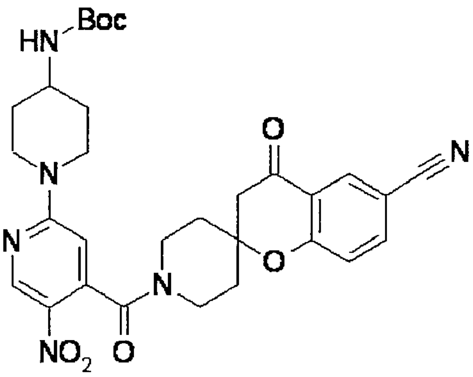

The title compound (455 mg, yield 41%) was obtained as a powder in the same manner as in Example 1 and from 4-oxo-3,4-dihydrospiro[chromene-2,4'-piperidine]-6-carbonitrile hydrochloride (350 mg, 1.25 mmol) and 2-{4-[(tert-butoxycarbonyl)amino]piperidin-1-yl}-5-nitropyridine-4-carboxylic acid (691 mg, 1.88 mmol) obtained in Reference Example 35.
EI(pos) 591.1 $[M+H]^+$

Example 47

tert-butyl (1-{4-[(6-cyano-4-oxo-3,4-dihydro-1'H-spiro[chromene-2,4'-piperidin]-1'-yl)carbonyl]-5-[(methylcarbamoyl)amino]pyridin-2-yl}piperidin-4-yl)carbamate

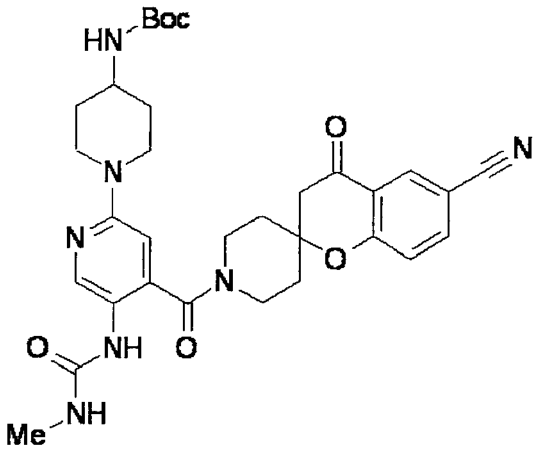

An aniline derivative (372 mg) was obtained as a powder in the same manner as in Example 24 and from tert-butyl (1-{4-[(6-cyano-4-oxo-3,4-dihydro-1'H-spiro[chromene-2,4'-piperidin]-1'-yl)carbonyl]-5-nitropyridin-2-yl}piperidin-4-yl)carbamate (455 mg, 0.770 mmol) obtained in Example 46 and 10% palladium carbon (50% water-containing product (120 mg). The title compound (135 mg, yield 61%) was obtained as a powder by an operation in the same manner as in Example 2 and using the obtained tert-butyl (1-{5-amino-4-[(6-cyano-4-oxo-3,4-dihydro-1'H-spiro[chromene-2,4'-piperidin]-1'-yl)carbonyl]pyridin-2-yl}piperidin-4-yl)carbamate (200 mg, 0.357 mmol) and methyl isocyanate (0.424 mL, 0.713 mmol). EI(pos) 618.2 $[M+H]^+$

Example 48

1-{6-(4-aminopiperidin-1-yl)-4-[(6-cyano-4-oxo-3,4-dihydro-1'H-spiro[chromene-2,4'-piperidin]-1'-yl)carbonyl] pyridin-3-yl}-3-methylurea

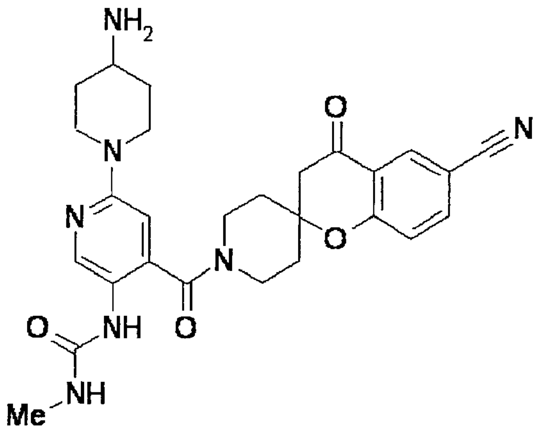

The title compound (54 mg, yield 48%) was obtained as a powder in the same manner as in Example 4 and from tert-butyl (1-{4-[(6-cyano-4-oxo-3,4-dihydro-1'H-spiro[chromene-2,4'-piperidin]-1'-yl)carbonyl]-5-[(methylcarbamoyl)amino]pyridin-2-yl}piperidin-4-yl)carbamate (135 mg, 0.219 mmol) obtained in Example 47 and trifluoroacetic acid (1 mL).
EI(pos) 518.1 $[M+H]^+$

Example 49

1'-({2-[4-(benzyloxy)piperidin-1-yl]-5-nitropyridin-4-yl}carbonyl)-4-oxo-3,4-dihydrospiro[chromene-2,4'-piperidine]-6-carbonitrile

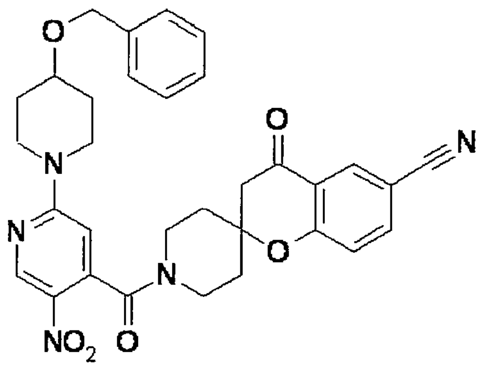

The title compound (400 mg, yield 65%) was obtained as a powder in the same manner as in Example 1 and from 4-oxo-3,4-dihydrospiro[chromene-2,4'-piperidine]-6-carbonitrile hydrochloride (294 mg, 1.06 mmol) and 2-[4-(benzyloxy)piperidin-1-yl]-5-nitropyridine-4-carboxylic acid (568 mg, 1.59 mmol) obtained in Reference Example 36. EI(pos) 582.11 $[M+H]^+$

Example 50

1-{6-[4-(benzyloxy)piperidin-1-yl]-4-[(6-cyano-4-oxo-3,4-dihydro-1'H-spiro[chromene-2,4'-piperidin]-1'-yl)carbonyl]pyridin-3-yl}-3-methylurea

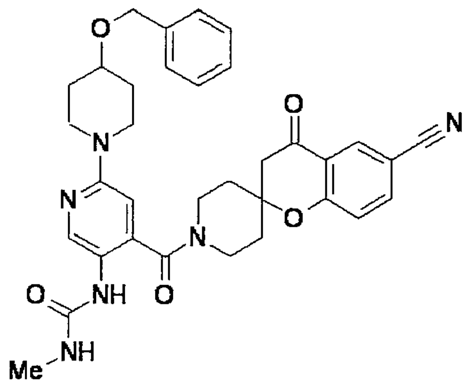

A solution of 1'-({2-[4-(Benzyloxy)piperidin-1-yl]-5-nitropyridin-4-yl}carbonyl)-4-oxo-3,4-dihydrospiro[chromene-2,4'-piperidine]-6-carbonitrile (325 mg, 0.559 mmol) obtained in Example 49, reduced iron (306 mg, 5.60 mmol) and calcium chloride (61.6 mg, 0.560 mmol) in a mixed solvent of ethanol (13 mL), THF (13 mL) and 1N aqueous hydrochloric acid solution (1.1 mL) was stirred with heating at 80°C for 7 hr. After completion of the reaction, the insoluble material was filtered off, and the filtrate was concentrated under reduced pressure. The residue was dissolved in ethyl acetate, and the solution was washed with saturated brine, and dried over anhydrous sodium sulfate. The solvent was evaporated under reduced pressure, and the obtained residue was purified by silica gel column chromatography (ethyl acetate:hexane=7:3 to 1:0) to give an aniline derivative (210 mg, yield 68%) as a powder. The title compound (134 mg, yield 58%) was obtained as a powder by an operation in the same manner as in Example 2 and using the obtained 1'-({5-amino-2-[4-(benzyloxy)piperidin-1-yl]pyridin-4-yl}carbonyl)-4-oxo-3,4-dihydrospiro[chromene-2,4'-piperidine]-6-carbonitrile (210 mg, 0.381 mmol) and methyl isocyanate (0.056 mL, 0.953 mmol).
EI(pos) 609.2 $[M+H]^+$

Example 51

1-{6-(4-hydroxypiperidin-1-yl)-4-[(6-methyl-4-oxo-3,4-dihydro-1'H-spiro[chromene-2,4'-piperidin]-1'-yl)carbonyl]pyridin-3-yl}-3-methylurea

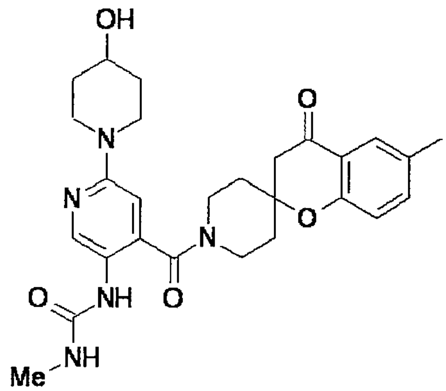

THF (2 mL) was added to 1-{6-[4-(benzyloxy)piperidin-1-yl]-4-[(6-cyano-4-oxo-3,4-dihydro-1'H-spiro [chromene-2,4'-piperidin]-1'-yl)carbonyl]pyridin-3-yl}-3-methylurea (134 mg, 0.219 mmol) obtained in Example 50 and 10% palladium carbon (50% water-containing product, 130 mg), and the mixture was stirred with heating at 65°C for 16 hr under a hydrogen atmosphere. The reaction mixture was filtered, and the filtrate was concentrated under reduced pressure. The obtained residue was purified by basic silica gel column chromatography (ethyl acetate:methanol=1:0 to 17:3) to give the title compound (30.0 mg, yield 27%) as a powder.
EI(pos) 508.5 $[M+H]^+$

Example 52

tert-butyl {5-bromo-3-[(6-cyano-4-oxo-3,4-dihydro-1'H-spiro[chromene-2,4'-piperidin]-1'-yl)carbonyl]thiophen-2-yl}carbamate
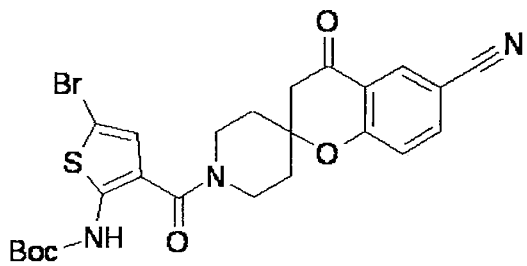

The title compound (478 mg, yield 80%) was obtained as a powder in the same manner as in Example 1 and from 4-oxo-3,4-dihydrospiro[chromene-2,4'-piperidine]-6-carbonitrile hydrochloride (306 mg, 1.10 mmol) and 5-bromo-2-[(tert-butoxycarbonyl)amino]thiophene-3-carboxylic acid (350 mg, 1.10 mmol) (produced by the method described in WO07/013691). EI(pos) 447.9 $[M+H]^+$

Example 53

1'-{[2-amino-5-(1-methyl-1H-pyrazol-4-yl)thiophen-3-yl]carbonyl}-4-oxo-3,4-dihydrospiro[chromene-2,4'-piperidine]-6-carbonitrile
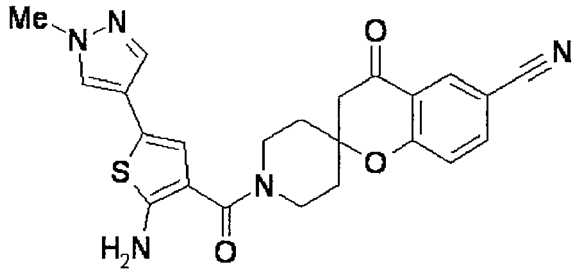

To a solution of tert-butyl {5-bromo-3-[(6-cyano-4-oxo-3,4-dihydro-1'H-spiro[chromene-2,4'-piperidin]-1'-yl)carbonyl]thiophen-2-yl}carbamate (232 mg, 0.425 mmol) obtained in Example 52, (1-methyl-1H-pyrazol-4-yl)boric acid (220 mg, 1.06 mmol) and potassium carbonate (117 mL, 0.850 mmol) in a mixed solvent of 1,4-dioxane (1.4 mL) and water (0.14 mL) was added dichlorobistriphenylphosphine palladium (14.9 mg, 0.0213 mmol), and the mixture was stirred with heating under a nitrogen atmosphere at 100°C for 3 hr. After completion of the reaction, the reaction mixture was diluted with ethyl acetate, and the mixture was washed with saturated brine, and dried over anhydrous sodium sulfate. The residue was subjected to silica gel column chromatography (ethyl acetate). The title compound (97.6 mg, yield 51%, 2 steps) was obtained as a powder by an operation in the same manner as in Example 4 and using the obtained tert-butyl {3-[(6-cyano-4-oxo-3,4-dihydro-1'H-spiro[chromene-2,4'-piperidin]-1'-yl)carbonyl]-5-(1-methyl-1H-pyrazol-4-yl)thiophen-2-yl}carbamate and trifluoroacetic acid (2 mL).
EI(pos) 448.0 $[M+H]^+$

Example 54

1-{3-[(6-cyano-4-oxo-3,4-dihydro-1'H-spiro[chromene-2,4'-piperidin]-1'-yl)carbonyl]-5-(1-methyl-1H-pyrazol-4-yl)thiophen-2-yl}-3-methylurea
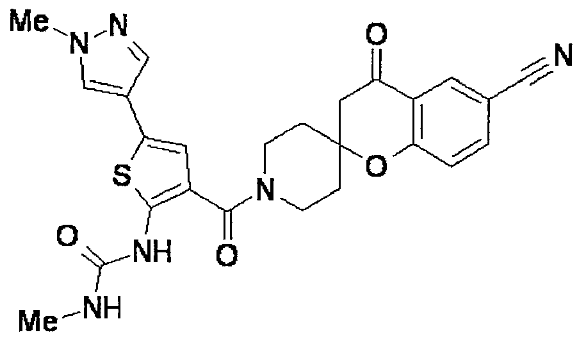

The title compound (46.5 mg, yield 57%) was obtained as a powder in the same manner as in Example 2 and from 1'-{[2-amino-5-(1-methyl-1H-pyrazol-4-yl)thiophen-3-yl]carbonyl}-4-oxo-3,4-dihydrospiro[chromene-2,4'-piperidine]-6-carbonitrile (72.8 mg, 0.163 mmol) obtained in Example 53 and methyl isocyanate (0.019 mL, 0.326 mmol).
EI(pos) 505.1 $[M+H]^+$

Example 55

1-{3-[(2,4-dioxo-1-oxa-3,9-diazaspiro[5.5]undec-9-yl)carbonyl]-1-benzothiophen-2-yl}-3-ethylurea
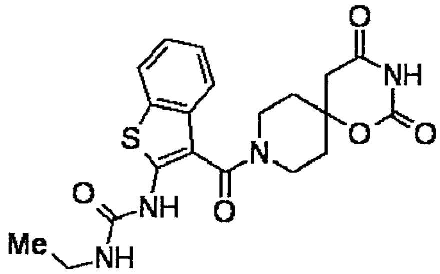

To a solution of benzyl [4-(carbamoyloxy)-1-({2-[(ethylcarbamoyl)amino]-1-benzothiophen-3-yl}carbonyl)piperidin-4-yl]acetate (252 mg, 0.468 mmol) obtained in Reference Example 41 in THF (3 mL) was added potassium tert-butoxide (52.5 mg, 0.468 mmol), and the mixture was stirred at room temperature for 2 hr. The reaction mixture was diluted with ethyl acetate, and the mixture was washed with 0.5N hydrochloric acid and saturated brine, and dried over anhydrous sodium sulfate. The solvent was evaporated under reduced pressure, and the residue was purified by silica gel column chromatography (hexane:ethyl acetate=1:1 to 0:1) to give the title compound (130 mg, yield 65%) as a powder.
EI(pos) 431.1 $[M+H]^+$

Example 56

benzyl {9-[(2-amino-1-benzothiophen-3-yl)carbonyl]-2,4-dioxo-1-oxa-3,9-diazaspiro[5.5]undec-3-yl}acetate
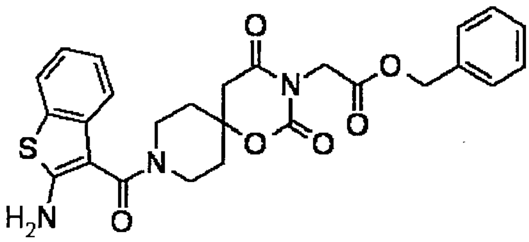

The title compound (298 mg, yield 91%) was obtained as a powder in the same manner as in Example 1 and from benzyl (2,4-dioxo-1-oxa-3,9-diazaspiro[5.5]undec-3-yl)acetate hydrochloride (239 mg, 0.648 mmol) obtained in Reference Example 44 and 2-amino-1-benzothiophene-3-carboxylic acid (125 mg, 0.648 mmol) (produced by the method described in WO07/013691).
EI(pos) 508.5 $[M+H]^+$

Example 57

benzyl [9-({2-[(ethylcarbamoyl)amino]-1-benzothiophen-3-yl}carbonyl)-2,4-dioxo-1-oxa-3,9-diazaspiro[5.5]undec-3-yl]acetate
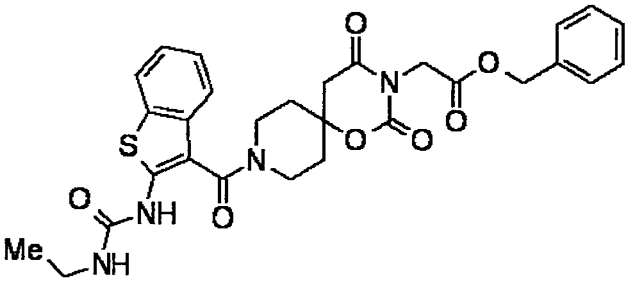

The title compound (237 mg, yield 72%) was obtained as a powder in the same manner as in Example 2 and from benzyl {9-[(2-amino-1-benzothiophen-3-yl)carbonyl]-2,4-dioxo-1-oxa-3,9-diazaspiro[5.5]undec-3-yl}acetate (288 mg, 0.567 mmol) obtained in Example 56 and ethyl isocyanate (0.180 mL, 2.27 mmol).
EI(pos) 579.5 $[M+H]^+$

Example 58

[9-({2-[(ethylcarbamoyl)amino]-1-benzothiophen-3-yl}carbonyl)-2,4-dioxo-1-oxa-3,9-diazaspiro[5.5]undec-3-yl]acetic acid
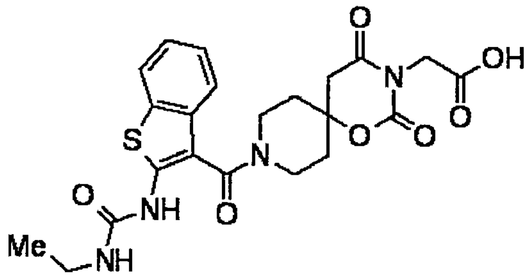

The title compound (197 mg, quantitative) was obtained as a powder in the same manner as in Example 24 and from benzyl [9-({2-[(ethylcarbamoyl)amino]-1-benzothiophen-3-yl}carbonyl)-2,4-dioxo-1-oxa-3,9-diazaspiro[5.5]undec-3-yl]acetate (233 mg, 0.403 mmol) obtained in Example 57.
EI(pos) 489.4 $[M+H]^+$

Example 59

benzyl 3-{9-[(2-amino-1-benzothiophen-3-yl)carbonyl]-2,4-dioxo-1-oxa-3,9-diazaspiro[5.5]undec-3-yl}propanoate
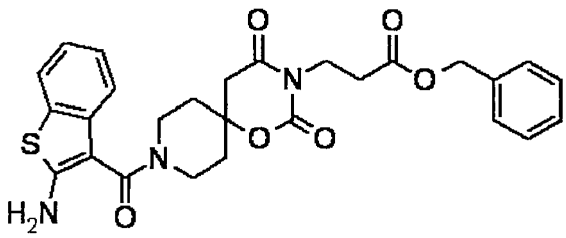

The title compound (234 mg, yield 91%) was obtained as an oil in the same manner as in Example 1 and from benzyl 3-(2,4-dioxo-1-oxa-3,9-diazaspiro[5.5]undec-3-yl)propanoate hydrochloride (189 mg, 0.494 mmol) obtained in Reference Example 46 and 2-amino-1-benzothiophene-3-carboxylic acid (95.4 mg, 0.494 mmol) (produced by the method described in WO07/013691).
EI(pos) 522.1 $[M+H]^+$

Example 60

benzyl 3-[9-({2-[(ethylcarbamoyl)amino]-1-benzothiophen-3-yl}carbonyl)-2,4-dioxo-1-oxa-3,9-diazaspiro[5.5]undec-3-yl]propanoate
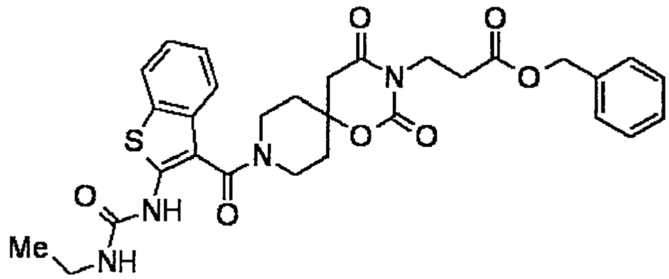

The title compound (178 mg, yield 67%) was obtained as a powder in the same manner as in Example 2 and from benzyl 3-{9-[(2-amino-1-benzothiophen-3-yl)carbonyl]-2,4-dioxo-1-oxa-3,9-diazaspiro[5.5]undec-3-yl}propanoate (234 mg, 0.449 mmol) obtained in Example 59 and ethyl isocyanate (0.142 mL, 1.79 mmol).
EI(pos) 593.6 $[M+H]^+$

Example 61

3-[9-({2-[(ethylcarbamoyl)amino]-1-benzothiophen-3-yl}carbonyl)-2,4-dioxo-1-oxa-3,9-diazaspiro[5.5]undec-3-yl]propanoic acid
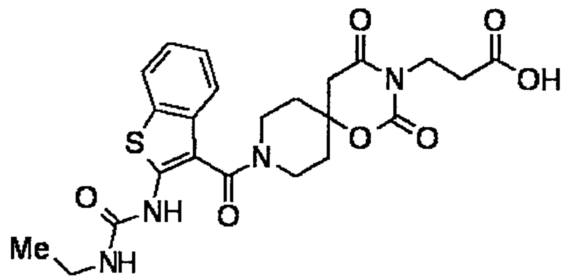

The title compound (104 mg, yield 71%) was obtained as a powder in the same manner as in Example 24 and from benzyl 3-[9-({2-[(ethylcarbamoyl)amino]-1-benzothiophen-3-yl}carbonyl)-2,4-dioxo-1-oxa-3,9-diazaspiro[5.5]undec-3-yl]propanoate (173 mg, 0.292 mmol) obtained in Example 60.
EI(pos) 503.5 $[M+H]^+$

Example 62

9-[(2-amino-1-benzothiophen-3-yl)carbonyl]-3-ethyl-1-oxa-3,9-diazaspiro[5.5]undecane-2,4-dione
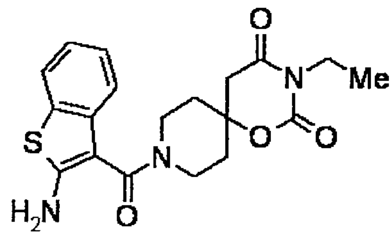

The title compound (695 mg, yield 73%) was obtained as an oil in the same manner as in Example 1 and from 3-ethyl-1-oxa-3,9-diazaspiro[5.5]undecane-2,4-dione hydrochloride (606 mg, 2.44 mmol) obtained in Reference Example 48 and 2-amino-1-benzothiophene-3-carboxylic acid (470 mg, 2.44 mmol) (produced by the method described in WO07/013691).

EI(pos) 388 [M+H]+

Example 63

1-ethyl-3-{3-[(3-ethyl-2,4-dioxo-1-oxa-3,9-diazaspiro[5.5]undec-9-yl)carbonyl]-1-benzothiophen-2-yl}urea
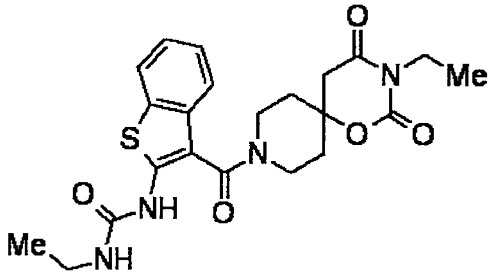

The title compound (702 mg, yield 85%) was obtained as an amorphous solid in the same manner as in Example 2 and from 9-[(2-amino-1-benzothiophen-3-yl)carbonyl]-3-ethyl-1-oxa-3,9-diazaspiro[5.5]undecane-2,4-dione (695 mg, 1.79 mmol) obtained in Example 62 and ethyl isocyanate (0.426 mL, 5.38 mmol).
EI(pos) 459 [M+H]+

Example 64

tert-butyl {6-chloro-4-[(6-cyano-4-oxo-3,4-dihydro-1'H-spiro[chromene-2,4'-piperidin]-1'-yl)carbonyl]pyridin-3-yl}carbamate
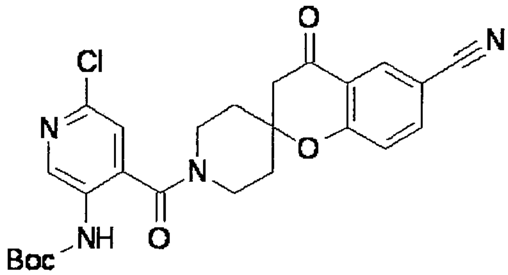

The title compound (243 mg, yield 67%) was obtained as a powder in the same manner as in Example 1 and from 4-oxo-3,4-dihydrospiro[chromene-2,4'-piperidine]-6-carbonitrile hydrochloride (204 mg, 0.735 mmol) and 5-[(tert-butoxycarbonyl)amino]-2-chloropyridine-4-carboxylic acid (200 mg, 0.735 mmol).
EI(pos) 441 [M+H]+

Example 65

1'-[(5-amino-2-phenylpyridin-4-yl)carbonyl]-4-oxo-3,4-dihydrospiro[chromene-2,4'-piperidine]-6-carbonitrile
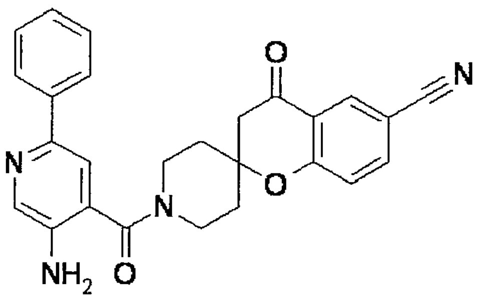

A coupled compound was obtained in the same manner as in Example 53 and from tert-butyl {6-chloro-4-[(6-cyano-4-oxo-3,4-dihydro-1'H-spiro[chromene-2,4'-piperidin]-1'-yl)carbonyl]pyridin-3-yl}carbamate (233 mg, 0.469 mmol) obtained in Example 64 and phenylboric acid (113 mg, 0.938 mmol), and BOC was eliminated with trifluoroacetic acid (3 mL) to give the title compound (163 mg, yield 80%) as a powder.
EI(pos) 439.1 $[M+H]^+$

Example 66

1-{4-[(6-cyano-4-oxo-3,4-dihydro-1'H-spiro[chromene-2,4'-piperidin]-1'-yl)carbonyl]-6-phenylpyridin-3-yl}-3-ethylurea
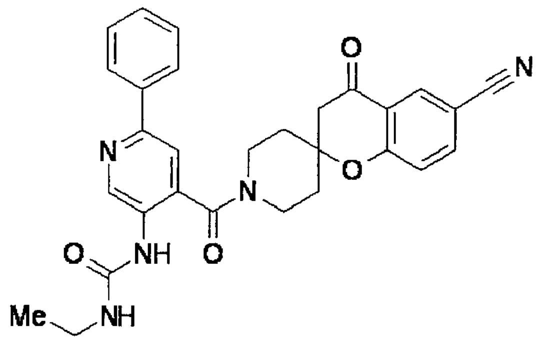

The title compound (106 mg, yield 58%) was obtained as a powder in the same manner as in Example 2 and from 1'-[(5-amino-2-phenylpyridin-4-yl)carbonyl]-4-oxo-3,4-dihydrospiro[chromene-2,4'-piperidine]-6-carbonitrile (158 mg, 0.361 mmol) obtained in Example 65 and ethyl isocyanate (0.071 mL, 0.902 mmol).
EI(pos) 510.1 $[M+H]^+$

Example 67

1'-[(2-amino-1-benzothiophen-3-yl)carbonyl]-4-oxo-3,4-dihydrospiro[chromene-2,4'-piperidine]-6-carbaldehyde
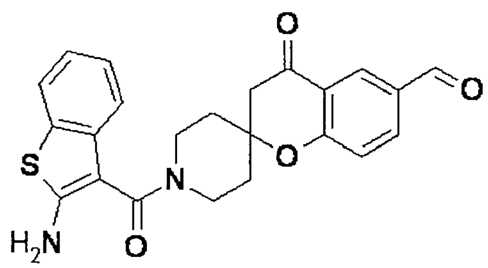

The title compound (233 mg, yield 31%) was obtained as an oil in the same manner as in Example 1 and from 4-oxo-3,4-dihydrospiro[chromene-2,4'-piperidine]-6-carbaldehyde hydrochloride (490 mg, 1.74 mmol) obtained in Reference Example 51 and 2-amino-1-benzothiophene-3-carboxylic acid (370 mg, 1.92 mmol) (produced by the method described in WO07/013691).
EI(pos) 421 $[M+H]^+$

Example 68

1-ethyl-3-{3-[(6-formyl-4-oxo-3,4-dihydro-1'H-spiro[chromene-2,4'-piperidin]-1'-yl)carbonyl]-1-benzothiophen-2-yl}urea
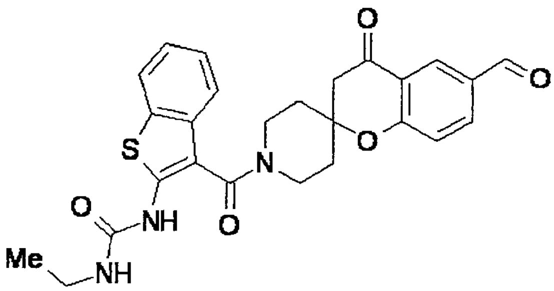

The title compound (246 mg, yield 90%) was obtained as an amorphous solid in the same manner as in Example 2 and from 1'-[(2-amino-1-benzothiophen-3-yl)carbonyl]-4-oxo-3,4-dihydrospiro[chromene-2,4'-piperidine]-6-carbaldehyde (233 mg, 0.555 mmol) obtained in Example 67 and ethyl isocyanate (0.088 mL, 1.11 mmol).
EI(pos) 492 $[M+H]^+$

Example 69

1-[3-({6-[(2,4-dioxo-1,3-thiazolidin-5-ylidene)methyl]-4-oxo-3,4-dihydro-1'H-spiro[chromene-2,4'-piperidin]-1'-yl}carbonyl)-1-benzothiophen-2-yl]-3-ethylurea
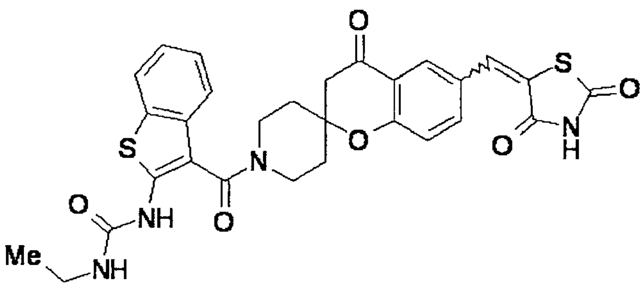

A solution of 1-ethyl-3-{3-[(6-formyl-4-oxo-3,4-dihydro-1'H-spiro[chromene-2,4'-piperidin]-1'-yl)carbonyl]-1-benzothiophen-2-yl}urea (230 mg, 0.468 mmol) obtained in Example 68, thiazolidine-2,4-dione (60.3 mg, 0.515 mmol), pyrrolidine (0.012 mL, 0.141 mmol) and acetic acid (0.008 mL, 0.141 mmol) in toluene (5 mL) was stirred with heating at 100°C for 3 hr. After completion of the reaction, the reaction mixture was diluted with ethyl acetate, and the mixture was washed with 0.5N hydrochloric acid and saturated brine, and dried over anhydrous magnesium sulfate. The solvent was evaporated under reduced pressure, and the obtained residue was purified by silica gel column chromatography (ethyl acetate:hexane=1:1 to 1:0) to give the title compound (146 mg, yield 52%) as a powder.
EI(pos) 591 $[M+H]^+$

Example 70

1'-[(2-amino-1-benzothiophen-3-yl)carbonyl]-8-chlorospiro[chromene-2,4'-piperidin]-4(3H)-one

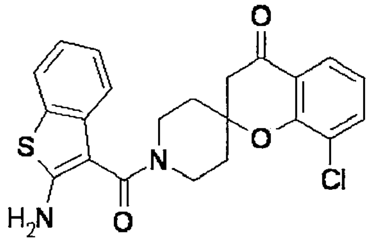

The title compound (460 mg, yield 77%) was obtained as an oil in the same manner as in Example 1 and from 8-chlorospiro[chromene-2,4'-piperidin]-4(3H)-one hydrochloride (400 mg, 1.39 mmol) obtained in Reference Example 53 and 2-amino-1-benzothiophene-3-carboxylic acid (269 mg, 1.39 mmol) (produced by the method described in WO07/013691).
EI(pos) 427 $[M+H]^+$

Example 71

1-{3-[(8-chloro-4-oxo-3,4-dihydro-1'H-spiro[chromene-2,4'-piperidin]-1'-yl)carbonyl]-1-benzothiophen-2-yl}-3-ethylurea
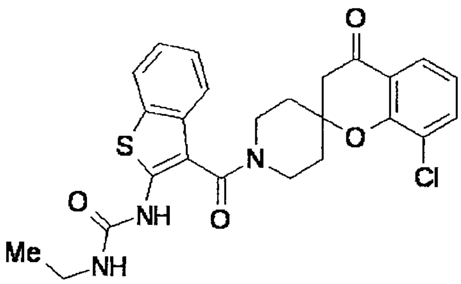

The title compound (360 mg, yield 66%) was obtained as an amorphous solid in the same manner as in Example 2 and from 1'-[(2-amino-1-benzothiophen-3-yl)carbonyl]-8-chlorospiro[chromene-2,4'-piperidin]-4(3H)-one (460 mg, 1.08 mmol) obtained in Example 70 and ethyl isocyanate (0.255 mL, 3.24 mmol).
EI(pos) 498 $[M+H]^+$

Example 72

1'-[(2-amino-1-benzothiophen-3-yl)carbonyl]-6-(benzyloxy)spiro[chromene-2,4'-piperidin]-4(3H)-one
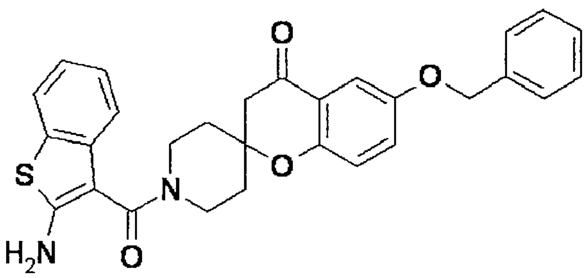

The title compound (610 mg, yield 88%) was obtained as an oil in the same manner as in Example 1 and from

6-(benzyloxy)spiro[chromene-2,4'-piperidin]-4(3H)-one hydrochloride (500 mg, 1.39 mmol) obtained in Reference Example 55 and 2-amino-1-benzothiophene-3-carboxylic acid (269 mg, 1.39 mmol) (produced by the method described in WO07/013691).
EI(pos) 499 $[M+H]^+$

Example 73

1-(3-{[6-(benzyloxy)-4-oxo-3,4-dihydro-1'H-spiro[chromene-2,4'-piperidin]-1'-yl]carbonyl}-1-benzothiophen-2-yl)-3-ethylurea

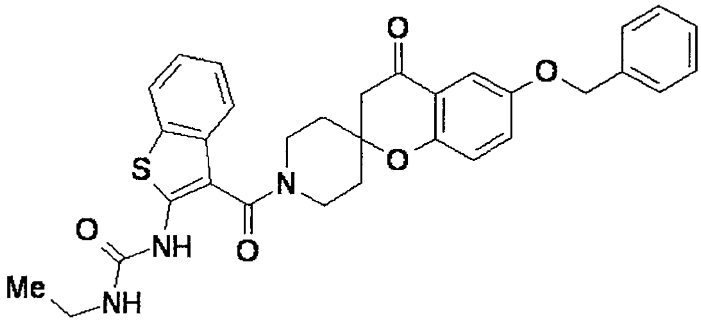

The title compound (396 mg, yield 56%) was obtained as an amorphous solid in the same manner as in Example 2 and from 1'-[(2-amino-1-benzothiophen-3-yl)carbonyl]-6-(benzyloxy)spiro[chromene-2,4'-piperidin]-4(3H)-one (610 mg, 1.23 mmol) obtained in Example 72 and ethyl isocyanate (0.291 mL, 3.67 mmol).
EI(pos) 570 $[M+H]^+$

Example 74

1-ethyl-3-{3-[(6-hydroxy-4-oxo-3,4-dihydro-1'H-spiro[chromene-2,4'-piperidin]-1'-yl)carbonyl]-1-benzothiophen-2-yl}urea

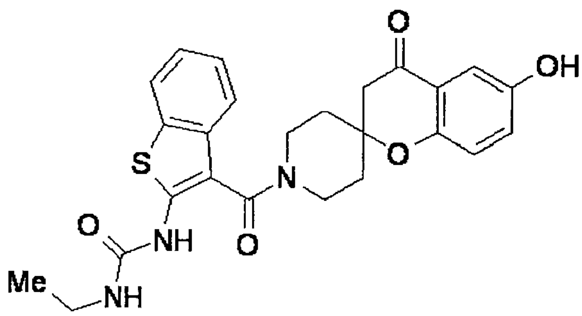

The title compound (167 mg, yield 50%) was obtained as an amorphous solid in the same manner as in Example 24 and from 1-(3-{[6-(benzyloxy)-4-oxo-3,4-dihydro-1'H-spiro[chromene-2,4'-piperidin]-1'-yl]carbonyl}-1-benzothiophen-2-yl)-3-ethylurea (396 mg, 0.449 mmol) obtained in Example 73.
EI(pos) 480 $[M+H]^+$

Example 75

1'-[(2-amino-1-benzothiophen-3-yl)carbonyl]spiro[chromene-2,3'-pyrrolidin]-4(3H)-one

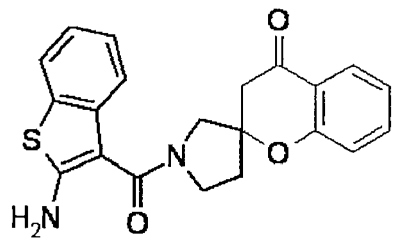

The title compound (820 mg, yield 98%) was obtained as an oil in the same manner as in Example 1 and from spiro[chromene-2,3'-pyrrolidin]-4(3H)-one hydrochloride (530 mg, 2.21 mmol) obtained in Reference Example 57 and 2-amino-1-benzothiophene-3-carboxylic acid (428 mg, 2.21 mmol) (produced by the method described in WO07/013691).
EI(pos) 379 $[M+H]^+$

Example 76

1-ethyl-3-{3-[(4-oxo-3,4-dihydro-1'H-spiro[chromene-2,3'-pyrrolidin]-1'-yl)carbonyl]-1-benzothiophen-2-yl}urea

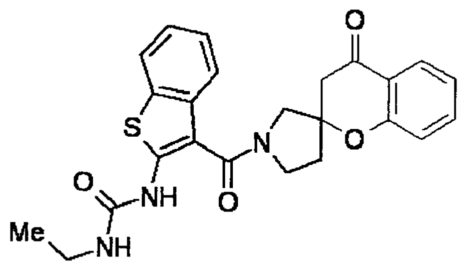

The title compound (612 mg, yield 63%) was obtained as an amorphous solid in the same manner as in Example 2 and from 1'-[(2-amino-1-benzothiophen-3-yl)carbonyl]spiro[chromene-2,3'-pyrrolidin]-4(3H)-one (820 mg, 2.17 mmol) obtained in Example 75 and ethyl isocyanate (0.344 mL, 4.34 mmol).
EI(pos) 450 $[M+H]^+$

Example 77

1'-({2-amino-5-[4-(benzyloxy)phenyl]thiophen-3-yl}carbonyl)-4-oxo-3,4-dihydrospiro[chromene-2,4'-piperidine]-6-carbonitrile

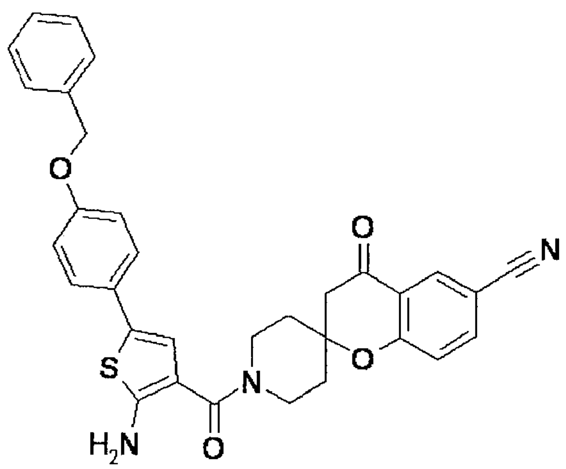

A coupled compound was obtained in the same manner as in Example 53 and from tert-butyl {5-bromo-3-[(6-cyano-4-oxo-3,4-dihydro-1'H-spiro[chromene-2,4'-piperidin]-1'-yl)carbonyl]thiophen-2-yl}carbamate (230 mg, 0.421 mmol) obtained in Example 52 and [4-(benzyloxy)phenyl]boric acid (191 mg, 0.842 mmol), and BOC was eliminated with trifluoroacetic acid (2 mL) to give the title compound (138 mg, yield 71%) as a powder.
EI(pos) 550.1 $[M+H]^+$

Example 78

1-{5-[4-(benzyloxy)phenyl]-3-[(6-cyano-4-oxo-3,4-dihydro-1'H-spiro[chromene-2,4'-piperidin]-1'-yl)carbonyl]thiophen-2-yl}-3-methylurea
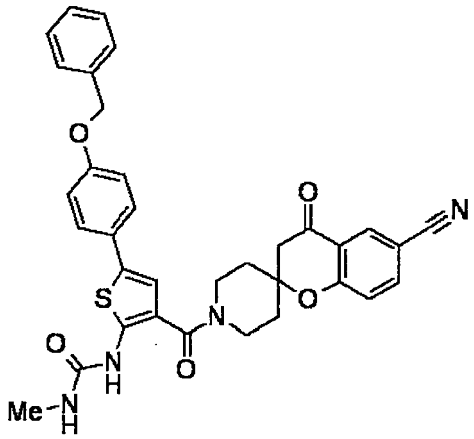

The title compound (93.6 mg, yield 82%) was obtained as a powder in the same manner as in Example 2 and from 1'-({2-amino-5-[4-(benzyloxy)phenyl]thiophen-3-yl}carbonyl)-4-oxo-3,4-dihydrospiro[chromene-2,4'-piperidine]-6-carbonitrile (104 mg, 0.189 mmol) obtained in Example 77 and methyl isocyanate (0.024 mL, 0.378 mmol).
EI(pos) 607.1 $[M+H]^+$

Example 79

1-{3-[(6-cyano-4-oxo-3,4-dihydro-1'H-spiro[chromene-2,4'-piperidin]-1'-yl)carbonyl]-5-(4-hydroxyphenyl)thiophen-2-yl}-3-methylurea
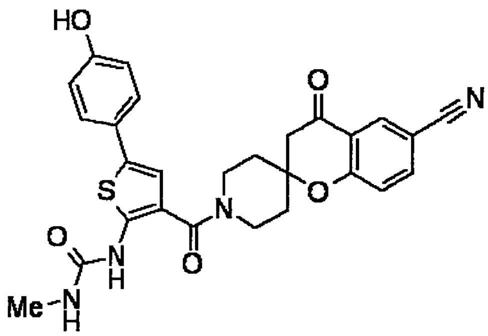

The title compound (18.4 mg, yield 23%) was obtained as a powder in the same manner as in Example 24 and from 1-{5-[4-(benzyloxy)phenyl]-3-[(6-cyano-4-oxo-3,4-dihydro-1'H-spiro[chromene-2,4'-piperidin]-1'-yl)carbonyl]thiophen-2-yl}-3-methylurea (93.6 mg, 0.154 mmol) obtained in Example 78.
$^1$H NMR (DMSO-$d_6$) δ1.75-1.9 (2H, m), 1.95-2.07 (2H, m), 2.66 (3H, d, J = 4.52 Hz), 2.99 (2H, s), 3.30-3.45 (2H, m), 3.90-4.10 (2H, m), 6.76 (2H, d, J = 8.67 Hz), 6.99 (1H, s), 7.23-7.30 (1H, br), 7.31 (1H, d, J = 8.67 Hz), 7.36 (2H, d, J = 8.67 Hz), 8.01 (1H, dd, J = 8.67, 2.07 Hz), 8.14 (1H, d, J = 2.07 Hz), 9.51 (1H, s), 9.53 (1H, s).

Example 80

1-[(2-amino-1-benzothiophen-3-yl)carbonyl]spiro[piperidine-4,2'-pyrano[3,2-b]pyridin]-4'(3'H)-one

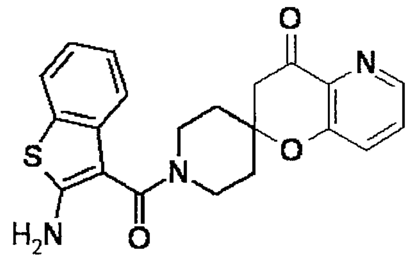

The title compound (286 mg, yield 24%) was obtained as an oil in the same manner as in Example 1 and from spiro[piperidine-4,2'-pyrano[3,2-b]pyridin]-4'(3'H)-one dihydrochloride (879 mg, 3.02 mmol) obtained in Reference Example 60 and 2-amino-1-benzothiophene-3-carboxylic acid (583 mg, 3.02 mmol) (produced by the method described in WO07/013691).
EI(pos) 394 $[M+H]^+$

Example 81

1-ethyl-3-{3-[(4'-oxo-3',4'-dihydro-1H-spiro[piperidine-4,2'-pyrano[3,2-b]pyridin]-1-yl)carbonyl]-1-benzothiophen-2-yl}urea

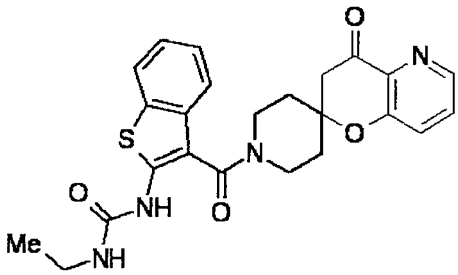

The title compound (305 mg, yield 90%) was obtained as an amorphous solid in the same manner as in Example 2 and from 1-[(2-amino-1-benzothiophen-3-yl)carbonyl]spiro[piperidine-4,2'-pyrano[3,2-b]pyridin]-4'(3'H)-one (286 mg, 0.727 mmol) obtained in Example 80 and ethyl isocyanate (0.144 mL, 1.82 mmol).
EI(pos) 465 $[M+H]^+$

Example 82

1'-[(2-amino-1-benzothiophen-3-yl)carbonyl]-6-(5-oxo-4,5-dihydro-1,2,4-oxadiazol-3-yl)spiro[chromene-2,4'-piperidin]-4(3H)-one

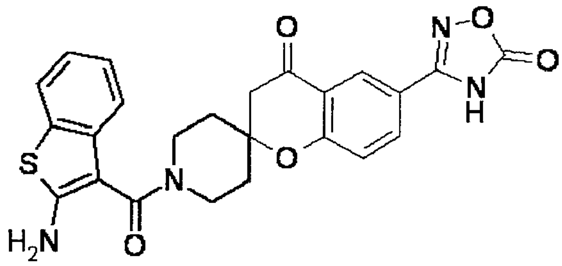

To a solution of 6-(5-oxo-4,5-dihydro-1,2,4-oxadiazol-3-yl)spiro[chromene-2,4'-piperidin]-4(3H)-one hydrochloride (230 mg, 0.681 mmol) obtained in Reference Example 64, 2-amino-1-benzothiophene-3-carboxylic acid (145 mg, 0.749 mmol) (produced by the method described in WO07/013691) and 1-hydroxybenzotriazole (101 mg, 0.749 mmol) in DMF (3 mL) were added 1-ethyl-3-(3'-dimethylaminopropyl)carbodiimide hydrochloride (144 mg, 0.749 mmol) and triethylamine (0.104 mL, 0.749 mmol), and the mixture was stirred at room temperature for 3 days. The reaction mixture was diluted with ethyl acetate, and the mixture was washed with saturated brine, and concentrated under reduced pressure. To the residue was added chloroform and the resulting solid was collected to give the title compound (278 mg, yield 86%).
EI(pos) 477.0 $[M+H]^+$

Example 83

1-ethyl-3-(3-{[4-oxo-6-(5-oxo-4,5-dihydro-1,2,4-oxadiazol-3-yl)-3,4-dihydro-1'H-spiro[chromene-2,4'-piperidin]-1'-yl]carbonyl}-1-benzothiophen-2-yl)urea
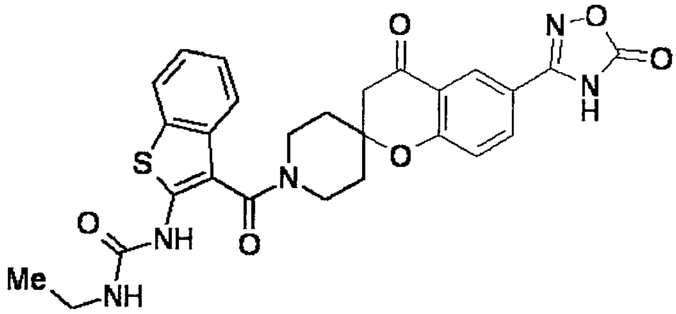

To a solution of 1'-[(2-amino-1-benzothiophen-3-yl)carbonyl]-6-(5-oxo-4,5-dihydro-1,2,4-oxadiazol-3-yl)spiro[chromene-2,4'-piperidin]-4(3H)-one (270 mg, 0.567 mmol) obtained in Example 82 in pyridine (2 mL) was added ethyl isocyanate (0.179 mL, 2.27 mmol), and the mixture was stirred at 70°C for 16 hr. The reaction mixture was diluted with ethyl acetate, and the mixture was washed with 1N hydrochloric acid and saturated brine, and triturated with ethyl acetate and diisopropyl ether to give the title compound (180 mg, yield 58%).
EI(pos) 548.1 $[M+H]^+$

Example 84

1'-[(2-amino-1-benzothiophen-3-yl)carbonyl]-6-bromospiro[1,3-benzoxazin-2,4'-piperidin]-4(3H)-one
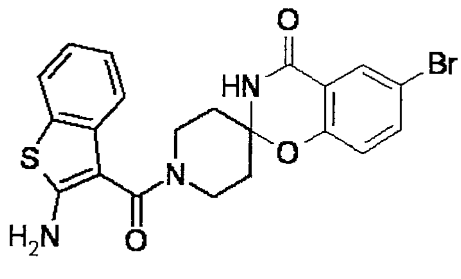

The title compound (576 mg, quantitative) was obtained as a powder in the same manner as in Example 1 and from 6-bromospiro[1,3-benzoxazin-2,4'-piperidin]-4(3H)-one trifluoroacetate (500 mg, 1.22 mmol) and 2-amino-1-benzothiophene-3-carboxylic acid (235 mg, 1.22 mmol) (produced by the method described in WO07/013691).
EI(pos) 474 $[M+H]^+$

Example 85

1-{3-[(6-bromo-4-oxo-3,4-dihydro-1'H-spiro[1,3-benzoxazin-2,4'-piperidin]-1'-yl)carbonyl]-1-benzothiophen-2-

yl}-3-ethylurea

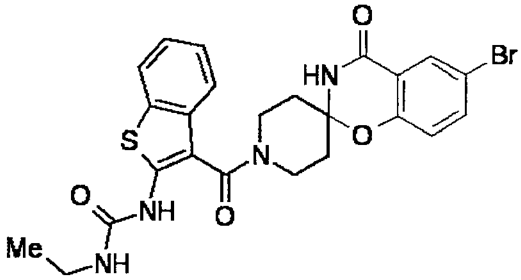

The title compound (299 mg, yield 45%) was obtained as an amorphous solid in the same manner as in Example 2 and from 1'-[(2-amino-1-benzothiophen-3-yl)carbonyl]-6-bromospiro[1,3-benzoxazin-2,4'-piperidin]-4(3H)-one (576 mg, 1.22 mmol) obtained in Example 84 and ethyl isocyanate (0.29 mL, 3.66 mmol).
EI(pos) 545 $[M+H]^+$

Example 86

methyl {1'-[(2-amino-1-benzothiophen-3-yl)carbonyl]-4-oxo-3,4-dihydrospiro[chromene-2,4'-piperidin]-6-yl}acetate

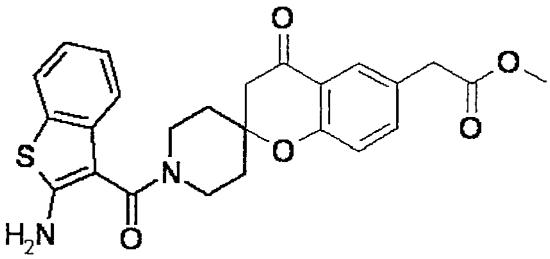

The title compound (602 mg, yield 68%) was obtained as a powder in the same manner as in Example 1 and from methyl (4-oxo-3,4-dihydrospiro[chromene-2,4'-piperidin]-6-yl)acetate (555 mg, 1.92 mmol) obtained in Reference Example 66 and 2-amino-1-benzothiophene-3-carboxylic acid (371 mg, 1.92 mmol) (produced by the method described in WO07/013691).
EI(pos) 465.2 $[M+H]^+$

Example 87

methyl [1'-({2-[(ethylcarbamoyl)amino]-1-benzothiophen-3-yl}carbonyl)-4-oxo-3,4-dihydrospiro[chromene-2,4'-piperidin]-6-yl]acetate

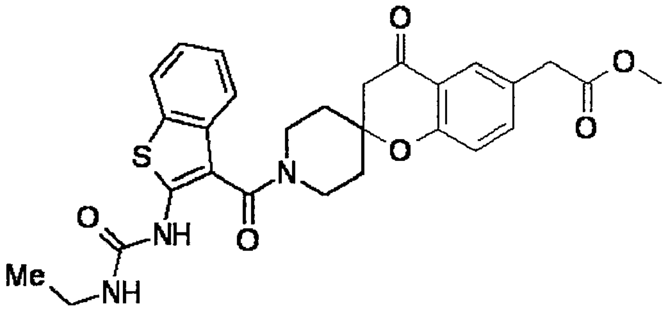

The title compound (539 mg, yield 78%) was obtained as a powder in the same manner as in Example 2 and from methyl {1'-[(2-amino-1-benzothiophen-3-yl)carbonyl]-4-oxo-3,4-dihydrospiro[chromene-2,4'-piperidin]-6-yl}acetate (600 mg, 1.29 mmol) obtained in Example 86 and ethyl isocyanate (0.409 mL, 5.17 mmol).
EI(pos) 536.6 $[M+H]^+$

Example 88

[1'-({2-[(ethylcarbamoyl)amino]-1-benzothiophen-3-yl}carbonyl)-4-oxo-3,4-dihydrospiro[chromene-2,4'-piperidin]-6-yl]acetic acid
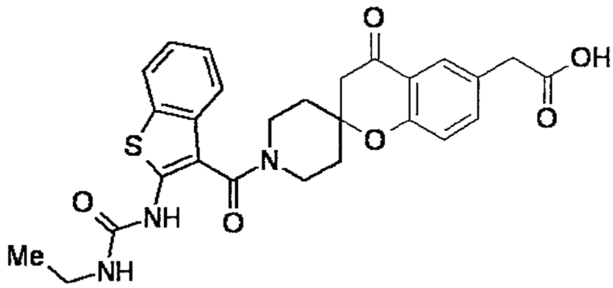

To a solution of methyl [1'-({2-[(ethylcarbamoyl)amino]-1-benzothiophen-3-yl}carbonyl)-4-oxo-3,4-dihydrospiro[chromene-2,4'-piperidin]-6-yl]acetate (130 mg, 0.243 mmol) obtained in Example 87 in methanol (3 mL) was added 1N aqueous sodium hydroxide solution (0.534 mL), and the mixture was stirred at room temperature for 4 hr. After completion of the reaction, the reaction mixture was acidified with 1N hydrochloric acid and extracted with ethyl acetate. The extract was washed with saturated brine, and dried over anhydrous sodium sulfate. The solvent was evaporated under reduced pressure, and the obtained residue was triturated with diisopropyl ether to give the title compound (74.2 mg, yield 58%).
EI(pos) 522.5 $[M+H]^+$

Example 89

1-[(2-amino-1-benzothiophen-3-yl)carbonyl]-1'H-spiro[piperidine-4,2'-quinazolin]-4'(3'H)-one
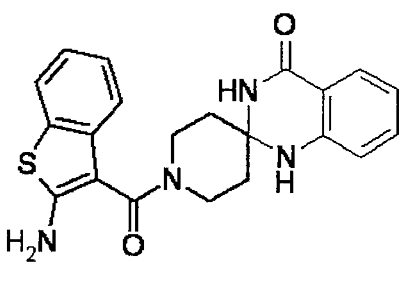

The title compound (500 mg, yield 31%) was obtained as an oil in the same manner as in Example 1 and from 1'H-spiro[piperidine-4,2'-quinazolin]-4'(3'H)-one hydrochloride (1.16 g, 4.00 mmol) obtained in Reference Example 68 and 2-amino-1-benzothiophene-3-carboxylic acid (773 mg, 4.00 mmol) (produced by the method described in WO07/013691).
EI(pos) 393 $[M+H]^+$

Example 90

1-ethyl-3-{3-[(4'-oxo-3',4'-dihydro-1H,1'H-spiro[piperidine-4,2'-quinazolin]-1-yl)carbonyl]-1-benzothiophen-2-yl}urea
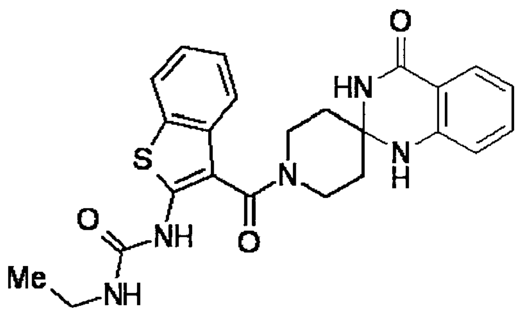

The title compound (230 mg, yield 38%) was obtained as an amorphous solid in the same manner as in Example 2 and from 1-[(2-amino-1-benzothiophen-3-yl)carbonyl]-1'H-spiro[piperidine-4,2'-quinazolin]-4'(3'H)-one (500 mg, 1.28 mmol) obtained in Example 89 and ethyl isocyanate (0.20 mL, 2.55 mmol).
EI(pos) 464 $[M+H]^+$

Example 91

1'-[(2-amino-7-methoxy-1-benzothiophen-3-yl)carbonyl]-6-(5-oxo-4,5-dihydro-1,2,4-oxadiazol-3-yl)spiro[chromene-2,4'-piperidin]-4(3H)-one
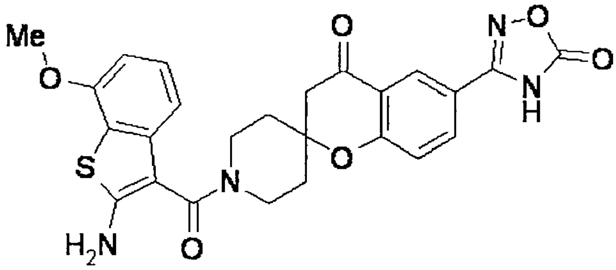

The title compound (152 mg, yield 90%) was obtained as a powder in the same manner as in Example 82 and from 6-(5-oxo-4,5-dihydro-1,2,4-oxadiazol-3-yl)spiro[chromene-2,4'-piperidin]-4(3H)-one hydrochloride (152 mg, 0.450 mmol) obtained in Reference Example 64 and 2-amino-7-methoxy-1-benzothiophene-3-carboxylic acid (111 mg, 0.495 mmol) (produced by the method described in WO07/119833).
EI(pos) 507.1 $[M+H]^+$

Example 92

1-ethyl-3-(7-methoxy-3-{[4-oxo-6-(5-oxo-4,5-dihydro-1,2,4-oxadiazol-3-yl)-3,4-dihydro-1'H-spiro[chromene-2,4'-piperidin]-1'-yl]carbonyl}-1-benzothiophen-2-yl)urea
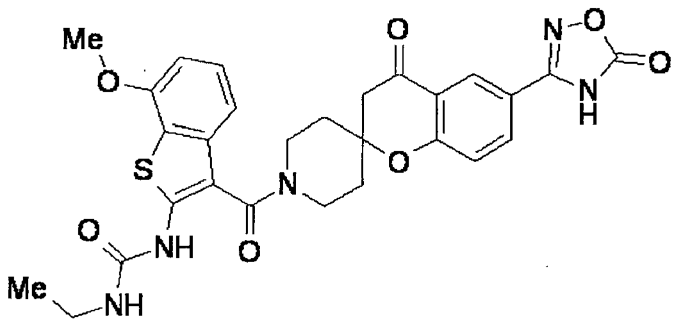

The title compound (70.2 mg, yield 88%) was obtained as a powder in the same manner as in Example 83 and from 1'-[(2-amino-7-methoxy-1-benzothiophen-3-yl)carbonyl]-6-(5-oxo-4,5-dihydro-1,2,4-oxadiazol-3-yl)spiro [chromene-2,4'-piperidin]-4(3H)-one (70 mg, 0.138 mmol) obtained in Example 91 and ethyl isocyanate (0.044 mL, 0.553 mmol).
EI(pos) 578.2 $[M+H]^+$

Example 93

1'-[(2-amino-7-methoxy-1-benzothiophen-3-yl) carbonyl]-6-(5-oxo-4,5-dihydro-1,2,4-thiadiazol-3-yl) spiro [chromene-2,4'-piperidin]-4(3H)-one

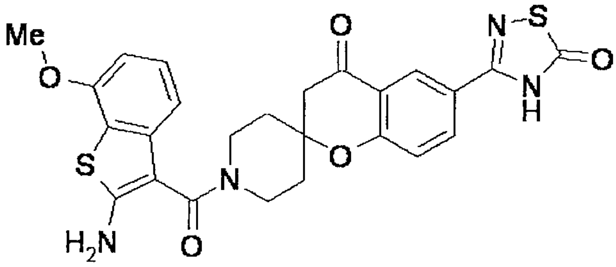

The title compound (86.8 mg, yield 65%) was obtained as a powder in the same manner as in Example 82 and from 6-(5-oxo-4,5-dihydro-1,2,4-thiadiazol-3-yl)spiro[chromene-2,4'-piperidin]-4(3H)-one hydrochloride (90.0 mg, 0.254 mmol) obtained in Reference Example 70 and 2-amino-7-methoxy-1-benzothiophene-3-carboxylic acid (62.5 mg, 0.280 mmol) (produced by the method described in WO07/119833).
EI(pos) 523.5 $[M+H]^+$

Example 94

1-ethyl-3-(7-methoxy-3-{[4-oxo-6-(5-oxo-4,5-dihydro-1,2,4-thiadiazol-3-yl)-3,4-dihydro-1'H-spiro[chromene-2,4'-piperidin]-1'-yl]carbonyl}-1-benzothiophen-2-yl)urea

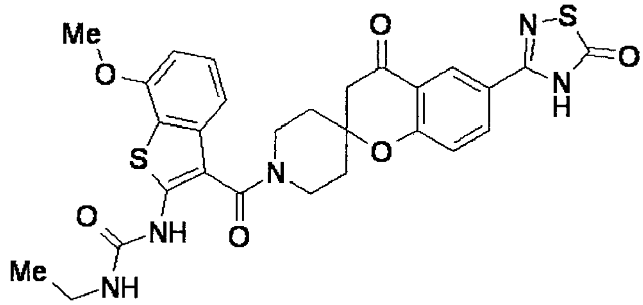

The title compound (77.9 mg, yield 86%) was obtained as a powder in the same manner as in Example 83 and from 1'-[(2-amino-7-methoxy-1-benzothiophen-3-yl)carbonyl]-6-(5-oxo-4,5-dihydro-1,2,4-thiadiazol-3-yl)spiro [chromene-2,4'-piperidin]-4(3H)-one (80 mg, 0.153 mmol) obtained in Example 93 and ethyl isocyanate (0.049 mL, 0.612 mmol).
EI(pos) 594.6 $[M+H]^+$

Example 95

2,2,2-trifluoro-N-(7-methoxy-3-{[4-oxo-6-(5-oxo-4,5-dihydro-1,2,4-oxadiazol-3-yl)-3,4-dihydro-1'H-spiro [chromene-2,4'-piperidin]-1'-yl]carbonyl}-1-benzothiophen-2-yl)acetamide
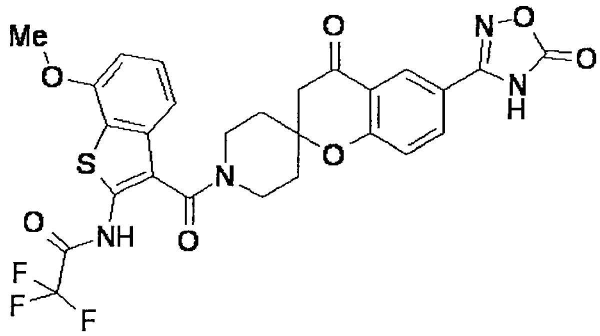

To a solution of 1'-[(2-amino-7-methoxy-1-benzothiophen-3-yl)carbonyl]-6-(5-oxo-4,5-dihydro-1,2,4-oxadiazol-3-yl)spiro[chromene-2,4'-piperidin]-4(3H)-one (77 mg, 0.152 mmol) obtained in Example 91 in pyridine (1 mL) was added trifluoroacetic acid anhydride (0.095 mL, 0.684 mmol), and the mixture was stirred at room temperature for 3 hr. The reaction mixture was diluted with ethyl acetate, and the mixture was washed with 1N hydrochloric acid and saturated brine, and dried over anhydrous sodium sulfate. The residue was purified by silica gel column chromatography (ethyl acetate:hexane=1:1 to 1:0), and triturated with diisopropyl ether to give the title compound (61.3 mg, yield 67%).
EI(pos) 603.5 $[M+H]^+$

Example 96

1'-[(2-amino-6-methylthieno[2,3-b]pyridin-3-yl)carbonyl]-4-oxo-3,4-dihydrospiro[chromene-2,4'-piperidine]-6-carbonitrile
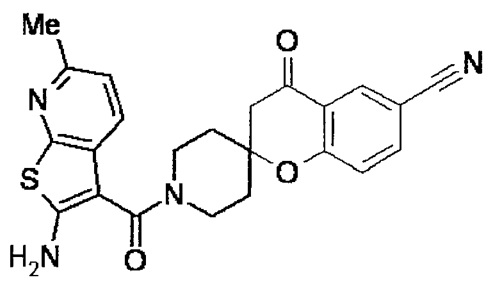

The title compound (797 mg, yield 77%) was obtained as a powder in the same manner as in Example 1 and from 4-oxo-3,4-dihydrospiro[chromene-2,4'-piperidine]-6-carbonitrile hydrochloride (673 mg, 2.41 mmol) and 2-amino-6-methylthieno[2,3-b]pyridine-3-carboxylic acid trifluoroacetate (778 mg, 2.41 mmol) obtained in Reference Example 11.
$^1H$ NMR (DMSO-$d_6$) δ1.76-1.99 (4H, m), 2.44 (3H, s), 2.93-2.99 (2H, m), 3.30-3.32 (2H, m), 3.75-3.79 (2H, m), 6.80 (2H, br), 7.08 (1H, d, J = 8.1 Hz), 7.23-7.31 (1H, m), 7.43-7.55 (1H, m), 8.00 (1H, dd, J = 8.7, 2.1 Hz), 8.12 (1H, d, J = 2.1 Hz).

Example 97

1-{3-[(6-cyano-4-oxo-3,4-dihydro-1'H-spiro[chromene-2,4'-piperidin]-1'-yl)carbonyl]-6-methylthieno[2,3-b]pyridin-2-yl}-3-ethylurea

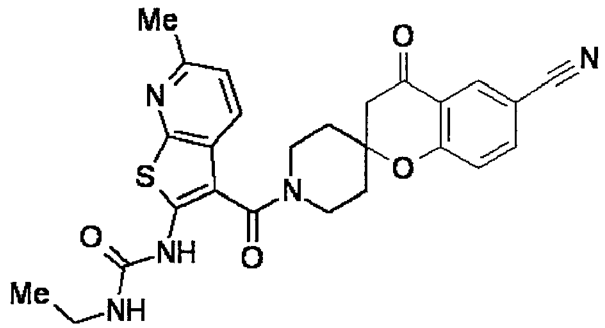

The title compound (206 mg, yield 59%) was obtained as a powder in the same manner as in Example 2 and from 1'-[(2-amino-6-methylthieno[2,3-b]pyridin-3-yl)carbonyl]-4-oxo-3,4-dihydrospiro[chromene-2,4'-piperidine]-6-carbonitrile (300 mg, 0.694 mmol) obtained in Example 96 and ethyl isocyanate (0.165 mL, 20.8 mmol).
$^1$H NMR (DMSO-$d_6$) δ1.10 (3H, t, J = 6.9 Hz), 1.70-1.99 (4H, m), 2.52 (3H, m), 2.94-3.03 (2H, m), 3.11-3.20 (2H, m), 3.20-3.41 (2H, m), 3.79 (2H, m), 7.19-7.40 (3H, m), 7.66-7.78 (1H, m), 7.99-8.04 (1H, m), 8.12 (1H, d, J = 2.1 Hz), 9.32-9.46 (1H, m).

Example 98

benzyl 4-[(6-cyano-4-oxo-3,4-dihydro-1'H-spiro[chromene-2,4'-piperidin]-1'-yl)carbonyl]-3-methyl-5-{[(2,2,2-trichloroethoxy)carbonyl]amino}thiophene-2-carboxylate

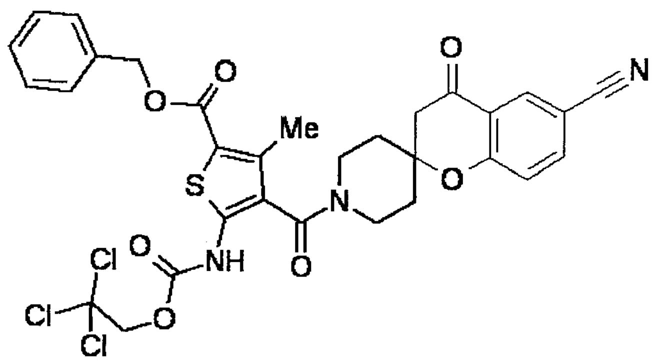

The title compound (81.8 mg, yield 24%) was obtained as a powder in the same manner as in Example 1 and from 4-oxo-3,4-dihydrospiro[chromene-2,4'-piperidine]-6-carbonitrile hydrochloride (135 mg, 0.489 mmol) and 5-[(benzyloxy)carbonyl]-4-methyl-2-{[(2,2,2-trichloroethoxy)carbonyl]amino}thiophene-3-carboxylic acid (228 mg, 0.489 mmol) obtained in Reference Example 72.
EI(pos) 692.4 [M+H]$^+$

Example 99

4-[(6-cyano-4-oxo-3,4-dihydro-1'H-spiro[chromene-2,4'-piperidin]-1'-yl)carbonyl]-3-methyl-5-{[(2,2,2-trichloroethoxy)carbonyl]amino}thiophene-2-carboxylic acid

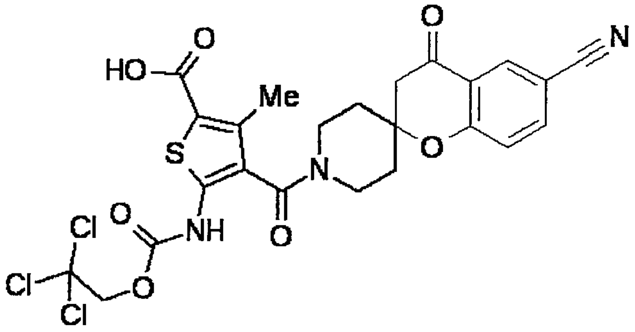

The title compound (11.8 mg, yield 22%) was obtained as a powder in the same manner as in Example 24 and from benzyl 4-[(6-cyano-4-oxo-3,4-dihydro-1'H-spiro[chromene-2,4'-piperidin]-1'-yl)carbonyl]-3-methyl-5-{[(2,2,2-trichloroethoxy)carbonyl]amino}thiophene-2-carboxylate (61.8 mg, 0.090 mmol) obtained in Example 98.
EI(pos) 602.4 $[M+H]^+$

Example 100

benzyl 5-(acetylamino)-4-[(6-cyano-4-oxo-3,4-dihydro-1'H-spiro[chromene-2,4'-piperidin]-1'-yl)carbonyl]-3-methylthiophene-2-carboxylate
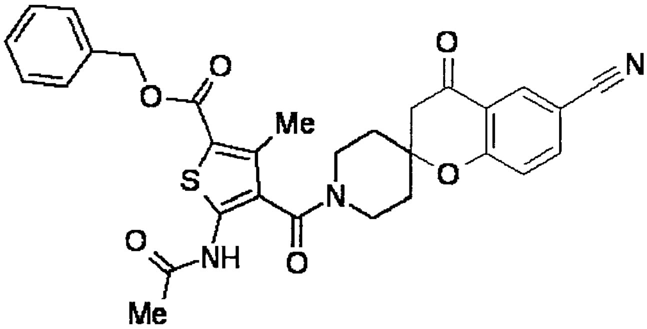

The title compound (8.3 mg, yield 3.3%) was obtained as a powder in the same manner as in Example 1 and from 4-oxo-3,4-dihydrospiro[chromene-2,4'-piperidine]-6-carbonitrile hydrochloride (137 mg, 0.449 mmol) and 2-(acetylamino)-5-[(benzyloxy)carbonyl]-4-methylthiophene-3-carboxylic acid (150 mg, 0.449 mmol) obtained in Reference Example 74.
EI(pos) 558.6 $[M+H]^+$

Example 101

9-[(2-amino-1-benzothiophen-3-yl)carbonyl]-3-ethyl-3,9-diazaspiro[5.5]undecan-2-one
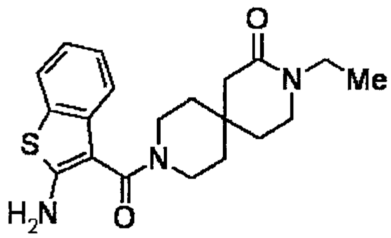

The title compound (618 mg, yield 59%) was obtained as a powder in the same manner as in Example 1 and

from 3-ethyl-3,9-diazaspiro[5.5]undecan-2-one hydrochloride (628 mg, 2.70 mmol) obtained in Reference Example 78 and 2-amino-7-methoxy-1-benzothiophene-3-carboxylic acid (522 mg, 2.70 mmol) (produced by the method described in WO07/119833)
EI(pos) 372 $[M+H]^+$

Example 102

1-ethyl-3-{3-[(9-ethyl-8-oxo-3,9-diazaspiro[5.5]undec-3-yl)carbonyl]-1-benzothiophen-2-yl}urea
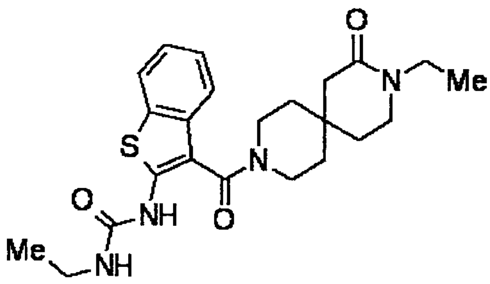

The title compound (453 mg, yield 63%) was obtained as an amorphous solid in the same manner as in Example 2 and from 9-[(2-amino-1-benzothiophen-3-yl)carbonyl]-3-ethyl-3,9-diazaspiro[5.5]undecan-2-one (618 mg, 1.61 mmol) obtained in Example 101 and ethyl isocyanate (0.381 mL, 4.81 mmol). EI(pos) 443 $[M+H]^+$

Example 103

1-ethyl-3-{3-[(9-ethyl-8,10-dioxo-3,9-diazaspiro[5.5]undec-3-yl)carbonyl]-1-benzothiophen-2-yl}urea
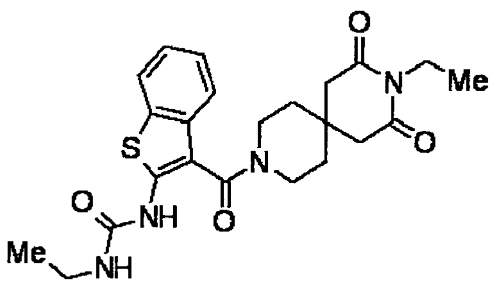

To a solution of ethyl {4-[2-(ethylamino)-2-oxoethyl]-1-({2-[(ethylcarbamoyl)amino]-1-benzothiophen-3-yl}carbonyl)piperidin-4-yl}acetate (673 mg, 1.34 mmol) obtained in Reference Example 82 in DMF (5 mL) was added 60% sodium hydride (53.6 mg, 13.4 mmol), and the mixture was stirred at room temperature for 15 min. The reaction mixture was neutralized with 1N hydrochloric acid, diluted with ethyl acetate, and the mixture was washed 3 times with saturated brine. The organic layer was dried over anhydrous sodium sulfate and the solvent was evaporated under reduced pressure. The obtained residue was purified by basic silica gel column chromatography (ethyl acetate:hexane=7:3 to 1:0) to give the title compound (415 mg, yield 67%) as a powder.
EI(pos) 457 $[M+H]^+$

Example 104

1-ethyl-3-(3-{[9-(1-methylethyl)-8,10-dioxo-3,9-diazaspiro[5.5]undec-3-yl]carbonyl}-1-benzothiophen-2-yl)urea
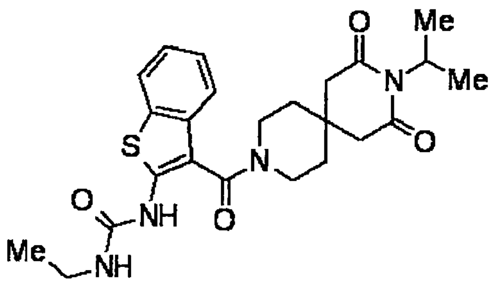

The title compound (267 mg, yield 53%) was obtained as an amorphous solid in the same manner as in Example 103 and from ethyl [1-({2-[(ethylcarbamoyl)amino]-1-benzothiophen-3-yl}carbonyl)-4-{2-[(1-methylethyl)amino]-2-oxoethyl}piperidin-4-yl]acetate (550 mg, 1.07 mmol) obtained in Reference Example 86.
EI(pos) 471 $[M+H]^+$

Example 105

1-ethyl-3-(3-{[9-(2-methylpropyl)-8,10-dioxo-3,9-diazaspiro[5.5]undec-3-yl]carbonyl}-1-benzothiophen-2-yl) urea

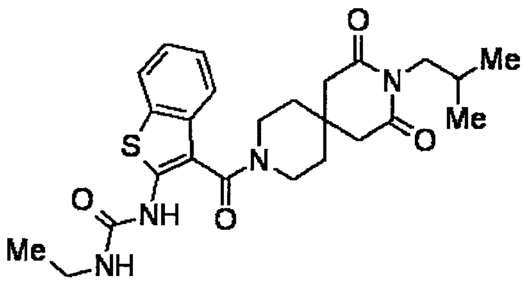

The title compound (525 mg, yield 75%) was obtained as an amorphous solid in the same manner as in Example 103 and from ethyl [1-({2-[(ethylcarbamoyl)amino]-1-benzothiophen-3-yl}carbonyl)-4-{2-[(2-methylpropyl)amino]-2-oxoethyl}piperidin-4-yl]acetate (765 mg, 1.44 mmol) obtained in Reference Example 90.
EI(pos) 485 $[M+H]^+$

Example 106

1-[(2-amino-1-benzothiophen-3-yl)carbonyl]spiro[azepane-4,2'-chromen]-4'(3'H)-one

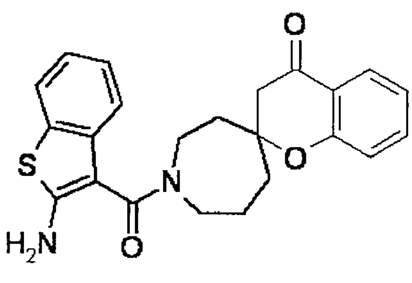

The title compound (242 mg, yield 53%) was obtained as a powder in the same manner as in Example 1 and from spiro[azepane-4,2'-chromen]-4'(3'H)-one hydrochloride (300 mg, 1.12 mmol) obtained in Reference Example 91 and 2-amino-1-benzothiophene-3-carboxylic acid (216 mg, 1.12 mmol) (produced by the method described in WO07/013691).

$^1H$ NMR ($CDCl_3$) δ1.61-1.64 (2H, m), 2.04-2.20 (4H, m), 2.63-2.82 (2H, m), 3.49-3.66 (3H, m), 3.92 (1H, m), 4.90-5.02 (2H, m), 6.95-7.00 (2H, m), 7.07-7.12 (1H, m), 7.25-7.27 (2H, m), 7.44-7.50 (1H, m), 7.54 (1H, d, J = 8.1 Hz), 7.83 (1H, d, J = 7.8 Hz).

Example 107

1-ethyl-3-{3-[(4'-oxo-3',4'-dihydro-1H-spiro[azepane-4,2'-chromen]-1-yl)carbonyl]-1-benzothiophen-2-yl}urea
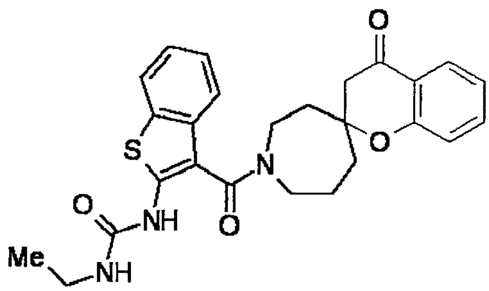

The title compound (216 mg, yield 79%) was obtained as a powder in the same manner as in Example 2 and from 1-[(2-amino-1-benzothiophen-3-yl)carbonyl]spiro[azepane-4,2'-chromen]-4'(3'H)-one (233 mg, 0.573 mmol) obtained in Example 106 and ethyl isocyanate (0.181 mL, 2.29 mmol).
EI(pos) 478.0 $[M+H]^+$

Example 108

tert-butyl {5-bromo-3-[(4-oxo-3,4-dihydro-1'H-spiro[chromene-2,4'-piperidin]-1'-yl)carbonyl]thiophen-2-yl}carbamate
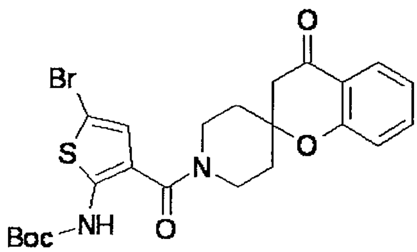

The title compound (4.00 g, yield 88%) was obtained as an amorphous solid in the same manner as in Example 1 and from spiro[chromene-2,4'-piperidin]-4(3H)-one hydrochloride (2.19 g, 8.63 mmol) and 5-bromo-2-[(tert-butoxycarbonyl)amino]thiophene-3-carboxylic acid (2.78 g, 8.63 mmol) (produced by the method described in WO07/013691) EI (pos) 522 $[M+H]^+$

Example 109

benzyl 4-{5-[(tert-butoxycarbonyl)amino]-4-[(4-oxo-3,4-dihydro-1'H-spiro[chromene-2,4'-piperidin]-1'-yl)carbonyl]thiophen-2-yl}benzoate
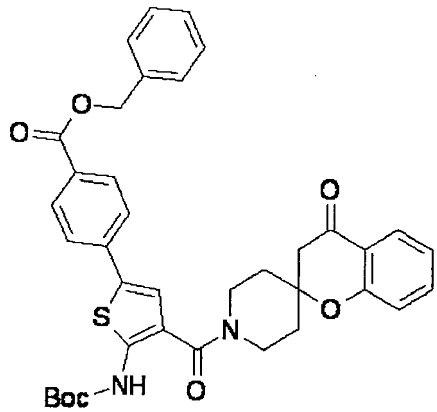

The title compound (940 mg, yield 50%) was obtained as an oil in the same manner as in Example 10 and from tert-butyl {5-bromo-3-[(4-oxo-3,4-dihydro-1'H-spiro[chromene-2,4'-piperidin]-1'-yl)carbonyl]thiophen-2-yl}carbamate (1.50 g, 2.88 mmol) obtained in Example 108 and {4-[(benzyloxy)carbonyl]phenyl}boric acid (1.47 g, 5.75 mmol).
EI(pos) 653 $[M+H]^+$

Example 110

benzyl 4-{5-amino-4-[(4-oxo-3,4-dihydro-1'H-spiro[chromene-2,4'-piperidin]-1'-yl)carbonyl]thiophen-2-yl}benzoate

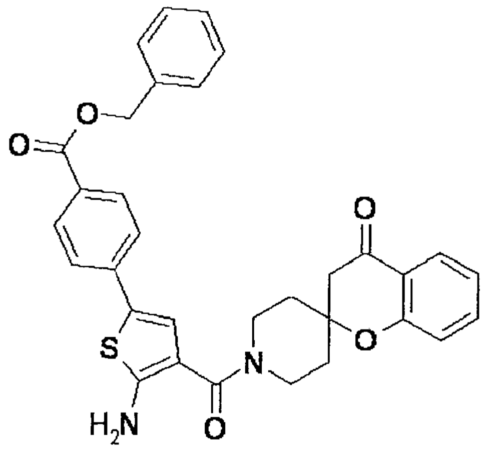

The title compound (580 mg, yield 72%) was obtained as an oil in the same manner as in Example 4 and from benzyl 4-{5-[(tert-butoxycarbonyl)amino]-4-[(4-oxo-3,4-dihydro-1'H-spiro[chromene-2,4'-piperidin]-1'-yl)carbonyl]thiophen-2-yl}benzoate (940 mg, 1.44 mmol) obtained in Example 109 and trifluoroacetic acid (5 mL).
EI(pos) 553 $[M+H]^+$

Example 111

benzyl 4-{5-[(ethylcarbamoyl)amino]-4-[(4-oxo-3,4-dihydro-1'H-spiro[chromene-2,4'-piperidin]-1'-yl)carbonyl]thiophen-2-yl}benzoate

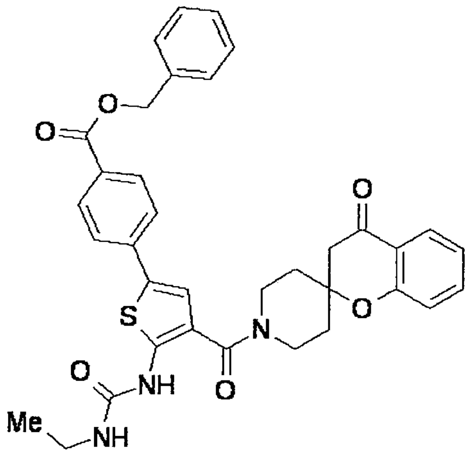

The title compound (639 mg, yield 97%) was obtained as an oil in the same manner as in Example 2 and from benzyl 4-{5-amino-4-[(4-oxo-3,4-dihydro-1'H-spiro[chromene-2,4'-piperidin]-1'-yl)carbonyl]thiophen-2-yl}benzoate (580 mg, 1.05 mmol) obtained in Example 110 and ethyl isocyanate (0.25 mL, 3.15 mmol).
EI(pos) 624 $[M+H]^+$

Example 112

4-{5-[(ethylcarbamoyl) amino]-4-[(4-oxo-3,4-dihydro-1'H-spiro [chromene-2,4'-piperidin]-1'-yl) carbonyl] thiophen-2-yl}benzoic acid
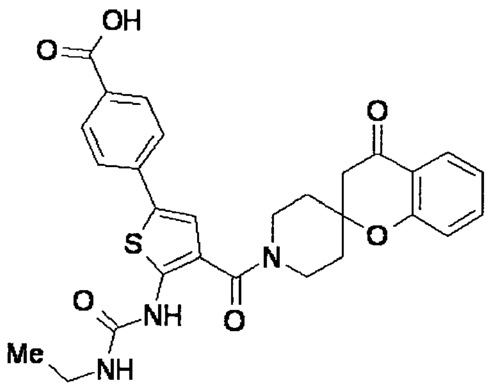

The title compound (221 mg, yield 41%) as a powder was obtained in the same manner as in Example 24 and from benzyl 4-{5-[(ethylcarbamoyl)amino]-4-[(4-oxo-3,4-dihydro-1'H-spiro[chromene-2,4'-piperidin]-1'-yl)carbonyl]thiophen-2-yl}benzoate (639 mg, 1.02 mmol) obtained in Example 111.
EI(pos) 534 $[M+H]^+$

Example 113

1'-[(2-amino-1-benzothiophen-3-yl)carbonyl]spiro[1,3-benzoxazin-2,4'-piperidin]-4(3H)-one
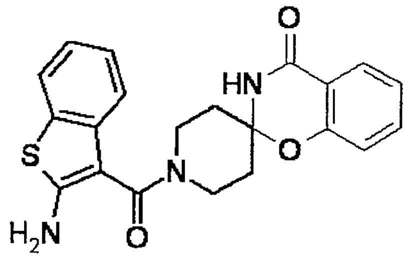

The title compound (227 mg, yield 69%) was obtained as a powder in the same manner as in Example 1 and from spiro[1,3-benzoxazin-2,4'-piperidin]-4(3H)-one (183 mg, 0.838 mmol) obtained in Reference Example 92 and 2-amino-1-benzothiophene-3-carboxylic acid (162 mg, 0.838 mmol) (produced by the method described in WO07/013691).
EI(pos) 394.1 $[M+H]^+$

Example 114

1-ethyl-3-{3-[(4-oxo-3,4-dihydro-1'H-spiro[1,3-benzoxazin-2,4'-piperidin]-1'-yl)carbonyl]-1-benzothiophen-2-yl}urea
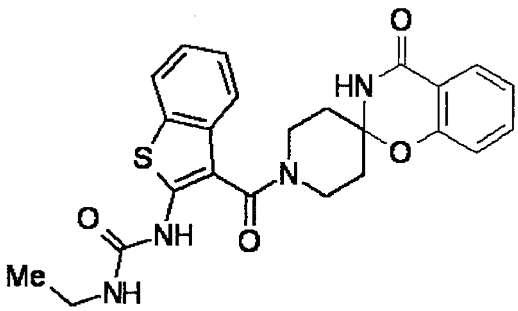

The title compound (174 mg, yield 66%) was obtained as a powder in the same manner as in Example 2 and from 1'-[(2-amino-1-benzothiophen-3-yl)carbonyl]spiro[1,3-benzoxazin-2,4'-piperidin]-4(3H)-one (224 mg, 0.569 mmol) obtained in Example 113 and ethyl isocyanate (0.180 mL, 2.28 mmol).
EI(pos) 465.5 $[M+H]^+$

Example 115

1-[(2-amino-1-benzothiophen-3-yl)carbonyl]spiro[piperidine-4,2'-thiochromen]-4'(3'H)-one
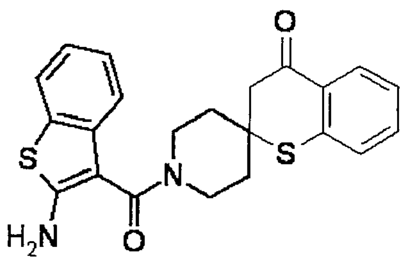

The title compound (723 mg, quantitative) was obtained as an oil in the same manner as in Example 1 and from spiro[piperidine-4,2'-thiochromen]-4'(3'H)-one hydrochloride (477 mg, 1.77 mmol) obtained in Reference Example 93 and 2-amino-1-benzothiophene-3-carboxylic acid (342 mg, 1.77 mmol) (produced by the method described in WO07/013691).
EI(pos) 409 $[M+H]^+$

Example 116

1-ethyl-3-{3-[(4'-oxo-3',4'-dihydro-1H-spiro[piperidine-4,2'-thiochromen]-1-yl)carbonyl]-1-benzothiophen-2-yl} urea

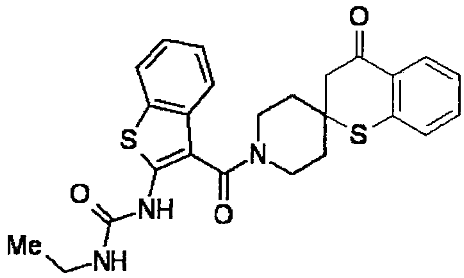

The title compound (687 mg, yield 73%) was obtained as an amorphous solid in the same manner as in Example 2 and from 1-[(2-amino-1-benzothiophen-3-yl)carbonyl]spiro[piperidine-4,2'-thiochromen]-4'(3'H)-one (800 mg, 1.96 mmol) obtained in Example 115 and ethyl isocyanate (0.465 mL, 5.87 mmol).
EI(pos) 480 $[M+H]^+$

Example 117

1-[(2-amino-1-benzothiophen-3-yl)carbonyl]spiro[piperidine-4,2'-thiochromen]-4'(3'H)-one 1'-oxide

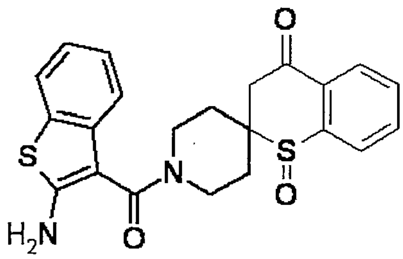

The title compound (390 mg, yield 63%) was obtained as an oil in the same manner as in Example 1 and from spiro[piperidine-4,2'-thiochromen]-4'(3'H)-one 1'-oxide hydrochloride (411 mg, 1.44 mmol) obtained in Reference Example 95 and 2-amino-1-benzothiophene-3-carboxylic acid (277 mg, 1.44 mmol) (produced by the method described in WO07/013691).
EI(pos) 425 $[M+H]^+$

Example 118

1-ethyl-3-{3-[(1'-oxido-4'-oxo-3',4'-dihydro-1H-spiro[piperidine-4,2'-thiochromen]-1-yl)carbonyl]-1-benzothiophen-2-yl}urea

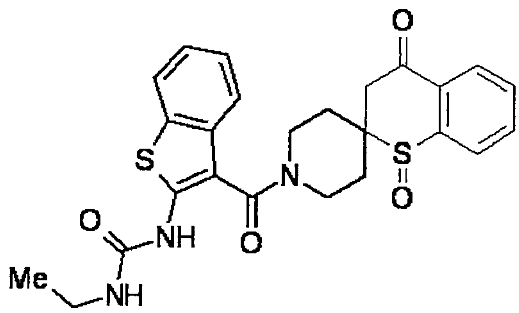

The title compound (116 mg, yield 25%) was obtained as an amorphous solid in the same manner as in Example 2 and from 1-[(2-amino-1-benzothiophen-3-yl)carbonyl]spiro[piperidine-4,2'-thiochromen]-4'(3'H)-one 1'-oxide (390 mg, 0.919 mmol) obtained in Example 117 and ethyl isocyanate (0.220 mL, 2.76 mmol).
EI(pos) 496 $[M+H]^+$

Example 119

1-[(2-amino-1-benzothiophen-3-yl)carbonyl]spiro[piperidine-4,2'-thiochromen]-4'(3'H)-one 1',1'-dioxide
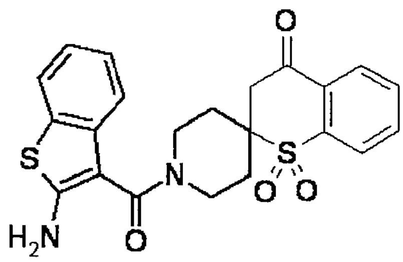

The title compound (615 mg, yield 52%) was obtained as an oil in the same manner as in Example 1 and from spiro[piperidine-4,2'-thiochromen]-4'(3'H)-one 1',1'-dioxide hydrochloride (800 mg, 2.65 mmol) obtained in Reference Example 96 and 2-amino-1-benzothiophene-3-carboxylic acid (513 mg, 2.65 mmol) (produced by the method described in WO07/013691).
EI(pos) 441 $[M+H]^+$

Example 120

1-{3-[(1',1'-dioxido-4'-oxo-3',4'-dihydro-1H-spiro[piperidine-4,2'-thiochromen]-1-yl)carbonyl]-1-benzothiophen-2-yl}-3-ethylurea
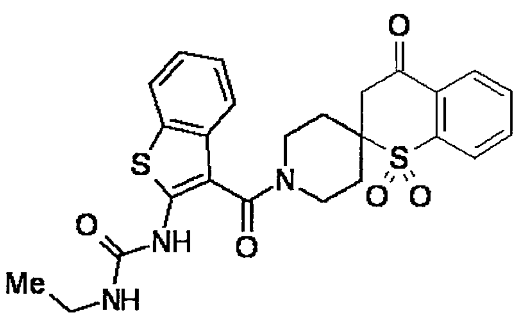

The title compound (443 mg, yield 61%) was obtained as an amorphous solid in the same manner as in Example

2 and from 1-[(2-amino-1-benzothiophen-3-yl)carbonyl]spiro[piperidine-4,2'-thiochromen]-4'(3'H)-one 1',1'-dioxide (615 mg, 1.40mmol) obtained in Example 119 and ethyl isocyanate (0.332 mL, 4.19 mmol).
EI(pos) 512 [M+H]$^+$

Example 121

1-[(2-amino-7-methoxy-1-benzothiophen-3-yl)carbonyl]spiro[piperidine-4,2'-thiochromen]-4'(3'H)-one
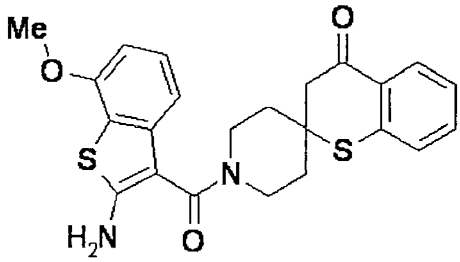

The title compound (314 mg, quantitative) was obtained as an oil in the same manner as in Example 1 and from spiro[piperidine-4,2'-thiochromen]-4'(3'H)-one hydrochloride (193 mg, 0.717 mmol) obtained in Reference Example 93 and 2-amino-7-methoxy-1-benzothiophene-3-carboxylic acid (160 mg, 0.717 mmol) (produced by the method described in WO07/119833).
EI(pos) 439 [M+H]$^+$

Example 122

1-ethyl-3-{7-methoxy-3-[(4'-oxo-3',4'-dihydro-1H-spiro[piperidine-4,2'-thiochromen]-1-yl)carbonyl]-1-benzothiophen-2-yl}urea
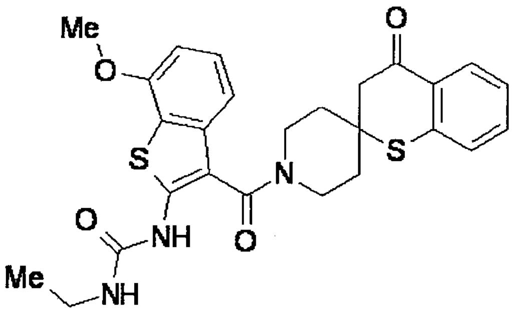

The title compound (311 mg, yield 74%) was obtained as an amorphous solid in the same manner as in Example 2 and from 1-[(2-amino-7-methoxy-1-benzothiophen-3-yl)carbonyl]spiro[piperidine-4,2'-thiochromen]-4'(3'H)-one (360 mg, 0.821 mmol) obtained in Example 121 and ethyl isocyanate (0.200 mL, 2.46 mmol).
EI(pos) 510 [M+H]$^+$

Example 123

1-[(2-amino-6-methylthieno[2,3-b]pyridin-3-yl)carbonyl]spiro[piperidine-4,2'-thiochromen]-4'(3'H)-one
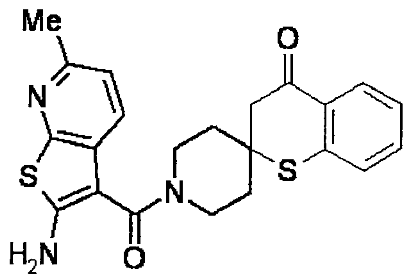

The title compound (597 mg, quantitative) was obtained as a powder in the same manner as in Example 1 and from spiro[piperidine-4,2'-thiochromen]-4'(3'H)-one hydrochloride (269 mg, 1.00 mmol) obtained in Reference Example 93 and 2-amino-6-methylthieno[2,3-b]pyridine-3-carboxylic acid trifluoroacetate (308 mg, 1.00 mmol) obtained in Reference Example 11.
EI(pos) 523.8 $[M+H]^+$

Example 124

1-{6-methyl-3-[(4'-oxo-3',4'-dihydro-1H-spiro[piperidine-4,2'-thiochromen]-1-yl)carbonyl]thieno[2,3-b]pyridin-2-y1}urea

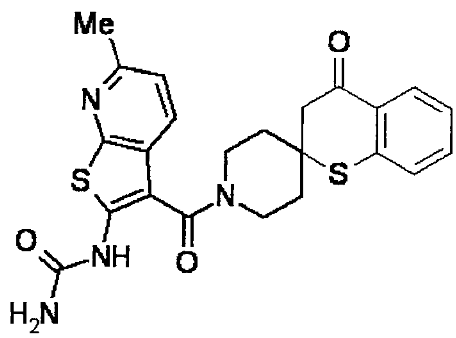

The title compound (153 mg, yield 68%) was obtained as a powder in the same manner as in Reference Example 38 and from 1-[(2-amino-6-methylthieno[2,3-b]pyridin-3-yl)carbonyl]spiro[piperidine-4,2'-thiochromen]-4'(3'H)-one (187 mg, 0.482 mmol) obtained in Example 123, trichloroacetyl isocyanate (0.086 mL, 0.723 mmol) and 7M ammonia-methanol (1 mL).
EI(pos) 467.0 $[M+H]^+$

Example 125

1-methyl-3-{6-methyl-3-[(4'-oxo-3',4'-dihydro-1H-spiro[piperidine-4,2'-thiochromen]-1-yl)carbonyl]thieno[2,3-b]pyridin-2-yl}urea

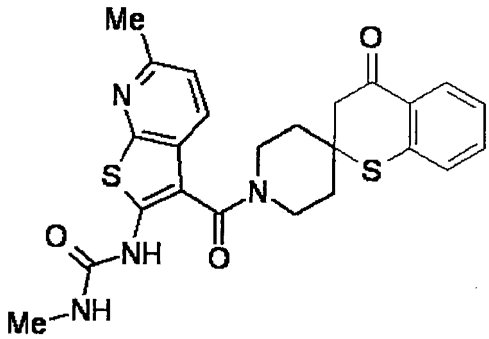

The title compound (134 mg, yield 56%) was obtained as a powder in the same manner as in Example 2 and from 1-[(2-amino-6-methylthieno[2,3-b]pyridin-3-yl)carbonyl]spiro[piperidine-4,2'-thiochromen]-4'(3'H)-one (204 mg, 0.482 mmol) obtained in Example 123 and methyl isocyanate (0.072 mL, 1.21 mmol).
EI(pos) 481.0 $[M+H]^+$

Example 126

1-ethyl-3-{6-methyl-3-[(4'-oxo-3',4'-dihydro-1H-spiro[piperidine-4,2'-thiochromen]-1-yl)carbonyl]thieno[2,3-b]pyridin-2-yl}urea
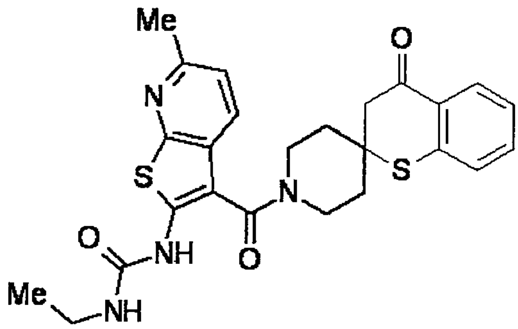

The title compound (205 mg, yield 55%) was obtained as a powder in the same manner as in Example 2 and from 1-[(2-amino-6-methylthieno[2,3-b]pyridin-3-yl)carbonyl]spiro[piperidine-4,2'-thiochromen]-4'(3'H)-one (314 mg, 0.742 mmol) obtained in Example 123 and ethyl isocyanate (0.146 mL, 1.85 mmol).
EI(pos) 494.8 $[M+H]^+$

Example 127

tert-butyl {5,6-dimethyl-3-[(4'-oxo-3',4'-dihydro-1H-spiro[piperidine-4,2'-thiochromen]-1-yl)carbonyl]thieno[2,3-b]pyridin-2-yl}carbamate
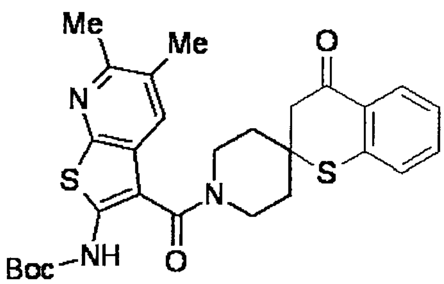

The title compound (710 mg, quantitative) was obtained as an oil in the same manner as in Example 1 and from spiro[piperidine-4,2'-thiochromen]-4'(3'H)-one hydrochloride (269 mg, 1.00 mmol) obtained in Reference Example 93 and 2-[(tert-butoxycarbonyl)amino]-5,6-dimethylthieno[2,3-b]pyridine-3-carboxylic acid (322 mg, 1.00 mmol) obtained in Reference Example 17.
EI(pos) 537.8 $[M+H]^+$

Example 128

1-[(2-amino-5,6-dimethylthieno[2,3-b]pyridin-3-yl)carbonyl]spiro[piperidine-4,2'-thiochromen]-4'(3'H)-one
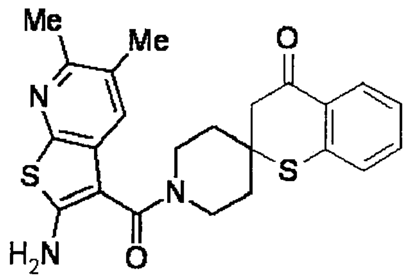

The title compound (300 mg, yield 69%) was obtained as a powder in the same manner as in Example 4 and from tert-butyl {5,6-dimethyl-3-[(4'-oxo-3',4'-dihydro-1H-spiro[piperidine-4,2'-thiochromen]-1-yl)carbonyl]thieno[2,3-b]pyridin-2-yl}carbamate (710 mg, 1.00 mmol) obtained in Example 127 and trifluoroacetic acid (5 mL).
EI(pos) 437.9 $[M+H]^+$

Example 129

1-{5,6-dimethyl-3-[(4'-oxo-3',4'-dihydro-1H-spiro[piperidine-4,2'-thiochromen]-1-yl)carbonyl]thieno[2,3-b]pyridin-2-yl}-3-ethylurea
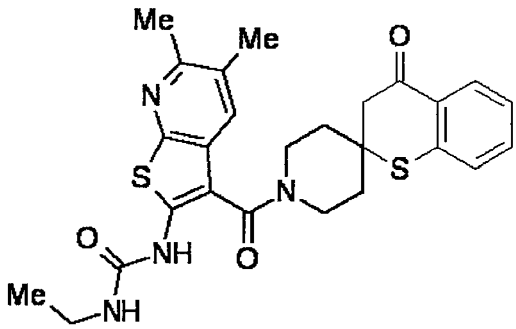

The title compound (146 mg, yield 43%) was obtained as a powder in the same manner as in Example 2 and from 1-[(2-amino-5,6-dimethylthieno[2,3-b]pyridin-3-yl)carbonyl]spiro[piperidine-4,2'-thiochromen]-4'(3'H)-one (300 mg, 0.686 mmol) obtained in Example 128 and ethyl isocyanate (0.135 mL, 1.72 mmol).
EI(pos) 508.8 $[M+]^+$

Example 130

1-[(2- amino- 7- methoxy- 1- benzothiophen- 3- yl) carbonyl]- 6'- bromospiro [piperidine- 4,2'- thiochromen]- 4' (3'H)-one
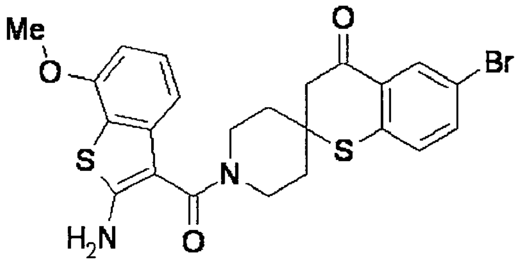

The title compound (836 mg, yield 94%) was obtained as a powder in the same manner as in Example 1 and from 6'-bromospiro[piperidine-4,2'-thiochromen]-4'(3'H)-one hydrochloride (600 mg, 1.72 mmol) obtained in Reference Example 99 and 2-amino-7-methoxy-1-benzothiophene-3-carboxylic acid (423 mg, 1.89 mmol) (produced by the method described in WO07/119833).
EI(pos) 518.8 $[M+H]^+$

Example 131

1-{3-[(6'-bromo-4'-oxo-3',4'-dihydro-1H-spiro[piperidine-4,2'-thiochromen]-1-yl)carbonyl]-7-methoxy-1-benzothiophen-2-yl}-3-ethylurea
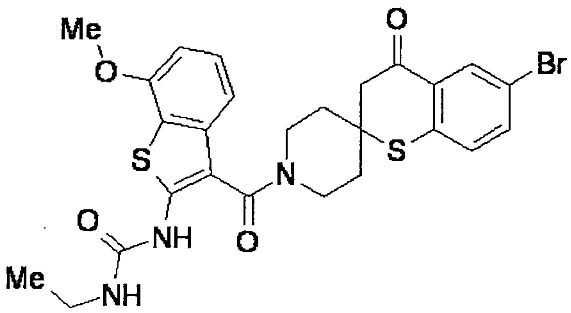

The title compound (650 mg, yield 69%) was obtained as a powder in the same manner as in Example 2 and from 1-[(2-amino-7-methoxy-1-benzothiophen-3-yl)carbonyl]-6'-bromospiro[piperidine-4,2'-thiochromen]-4'(3'H)-one (834 mg, 1.61 mmol) obtained in Example 130 and ethyl isocyanate (0.510 mL, 6.45 mmol).
EI(pos) 589.8 $[M+H]^+$

Example 132

1-{3-[(6'-cyano-4'-oxo-3',4'-dihydro-1H-spiro[piperidine-4,2'-thiochromen]-1-yl)carbonyl]-7-methoxy-1-benzothiophen-2-yl}-3-ethylurea
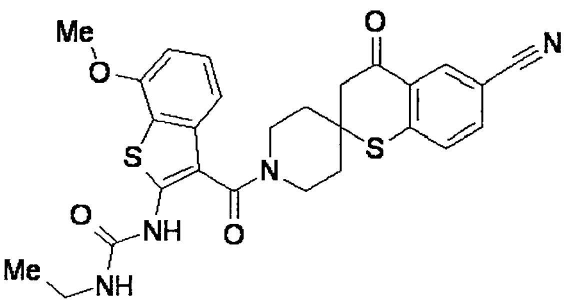

The title compound (477 mg, yield 90%) was obtained as a powder in the same manner as in Example 24 and

from 1-{3-[(6'-bromo-4'-oxo-3',4'-dihydro-1H-spiro[piperidine-4,2'-thiochromen]-1-yl)carbonyl]-7-methoxy-1-benzothiophen-2-yl}-3-ethylurea (586 mg, 0.996 mmol) obtained in Example 131 and zinc cyanide (175 mg, 1.49 mmol).
EI(pos) 535.0 [M+H]+

Example 133

1-[(2- amino- 6- methylthieno [2,3- b] pyridin- 3- yl) carbonyl]- 4'- oxo- 3', 4'- dihydrospiro [piperidine- 4,2'- thiochromene]-6'-carbonitrile

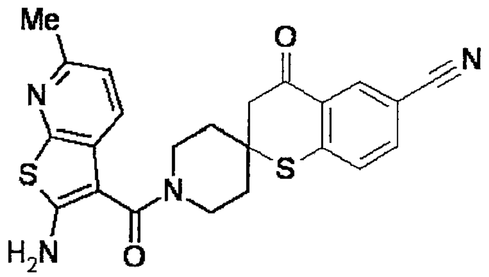

The title compound (173 mg, yield 38%) was obtained as a powder in the same manner as in Example 1 and from 4'-oxo-3',4'-dihydrospiro[piperidine-4,2'-thiochromene]-6'-carbonitrile hydrochloride (294 mg, 1.00 mmol) obtained in Reference Example 103 and 2-amino-6-methylthieno[2,3-b]pyridine-3-carboxylic acid trifluoroacetate (322 mg, 1.00 mmol) obtained in Reference Example 11.
EI(pos) 448.8 [M+H]+

Example 134

1-{3-[(6'-cyano-4'-oxo-3',4'-dihydro-1H-spiro[piperidine-4,2'-thiochromen]-1-yl)carbonyl]-6-methylthieno[2,3-b]pyridin-2-yl}-3-ethylurea

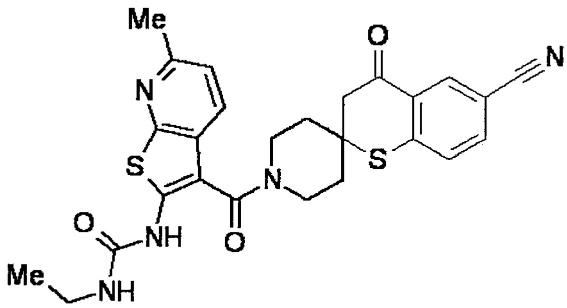

The title compound (94.2 mg, yield 47%) was obtained as a powder in the same manner as in Example 2 and from 1-[(2-amino-6-methylthieno[2,3-b]pyridin-3-yl)carbonyl]-4'-oxo-3',4'-dihydrospiro[piperidine-4,2'-thiochromene]-6'-carbonitrile (173 mg, 0.386 mmol) obtained in Example 133 and ethyl isocyanate (0.304 mL, 3.85 mmol).
EI(pos) 520.0 [M+H]+

Example 135

1-[(2-amino-1-benzothiophen-3-yl)carbonyl]-6'-(5-oxo-4,5-dihydro-1,2,4-oxadiazol-3-yl)spiro[piperidine-4,2'-thiochromen]-4'(3'H)-one

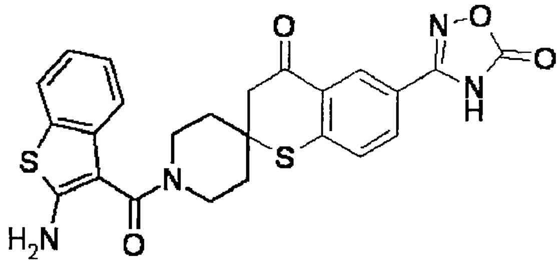

The title compound (352 mg, yield 84%) was obtained as a powder in the same manner as in Example 82 and from 6'-(5-oxo-4,5-dihydro-1,2,4-oxadiazol-3-yl)spiro[piperidine-4,2'-thiochromen]-4'(3'H)-one hydrochloride (300 mg, 0.848 mmol) obtained in Reference Example 108 and 2-amino-1-benzothiophene-3-carboxylic acid (164 mg, 0.848 mmol) (produced by the method described in WO07/013691).

$^1$H NMR (DMSO-$d_6$) δ1.75-1.92 (4H, m), 3.10-3.13 (2H, m), 3.33-3.39 (2H, m), 3.81-3.86 (2H, m), 6.60 (2H, br), 7.00-7.02 (1H, m), 7.16-7.36 (2H, m), 7.55-7.62 (2H, m), 7.88 (1H, m), 8.36 (1H, d, J = 2.4 Hz), 13.06 (1H, br).

Example 136

1-ethyl-3-(3-{[4'-oxo-6'-(5-oxo-4,5-dihydro-1,2,4-oxadiazol-3-yl)-3',4'-dihydro-1H-spiro[piperidine-4,2'-thiochromen]-1-yl]carbonyl}-1-benzothiophen-2-yl)urea

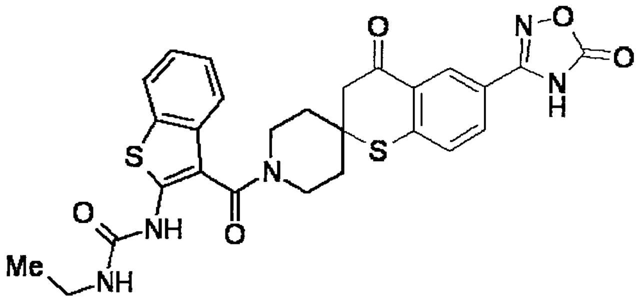

The title compound (163 mg, yield 48%) was obtained as a powder in the same manner as in Example 2 and from 1-[(2-amino-1-benzothiophen-3-yl)carbonyl]-6'-(5-oxo-4,5-dihydro-1,2,4-oxadiazol-3-yl)spiro[piperidine-4,2'-thiochromen]-4'(3'H)-one (300 mg, 0.609 mmol) obtained in Example 135 and ethyl isocyanate (0.193 mL, 2.44 mmol).

$^1$H NMR (DMSO-$d_6$) δ1.02-1.11 (3H, m), 1.90 (4H, m), 3.09-3.43 (6H, m), 3.81 (2H, m), 7.11-7.34 (3H, m), 7.40-7.53 (1H, m), 7.59-7.62 (1H, m), 7.78 (1H, d, J = 8.1 Hz), 7.89 (1H, d, J = 8.7 Hz), 8.36 (1H, s), 9.21, 9.28 (1H, sx2), 13.07 (1H, br).

Example 137

1-ethyl-3-(7-methoxy-3-{[9-(1-methylethyl)-8,10-dioxo-3,9-diazaspiro[5.5]undec-3-yl]carbonyl}-1-benzothiophen-2-yl)urea

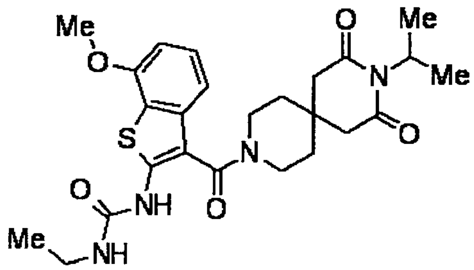

The title compound (454 mg, yield 60%) was obtained as a powder in the same manner as in Example 103 and from ethyl [1-({2-[(ethylcarbamoyl)amino]-7-methoxy-1-benzothiophen-3-yl}carbonyl)-4-{2-[(1-methylethyl)amino]-2-oxoethyl}piperidin-4-yl]acetate (814 mg, 1.49 mmol) obtained in Reference Example 110.
EI(pos) 501 $[M+H]^+$

Example 138

8-[(2-amino-7-methoxy-1-benzothiophen-3-yl)carbonyl]-3-ethyl-1,3,8-triazaspiro[4.5]decane-2,4-dione
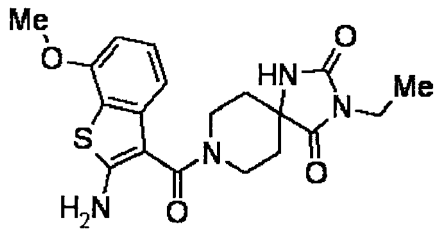

The title compound (598 mg, yield 69%) was obtained as a powder in the same manner as in Example 1 and from 3-ethyl-1,3,8-triazaspiro[4.5]decane-2,4-dione hydrochloride (500 mg, 2.14 mmol) obtained in Reference Example 112 and 2-amino-7-methoxy-1-benzothiophene-3-carboxylic acid (525 mg, 2.36 mmol) (produced by the method described in WO07/119833).
EI(pos) 403 $[M+H]^+$

Example 139

1-ethyl-3-{3-[(3-ethyl-2,4-dioxo-1,3,8-triazaspiro[4.5]dec-8-yl)carbonyl]-7-methoxy-1-benzothiophen-2-yl}urea
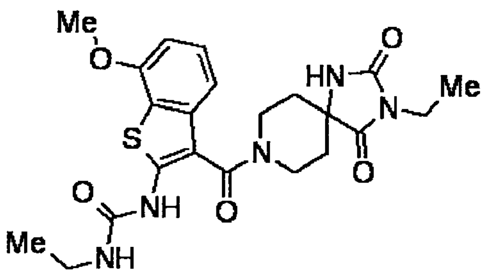

The title compound (403 mg, yield 57%) was obtained as an amorphous solid in the same manner as in Example 2 and from 8-[(2-amino-7-methoxy-1-benzothiophen-3-yl)carbonyl]-3-ethyl-1,3,8-triazaspiro[4.5]decane-2,4-dione (598 mg, 1.49 mmol) obtained in Example 138 and ethyl isocyanate (0.236 mL, 2.98 mmol).
EI(pos) 474 $[M+H]^+$

Example 140

1-ethyl-3-(3-{[6-(1-methyl-1H-pyrazol-4-yl)-4-oxo-3,4-dihydro-1'H-spiro[chromene-2,4'-piperidin]-1'-yl]carbonyl}-1-benzothiophen-2-yl)urea
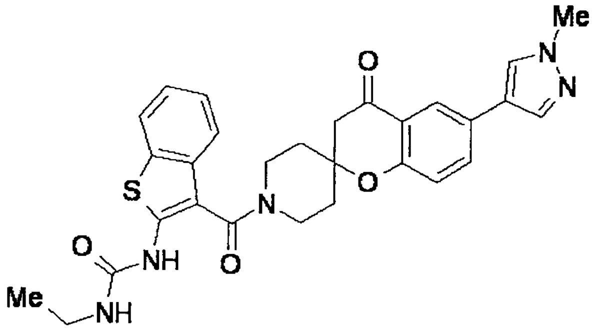

The title compound (277 mg, yield 55%) was obtained as an amorphous solid in the same manner as in Example 2 and from 1'-[(2-amino-1-benzothiophen-3-yl)carbonyl]-6-(1-methyl-1H-pyrazol-4-yl)spiro[chromene-2,4'-piperidin]-4(3H)-one (440 mg, 0.932 mmol) obtained in Example 28 and ethyl isocyanate (0.148 mL, 1.87 mmol).
EI(pos) 544 $[M+H]^+$

Example 141

1'-[(2-amino-1-benzothiophen-3-yl)carbonyl]-3,3-dimethylspiro[chromene-2,4'-piperidin]-4(3H)-one
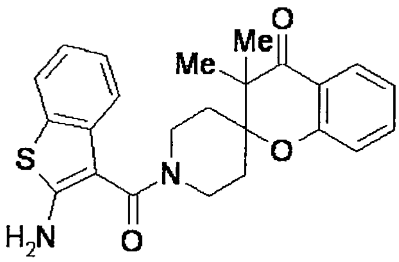

The title compound (128 mg, yield 29%) was obtained as a powder in the same manner as in Example 1 and from 3,3-dimethylspiro[chromene-2,4'-piperidin]-4(3H)-one hydrochloride (300 mg, 1.06 mmol) obtained in Reference Example 134 and 2-amino-1-benzothiophene-3-carboxylic acid (206 mg, 1.06 mmol) (produced by the method described in WO07/013691).
$^1$H NMR ($CDCl_3$) δ1.16, 1.24 (6H, sx2), 1.64-2.04 (4H, m), 3.24-3.40 (2H, m), 4.08-4.13 (2H, m), 5.15, 5.37 (2H, brx2), 6.94-7.10 (3H, m), 7.25-7.33 (2H, m), 7.39-7.53 (2H, m), 7.85 (1H, d, J = 7.8 Hz).

Example 142

1-{3-[(3,3-dimethyl-4-oxo-3,4-dihydro-1'H-spiro[chromene-2,4'-piperidin]-1'-yl)carbonyl]-1-benzothiophen-2-yl}-3-ethylurea
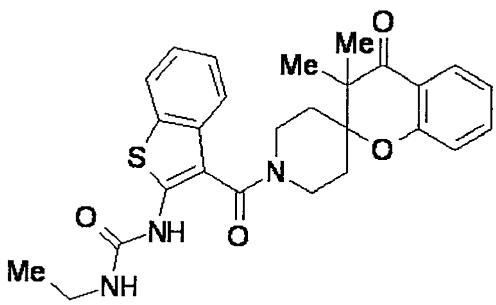

The title compound (100 mg, yield 69%) was obtained as a powder in the same manner as in Example 2 and

from 1'-[(2-amino-1-benzothiophen-3-yl)carbonyl]-3,3-dimethylspiro[chromene-2,4'-piperidin]-4(3H)-one (125 mg, 0.297 mmol) obtained in Example 141 and ethyl isocyanate (0.141 mL, 1.98 mmol).
$^1$H NMR ($CDCl_3$) δ0.94-1.07 (3H, m), 1.12-1.28 (6H, m), 1.56-2.05 (4H, m), 3.12-3.45 (4H, m), 4.08-4.34 (2H, m), 5.29-5.36 (1H, m), 6.90-7.06 (2H, m), 7.17-7.35 (2H, m), 7.39-7.55 (2H, m), 7.70 (1H, d, J = 7.5 Hz), 7.85 (1H, d, J = 7.8 Hz), 9.32, 9.46 (1H, brx2).

Example 143

tert-butyl {5-bromo-3-[(4'-oxo-3',4'-dihydro-1H-spiro[piperidine-4,2'-thiochromen]-1-yl)carbonyl]thiophen-2-yl} carbamate
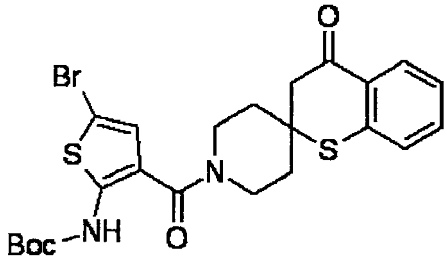

The title compound (2.39 g, yield 89%) was obtained as an oil in the same manner as in Example 1 and from spiro[piperidine-4,2'-thiochromen]-4'(3'H)-one hydrochloride (1.34 g, 4.97 mmol) obtained in Reference Example 93 and 5-bromo-2-[(tert-butoxycarbonyl)amino]thiophene-3-carboxylic acid (1.66 g, 4.97 mmol) (produced by the method described in WO07/013691).
EI(pos) 538 $[M+H]^+$

Example 144

benzyl 4-{5-[(tert-butoxycarbonyl)amino]-4-[(4'-oxo-3',4'-dihydro-1H-spiro[piperidine-4,2'-thiochromen]-1-yl) carbonyl]thiophen-2-yl}benzoate
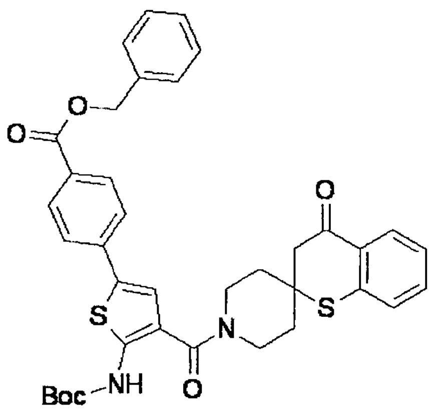

The title compound (960 mg, yield 55%) was obtained as an oil in the same manner as in Example 10 and from tert-butyl {5-bromo-3-[(4'-oxo-3',4'-dihydro-1H-spiro[piperidine-4,2'-thiochromen]-1-yl)carbonyl]thiophen-2-yl}carbamate (1.39 g, 2.59 mmol) obtained in Example 143 and {4-[(benzyloxy)carbonyl]phenyl}boric acid (1.33 g, 5.17 mmol).
EI(pos) 668 $[M+H]^+$

Example 145

benzyl 4-{5-amino-4-[(4'-oxo-3',4'-dihydro-1H-spiro[piperidine-4,2'-thiochromen]-1-yl)carbonyl]thiophen-2-yl} benzoate

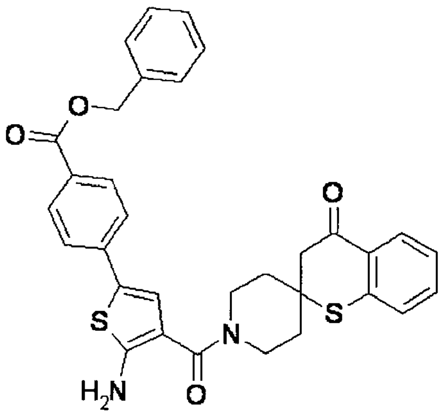

The title compound (528 mg, yield 64%) was obtained as an oil in the same manner as in Example 4 and from benzyl 4-{5-[(tert-butoxycarbonyl)amino]-4-[(4'-oxo-3',4'-dihydro-1H-spiro[piperidine-4,2'-thiochromen]-1-yl)carbonyl] thiophen-2-yl}benzoate (960 mg, 1.44 mmol) obtained in Example 144 and trifluoroacetic acid (5 mL).
EI(pos) 568 $[M+H]^+$

Example 146

benzyl 4-{5-[(ethylcarbamoyl)amino]-4-[(4'-oxo-3',4'-dihydro-1H-spiro[piperidine-4,2'-thiochromen]-1-yl)carbonyl]thiophen-2-yl}benzoate

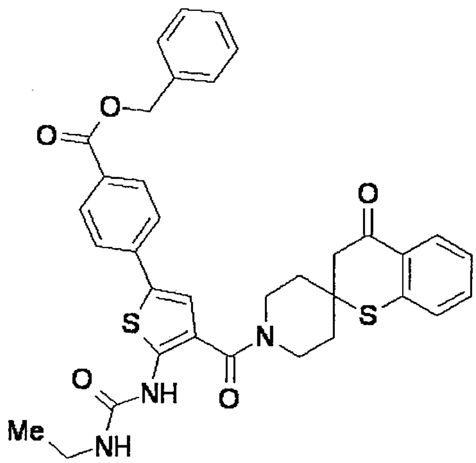

The title compound (555 mg, yield 93%) was obtained as an oil in the same manner as in Example 2 and from benzyl 4-{5-amino-4-[(4'-oxo-3',4'-dihydro-1H-spiro[piperidine-4,2'-thiochromen]-1-yl)carbonyl]thiophen-2-yl}benzoate (528 mg, 0.929 mmol) obtained in Example 145 and ethyl isocyanate (0.221 mL, 2.79 mmol).
EI(pos) 640 $[M+H]^+$

Example 147

4-{5-[(ethylcarbamoyl)amino]-4-[(4'-oxo-3',4'-dihydro-1H-spiro[piperidine-4,2'-thiochromen]-1-yl)carbonyl]thiophen-2-yl}benzoic acid

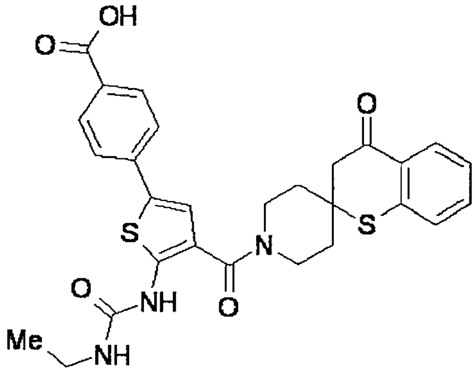

The title compound (131 mg, yield 27%) was obtained as a powder in the same manner as in Example 24 and from benzyl 4-{5-[(ethylcarbamoyl)amino]-4-[(4'-oxo-3',4'-dihydro-1H-spiro[piperidine-4,2'-thiochromen]-1-yl)carbonyl] thiophen-2-yl}benzoate (555 mg, 0.867 mmol) obtained in Example 146.
EI(pos) 550 $[M+H]^+$

Example 148

tert-butyl {3-[(6'-cyano-4'-oxo-3',4'-dihydro-1H-spiro[piperidine-4,2'-thiochromen]-1-yl)carbonyl]-6-methoxy-1-benzothiophen-2-yl}carbamate

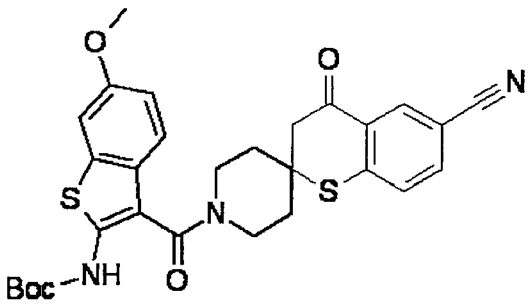

The title compound (900 mg, yield 94%) was obtained as an oil in the same manner as in Example 1 and from 4'-oxo-3',4'-dihydrospiro[piperidine-4,2'-thiochromene]-6'-carbonitrile hydrochloride (503 mg, 1.71 mmol) obtained in Reference Example 103 and 2-[(tert-butoxycarbonyl)amino]-6-methoxy-1-benzothiophene-3-carboxylic acid (552 mg, 1.71 mmol) obtained in Reference Example 118.
$^1$H NMR ($CDCl_3$) δ1.54, 1.56 (9H, sx2), 1.71-1.79 (1H, m), 1.92-1.98 (3H, m), 2.96, 3.11 (2H, sx2), 3.36-3.49 (2H, m), 3.84-3.91 (4H, m), 4.05 (1H, m), 6.94-7.00 (1H, m), 7.18-7.20 (1H, m), 7.30-7.41 (2H, m), 7.59-7.64 (1H, m), 8.31-8.33 (1H, m), 8.87, 9.00 (1H, brx2).

Example 149

1-[(2- amino- 6- methoxy- 1- benzothiophen- 3- yl) carbonyl]- 4'- oxo- 3', 4'- dihydrospiro [piperidine- 4,2'- thiochromene]-6'-carbonitrile

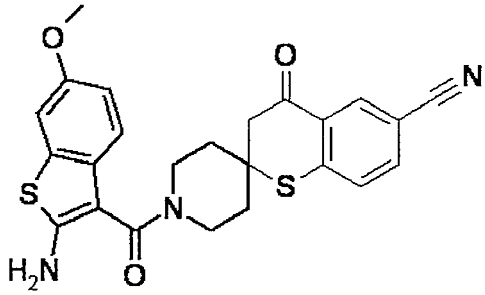

The title compound (593 mg, yield 82%) was obtained as a powder in the same manner as in Example 4 and from tert-butyl {3-[(6'-cyano-4'-oxo-3',4'-dihydro-1H-spiro[piperidine-4,2'-thiochromen]-1-yl)carbonyl]-6-methoxy-1-benzothiophen-2-yl}carbamate (880 mg, 1.56 mmol) obtained in Example 148 and trifluoroacetic acid (8 mL).
$^1$H NMR ($CDCl_3$) δ1.69-1.79 (1H, m), 1.84-2.02 (3H, m), 2.95, 3.08 (2H, sx2), 3.37-3.45 (2H, m), 3.81-3.91 (4H, m), 4.02-4.07 (1H, m), 5.09, 5.21 (2H, sx2), 6.87-6.93 (1H, m), 7.06-7.07 (1H, m), 7.18-7.27 (1H, m), 7.34-7.40 (1H, m), 7.59-7.63 (1H, m), 8.31-8.33 (1H, m).

Example 150

1-{3-[(6'-cyano-4'-oxo-3',4'-dihydro-1H-spiro[piperidine-4,2'-thiochromen]-1-yl)carbonyl]-6-methylthieno[2,3-b]pyridin-2-yl}-3-methylurea
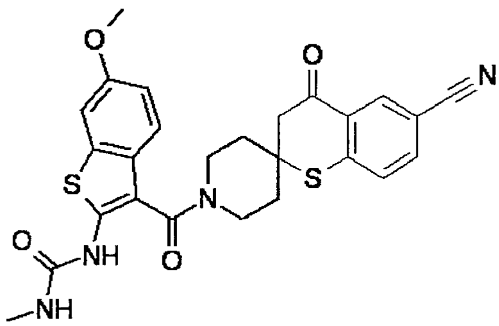

The title compound (178 mg, yield 79%) was obtained as a powder in the same manner as in Example 2 and from 1-[(2-amino-6-methoxy-1-benzothiophen-3-yl)carbonyl]-4'-oxo-3',4'-dihydrospiro[piperidine-4,2'-thiochromene]-6'-carbonitrile (200 mg, 0.431 mmol) obtained in Example 149 and methyl isocyanate (0.076 mL, 1.29 mmol).
$^1$H NMR (DMSO-$d_6$) δ1.87 (4H, m), 2.67-2.69 (3H, m), 3.08, 3.17 (2H, sx2), 3.27-3.39 (2H, m), 3.77 (5H, m), 6.90-7.09 (2H, m), 7.28-7.43 (2H, m), 7.59-7.63 (1H, m), 7.89-7.92 (1H, m), 8.23 (1H, m), 9.19-9.24 (1H, brx2).

Example 151

1-{3-[(6'-cyano-4'-oxo-3',4'-dihydro-1H-spiro[piperidine-4,2'-thiochromen]-1-yl)carbonyl]-6-methoxy-1-benzothiophen-2-yl}urea
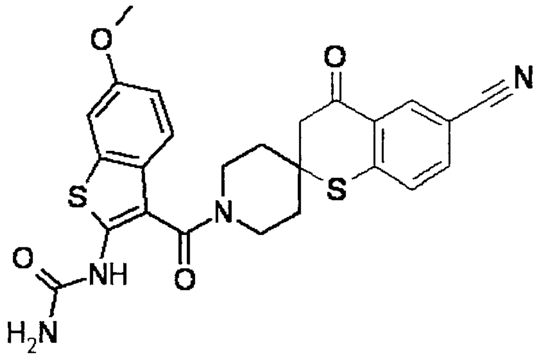

The title compound (236 mg, yield 89%) was obtained as a powder in the same manner as in Reference Example 38 and from 1-[(2-amino-6-methoxy-1-benzothiophen-3-yl)carbonyl]-4'-oxo-3',4'-dihydrospiro[piperidine-4,2'-thiochromene]-6'-carbonitrile (243 mg, 0.524 mmol) obtained in Example 149, trichloroacetyl isocyanate (0.125 mL, 1.05 mmol) and 7M ammonia-methanol (2 mL).
$^1$H NMR (DMSO-$d_6$) δ1.89 (4H, m), 3.08, 3.18 (2H, sx2), 3.30-3.40 (2H, m), 3.77 (5H, m), 6.60, 6.67 (2H, brx2), 6.91-6.95 (1H, m), 7.29-7.44 (2H, m), 7.60-7.63 (1H, m), 7.89-7.93 (1H, m), 8.23 (1H, m), 9.16 (1H, br).

Example 152

N-{3-[(6'-cyano-4'-oxo-3',4'-dihydro-1H-spiro[piperidine-4,2'-thiochromen]-1-yl)carbonyl]-6-methoxy-1-benzothiophen-2-yl}acetamide
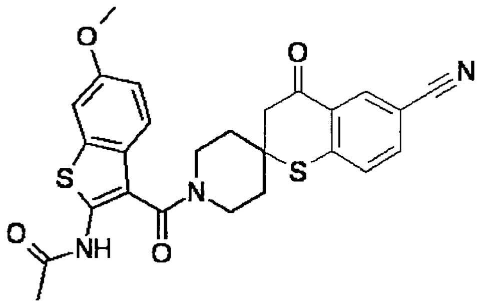

To a solution of 1-[(2-amino-6-methoxy-1-benzothiophen-3-yl)carbonyl]-4'-oxo-3',4'-dihydrospiro[piperidine-4,2'-thiochromene]-6'-carbonitrile (125 mg, 0.270 mmol) obtained in Example 149 in pyridine (1 mL) was added acetic anhydride (0.255 mL, 2.70 mmol), and the mixture was stirred with heating at 70°C for 2 hr. The reaction mixture was concentrated under reduced pressure, and the residue was purified by basic silica gel column chromatography (ethyl acetate:hexane=1:3 to 1:0), and triturated with diisopropyl ether to give the title compound (119 mg, yield 88%).
$^1$H NMR (DMSO-$d_6$) δ1.87 (4H, m), 2.16, 2.18 (3H, sx2), 3.08, 3.13 (2H, sx2), 3.25-3.79 (7H, m), 6.94-6.98 (1H, m), 7.34-7.46 (2H, m), 7.59-7.64 (1H, m), 7.89-7.93 (1H, m), 8.22-8.23 (1H, m), 10.65 (1H, br).

Example 153

1-[(2- amino- 6- methylthieno [2,3- b] pyridin- 3- yl) carbonyl]- 6'- chlorospiro [piperidine- 4,2'- thiochromen]- 4' (3'H)-one
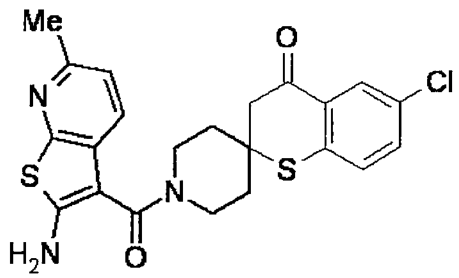

The title compound (43.0 mg, yield 5%) was obtained as a powder in the same manner as in Example 1 and from 6'-chlorospiro[piperidine-4,2'-thiochromen]-4'(3'H)-one hydrochloride (400 mg, 1.32 mmol) obtained in Reference Example 121 and 2-amino-6-methylthieno[2,3-b]pyridine-3-carboxylic acid trifluoroacetate (552 mg, 1.72 mmol) obtained in Reference Example 11.
EI(pos) 458 $[M+H]^+$

Example 154

1-{3-[(6'-chloro-4'-oxo-3',4'-dihydro-1H-spiro[piperidine-4,2'-thiochromen]-1-yl)carbonyl]-6-methylthieno[2,3-b]pyridin-2-yl}urea
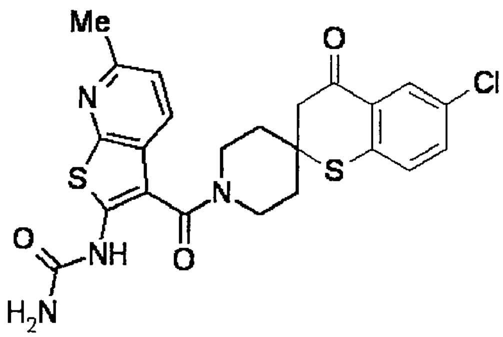

The title compound (22.6 mg, yield 48%) was obtained as a powder in the same manner as in Reference Example 38 and from 1-[(2-amino-6-methylthieno[2,3-b]pyridin-3-yl)carbonyl]-6'-chlorospiro[piperidine-4,2'-thiochromen]-4'(3'H)-one (43.0 mg, 0.0938 mmol) obtained in Example 153, trichloroacetyl isocyanate (0.017 mL, 0.141 mmol) and 7M ammonia-methanol (0.6 mL).
EI(pos) 501 $[M+H]^+$

Example 155

1-[(2- amino- 6- methylthieno [2,3- b] pyridin- 3- yl) carbonyl]- 7'- chlorospiro [piperidine- 4,2'- thiochromen]- 4' (3'H)-one
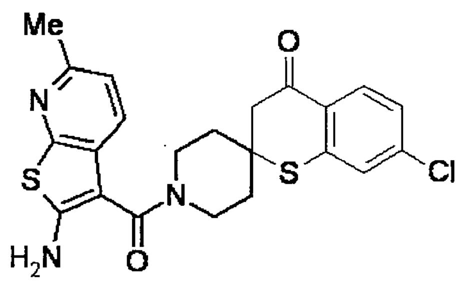

The title compound (42.4 mg, yield 5.4%) was obtained as a powder in the same manner as in Example 1 and from 7'-chlorospiro[piperidine-4,2'-thiochromen]-4'(3'H)-one hydrochloride (400 mg, 1.32 mmol) obtained in Reference Example 124 and 2-amino-6-methylthieno[2,3-b]pyridine-3-carboxylic acid trifluoroacetate (552 mg, 1.72 mmol) obtained in Reference Example 11.
EI(pos) 458 $[M+H]^+$

Example 156

1-{3-[(7'-chloro-4'-oxo-3',4'-dihydro-1H-spiro[piperidine-4,2'-thiochromen]-1-yl)carbonyl]-6-methylthieno[2,3-b]pyridin-2-yl}urea
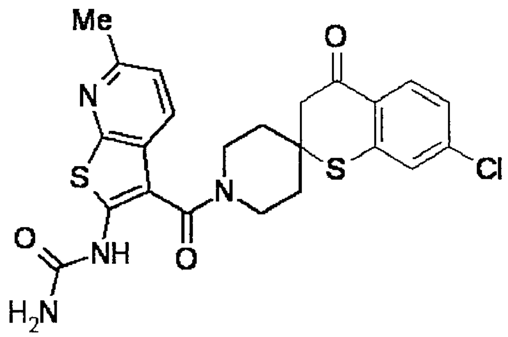

The title compound (11.5 mg, yield 25%) was obtained as a powder in the same manner as in Reference Example 38 and from 1-[(2-amino-6-methylthieno[2,3-b]pyridin-3-yl)carbonyl]-7'-chlorospiro[piperidine-4,2'-thiochromen]-4'(3'H)-one (42.4 mg, 0.093 mmol) obtained in Example 155, trichloroacetyl isocyanate (0.017 mL, 0.139 mmol) and 7M ammonia-methanol (0.6 mL).
EI(pos) 501 $[M+H]^+$

Example 157

1-[(2- amino- 6- methylthieno [2,3- b] pyridin- 3- yl) carbonyl]- 6'- methylspiro [piperidine- 4,2'- thiochromen]- 4' (3'H)-one
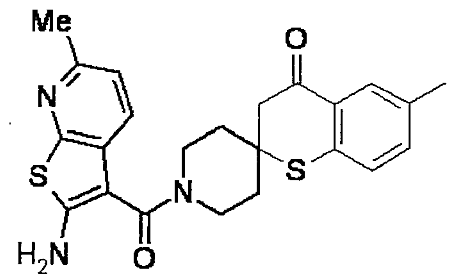

The title compound (69.5 mg, yield 10%) was obtained as a powder in the same manner as in Example 1 and from 6'-methylspiro[piperidine-4,2'-thiochromen]-4'(3'H)-one hydrochloride (337 mg, 1.19 mmol) obtained in Reference Example 127 and 2-amino-6-methylthieno[2,3-b]pyridine-3-carboxylic acid trifluoroacetate (498 mg, 1.55 mmol) obtained in Reference Example 11.
EI(pos) 438 $[M+H]^+$

Example 158

1-{6-methyl-3-[(6'-methyl-4'-oxo-3',4'-dihydro-1H-spiro[piperidine-4,2'-thiochromen]-1-yl)carbonyl]thieno[2,3-b]pyridin-2-yl}urea
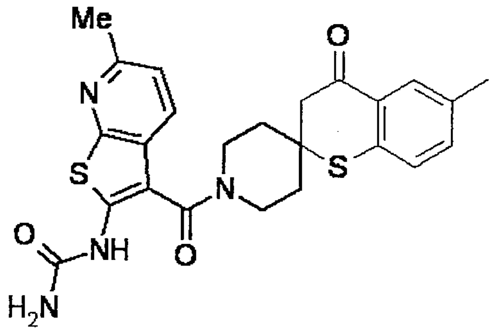

The title compound (43.1 mg, yield 57%) was obtained as a powder in the same manner as in Reference Example 38 and from 1-[(2-amino-6-methylthieno[2,3-b]pyridin-3-yl)carbonyl]-6'-methylspiro[piperidine-4,2'-thiochromen]-4'(3'H)-one (69.5 mg, 0.158 mmol) obtained in Example 157, trichloroacetyl isocyanate (0.028 mL, 0.237 mmol) and 7M ammonia-methanol (0.6 mL).
EI(pos) 481 $[M+H]^+$

Example 159

1-[(2-amino-6-methylthieno[2,3-b]pyridin-3-yl)carbonyl]-6'-methoxyspiro[piperidine-4,2'-thiochromen]-4'(3'H)-one
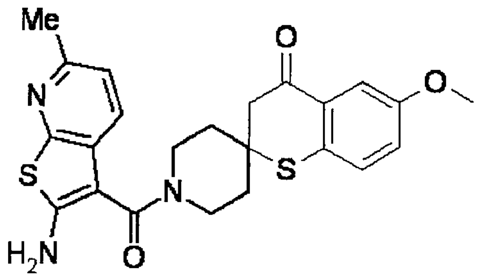

The title compound (31.9 mg, yield 20%) was obtained as a powder in the same manner as in Example 1 and from 6'-methoxyspiro[piperidine-4,2'-thiochromen]-4'(3'H)-one hydrochloride (81.0 mg, 0.271 mmol) obtained in Reference Example 130 and 2-amino-6-methylthieno[2,3-b]pyridine-3-carboxylic acid trifluoroacetate (113 mg, 0.271 mmol) obtained in Reference Example 11.
EI(pos) 454 $[M+H]^+$

Example 160

1-{3-[(6'-methoxy-4'-oxo-3',4'-dihydro-1H-spiro[piperidine-4,2'-thiochromen]-1-yl)carbonyl]-6-methylthieno[2,3-b]pyridin-2-yl}urea
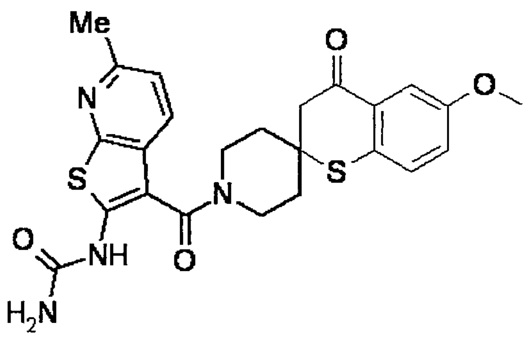

The title compound (17.3 mg, yield 51%) was obtained as a powder in the same manner as in Reference Example 38 and from 1-[(2-amino-6-methylthieno[2,3-b]pyridin-3-yl)carbonyl]-6'-methoxyspiro[piperidine-4,2'-thiochromen]-4'(3'H)-one (31.9 mg, 0.069 mmol) obtained in Example 159, trichloroacetyl isocyanate (0.012 mL, 0.103 mmol) and 7M ammonia-methanol (0.6 mL).
EI(pos) 497 $[M+H]^+$

Example 161

1'-[(2-amino-1-benzothiophen-3-yl)carbonyl]-3-methylspiro[chromene-2,4'-piperidin]-4(3H)-one
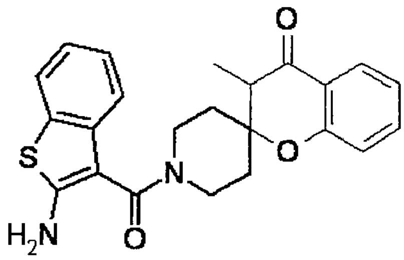

The title compound (200 mg, yield 46%) was obtained as a powder in the same manner as in Example 1 and from 3-methylspiro[chromene-2,4'-piperidin]-4(3H)-one hydrochloride (285 mg, 1.06 mmol) obtained in Reference Example 132 and 2-amino-1-benzothiophene-3-carboxylic acid (206 mg, 1.06 mmol) (produced by the method described in WO07/013691).
$^1$H NMR ($CDCl_3$) δ1.16-1.26 (3H, m), 1.62 (9H, s), 1.51-1.90 (2H, m), 1.96-2.09 (2H, m), 2.58-2.74 (1H, m), 3.25-3.47 (2H, m), 5.18, 5.37 (2H, brx2), 6.95-7.13 (3H, m), 7.25-7.53 (4H, m), 7.83-7.86 (1H, m).

Example 162

1-ethyl-3-{3-[(3-methyl-4-oxo-3,4-dihydro-1'H-spiro[chromene-2,4'-piperidin]-1'-yl)carbonyl]-1-benzothiophen-2-yl}urea
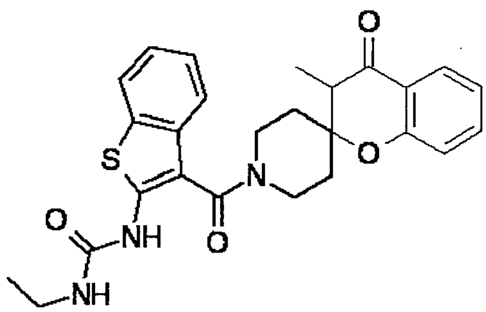

The title compound (159 mg, yield 80%) was obtained as a powder in the same manner as in Example 2 and from 1'-[(2-amino-1-benzothiophen-3-yl)carbonyl]-3-methylspiro[chromene-2,4'-piperidin]-4(3H)-one (169 mg, 0.416 mmol) obtained in Example 161 and ethyl isocyanate (0.132 mL, 1.67 mmol).
EI(pos) 478.0 $[M+H]^+$

Example 163

tert-butyl {3-[(6'-cyano-4'-oxo-3',4'-dihydro-1H-spiro[piperidine-4,2'-thiochromen]-1-yl)carbonyl]thieno[2,3-b]pyridin-2-yl}carbamate
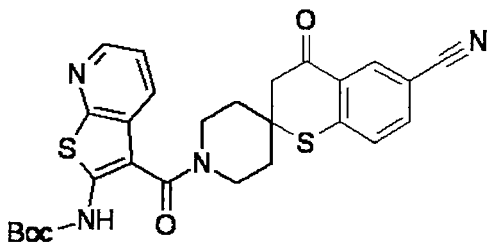

The title compound (771 mg, yield 85%) was obtained as a powder in the same manner as in Example 1 and from 4'-oxo-3',4'-dihydrospiro[piperidine-4,2'-thiochromene]-6'-carbonitrile hydrochloride (500 mg, 1.70 mmol) obtained in Reference Example 103 and 2-[(tert-butoxycarbonyl)amino]thieno[2,3-b]pyridine-3-carboxylic acid (499 mg, 1.70 mmol) obtained in Reference Example 5.
$^1$H NMR ($CDCl_3$) δ1.56, 1.57 (9H, sx2), 1.71-1.81 (1H, m), 1.87-2.07 (3H, m), 2.97, 3.12 (2H, sx2), 3.41-3.50 (2H, m), 3.79-3.84 (1H, m), 4.04-4.08 (1H, m), 7.25-7.42 (2H, m), 7.60-7.72 (2H, m), 8.32-8.33 (1H, m), 8.41-8.46 (1H, m), 9.16, 9.31 (1H, brx2).

Example 164

1-[(2-aminothieno[2,3-b]pyridin-3-yl)carbonyl]-4'-oxo-3',4'-dihydrospiro[piperidine-4,2'-thiochromene]-6'-carbonitrile
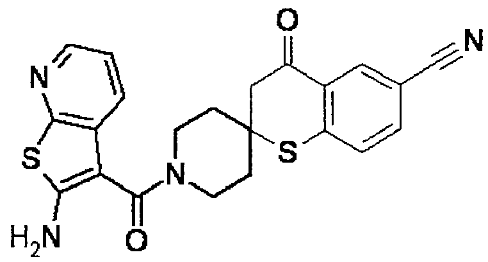

The title compound (558 mg, yield 91%) was obtained as a powder in the same manner as in Example 4 and from tert-butyl {3-[(6'-cyano-4'-oxo-3',4'-dihydro-1H-spiro[piperidine-4,2'-thiochromen]-1-yl)carbonyl]thieno[2,3-b]pyridin-2-yl}carbamate (751 mg, 1.40 mmol) obtained in Example 163 and trifluoroacetic acid (7 mL).
$^1$H NMR ($CDCl_3$) δ1.53-1.99 (4H, m), 2.96, 3.06 (2H, sx2), 3.39-3.47 (2H, m), 3.81-3.86 (1H, m), 4.04-4.08 (1H, m), 5.55, 5.70 (2H, brx2), 7.16-7.23 (1H, m), 7.35-7.41 (1H, m), 7.50-7.63 (2H, m), 8.22-8.33 (2H, m).

Example 165

1-{3-[(6'-cyano-4'-oxo-3',4'-dihydro-1H-spiro[piperidine-4,2'-thiochromen]-1-yl)carbonyl]thieno[2,3-b]pyridin-2-yl}-3-ethylurea
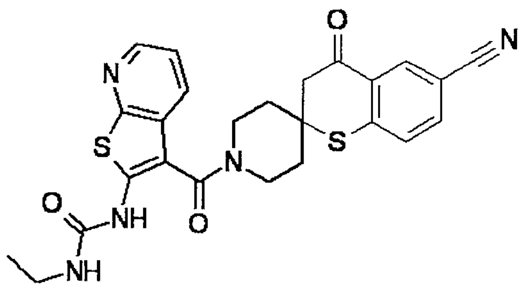

The title compound (109 mg, yield 47%) was obtained as a powder in the same manner as in Example 2 and from 1-[(2-aminothieno[2,3-b]pyridin-3-yl)carbonyl]-4'-oxo-3',4'-dihydrospiro[piperidine-4,2'-thiochromene]-6'-carbonitrile (200 mg, 0.460 mmol) obtained in Example 164 and ethyl isocyanate (0.146 mL, 1.84 mmol).
$^1$H NMR (DMSO-$d_6$) δ1.09 (3H, t, J = 7.5 Hz), 1.88 (4H, m), 3.09-3.18 (4H, m), 3.33-3.43 (2H, m), 3.81 (2H, m), 7.22-7.39 (2H, m), 7.61 (1H, d, J = 8.1 Hz), 7.78-7.93 (2H, m), 8.23 (1H, d, J = 1.5 Hz), 8.33 (1H, m), 9.40, 9.48 (1H, brx2).

Example 166

1-{3-[(6'-cyano-4'-oxo-3',4'-dihydro-1H-spiro[piperidine-4,2'-thiochromen]-1-yl)carbonyl]thieno[2,3-b]pyridin-2-yl}-3-methylurea

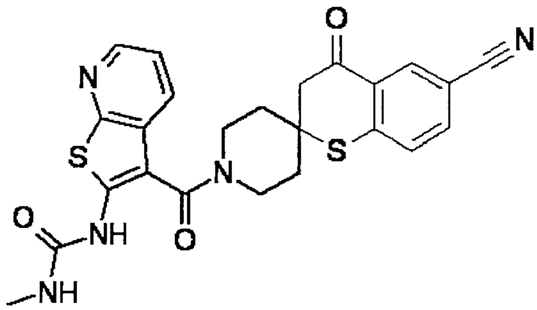

The title compound (147 mg, yield 65%) was obtained as a powder in the same manner as in Example 2 and from 1-[(2-aminothieno[2,3-b]pyridin-3-yl)carbonyl]-4'-oxo-3',4'-dihydrospiro[piperidine-4,2'-thiochromene]-6'-carbonitrile (200 mg, 0.460 mmol) obtained in Example 164 and methyl isocyanate (0.109 mL, 1.84 mmol).
$^1$H NMR (DMSO-$d_6$) δ1.88 (4H, m), 2.70, 2.72 (3H, sx2), 3.09, 3.17 (2H, sx2), 3.31-3.43 (2H, m), 3.83 (2H, m), 7.07-7.26 (1H, m), 7.32-7.40 (1H, m), 7.61 (1H, d, J = 8.1 Hz), 7.76-7.93 (2H, m), 8.23 (1H, d, J = 1.8 Hz), 8.34 (1H, m), 9.48, 9.54 (1H, brx2).

Example 167

1-[(2-amino-7-oxo-4,5,6,7-tetrahydro-1-benzothiophen-3-yl)carbonyl]-4'-oxo-3',4'-dihydrospiro[piperidine-4,2'-thiochromene]-6'-carbonitrile

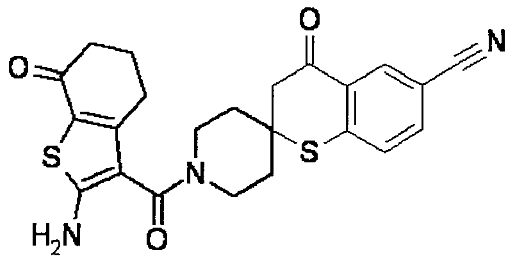

The title compound (310 mg, yield 58%) was obtained as a powder in the same manner as in Example 1 and from 4'-oxo-3',4'-dihydrospiro[piperidine-4,2'-thiochromene]-6'-carbonitrile hydrochloride (350 mg, 1.19 mmol) obtained in Reference Example 103 and 2-amino-7-oxo-4,5,6,7-tetrahydro-1-benzothiophene-3-carboxylic acid (251 mg, 1.19 mmol) (produced by the method described in WO07/013691).
EI(pos) 451.9 $[M+H]^+$

Example 168

1-{3-[(6'-cyano-4'-oxo-3',4'-dihydro-1H-spiro[piperidine-4,2'-thiochromen]-1-yl)carbonyl]-7-oxo-4,5,6,7-tetrahydro-1-benzothiophen-2-yl}-3-ethylurea

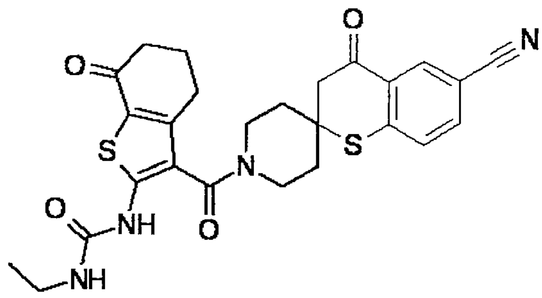

The title compound (229 mg, yield 66%) was obtained as a powder in the same manner as in Example 2 and from 1-[(2-amino-7-oxo-4,5,6,7-tetrahydro-1-benzothiophen-3-yl)carbonyl]-4'-oxo-3',4'-dihydrospiro[piperidine-4,2'-thiochromene]-6'-carbonitrile (300 mg, 0.664 mmol) obtained in Example 167 and ethyl isocyanate (0.210 mL, 2.66 mmol). EI(pos) 523.0 $[M+H]^+$

Example 169

N-{3-[(6-cyano-4-oxo-3,4-dihydro-1'H-spiro[chromene-2,4'-piperidin]-1'-yl)carbonyl]-7-methoxy-1-benzothiophen-2-yl}acetamide

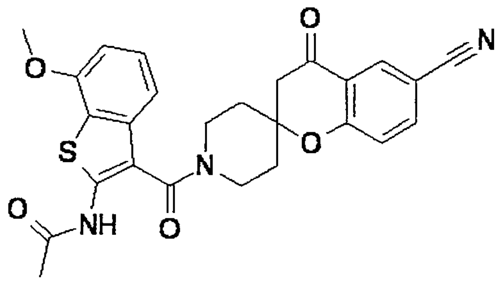

The title compound (196 mg, yield 62%) was obtained as a powder in the same manner as in Example 152 and from 1'-[(2-amino-7-methoxy-1-benzothiophen-3-yl)carbonyl]-4-oxo-3,4-dihydrospiro[chromene-2,4'-piperidine]-6-carbonitrile (290 mg, 0.648 mmol) obtained in Example 14, acetyl chloride (0.057 mL, 0.778 mmol) and triethylamine (0.179 mL, 1.23 mmol).
EI(pos) 490 $[M+H]^+$

Example 170

1-{3-[(6-cyano-4-oxo-3,4-dihydro-1'H-spiro[chromene-2,4'-piperidin]-1'-yl)carbonyl]-7-methoxy-1-benzothiophen-2-yl}urea

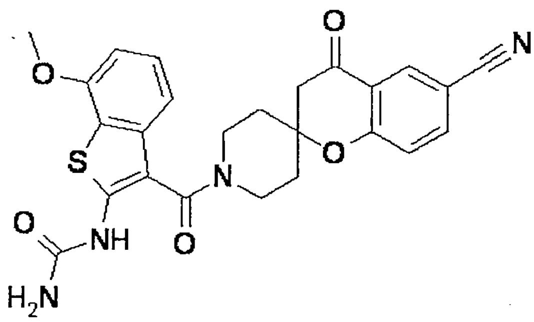

The title compound (187 mg, yield 56%) was obtained as a powder in the same manner as in Reference Example 38 and from 1'-[(2-amino-7-methoxy-1-benzothiophen-3-yl)carbonyl]-4-oxo-3,4-dihydrospiro[chromene-2,4'-piperidine]-6-carbonitrile (300 mg, 0.671 mmol) obtained in Example 14, trichloroacetyl isocyanate (0.119 mL, 1.01 mmol) and 7M ammonia-methanol (0.7 mL).
EI(pos) 491 $[M+H]^+$

Example 171

tert-butyl {3-[(6-cyano-4-oxo-3,4-dihydro-1'H-spiro[chromene-2,4'-piperidin]-1'-yl)carbonyl]-6-methoxy-1-benzothiophen-2-yl}carbamate
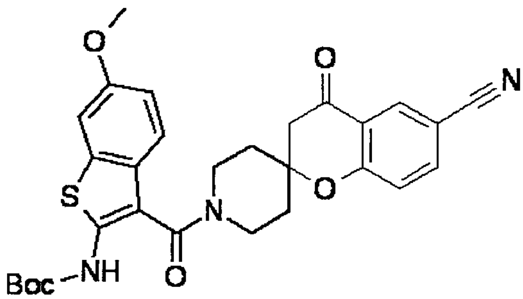

The title compound (807 mg, quantitative) was obtained as an oil in the same manner as in Example 1 and from 4-oxo-3,4-dihydrospiro[chromene-2,4'-piperidine]-6-carbonitrile hydrochloride (439 mg, 1.58 mmol) and 2-[(tert-butoxycarbonyl)amino]-6-methoxy-1-benzothiophene-3-carboxylic acid (510 mg, 1.58 mmol) obtained in Reference Example 118.
EI(pos) 492 $[M+H]^+$

Example 172

1'-[(2-amino-6-methoxy-1-benzothiophen-3-yl)carbonyl]-4-oxo-3,4-dihydrospiro[chromene-2,4'-piperidine]-6-carbonitrile
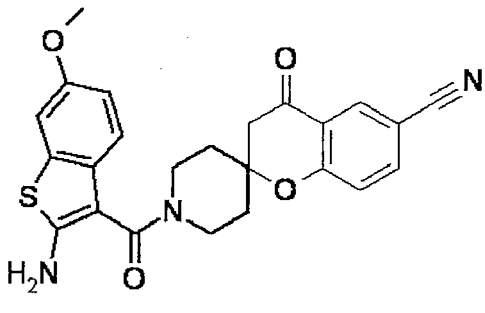

The title compound (422 mg, yield 63%) was obtained as a powder in the same manner as in Example 4 and from tert-butyl {3-[(6-cyano-4-oxo-3,4-dihydro-1'H-spiro[chromene-2,4'-piperidin]-1'-yl)carbonyl]-6-methoxy-1-benzothiophen-2-yl}carbamate (807 mg, 1.48 mmol) obtained in Example 171 and trifluoroacetic acid (7 mL).
EI(pos) 448 $[M+H]^+$

Example 173

N-{3-[(6-cyano-4-oxo-3,4-dihydro-1'H-spiro[chromene-2,4'-piperidin]-1'-yl)carbonyl]-6-methoxy-1-benzothiophen-2-yl}acetamide
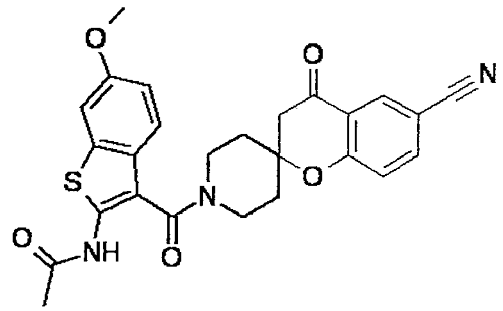

The title compound (136 mg, yield 62%) was obtained as a powder in the same manner as in Example 152 and from 1'-[(2-amino-6-methoxy-1-benzothiophen-3-yl)carbonyl]-4-oxo-3,4-dihydrospiro[chromene-2,4'-piperidine]-6-carbonitrile (200 mg, 0.447 mmol) obtained in Example 172, acetyl chloride (0.048 mL, 0.671 mmol) and triethylamine (0.128 mL, 0.894 mmol).
EI(pos) 490 $[M+H]^+$

Example 174

1-{3-[(6-cyano-4-oxo-3,4-dihydro-1'H-spiro[chromene-2,4'-piperidin]-1'-yl)carbonyl]-6-methoxy-1-benzothiophen-2-yl}urea
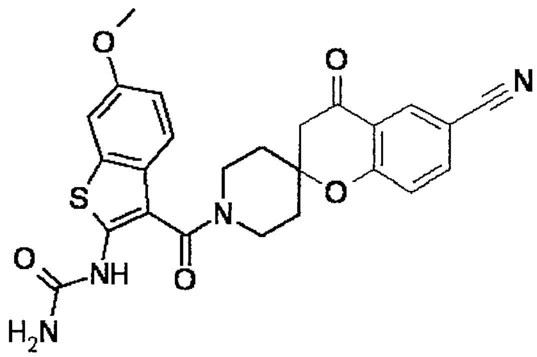

The title compound (166 mg, yield 76%) was obtained as a powder in the same manner as in Reference Example 38 and from 1'-[(2-amino-6-methoxy-1-benzothiophen-3-yl)carbonyl]-4-oxo-3,4-dihydrospiro[chromene-2,4'-piperidine]-6-carbonitrile (200 mg, 0.447 mmol) obtained in Example 172, trichloroacetyl isocyanate (0.079 mL, 0.671 mmol) and 7M ammonia-methanol (0.7 mL).
EI(pos) 491 $[M+H]^+$

Example 175

tert-butyl {3-[(6'-chloro-4'-oxo-3',4'-dihydro-1H-spiro[piperidine-4,2'-thiochromen]-1-yl)carbonyl]-6-methoxy-1-benzothiophen-2-yl}carbamate
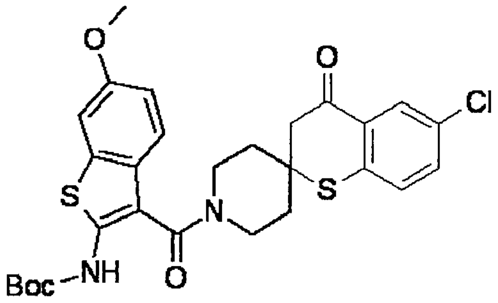

The title compound (595 mg, quantitative) was obtained as an oil in the same manner as in Example 1 and from 6'-chlorospiro[piperidine-4,2'-thiochromen]-4'(3'H)-one hydrochloride (316 mg, 1.04 mmol) obtained in Reference Example 121 and 2-[(tert-butoxycarbonyl)amino]-6-methoxy-1-benzothiophene-3-carboxylic acid (336 mg, 1.04 mmol) obtained in Reference Example 118.
$^1$H NMR ($CDCl_3$) δ1.54, 1.56 (9H, sx2), 1.47-1.75 (2H, m), 1.83-2.03 (2H, m), 2.92, 3.06 (2H, sx2), 3.35-3.49 (2H, m), 3.84-4.04 (5H, m), 6.94-7.00 (1H, m), 7.19-7.40 (4H, m), 8.02-8.04 (1H, m), 8.84, 8.99 (1H, brx2).

Example 176

1-[(2- amino- 6- methoxy- 1- benzothiophen- 3- yl) carbonyl]- 6'- chlorospiro [piperidine- 4,2'- thiochromen]- 4' (3'H)-one
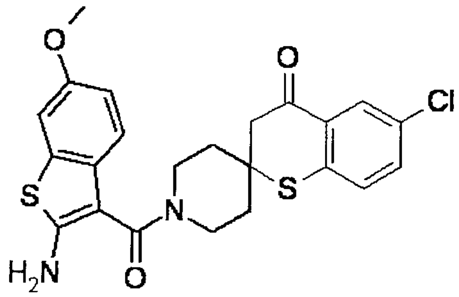

The title compound (343 mg, yield 70%) was obtained as a powder in the same manner as in Example 4 and from tert-butyl {3-[(6'-chloro-4'-oxo-3',4'-dihydro-1H-spiro[piperidine-4,2'-thiochromen]-1-yl)carbonyl]-6-methoxy-1-benzothiophen-2-yl}carbamate (595 mg, 1.04 mmol) obtained in Example 175 and trifluoroacetic acid (5 mL).
$^1$H NMR (DMSO-$d_6$) δ1.75-1.84 (4H, m), 3.04, 3.07 (2H, sx2), 3.27-3.33 (2H, m), 3.73 (3H, s), 3.78-3.83 (2H, m), 6.36 (2H, m), 6.80-6.83 (1H, m), 7.13-7.26 (2H, m), 7.43 (1H, d, J = 8.4 Hz), 7.57-7.60 (1H, m), 7.86 (1H, d, J = 2.4 Hz).

Example 177

1-{3-[(6'-chloro-4'-oxo-3',4'-dihydro-1H-spiro[piperidine-4,2'-thiochromen]-1-yl)carbonyl]-6-methoxy-1-benzothiophen-2-yl}urea
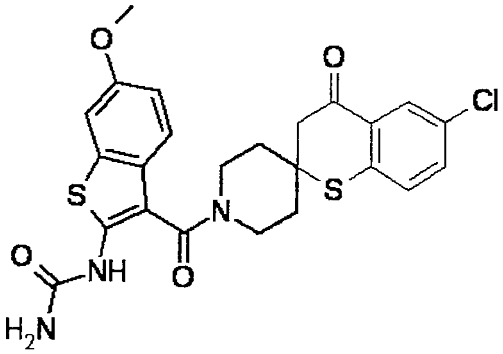

The title compound (305 mg, yield 84%) was obtained as a powder in the same manner as in Reference Example 38 and from 1-[(2-amino-6-methoxy-1-benzothiophen-3-yl)carbonyl]-6'-chlorospiro[piperidine-4,2'-thiochromen]-4'(3'H)-one (334 mg, 0.706 mmol) obtained in Example 176, trichloroacetyl isocyanate (0.168 mL, 1.41 mmol) and 7M ammonia-methanol (3 mL).
$^1$H NMR (DMSO-$d_6$) δ1.74-1.91 (4H, m), 3.03, 3.12 (2H, sx2), 3.27-3.39 (2H, m), 3.58-3.78 (5H, m), 6.60, 6.67 (2H, brx2), 6.91-6.95 (1H, m), 7.28-7.46 (3H, m), 7.57-7.61 (1H, m), 7.87 (1H, d, J = 2.1 Hz), 9.15 (1H, br).

Example 178

tert-butyl {3-[(6'-methyl-4'-oxo-3',4'-dihydro-1H-spiro[piperidine-4,2'-thiochromen]-1-yl)carbonyl]thieno[2,3-b]pyridin-2-yl}carbamate
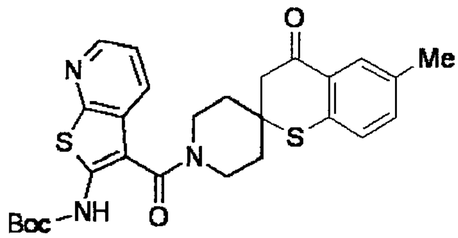

The title compound (714 mg, yield 57%) was obtained as an oil in the same manner as in Example 1 and from 6'-methylspiro[piperidine-4,2'-thiochromen]-4'(3'H)-one hydrochloride (673 mg, 2.38 mmol) obtained in Reference Example 127 and 2-[(tert-butoxycarbonyl)amino]thieno[2,3-b]pyridine-3-carboxylic acid (699 mg, 2.38 mmol) obtained in Reference Example 5.
EI(pos) 524 $[M+H]^+$

Example 179

1-[(2-aminothieno[2,3-b]pyridin-3-yl)carbonyl]-6'-methylspiro[piperidine-4,2'-thiochromen]-4'(3'H)-one
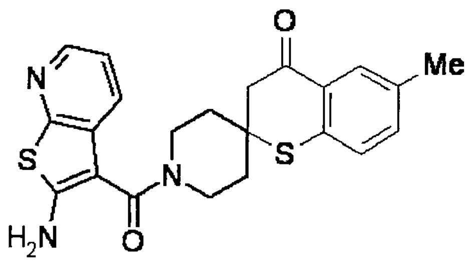

The title compound (635 mg, quantitative) was obtained as a powder in the same manner as in Example 4 and

from tert-butyl {3-[(6'-methyl-4'-oxo-3',4'-dihydro-1H-spiro[piperidine-4,2'-thiochromen]-1-yl)carbonyl]thieno[2,3-b]pyridin-2-yl}carbamate (714 mg, 1.36 mmol) obtained in Example 178 and trifluoroacetic acid (5 mL).
EI(pos) 424 $[M+H]^+$

Example 180

1-{3-[(6'-methyl-4'-oxo-3',4'-dihydro-1H-spiro[piperidine-4,2'-thiochromen]-1-yl)carbonyl]thieno[2,3-b]pyridin-2-yl}urea
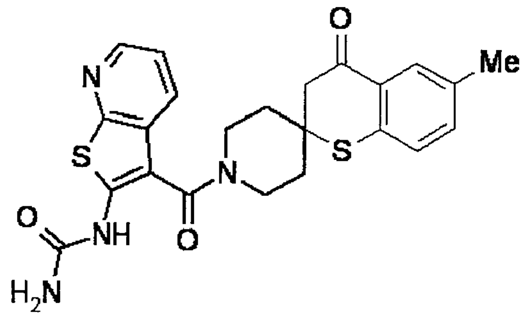

The title compound (105 mg, yield 48%) was obtained as a powder in the same manner as in Reference Example 38 and from 1-[(2-aminothieno[2,3-b]pyridin-3-yl)carbonyl]-6'-methylspiro[piperidine-4,2'-thiochromen]-4'(3'H)-one (200 mg, 0.473 mmol) obtained in Example 179, trichloroacetyl isocyanate (0.084 mL, 0.709 mmol) and 7M ammonia-methanol (0.6 mL).
EI(pos) 467 $[M+H]^+$
$^1$H NMR ($CDCl_3$) δ1.60-2.10 (4H, m), 2.32, 2.33 (3H, sx2), 2.89, 3.04 (2H, sx2), 3.35-3.55 (2H, m), 3.75-4.10 (2H, m), 5.57 (2H, br), 7.05-7.30 (3H, m), 7.60-7.75 (1H, m), 7.88 (1H, br), 8.35-8.46 (1H, m), 9.63, 9.73 (1H, brx2).

Example 181

1-ethyl-3-{3-[(6'-methyl-4'-oxo-3',4'-dihydro-1H-spiro[piperidine-4,2'-thiochromen]-1-yl)carbonyl]thieno[2,3-b]pyridin-2-yl}urea
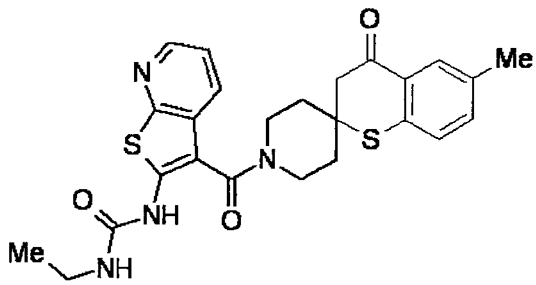

The title compound (119 mg, yield 51%) was obtained as a powder in the same manner as in Example 2 and from 1-[(2-aminothieno[2,3-b]pyridin-3-yl)carbonyl]-6'-methylspiro[piperidine-4,2'-thiochromen]-4'(3'H)-one (200 mg, 0.472 mmol) obtained in Example 179 and ethyl isocyanate (0.093 mL, 1.18 mmol).
EI(pos) 495 $[M+H]^+$
$^1$H NMR ($CDCl_3$) δ1.05-1.20 (3H, m), 1.60-2.10 (4H, m), 2.33 (3H, s), 2.89, 3.06 (2H, sx2), 3.10-3.55 (4H, m), 3.75-4.10 (2H, m), 5.33 (1H, br), 7.05-7.30 (3H, m), 7.60-7.75 (1H, m), 7.89 (1H, br), 8.35-8.46 (1H, m), 9.56, 9.68 (1H, brx2).

Example 182

1-[(5-acetyl-2-amino-4-methylthiophen-3-yl)carbonyl]-6'-methylspiro[piperidine-4,2'-thiochromen]-4'(3'H)-one

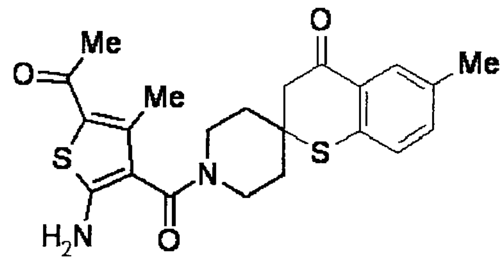

The title compound (46.2 mg, yield 14%) was obtained as a powder in the same manner as in Example 1 and from 6'-methylspiro[piperidine-4,2'-thiochromen]-4'(3'H)-one hydrochloride (300 mg, 0.783 mmol) obtained in Reference Example 127 and 5-acetyl-2-amino-4-methylthiophene-3-carboxylic acid (155 mg, 0.783 mmol).
EI(pos) 429 $[M+H]^+$

Example 183

1-{5-acetyl-4-methyl-3-[(6'-methyl-4'-oxo-3',4'-dihydro-1H-spiro[piperidine-4,2'-thiochromen]-1-yl)carbonyl] thiophen-2-yl}-3-ethylurea

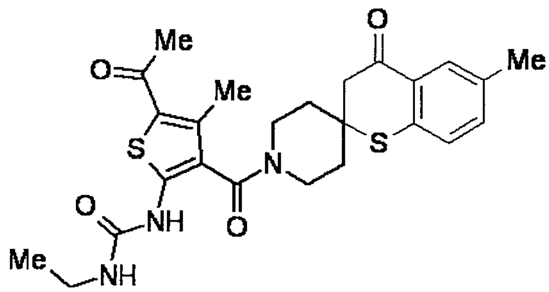

The title compound (6.4 mg, yield 12%) was obtained as a powder in the same manner as in Example 2 and from 1-[(5-acetyl-2-amino-4-methylthiophen-3-yl)carbonyl]-6'-methylspiro[piperidine-4,2'-thiochromen]-4'(3'H)-one (46.2 mg, 0.108 mmol) obtained in Example 182 and ethyl isocyanate (0.043 mL, 0.540 mmol).
EI(pos) 500 $[M+H]^+$
$^1H$ NMR ($CDCl_3$) δ1.05-1.20 (3H, m), 1.40-2.10 (4H, m), 2.34 (3H, s), 2.41-2.44 (6H, m), 2.85-3.55 (8H, m), 5.56, 5.68 (1H, brx2), 7.05-7.30 (2H, m), 7.89 (1H, m), 8.99 (1H, br).

Example 184

tert-butyl {3-[(6'-methyl-4'-oxo-3',4'-dihydro-1H-spiro[piperidine-4,2'-thiochromen]-1-yl)carbonyl]-6-(trifluoromethyl)thieno[2,3-b]pyridin-2-yl}carbamate

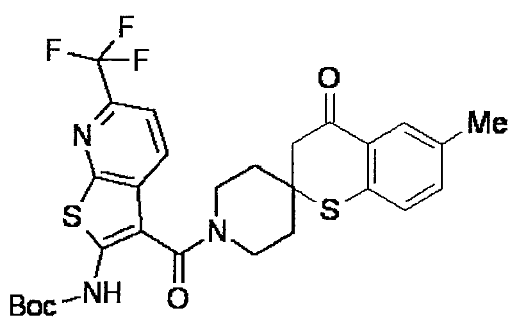

The title compound (523 mg, yield 42%) was obtained as an oil in the same manner as in Example 1 and from 6'-methylspiro[piperidine-4,2'-thiochromen]-4'(3'H)-one hydrochloride (600 mg, 2.12 mmol) obtained in Reference Example 127 and 2-[(tert-butoxycarbonyl)amino]-6-(trifluoromethyl)thieno[2,3-b]pyridine-3-carboxylic acid (767 mg, 2.12 mmol) obtained in Reference Example 138.
EI(pos) 592 $[M+H]^+$

Example 185

1-{[2-amino-6-(trifluoromethyl)thieno[2,3-b]pyridin-3-yl]carbonyl}-6'-methylspiro[piperidine-4,2'-thiochromen]-4'(3'H)-one
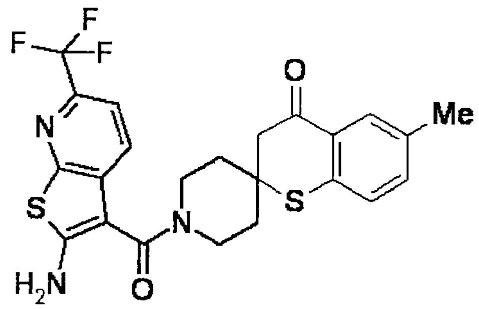

The title compound (237 mg, yield 55%) was obtained as a powder in the same manner as in Example 4 and from tert-butyl {3-[(6'-methyl-4'-oxo-3',4'-dihydro-1H-spiro[piperidine-4,2'-thiochromen]-1-yl)carbonyl]-6-(trifluoromethyl)thieno[2,3-b]pyridin-2-yl}carbamate (522 mg, 0.883 mmol) obtained in Example 184 and trifluoroacetic acid (5 mL).
EI(pos) 491.9 $[M+H]^+$

Example 186

1-{3-[(6'-methyl-4'-oxo-3',4'-dihydro-1H-spiro[piperidine-4,2'-thiochromen]-1-yl)carbonyl]-6-(trifluoromethyl)thieno[2,3-b]pyridin-2-yl}urea
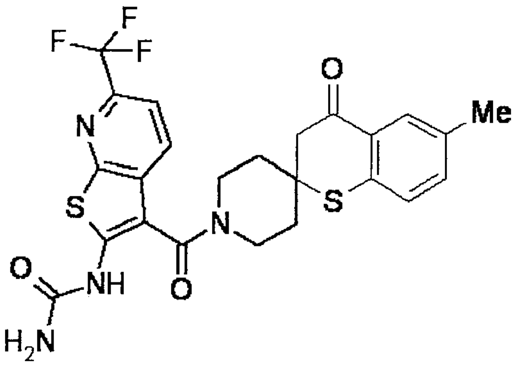

The title compound (66.6 mg, yield 61%) was obtained as a powder in the same manner as in Reference Example 38 and from 1-{[2-amino-6-(trifluoromethyl)thieno[2,3-b]pyridin-3-yl]carbonyl}-6'-methylspiro[piperidine-4,2'-thiochromen]-4'(3'H)-one (100 mg, 0.203 mmol) obtained in Example 185, trichloroacetyl isocyanate (0.036 mL, 0.305 mmol) and 7M ammonia-methanol (0.5 mL).
EI(pos) 535 $[M+H]^+$
$^1$H NMR ($CDCl_3$) δ1.40-2.10 (4H, m), 2.33 (3H, s), 2.89, 3.04 (2H, sx2), 3.35-3.55 (2H, m), 3.75-4.15 (2H, m), 5.76, 5.80 (2H, brx2), 7.05-7.30 (2H, m), 7.62-7.70 (1H, m), 7.70-7.85 (1H, m), 7.88 (1H, br), 9.75, 9.88 (1H, brx2).

Example 187

1-ethyl-3-{3-[(6'-methyl-4'-oxo-3',4'-dihydro-1H-spiro[piperidine-4,2'-thiochromen]-1-yl)carbonyl]-6-(trifluoromethyl)thieno[2,3-b]pyridin-2-yl}urea

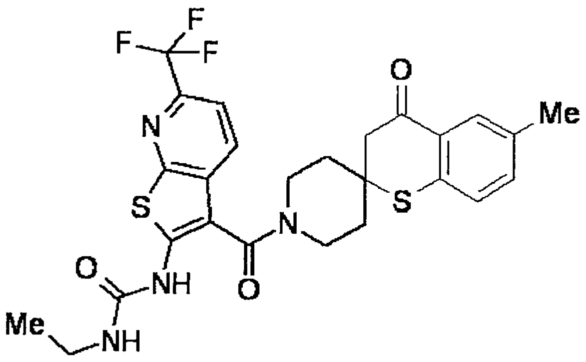

The title compound (38.3 mg, yield 24%) was obtained as a powder in the same manner as in Example 2 and from 1-{[2-amino-6-(trifluoromethyl)thieno[2,3-b]pyridin-3-yl]carbonyl}-6'-methylspiro[piperidine-4,2'-thiochromen]-4'(3'H)-one (137 mg, 0.279 mmol) obtained in Example 185 and ethyl isocyanate (0.055 mL, 0.698 mmol).
EI(pos) 563 $[M+H]^+$
$^1$H NMR ($CDCl_3$) δ1.05-1.20 (3H, m), 1.40-2.10 (4H, m), 2.33 (3H, s), 2.88, 3.05 (2H, sx2), 3.10-4.15 (6H, m), 5.65-5.70 (1H, br), 7.05-7.30 (2H, m), 7.62-7.70 (1H, m), 7.70-7.85 (1H, m), 7.88 (1H, br), 9.68, 9.80 (1H, brx2).

Example 188

N-[({3-[(6-cyano-4-oxo-3,4-dihydro-1'H-spiro[chromene-2,4'-piperidin]-1'-yl)carbonyl]-7-methoxy-1-benzothiophen-2-yl}amino)carbonyl]methanesulfonamide

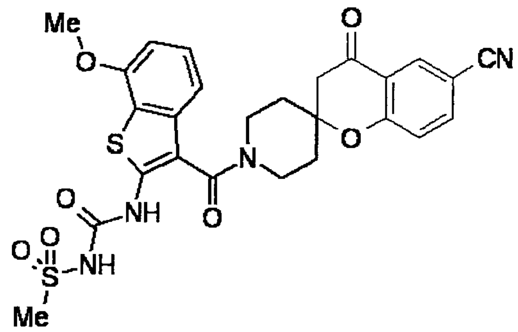

To a solution of 1'-[(2-amino-7-methoxy-1-benzothiophen-3-yl)carbonyl]-4-oxo-3,4-dihydrospiro[chromene-2,4'-piperidine]-6-carbonitrile (460 mg, 1.03 mmol) obtained in Example 14 in DMF (5 mL) were added pyridine (0.125 mL, 1.54 mmol) and phenyl chlorocarbonate (0.155 mL, 1.23 mmol) under ice-cooling, and the mixture was stirred at room temperature for 1 hr. To this solution were added methanesulfonamide (117 mg, 1.23 mmol), DBU (0.215 mL, 1.44 mmol) and 4-dimethylaminopyridine (176 mg, 1.44 mmol), and the mixture was stirred with heating at 60°C for 1 day. The reaction mixture was diluted with ethyl acetate, and the mixture was washed with 0.3N hydrochloric acid and saturated brine, and dried over anhydrous sodium sulfate. The solvent was evaporated under reduced pressure. The obtained residue was purified by silica gel column chromatography (ethyl acetate:hexane=1:1 to 1:0) to give the title compound (118 mg, yield 20%) as a powder.
$^1$H NMR ($CDCl_3$) δ1.66-2.15 (4H, m), 2.76, 2.88 (2H, sx2), 3.17, 3.20 (3H, sx2), 3.35-4.52 (7H, m), 6.71-6.75 (1H, m), 7.06-7.18 (2H, m), 7.31-7.37 (1H, m), 7.69-7.77 (1H, m), 8.18 (1H, m), 9.62, 9.72 (1H, brx2).

Example 189

ethyl ({3-[(6-cyano-4-oxo-3,4-dihydro-1'H-spiro[chromene-2,4'-piperidin]-1'-yl)carbonyl]-7-methoxy-1-benzothiophen-2-yl}amino)(oxo)acetate

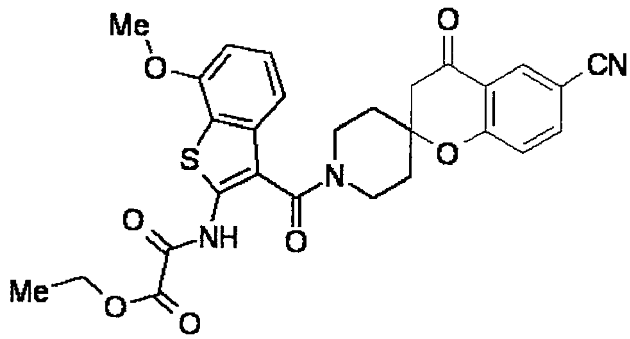

To a solution of 1'-[(2-amino-7-methoxy-1-benzothiophen-3-yl)carbonyl]-4-oxo-3,4-dihydrospiro[chromene-2,4'-piperidine]-6-carbonitrile (300 mg, 0.670 mmol) obtained in Example 14 in a mixed solvent of DMF (6 mL) and chloroform (4 mL) were added pyridine (6 mL) and ethyl chloro(oxo)acetate (0.336 mL, 3.03 mmol) under ice-cooling, and the mixture was stirred at room temperature for 1 day. The reaction mixture was diluted with ethyl acetate, and the mixture was washed with 1N hydrochloric acid, 10% aqueous potassium carbonate solution and saturated brine, dried over anhydrous sodium sulfate, and the solvent was evaporated under reduced pressure. The obtained residue was purified by silica gel column chromatography (ethyl acetate:hexane=1:1 to 1:0) to give the title compound (272 mg, yield 74%) as a powder.

$^1$H NMR ($CDCl_3$) δ1.45(3H, t, J = 7.2 Hz), 1.65-1.74 (2H, m), 1.84-1.94 (1H, m), 2.11 (1H, m), 2.77, 2.90 (2H, sx2), 3.35-4.53 (2H, m), 3.99-4.15 (5H. m), 4.46 (2H, q, J = 7.2 Hz), 6.75-6.79 (1H, m), 7.07-7.21 (2H, m), 7.34-7.40 (1H, m), 7.71-7.77 (1H, m), 8.18-8.19 (1H, m), 11.12, 11.33 (1H, brx2).

Example 190

({3-[(6-cyano-4-oxo-3,4-dihydro-1'H-spiro[chromene-2,4'-piperidin]-1'-yl)carbonyl]-7-methoxy-1-benzothiophen-2-yl}amino)(oxo)acetic acid

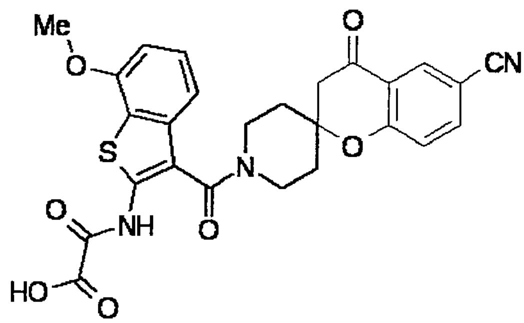

To a solution of ethyl ({3-[(6-cyano-4-oxo-3,4-dihydro-1'H-spiro[chromene-2,4'-piperidin]-1'-yl)carbonyl]-7-methoxy-1-benzothiophen-2-yl}amino)(oxo)acetate (162 mg, 0.296 mmol) obtained in Example 189 in a mixed solvent of EtOH (3 mL) and THF (1.5 mL) was added 2N aqueous sodium hydroxide solution (0.325 mL, 0.650 mmol) under ice-cooling, and the mixture was stirred at room temperature for 1 hr. The reaction mixture was diluted with ethyl acetate, and the mixture was washed with 1N hydrochloric acid and saturated brine, and dried over anhydrous sodium sulfate. The solvent was evaporated under reduced pressure and the obtained residue was triturated with diisopropyl ether to give the title compound (112 mg, yield 73%).

$^1$H NMR ($CDCl_3$) δ2.31-2.79 (4H, m), 3.24-3.28 (1H, m), 3.56-3.75 (3H, m), 3.88-4.13 (5H, m), 6.78-6.81 (1H, m), 7.04-7.23 (2H, m), 7.36-7.41 (1H, m), 7.66-7.72 (1H, m), 8.10-8.12 (1H, m), 11.23 (1H, br), 13.00 (1H, br).

Example 191

4-({3-[(6-cyano-4-oxo-3,4-dihydro-1'H-spiro[chromene-2,4'-piperidin]-1'-yl)carbonyl]-7-methoxy-1-benzothiophen-2-yl}amino)-4-oxobutanoic acid

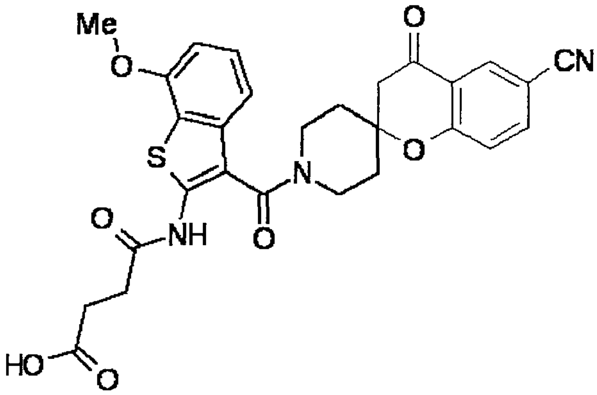

A solution of 1'-[(2-amino-7-methoxy-1-benzothiophen-3-yl)carbonyl]-4-oxo-3,4-dihydrospiro[chromene-2,4'-piperidine]-6-carbonitrile (300 mg, 0.670 mmol) obtained in Example 14 and maleic anhydride (168 mg, 1.68 mmol) in pyridine (5 mL) was stirred at 100°C for 1 day. The reaction mixture was diluted with ethyl acetate, and the mixture was washed with 1N hydrochloric acid and saturated brine, and dried over anhydrous sodium sulfate. The solvent was evaporated under reduced pressure. The obtained residue was purified by silica gel column chromatography (ethyl acetate:hexane=1:1 to 1:0) to give the title compound (77.3 mg, yield 25%) as a powder.
$^1$H NMR ($CDCl_3$) δ1.72-1.88 (2H, m), 2.05-2.10 (2H, m), 2.82-2.88 (6H, m), 3.35-3.50 (2H, m), 3.95 (5H, m), 6.70-6.74 (1H, m), 7.05-7.17 (2H, m), 7.29-7.35 (1H, m), 7.69-7.76 (1H, m), 8.15-8.17 (1H, m), 9.98, 10.06 (1H, brx2).

Example 192

tert-butyl [3-[(6'-methyl-1',1'-dioxido-4'-oxo-3',4'-dihydro-1H-spiro[piperidine-4,2'-thiochromen]-1-yl)carbonyl]-6-(trifluoromethyl)thieno[2,3-b]pyridin-2-yl]carbamate

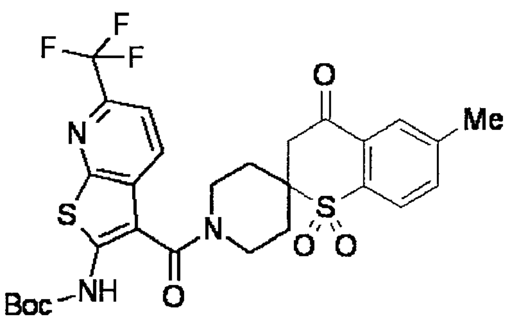

The title compound (364 mg, yield 61%) was obtained as a powder in the same manner as in Example 1 and from 6'-methylspiro[piperidine-4,2'-thiochromen]-4'(3'H)-one 1',1'-dioxide hydrochloride (300 mg, 0.952 mmol) obtained in Reference Example 140 and 2-[(tert-butoxycarbonyl)amino]-6-(trifluoromethyl)thieno[2,3-b]pyridine-3-carboxylic acid (344 mg, 0.952 mmol) obtained in Reference Example 138.
EI(pos) 623.8 $[M+H]^+$

Example 193

N-ethyl-N'-[3-[(6'-methyl-1',1'-dioxido-4'-oxo-3',4'-dihydro-1H-spiro[piperidine-4,2'-thiochromen]-1-yl)carbonyl]-6-(trifluoromethyl)thieno[2,3-b]pyridin-2-yl]urea

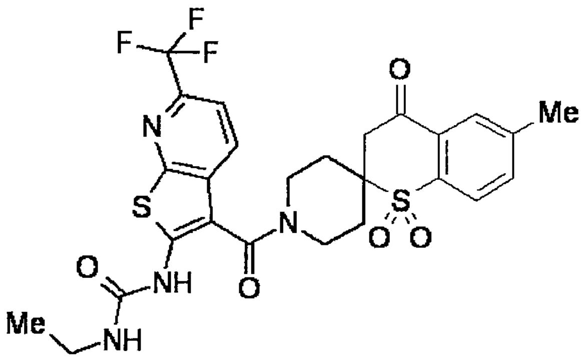

1-{[2-Amino-6-(trifluoromethyl)thieno[2,3-b]pyridin-3-yl]carbonyl}-6'-methylspiro[piperidine-4,2'-thiochromen]-4'(3'H)-one 1',1'-dioxide (258 mg, yield 85%) was obtained as a powder in the same manner as in Example 4 and from tert-butyl [3-[(6'-methyl-1',1'-dioxido-4'-oxo-3',4'-dihydro-1H-spiro[piperidine-4,2'-thiochromen]-1-yl)carbonyl]-6-(trifluoromethyl)thieno[2,3-b]pyridin-2-yl]carbamate (364 mg, 0.584 mmol) obtained in Example 192 and trifluoroacetic acid (4 mL). The title compound (141 mg, yield 48%) was obtained as a powder in the same manner as in Example 2 and from the obtained amino compound (258 mg, 0.493 mmol) and ethyl isocyanate (0.485 mL, 6.15 mmol).
EI(pos) 595 $[M+H]^+$

Example 194

1-[(2- amino- 6- methylthieno [2,3- b] pyridin- 3- yl) carbonyl]- 6'- fluorospiro [piperidine- 4,2'- thiochromen]- 4' (3'H)-one
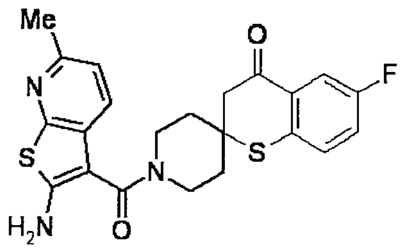

The title compound (530 mg, yield 69%) was obtained as a powder in the same manner as in Example 1 and from 6'-fluorospiro[piperidine-4,2'-thiochromen]-4'(3'H)-one hydrochloride (500 mg, 1.74 mmol) obtained in Reference Example 145 and 2-amino-6-methylthieno[2,3-b]pyridine-3-carboxylic acid trifluoroacetate (560 mg, 1.74 mmol) obtained in Reference Example 11.
EI(pos) 442 $[M+H]^+$

Example 195

N-{3-[(6'-fluoro-4'-oxo-3',4'-dihydro-1H-spiro[piperidine-4,2'-thiochromen]-1-yl)carbonyl]-6-methylthieno[2,3-b]pyridin-2-yl}urea
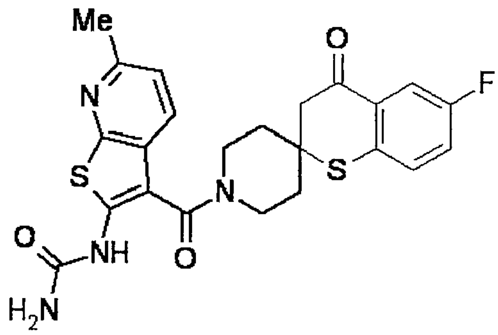

The title compound (138 mg, yield 68%) was obtained as a powder in the same manner as in Reference Example 38 and from 1-[(2-amino-6-methylthieno[2,3-b]pyridin-3-yl)carbonyl]-6'-fluorospiro[piperidine-4,2'-thiochromen]-4'(3'H)-one (185 mg, 0.419 mmol) obtained in Example 194, trichloroacetyl isocyanate (0.075 mL, 0.628 mmol) and 7M ammonia-methanol (0.7 mL).
EI(pos) 485 $[M+H]^+$

Example 196

N-ethyl-N'-{3-[(6'-fluoro-4'-oxo-3',4'-dihydro-1H-spiro[piperidine-4,2'-thiochromen]-1-yl)carbonyl]-6-methylthieno[2,3-b]pyridin-2-yl}urea
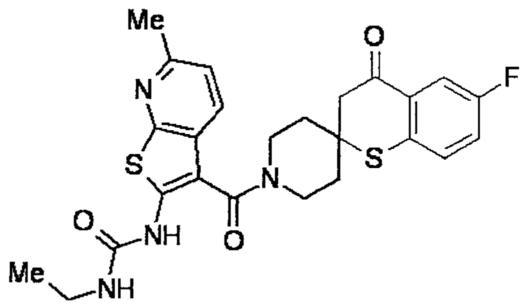

The title compound (153 mg, yield 73%) was obtained as a powder in the same manner as in Example 2 and from 1-[(2-amino-6-methylthieno[2,3-b]pyridin-3-yl)carbonyl]-6'-fluorospiro[piperidine-4,2'-thiochromen]-4'(3'H)-one (180 mg, 0.408 mmol) obtained in Example 194 and ethyl isocyanate (0.12 mL, 1.53 mmol).
EI(pos) 513 $[M+H]^+$

Example 197

1-[(5-acetyl-2-amino-4-methylthiophen-3-yl)carbonyl]-6'-fluorospiro[piperidine-4,2'-thiochromen]-4'(3'H)-one
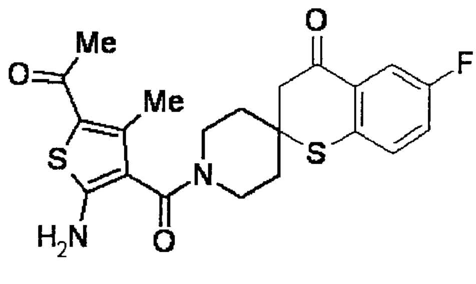

The title compound (224 mg, yield 37%) was obtained as a powder in the same manner as in Example 1 and from 6'-fluorospiro[piperidine-4,2'-thiochromen]-4'(3'H)-one hydrochloride (400 mg, 1.39 mmol) obtained in Reference Example 145 and 5-acetyl-2-amino-4-methylthiophene-3-carboxylic acid (332 mg, 1.67 mmol).
EI(pos) 432.9 $[M+H]^+$

Example 198

1-{5-acetyl-3-[(6'-fluoro-4'-oxo-3',4'-dihydro-1H-spiro[piperidine-4,2'-thiochromen]-1-yl)carbonyl]-4-methylthiophen-2-yl}-3-ethylurea
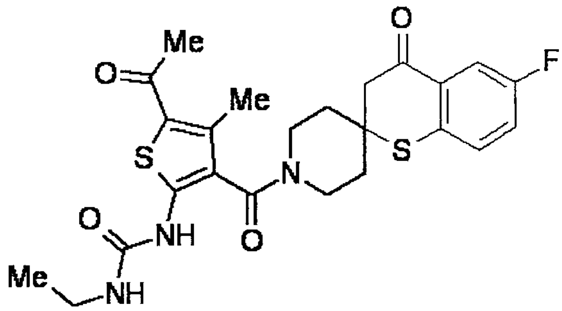

The title compound (206 mg, yield 82%) was obtained as a powder in the same manner as in Example 2 and from 1-[(5-acetyl-2-amino-4-methylthiophen-3-yl)carbonyl]-6'-fluorospiro[piperidine-4,2'-thiochromen]-4'(3'H)-one (216 mg, 0.499 mmol) obtained in Example 197 and ethyl isocyanate (0.237 mL, 3.00 mmol).
EI(pos) 503.9 $[M+H]^+$

Example 199

1-[(2-amino-7-oxo-4,5,6,7-tetrahydro-1-benzothiophen-3-yl)carbonyl]-6'-fluorospiro[piperidine-4,2'-thiochromen]-4'(3'H)-one
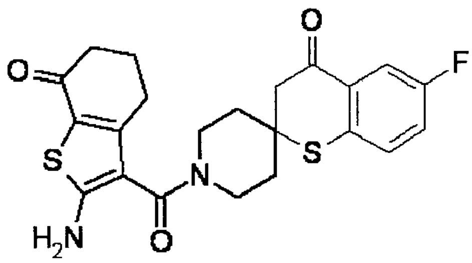

The title compound (448 mg, yield 75%) was obtained as a powder in the same manner as in Example 1 and from 6'-fluorospiro[piperidine-4,2'-thiochromen]-4'(3'H)-one hydrochloride (386 mg, 1.34 mmol) obtained in Reference Example 145 and 2-amino-7-oxo-4,5,6,7-tetrahydro-1-benzothiophene-3-carboxylic acid (283 mg, 1.34 mmol) (produced by the method described in WO07/013691).
EI(pos) 444.9 $[M+H]^+$

Example 200

1-ethyl-3-{3-[(6'-fluoro-4'-oxo-3',4'-dihydro-1H-spiro[piperidine-4,2'-thiochromen]-1-yl)carbonyl]-7-oxo-4,5,6,7-tetrahydro-1-benzothiophen-2-yl}urea
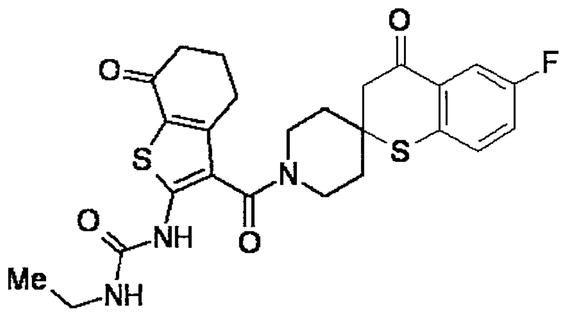

The title compound (166 mg, yield 71%) was obtained as a powder in the same manner as in Example 2 and from 1-[(2-amino-7-oxo-4,5,6,7-tetrahydro-1-benzothiophen-3-yl)carbonyl]-6'-fluorospiro[piperidine-4,2'-thiochromen]-4'(3'H)-one (200 mg, 0.450 mmol) obtained in Example 199 and ethyl isocyanate (0.214 mL, 2.70 mmol).
EI(pos) 515.9 $[M+H]^+$

Example 201

tert-butyl {3-[(6'-cyano-4'-oxo-3',4'-dihydro-1H-spiro[piperidine-4,2'-thiochromen]-1-yl)carbonyl]-6-(trifluoromethyl)thieno[2,3-b]pyridin-2-yl}carbamate
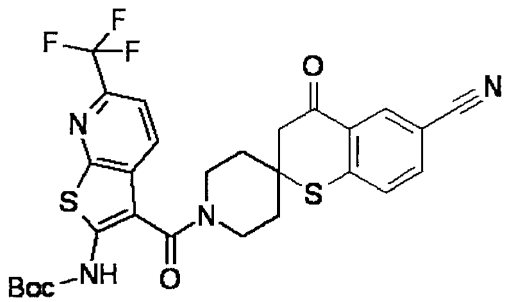

The title compound (820 mg, quantitative) was obtained as a powder in the same manner as in Example 1 and from 4'-oxo-3',4'-dihydrospiro[piperidine-4,2'-thiochromene]-6'-carbonitrile hydrochloride (401 mg, 1.36 mmol) obtained in Reference Example 103 and 2-[(tert-butoxycarbonyl)amino]-6-(trifluoromethyl)thieno[2,3-b]pyridine-3-carboxylic acid (493 mg, 1.36 mmol) obtained in Reference Example 138.
EI(pos) 602.9 $[M+H]^+$

Example 202

1-{[2-amino-6-(trifluoromethyl)thieno[2,3-b]pyridin-3-yl]carbonyl}-4'-oxo-3',4'-dihydrospiro[piperidine-4,2'-thiochromene]-6'-carbonitrile
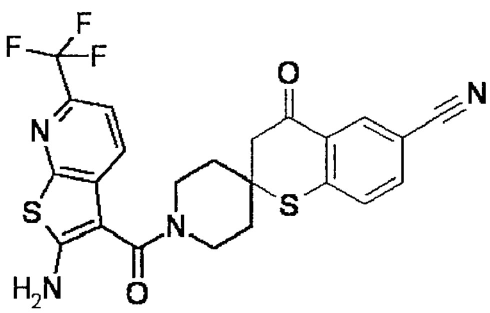

The title compound (279 mg, yield 41%) was obtained as a powder in the same manner as in Example 4 and from tert-butyl {3-[(6'-cyano-4'-oxo-3',4'-dihydro-1H-spiro[piperidine-4,2'-thiochromen]-1-yl)carbonyl]-6-(trifluoromethyl) thieno[2,3-b]pyridin-2-yl}carbamate (820 mg, 1.36 mmol) obtained in Example 201 and trifluoroacetic acid (7 mL).
EI(pos) 502.9 $[M+H]^+$

Example 203

1-{3-[(6'-cyano-4'-oxo-3',4'-dihydro-1H-spiro[piperidine-4,2'-thiochromen]-1-yl)carbonyl]-6-(trifluoromethyl) thieno[2,3-b]pyridin-2-yl}-3-methylurea

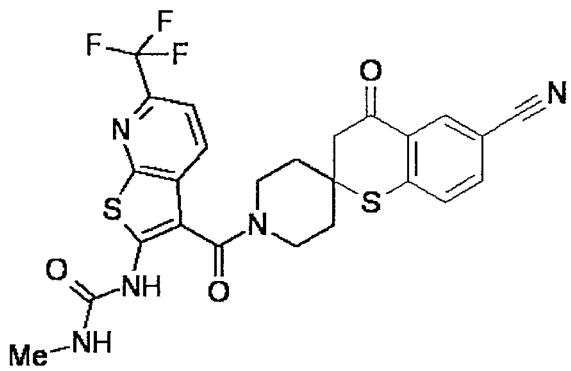

The title compound (115 mg, yield 79%) was obtained as a powder in the same manner as in Example 2 and from 1-{[2-amino-6-(trifluoromethyl)thieno[2,3-b]pyridin-3-yl]carbonyl}-4'-oxo-3',4'-dihydrospiro[piperidine-4,2'-thiochromene]-6'-carbonitrile (131 mg, 0.261 mmol) obtained in Example 202 and methyl isocyanate (0.124 mL, 2.08 mmol).
EI(pos) 559.8 $[M+H]^+$

Example 204

tert-butyl {3-[(6-fluoro-4-oxo-3,4-dihydro-1'H-spiro[chromene-2,4'-piperidin]-1'-yl)carbonyl]-6-(trifluoromethyl) thieno[2,3-b]pyridin-2-yl}carbamate

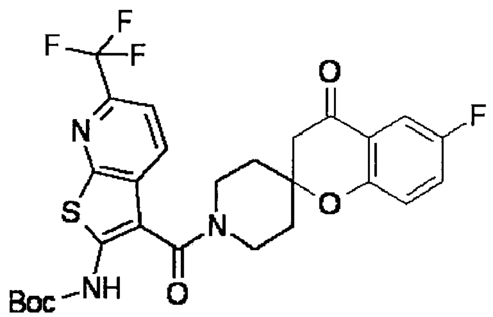

The title compound (835 mg, yield 78%) was obtained as a powder in the same manner as in Example 1 and from 6-fluorospiro[chromene-2,4'-piperidin]-4(3H)-one hydrochloride (500 mg, 1.84 mmol) and 2-[(tert-butoxycarbonyl) amino]-6-(trifluoromethyl)thieno[2,3-b]pyridine-3-carboxylic acid (667 mg, 1.84 mmol) obtained in Reference Example 138.
EI(pos) 579.9 $[M+H]^+$

Example 205

1'-{[2-amino-6-(trifluoromethyl)thieno[2,3-b]pyridin-3-yl]carbonyl}-6-fluorospiro[chromene-2,4'-piperidin]-4

(3H)-one
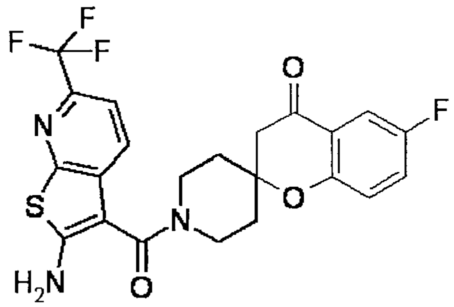

The title compound (584 mg, yield 86%) was obtained as a powder in the same manner as in Example 4 and from tert-butyl {3-[(6-fluoro-4-oxo-3,4-dihydro-1'H-spiro[chromene-2,4'-piperidin]-1'-yl)carbonyl]-6-(trifluoromethyl) thieno[2,3-b]pyridin-2-yl}carbamate (817 mg, 1.41 mmol) obtained in Example 204 and trifluoroacetic acid (7 mL).
EI(pos) 479.9 $[M+H]^+$

Example 206

1-{3-[(6-fluoro-4-oxo-3,4-dihydro-1'H-spiro[chromene-2,4'-piperidin]-1'-yl)carbonyl]-6-(trifluoromethyl)thieno [2,3-b]pyridin-2-yl}-3-methylurea
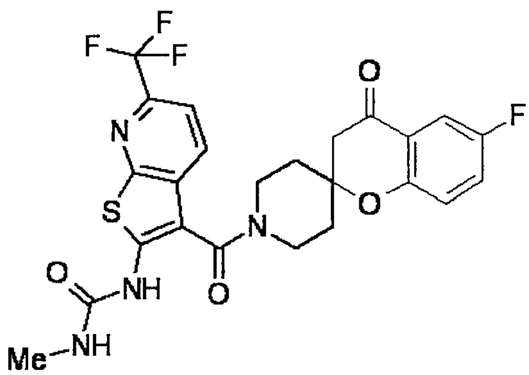

The title compound (160 mg, yield 72%) was obtained as a powder in the same manner as in Example 2 and from 1'-{[2-amino-6-(trifluoromethyl)thieno[2,3-b]pyridin-3-yl]carbonyl}-6-fluorospiro[chromene-2,4'-piperidin]-4 (3H)-one (200 mg, 0.417 mmol) obtained in Example 205 and methyl isocyanate (0.246 mL, 4.17 mmol).
EI(pos) 536.9 $[M+H]^+$

Example 207

1-{3-[(6-fluoro-4-oxo-3,4-dihydro-1'H-spiro[chromene-2,4'-piperidin]-1'-yl)carbonyl]-6-(trifluoromethyl)thieno [2,3-b]pyridin-2-yl}urea
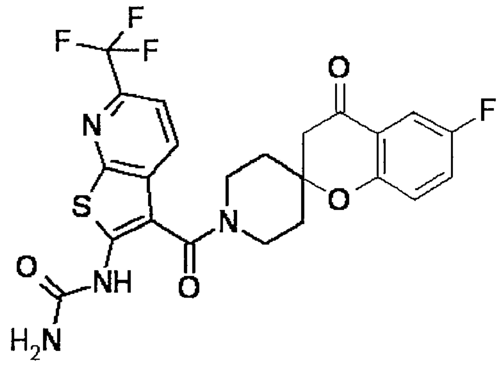

The title compound (197 mg, yield 84%) was obtained as a powder in the same manner as in Reference Example 38 and from 1'-{[2-amino-6-(trifluoromethyl)thieno[2,3-b]pyridin-3-yl]carbonyl}-6-fluorospiro[chromene-2,4'-piperidin]-4(3H)-one (216 mg, 0.450 mmol) obtained in Example 205, trichloroacetyl isocyanate (0.107 mL, 0.900 mmol) and 7M ammonia-methanol (2 mL).
EI(pos) 522.8 $[M+H]^+$

Example 208

tert-butyl {3-[(6-chloro-4-oxo-3,4-dihydro-1'H-spiro[chromene-2,4'-piperidin]-1'-yl)carbonyl]-6-(trifluoromethyl) thieno[2,3-b]pyridin-2-yl}carbamate
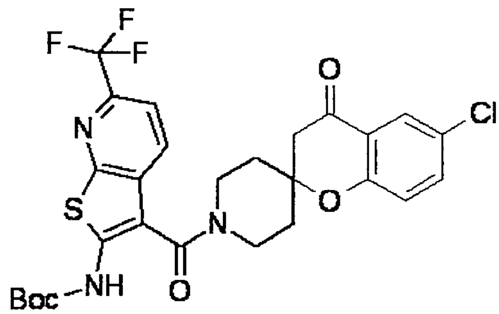

The title compound (816 mg, yield 79%) was obtained as a powder in the same manner as in Example 1 and from 6-chlorospiro[chromene-2,4'-piperidin]-4(3H)-one hydrochloride (500 mg, 1.74 mmol) and 2-[(tert-butoxycarbonyl) amino]-6-(trifluoromethyl)thieno[2,3-b]pyridine-3-carboxylic acid (629 mg, 1.74 mmol) obtained in Reference Example 138.
EI(pos) 595.8 $[M+H]^+$

Example 209

1'-{[2-amino-6-(trifluoromethyl)thieno[2,3-b]pyridin-3-yl]carbonyl}-6-chlorospiro[chromene-2,4'-piperidin]-4 (3H)-one
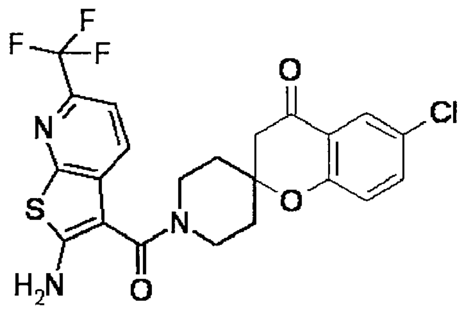

The title compound (572 mg, yield 86%) was obtained as a powder in the same manner as in Example 4 and from tert-butyl {3-[(6-chloro-4-oxo-3,4-dihydro-1'H-spiro[chromene-2,4'-piperidin]-1'-yl)carbonyl]-6-(trifluoromethyl) thieno[2,3-b]pyridin-2-yl}carbamate (803 mg, 1.35 mmol) obtained in Example 208 and trifluoroacetic acid (7 mL).
EI(pos) 495.8 $[M+H]^+$

Example 210

1-{3-[(6-chloro-4-oxo-3,4-dihydro-1'H-spiro[chromene-2,4'-piperidin]-1'-yl)carbonyl]-6-(trifluoromethyl)thieno [2,3-b]pyridin-2-yl}-3-methylurea
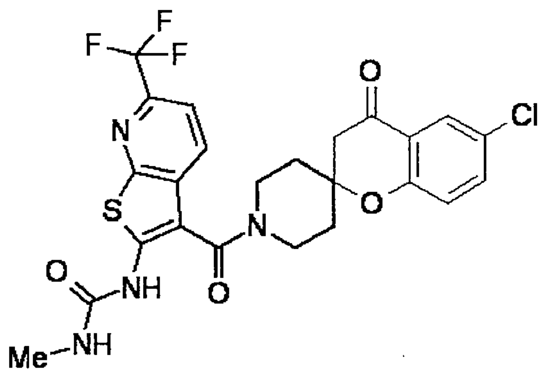

The title compound (129 mg, yield 58%) was obtained as a powder in the same manner as in Example 2 and from 1'-{[2-amino-6-(trifluoromethyl)thieno[2,3-b]pyridin-3-yl]carbonyl}-6-chlorospiro[chromene-2,4'-piperidin]-4 (3H)-one (200 mg, 0.403 mmol) obtained in Example 209 and methyl isocyanate (0.238 mL, 4.03 mmol).
EI(pos) 552.8 $[M+H]^+$

Example 211

1-{3-[(6-chloro-4-oxo-3,4-dihydro-1'H-spiro[chromene-2,4'-piperidin]-1'-yl)carbonyl]-6-(trifluoromethyl)thieno [2,3-b]pyridin-2-yl}urea
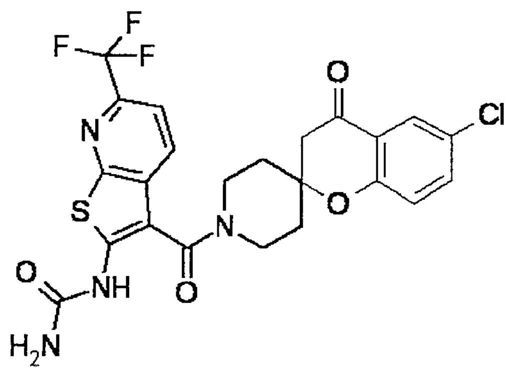

The title compound (152 mg, yield 62%) was obtained as a powder in the same manner as in Reference Example 38 and from 1'-{[2-amino-6-(trifluoromethyl)thieno[2,3-b]pyridin-3-yl]carbonyl}-6-chlorospiro[chromene-2,4'-piperidin]-4(3H)-one (223 mg, 0.450 mmol) obtained in Example 209, trichloroacetyl isocyanate (0.107 mL, 0.900 mmol) and 7M ammonia-methanol (2 mL).
EI(pos) 538.8 $[M+H]^+$

Example 212

tert-butyl {3-[(6-methyl-4-oxo-3,4-dihydro-1'H-spiro[chromene-2,4'-piperidin]-1'-yl)carbonyl]-6-(trifluoromethyl)thieno[2,3-b]pyridin-2-yl}carbamate
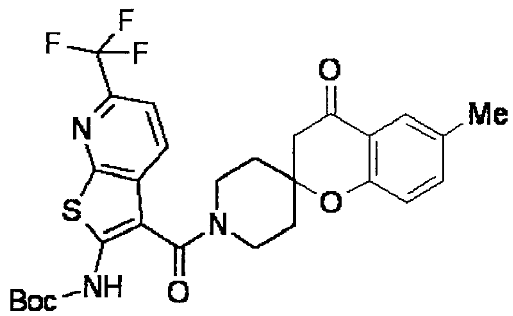

The title compound (897 mg, yield 84%) was obtained as a powder in the same manner as in Example 1 and from 6-methylspiro[chromene-2,4'-piperidin]-4(3H)-one hydrochloride (500 mg, 1.87 mmol) and 2-[(tert-butoxycarbonyl) amino]-6-(trifluoromethyl)thieno[2,3-b]pyridine-3-carboxylic acid (677 mg, 1.87 mmol) obtained in Reference Example 138.
EI (pos) 575.9 $[M+H]^+$

Example 213

1'-{[2-amino-6-(trifluoromethyl)thieno[2,3-b]pyridin-3-yl]carbonyl}-6-methylspiro[chromene-2,4'-piperidin]-4 (3H)-one
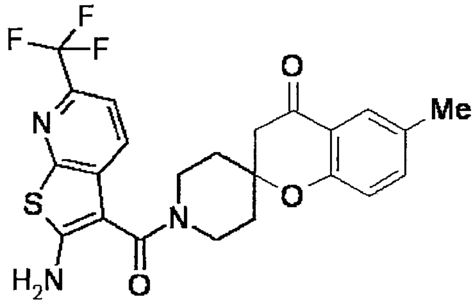

The title compound (540 mg, yield 75%) was obtained as a powder in the same manner as in Example 4 and from tert-butyl {3-[(6-methyl-4-oxo-3,4-dihydro-1'H-spiro[chromene-2,4'-piperidin]-1'-yl)carbonyl]-6-(trifluoromethyl) thieno[2,3-b]pyridin-2-yl}carbamate (876 mg, 1.52 mmol) obtained in Example 212 and trifluoroacetic acid (7 mL).
EI(pos) 475.9 $[M+H]^+$

Example 214

1-{3-[(6-methyl-4-oxo-3,4-dihydro-1'H-spiro[chromene-2,4'-piperidin]-1'-yl)carbonyl]-6-(trifluoromethyl)thieno [2,3-b]pyridin-2-yl}-3-methylurea
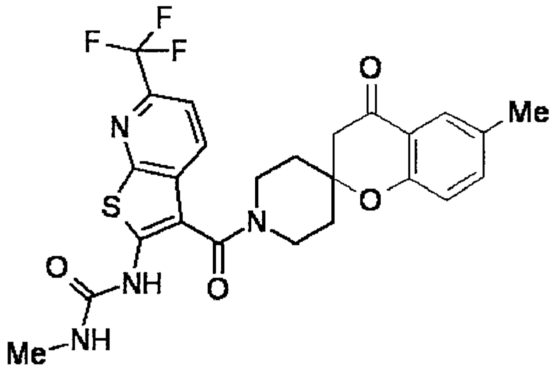

The title compound (118 mg, yield 53%) was obtained as a powder in the same manner as in Example 2 and from 1'-{[2-amino-6-(trifluoromethyl)thieno[2,3-b]pyridin-3-yl]carbonyl}-6-methylspiro[chromene-2,4'-piperidin]-4 (3H)-one (200 mg, 0.421 mmol) obtained in Example 213 and methyl isocyanate (0.248 mL, 4.21 mmol).
EI(pos) 532.9 $[M+H]^+$

Example 215

1-{3-[(6-methyl-4-oxo-3,4-dihydro-1'H-spiro[chromene-2,4'-piperidin]-1'-yl)carbonyl]-6-(trifluoromethyl)thieno [2,3-b]pyridin-2-yl}urea
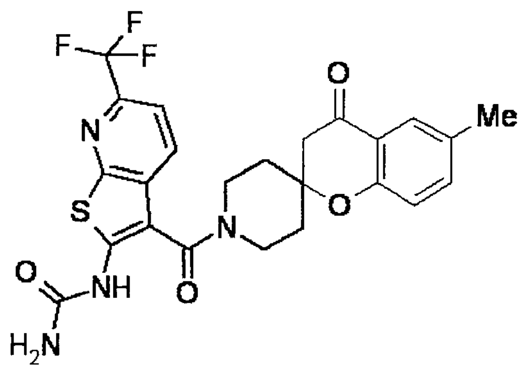

The title compound (161 mg, yield 69%) was obtained as a powder in the same manner as in Reference Example 38 and from 1'-{[2-amino-6-(trifluoromethyl)thieno[2,3-b]pyridin-3-yl]carbonyl}-6-methylspiro[chromene-2,4'-piperidin]-4(3H)-one (214 mg, 0.450 mmol) obtained in Example 213, trichloroacetyl isocyanate (0.107 mL, 0.900 mmol) and 7M ammonia-methanol (2 mL).
EI (pos) 518.9 $[M+H]^+$

Example 216

tert-butyl {3-[(6-cyano-4-oxo-3,4-dihydro-1'H-spiro[chromene-2,4'-piperidin]-1'-yl)carbonyl]-6-(trifluoromethyl) thieno[2,3-b]pyridin-2-yl}carbamate
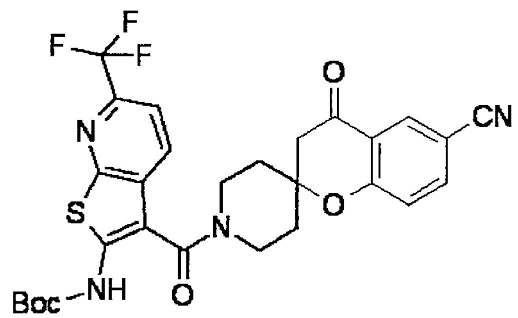

The title compound (1.25 g, yield 74%) was obtained as a powder in the same manner as in Example 1 and from 4-oxo-3,4-dihydrospiro[chromene-2,4'-piperidine]-6-carbonitrile hydrochloride (800 mg, 2.87 mmol) and 2-[(tert-butoxycarbonyl)amino]-6-(trifluoromethyl)thieno[2,3-b]pyridine-3-carboxylic acid (1.04 g, 2.87 mmol) obtained in Reference Example 138.
EI(pos) 587 $[M+H]^+$

Example 217

1'-{[2-amino-6-(trifluoromethyl)thieno[2,3-b]pyridin-3-yl]carbonyl}-4-oxo-3,4-dihydrospiro[chromene-2,4'-piperidine]-6-carbonitrile
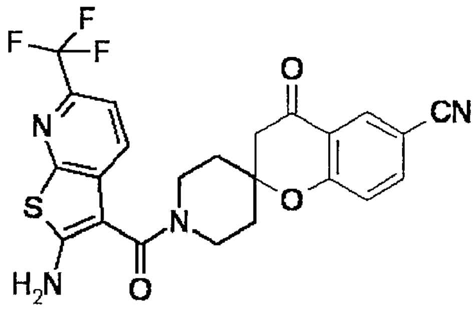

The title compound (927 mg, yield 89%) was obtained as a powder in the same manner as in Example 4 and from tert-butyl {3-[(6-cyano-4-oxo-3,4-dihydro-1'H-spiro[chromene-2,4'-piperidin]-1'-yl)carbonyl]-6-(trifluoromethyl) thieno[2,3-b]pyridin-2-yl}carbamate (1.25 g, 2.13 mmol) obtained in Example 216 and trifluoroacetic acid (5 mL).
EI(pos) 486 $[M+H]^+$

Example 218

1-{3-[(6-cyano-4-oxo-3,4-dihydro-1'H-spiro[chromene-2,4'-piperidin]-1'-yl)carbonyl]-6-(trifluoromethyl)thieno [2,3-b]pyridin-2-yl}-3-methylurea
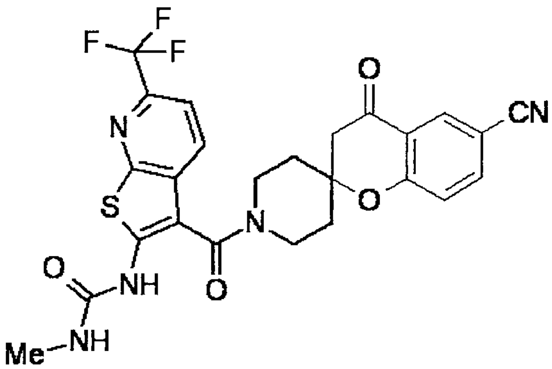

The title compound (199 mg, yield 41%) was obtained as a powder in the same manner as in Example 2 and from 1'-{[2-amino-6-(trifluoromethyl)thieno[2,3-b]pyridin-3-yl]carbonyl}-4-oxo-3,4-dihydrospiro[chromene-2,4'-piperidine]-6-carbonitrile (460 mg, 0.946 mmol) obtained in Example 217 and methyl isocyanate (0.336 mL, 5.68 mmol).
EI(pos) 544 $[M+H]^+$

Example 219

1-{3-[(6-cyano-4-oxo-3,4-dihydro-1'H-spiro[chromene-2,4'-piperidin]-1'-yl)carbonyl]-6-(trifluoromethyl)thieno [2,3-b]pyridin-2-yl}-3-ethylurea
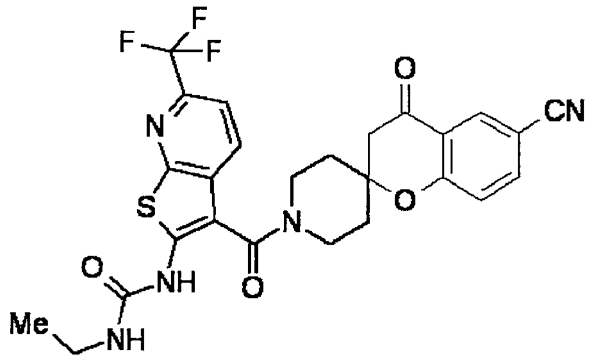

The title compound (433 mg, yield 82%) was obtained as a powder in the same manner as in Example 2 and from 1'-{[2-amino-6-(trifluoromethyl)thieno[2,3-b]pyridin-3-yl]carbonyl}-4-oxo-3,4-dihydrospiro[chromene-2,4'-piperidine]-6-carbonitrile (460 mg, 0.946 mmol) obtained in Example 217 and ethyl isocyanate (0.450 mL, 5.68 mmol).
EI(pos) 558 $[M+H]^+$

Example 220

1'-[(5-acetyl-2-amino-4-methylthiophen-3-yl)carbonyl]-6-fluorospiro[chromene-2,4'-piperidin]-4(3H)-one
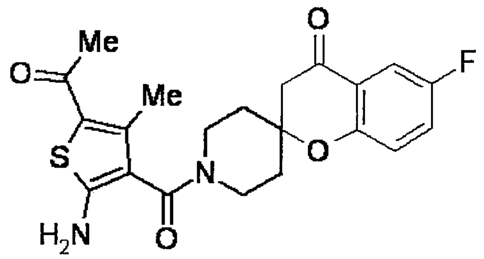

The title compound (253 mg, yield 37%) was obtained as a powder in the same manner as in Example 1 and from 6-fluorospiro[chromene-2,4'-piperidin]-4(3H)-one hydrochloride (447 mg, 1.65 mmol) and 5-acetyl-2-amino-4-methylthiophene-3-carboxylic acid (333 mg, 1.65 mmol).
EI(pos) 417 $[M+H]^+$

Example 221

1-{5-acetyl-3-[(6-fluoro-4-oxo-3,4-dihydro-1'H-spiro[chromene-2,4'-piperidin]-1'-yl)carbonyl]-4-methylthiophen-2-yl}-3-ethylurea
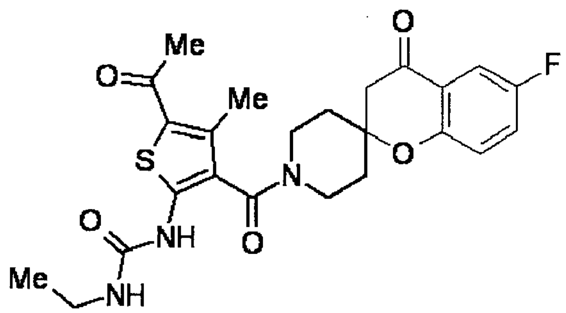

The title compound (145 mg, yield 49%) was obtained as a powder in the same manner as in Example 2 and from 1'-[(5-acetyl-2-amino-4-methylthiophen-3-yl)carbonyl]-6-fluorospiro[chromene-2,4'-piperidin]-4(3H)-one (253 mg,

0.608 mmol) obtained in Example 220 and ethyl isocyanate (0.192 mL, 2.44 mmol).
EI(pos) 488.2 $[M+H]^+$

Example 222

1'-[(5-acetyl-2-amino-4-methylthiophen-3-yl)carbonyl]-6-chlorospiro[chromene-2,4'-piperidin]-4(3H)-one
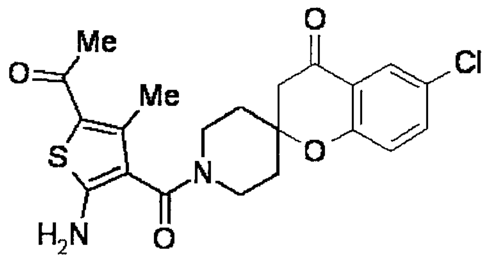

The title compound (272 mg, yield 38%) was obtained as a powder in the same manner as in Example 1 and from 6-chlorospiro[chromene-2,4'-piperidin]-4(3H)-one hydrochloride (475 mg, 1.65 mmol) and 5-acetyl-2-amino-4-methylthiophene-3-carboxylic acid (333 mg, 1.65 mmol).
EI(pos) 433 $[M+H]^+$

Example 223

1-{5-acetyl-3-[(6-chloro-4-oxo -3,4-dihydro-1'H-spiro[chromene-2,4'-piperidin]-1'-yl)carbonyl]-4-methylthiophen-2-yl}-3-ethylurea
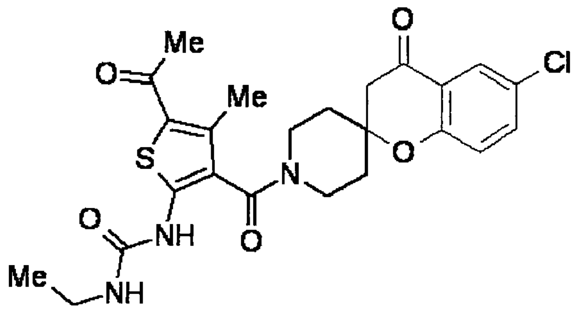

The title compound (198 mg, yield 63%) was obtained as a powder in the same manner as in Example 2 and from 1'-[(5-acetyl-2-amino-4-methylthiophen-3-yl)carbonyl]-6-chlorospiro[chromene-2,4'-piperidin]-4(3H)-one (272 mg, 0.629 mmol) obtained in Example 222 and ethyl isocyanate (0.198 mL, 2.52 mmol).
EI(pos) 504.1 $[M+H]^+$

Example 224

1'-[(5-acetyl-2-amino-4-methylthiophen-3-yl)carbonyl]-6-methylspiro[chromene-2,4'-piperidin]-4(3H)-one
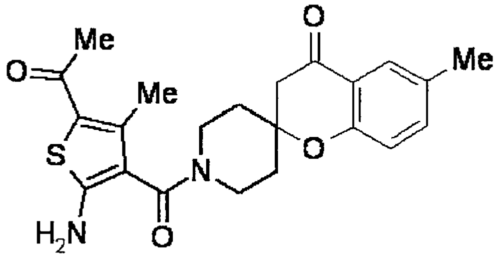

The title compound (190 mg, yield 27%) was obtained as a powder in the same manner as in Example 1 and from 6-methylspiro[chromene-2,4'-piperidin]-4(3H)-one hydrochloride (442 mg, 1.65 mmol) and 5-acetyl-2-amino-4-methylthiophene-3-carboxylic acid (333 mg, 1.65 mmol).
EI(pos) 413 $[M+H]^+$

Example 225

1-{5-acetyl-3-[(6-methyl-4-oxo-3,4-dihydro-1'H-spiro[chromene-2,4'-piperidin]-1'-yl)carbonyl]-4-methylthiophen-2-yl}-3-ethylurea
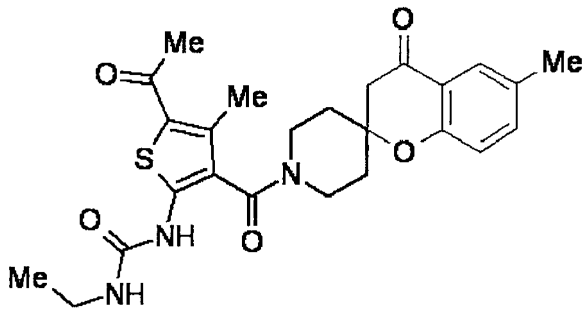

The title compound (138 mg, yield 63%) was obtained as a powder in the same manner as in Example 2 and from 1'-[(5-acetyl-2-amino-4-methylthiophen-3-yl)carbonyl]-6-methylspiro[chromene-2,4'-piperidin]-4(3H)-one (190 mg, 0.456 mmol) obtained in Example 224 and ethyl isocyanate (0.180 mL, 2.28 mmol).
EI(pos) 484 $[M+H]^+$

Example 226

1'-[(2-amino-7-oxo-4,5,6,7-tetrahydro-1-benzothiophen-3-yl)carbonyl]-6-fluorospiro[chromene-2,4'-piperidin]-4(3H)-one
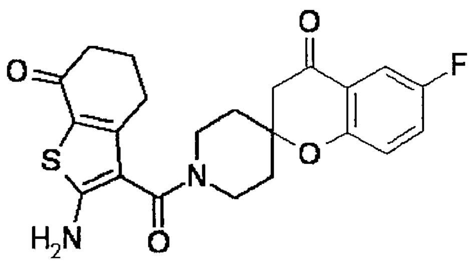

The title compound (350 mg, yield 69%) was obtained as a powder in the same manner as in Example 1 and from 6-fluorospiro[chromene-2,4'-piperidin]-4(3H)-one hydrochloride (319 mg, 1.18 mmol) and 2-amino-7-oxo-4,5,6,7-tetrahydro-1-benzothiophene-3-carboxylic acid (250 mg, 1.18 mmol) (produced by the method described in

WO07/013691).
EI(pos) 429.1 [M+H]+

Example 227

1-ethyl-3-{3-[(6-fluoro-4-oxo-3,4-dihydro-1'H-spiro[chromene-2,4'-piperidin]-1'-yl)carbonyl]-7-oxo-4,5,6,7-tetrahydro-1-benzothiophen-2-yl}urea
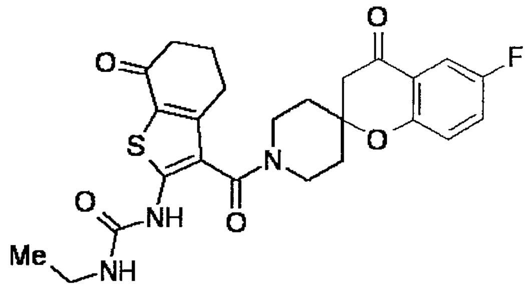

The title compound (147 mg, yield 62%) was obtained as a powder in the same manner as in Example 2 and from 1'-[(2-amino-7-oxo-4,5,6,7-tetrahydro-1-benzothiophen-3-yl)carbonyl]-6-fluorospiro[chromene-2,4'-piperidin]-4(3H)-one (200 mg, 0.467 mmol) obtained in Example 226 and ethyl isocyanate (0.220 mL, 2.80 mmol).
EI(pos) 499.9 [M+H]+

Example 228

1'-[(2-amino-7-oxo-4,5,6,7-tetrahydro-1-benzothiophen-3-yl)carbonyl]-6-chlorospiro[chromene-2,4'-piperidin]-4(3H)-one
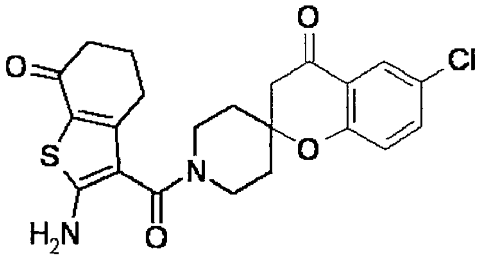

The title compound (464 mg, yield 86%) was obtained as a powder in the same manner as in Example 1 and from 6-chlorospiro[chromene-2,4'-piperidin]-4(3H)-one hydrochloride (341 mg, 1.18 mmol) and 2-amino-7-oxo-4,5,6,7-tetrahydro-l-benzothiophene-3-carboxylic acid (250 mg, 1.18 mmol) (produced by the method described in WO07/013691).
EI(pos) 444.9 [M+H]+

Example 229

1-ethyl-3-{3-[(6-chloro-4-oxo-3,4-dihydro-1'H-spiro[chromene-2,4'-piperidin]-1'-yl)carbonyl]-7-oxo-4,5,6,7-tetrahydro-1-benzothiophen-2-yl}urea
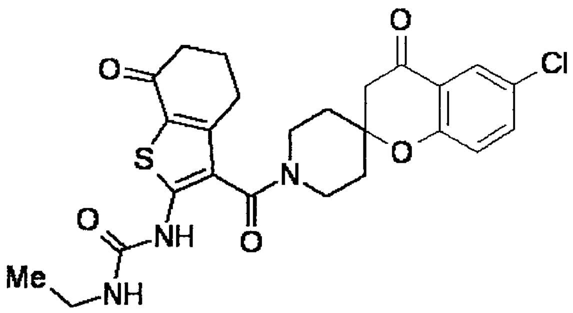

The title compound (177 mg, yield 58%) was obtained as a powder in the same manner as in Example 2 and from 1'-[(2-amino-7-oxo-4,5,6,7-tetrahydro-1-benzothiophen-3-yl)carbonyl]-6-chlorospiro[chromene-2,4'-piperidin]-4(3H)-one (264 mg, 0.593 mmol) obtained in Example 228 and ethyl isocyanate (0.282 mL, 3.58 mmol).
EI(pos) 515.9 $[M+H]^+$

Example 230

1'-[(2-amino-7-oxo-4,5,6,7-tetrahydro-1-benzothiophen-3-yl)carbonyl]-6-methylspiro[chromene-2,4'-piperidin]-4(3H)-one
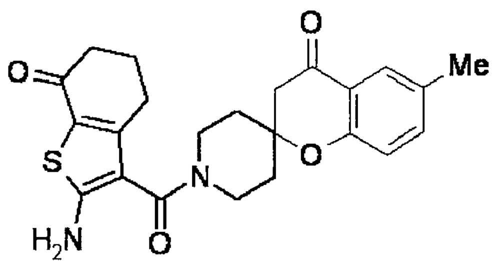

The title compound (165 mg, yield 42%) was obtained as a powder in the same manner as in Example 1 and from 6-methylspiro[chromene-2,4'-piperidin]-4(3H)-one hydrochloride (245 mg, 0.920 mmol) and 2-amino-7-oxo-4,5,6,7-tetrahydro-1-benzothiophene-3-carboxylic acid (196 mg, 0.920 mmol) (produced by the method described in WO07/013691).
EI(pos) 425 $[M+H]^+$

Example 231

1-ethyl-3-{3-[(6-methyl-4-oxo-3,4-dihydro-1'H-spiro[chromene-2,4'-piperidin]-1'-yl)carbonyl]-7-oxo-4,5,6,7-tetrahydro-1-benzothiophen-2-yl}urea
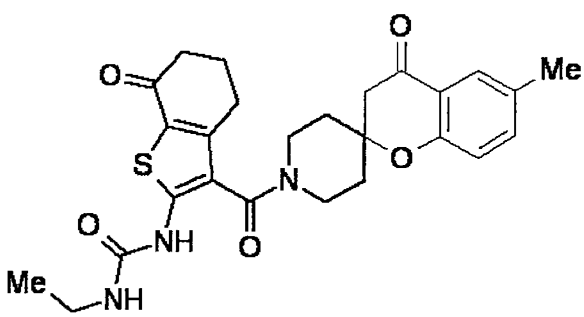

The title compound (119 mg, yield 62%) was obtained as a powder in the same manner as in Example 2 and

from 1'-[(2-amino-7-oxo-4,5,6,7-tetrahydro-1-benzothiophen-3-yl)carbonyl]-6-methylspiro[chromene-2,4'-piperidin]-4(3H)-one (165 mg, 0.387 mmol) obtained in Example 230 and ethyl isocyanate (0.157 mL, 1.98 mmol).
EI(pos)496 [M+H]+

Example 232

1'-[(2-amino-7-methoxy-1-benzothiophen-3-yl)carbonyl]-6-fluorospiro[chromene-2,4'-piperidin]-4(3H)-one
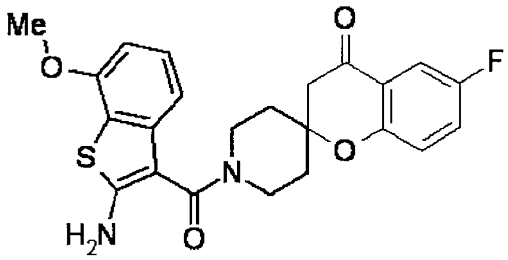

The title compound (967 mg, yield 77%) was obtained as a powder in the same manner as in Example 1 and from 6-fluorospiro[chromene-2,4'-piperidin]-4(3H)-one hydrochloride (778 mg, 2.86 mmol) and 2-amino-7-methoxy-1-benzothiophene-3-carboxylic acid (639 mg, 2.86 mmol) (produced by the method described in WO07/119833).
EI(pos) 440.9 [M+H]+

Example 233

tert-butyl N-({3-[(6-fluoro-4-oxo-3,4-dihydro-1'H-spiro[chromene-2,4'-piperidin]-1'-yl)carbonyl]-7-methoxy-1-benzothiophen-2-yl}carbamoyl)-D-alaninate
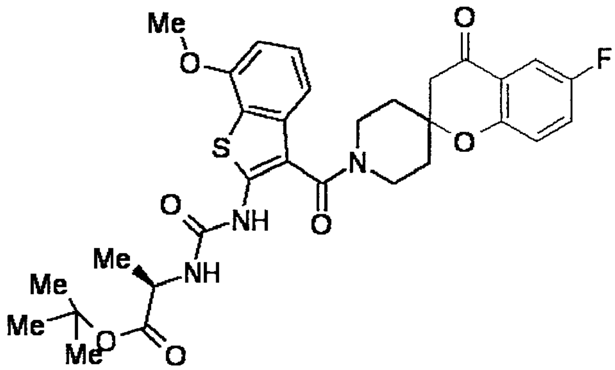

To a solution of 1'-[(2-amino-7-methoxy-1-benzothiophen-3-yl)carbonyl]-6-fluorospiro[chromene-2,4'-piperidin]-4(3H)-one (200 mg, 0.454 mmol) obtained in Example 232 and pyridine (1 mL) in DMF (3 mL) was added phenyl chlorocarbonate (0.0854 mL, 0.681 mmol) under ice-cooling, and the mixture was stirred for 30 min. To this solution were successively added a solution of tert-butyl D-alaninate (165 mg, 0.908 mmol) in DMF (1 mL) and DBU (0.136 mL, 0.908 mmol), and the mixture was stirred with heating at 60°C for 1 hr. The reaction mixture was diluted with ethyl acetate, and the mixture was washed with 0.5N hydrochloric acid, 10% aqueous potassium carbonate solution and saturated brine, dried over anhydrous sodium sulfate, and subjected to basic silica gel column chromatography (ethyl acetate). The solution was concentrated, and the obtained residue was purified by silica gel column chromatography (hexane:ethyl acetate=3:1 to 1:1) to give the title compound (214 mg, yield 77%) as an oil.
EI(pos) 612.0 [M+H]+

Example 234

N-({3-[(6-fluoro-4-oxo-3,4-dihydro-1'H-spiro[chromene-2,4'-piperidin]-1'-yl)carbonyl]-7-methoxy-1-benzothiophen-2-yl}carbamoyl)-D-alanine

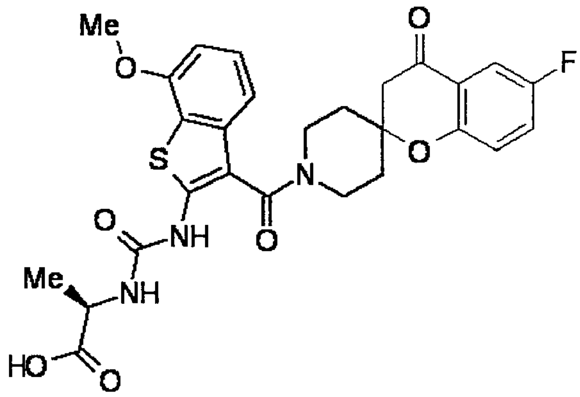

To tert-butyl N-({3-[(6-fluoro-4-oxo-3,4-dihydro-1'H-spiro[chromene-2,4'-piperidin]-1'-yl)carbonyl]-7-methoxy-1-benzothiophen-2-yl}carbamoyl)-D-alaninate (214 mg, 0.350 mmol) obtained in Example 233 was added TFA (2 mL), and the mixture was stirred for 1 hr. The solution was concentrated under reduced pressure, and the obtained residue was triturated with diisopropyl ether to give the title compound (182 mg, yield 94%).
EI(pos) 555.9 $[M+H]^+$

Example 235

tert-butyl N-({3-[(6-fluoro-4-oxo-3,4-dihydro-1'H-spiro[chromene-2,4'-piperidin]-1'-yl)carbonyl]-7-methoxy-1-benzothiophen-2-yl}carbamoyl)-L-alaninate

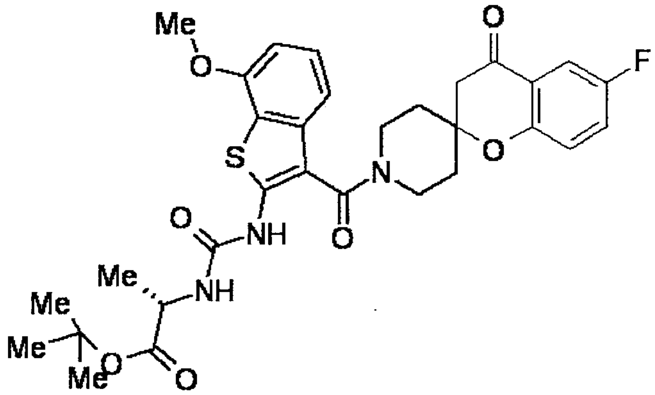

The title compound (267 mg, yield 96%) was obtained as an oil in the same manner as in Example 233 and using 1'-[(2-amino-7-methoxy-1-benzothiophen-3-yl)carbonyl]-6-fluorospiro[chromene-2,4'-piperidin]-4(3H)-one (200 mg, 0.454 mmol) obtained in Example 232, phenyl chlorocarbonate (0.0854 mL, 0.681 mmol) and tert-butyl L-alaninate (165 mg, 0.908 mmol).
EI(pos) 611.9 $[M+H]^+$

Example 236

N-({3-[(6-fluoro-4-oxo-3,4-dihydro-1'H-spiro[chromene-2,4'-piperidin]-1'-yl)carbonyl]-7-methoxy-1-benzothiophen-2-yl}carbamoyl)-L-alanine

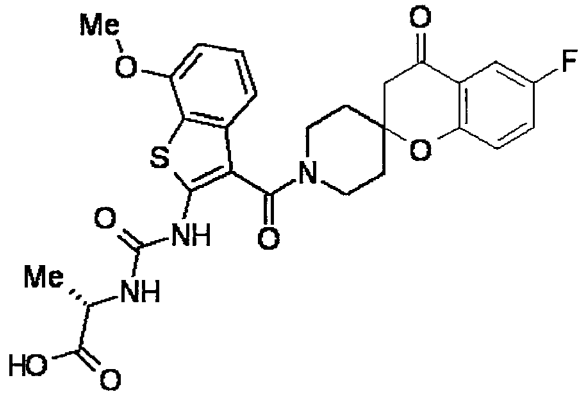

The title compound (211 mg, yield 87%) was obtained as a powder in the same manner as in Example 234 and using tert-butyl N-({3-[(6-fluoro-4-oxo-3,4-dihydro-1'H-spiro[chromene-2,4'-piperidin]-1'-yl)carbonyl]-7-methoxy-1-benzothiophen-2-yl}carbamoyl)-L-alaninate (266 mg, 0.435 mmol) obtained in Example 235 and TFA (2 mL).
EI(pos) 555.9 $[M+H]^+$

Example 237

tert-butyl N-({3-[(6-fluoro-4-oxo-3,4-dihydro-1'H-spiro[chromene-2,4'-piperidin]-1'-yl)carbonyl]-7-methoxy-1-benzothiophen-2-yl}carbamoyl)-2-methylalaninate
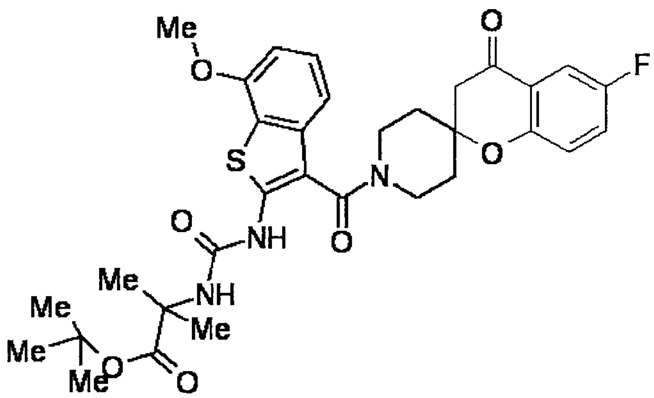

The title compound (253 mg, yield 89%) was obtained as an oil in the same manner as in Example 233 and using 1'-[(2-amino-7-methoxy-1-benzothiophen-3-yl)carbonyl]-6-fluorospiro[chromene-2,4'-piperidin]-4(3H)-one (200 mg, 0.454 mmol) obtained in Example 232, phenyl chlorocarbonate (0.0854 mL, 0.681 mmol) and tert-butyl 2-methylalaninate (178 mg, 0.908 mmol).
EI(pos) 626.0 $[M+H]^+$

Example 238

N-({3-[(6-fluoro-4-oxo-3,4-dihydro-1'H-spiro[chromene-2,4'-piperidin]-1'-yl)carbonyl]-7-methoxy-1-benzothiophen-2-yl}carbamoyl)-2-methylalanine
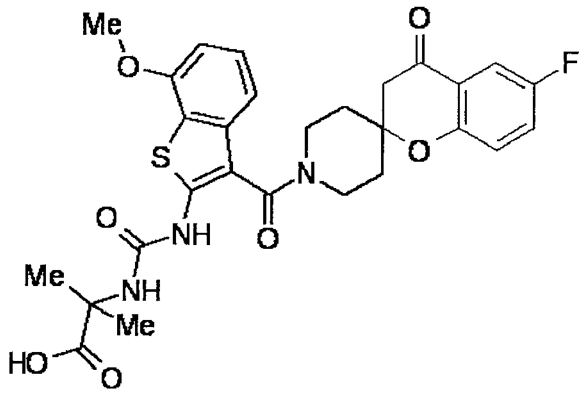

To tert-butyl N-({3-[(6-fluoro-4-oxo-3,4-dihydro-1'H-spiro[chromene-2,4'-piperidin]-1'-yl)carbonyl]-7-methoxy-1-benzothiophen-2-yl}carbamoyl)-2-methylalaninate (253 mg, 0.404 mmol) obtained in Example 237 was added TFA (2 mL), and the mixture was stirred at room temperature for 1 hr and concentrated under reduced pressure. To the obtained residue was added diisopropyl ether to give a powder (143 mg). The obtained powder (139 mg) was dissolved in a mixed solvent of methanol (2 mL) and THF (1 mL), 2N aqueous sodium hydroxide solution (0.488 mL, 0.976 mmol) was added, and the mixture was stirred with heating at 60°C for 1 hr. The reaction mixture was allowed to cool, 1N hydrochloric acid was added, and the mixture was extracted with ethyl acetate. The extract was washed with saturated brine, and dried over anhydrous sodium sulfate. The solution was concentrated, and the obtained residue was triturated with diisopropyl ether to give the title compound 108 mg (2 steps, yield 47%).
EI(pos) 569.9 $[M+H]^+$

Example 239

tert-butyl N-({3-[(6-fluoro-4-oxo-3,4-dihydro-1'H-spiro[chromene-2,4'-piperidin]-1'-yl)carbonyl]-7-methoxy-1-benzothiophen-2-yl}carbamoyl)glycinate
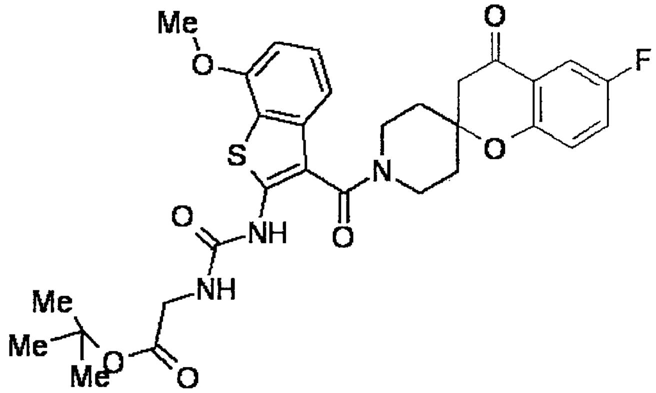

The title compound (224 mg, yield 83%) was obtained as a powder in the same manner as in Example 233 and using 1'-[(2-amino-7-methoxy-1-benzothiophen-3-yl)carbonyl]-6-fluorospiro[chromene-2,4'-piperidin]-4(3H)-one (200 mg, 0.454 mmol) obtained in Example 232, phenyl chlorocarbonate (0.0854 mL, 0.681 mmol) and tert-butyl glycinate (152 mg, 0.908 mmol).
EI(pos) 598.0 $[M+H]^+$

Example 240

N-({3-[(6-fluoro-4-oxo-3,4-dihydro-1'H-spiro[chromene-2,4'-piperidin]-1'-yl)carbonyl]-7-methoxy-1-benzothiophen-2-yl}carbamoyl)glycine

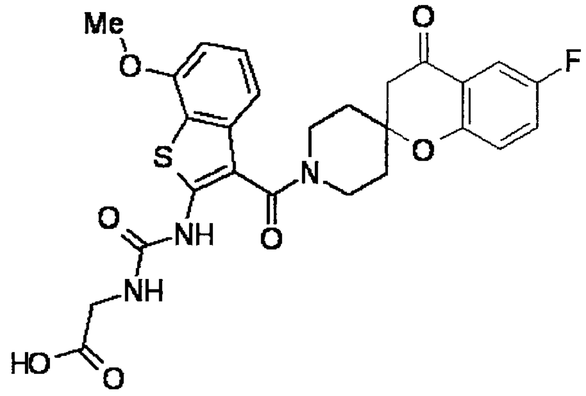

The title compound (224 mg, yield 83%) was obtained as a powder in the same manner as in Example 234 and using tert-butyl N-({3-[(6-fluoro-4-oxo-3,4-dihydro-1'H-spiro[chromene-2,4'-piperidin]-1'-yl)carbonyl]-7-methoxy-1-benzothiophen-2-yl}carbamoyl)glycinate (222 mg, 0.371 mmol) obtained in Example 239 and TFA (2 mL).
EI(pos) 541.9 $[M+H]^+$

Example 241

tert-butyl 2-methyl-N-({6-methyl-3-[(6'-methyl-4'-oxo-3',4'-dihydro-1H-spiro[piperidine-4,2'-thiochromen]-1-yl)carbonyl]thieno[2,3-b]pyridin-2-yl}carbamoyl)alaninate
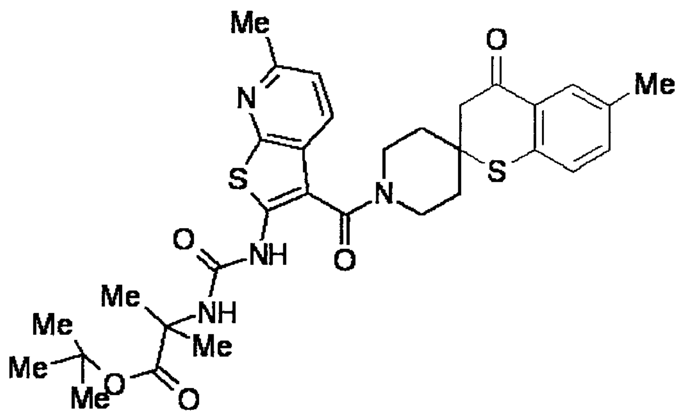

The title compound (199 mg, yield 70%) was obtained as an oil in the same manner as in Example 233 and using 1-[(2-amino-6-methylthieno[2,3-b]pyridin-3-yl)carbonyl]-6'-methylspiro[piperidine-4,2'-thiochromen]-4'(3'H)-one (199 mg, 0.454 mmol) obtained in Example 157, phenyl chlorocarbonate (0.0854 mL, 0.681 mmol) and tert-butyl 2-methylalaninate (178 mg, 0.908 mmol).
EI(pos) 623 $[M+H]^+$

Example 242

2-methyl-N-({6-methyl-3-[(6'-methyl-4'-oxo-3',4'-dihydro-1H-spiro[piperidine-4,2'-thiochromen]-1-yl)carbonyl]thieno[2,3-b]pyridin-2-yl}carbamoyl)alanine trifluoroacetate
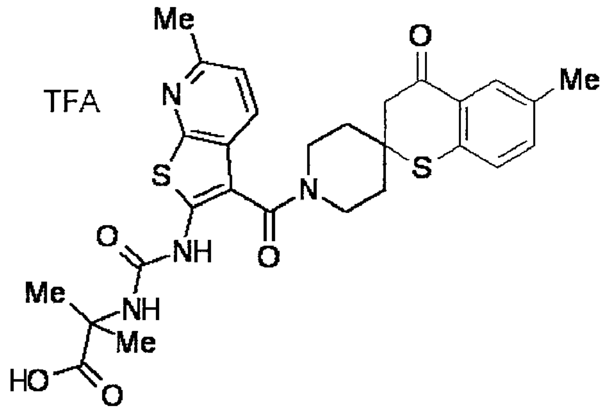

The title compound (186 mg, yield 86%) was obtained as a powder in the same manner as in Example 234 and using tert-butyl 2-methyl-N-({6-methyl-3-[(6'-methyl-4'-oxo-3',4'-dihydro-1H-spiro[piperidine-4,2'-thiochromen]-1-yl) carbonyl]thieno[2,3-b]pyridin-2-yl}carbamoyl)alaninate (198 mg, 0.318 mmol) obtained in Example 241 and TFA (2 mL).
EI(pos) 566.9 $[M+H]^+$

Example 243

1'-[(2-amino-7-oxo-4,5,6,7-tetrahydro-1-benzothiophen-3-yl)carbonyl]-6-(trifluoromethyl)spiro[chromene-2,4'-piperidin]-4(3H)-one
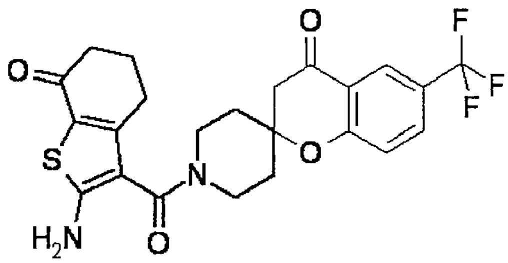

The title compound (412 mg, yield 42%) was obtained as a powder in the same manner as in Example 1 and from 6-(trifluoromethyl)spiro[chromene-2,4'-piperidin]-4(3H)-one hydrochloride (400 mg, 1.24 mmol) and 2-amino-7-oxo-4,5,6,7-tetrahydro-1-benzothiophene-3-carboxylic acid (263 mg, 1.24 mmol) (produced by the method described in WO07/013691).
EI(pos) 479.1 $[M+H]^+$

Example 244

1-ethyl-3-(7-oxo-3-{[4-oxo-6-(trifluoromethyl)-3,4-dihydro-1'H-spiro[chromene-2,4'-piperidin]-1'-yl]carbonyl}-4,5,6,7-tetrahydro-1-benzothiophen-2-yl)urea
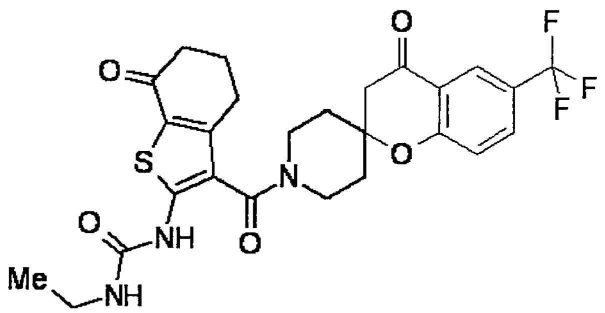

The title compound (168 mg, yield 73%) was obtained as a powder in the same manner as in Example 2 and from 1'-[(2-amino-7-oxo-4,5,6,7-tetrahydro-1-benzothiophen-3-yl)carbonyl]-6-(trifluoromethyl)spiro[chromene-2,4'-piperidin]-4(3H)-one (200 mg, 0.418 mmol) obtained in Example 243 and ethyl isocyanate (0.132 mL, 1.67 mmol).
EI(pos) 550.0 $[M+H]^+$

Experimental Example 1

The ACC1 inhibitory action of the compound of the present invention was evaluated by the following method.

(1) Cloning of human ACC1 gene and preparation of recombinant Baculovirus

Human ACC1 gene was cloned by PCR using a human liver cDNA library (Clontech) as a template and Primer 1 and Primer 2 shown below. Primer 1 and Primer 2 were prepared by adding SalI, NotI restriction enzyme recognition sequence based on the information of the base sequence (Genbank Accession U19822) of human ACC1 gene.

Primer 1 5'AAAAGTCGACCCACCATGGATGAACCTTCTCCCTTGGCCC (SEQ ID NO:1)
Primer 2 5'AAAAGCGGCCGCCTACGTAGAAGGGGAGTCCATAGTG (SEQ ID NO:2)

PCR was performed using a Pyrobest DNA polymerase (TAKARA BIO INC.) The obtained PCR product was cloned to pT7 Blue vector (Novagen) and, after confirmation of the base sequence, digested with restriction enzymes SalI and NotI. The obtained DNA fragment was inserted to pFAST-BacHTc (Invitrogen) digested with restriction enzymes SalI and NotI to give expression plasmid ACC1/pFAST-BacHTc.
Using the expression plasmid and BAC-TO-BAC Baculovirus Expression System (Invitrogen), virus stock BAC-ACC1 of recombinant Baculovirus was prepared.

(2) Preparation of ACC1 protein

SF-9 cells (Invitrogen) were inoculated to a medium (1 L) for insecT cells (Sf-900IISFM medium (Invitrogen) containing 10% fetal bovine serum (Trace), 50 mg/L Gentamicin (Invitrogen) and 0.1% Pluronic F-68 (Invitrogen)) at $1\times10^6$ cells/mL, and cultured with shaking at 27°C, 100 rpm in a 2 L Erlenmeyer flask.
After 24 hr of the culture, recombinant Baculovirus BAC-ACC1 (10 mL) was added, and the cells were further cultured for 3 days. The culture medium was centrifuged at 1000×g for 5 min to give virus-infected cells. The cells were washed with phosphate buffered saline (Invitrogen), and centrifuged under the same conditions, and the obtained cells were cryopreserved at -80°C.
The cryopreserved cells were thawed in ice and suspended in 100 mL of 25 mM HEPES buffer (pH 7.5) containing 10% Glycerol, 0.13 M NaCl, 1 mM EDTA, 25 mM Sodium β-Glycerophosphate and 1 mM Sodium Orthovanadate, and supplemented with Complete Protease Inhibitor (Nippon Boehringer Ingelheim Co., Ltd.). The obtained suspension was homogenized 3 times in a polytron homogenizer (Kinematica) at 20,000 rpm for 30 sec. The obtained cell disruption solution was clarified by centrifugation at 185700×g for 50 min, and filtered through a 0.45 μm filter. The filtrate was passed through a column packed with 12 mL of Ni-NTA Super Flow Gel (QUIAGEN) at a flow rate of about 5 mL/min. The column was washed with buffer A (50 mM HEPES (pH 7.5) containing 0.3 M NaCl), further washed with buffer A containing 20 mM Imidazole, and eluted with buffer A containing 100 mM Imidazole. The eluate was concentrated with Vivaspin 20 (Vivascience) with a molecular weight cut off of 30K. The obtained concentrate was dialyzed against Sephadex G-25 (Amersham Biosciences, 358 my) equilibrated with 50 mM HEPES (pH 7.5) containing 10 mM $MgCl_2$, 2 mM Dithiothreitol, 10 mM Tripotassium Citrate and 0.3 M NaCl. The inner dialysate was concentrated with Vivaspin 20 (Vivascience) with a molecular weight cut off of 30K, and the concentrate was filtered through a 0.22 μm filter to give

ACC1. The obtained ACC1 was cryopreserved at -80°C.

(3) Measurement of ACC1 inhibitory activity

ACC1 (0.93 mg/ml) obtained in the above-mentioned (2) was diluted with an enzyme reaction buffer (50 mM HEPES (pH 7.5), 10 mM $MgCl_2$, 10 mM Tripottasium Citrate, 2 mM Dithiothreitol, 0.75 mg/ml Fatty acid free BSA) to concentration of 8 μg/ml, and the mixture was added to each well of 384 well assay plate (Nunc 265196) by 10 μl. Thereafter, ACC1 inhibitory rate (%) was measured in the same manner as in the below-mentioned Experimental Example 2-(3), and $IC_{50}$ value was calculated. As a result, the compounds of Examples 2, 12 - 16, 18 - 21, 24, 27, 30, 37, 42, 43, 68, 69, 74, 79, 82, 83, 87, 91 - 94, 97, 112, 116, 122, 124 - 126, 129, 131, 132, 134 - 137, 140, 147, 150 to 152, 154, 1156, 158, 160, 165, 166, 168 - 170, 173, 174, 177, 180, 181, 183, 186, 188, 191, 195, 196, 198, 200, 203, 214, 215, 218, 219, 223, 225, 229, 231, 234, 236, 238, 240 and 242 showed an $IC_{50}$ value of 1 to 100 nM.
As shown above, the compound of the present invention has a superior ACC1 inhibitory action.

Experimental Example 2

The ACC2 inhibitory action of the compound of the present invention was evaluated by the following method.

(1) Cloning of human ACC2 gene and preparation of recombinant Baculovirus

Human ACC2 gene was cloned by PCR using a human skeletal muscle cDNA library (Clontech) as a template and Primer 1 and Primer 2 shown below. Primer 1 and Primer 2 were prepared by adding SalI, XbaI restriction enzyme recognition sequences based on the information of the base sequence (Genbank Accession U89344) of human ACC2 gene.

Primer 1 5'AAAAGTCGACCCACCATGGTCTTGCTTCTTTGTCTATCTTG (SEQ ID NO:3)
Primer 2 5'TTTTTCTAGATCAGGTAGAGGCCGGGCTGTCCATG (SEQ ID NO:4)

PCR was performed using Pyrobest DNA polymerase (TAKARA BIO INC.). The obtained PCR product was cloned to pT7 Blue vector (Novagen) and, after confirmation of the base sequence, digested with restriction enzymes SalI and XbaI. The obtained DNA fragment was inserted into pFAST-BacHTa (Invitrogen) digested with restriction enzymes SalI and XbaI to give expression plasmid ACC2/pFAST-BacHTa.
PCR was performed using the expression plasmid as a template and Primer 3 and Primer 4 shown below to prepare a plasmid to be used for expression of ACC2 free of mitochondrial targeting sequence.

Primer 3 5'CCAGGTCGACCCGCCAACGGGACTGGGACACAAGG (SEQ ID NO:5)
Primer 4 5'CGCACTCTCAGTTTCCCGGATTCCC (SEQ ID NO:6)

PCR was performed using Pyrobest-DNA polymerase (TAKARA BIO INC.). The obtained PCR product was cloned to pT7 Blue vector (Novagen) and, after confirmation of the base sequence, digested with restriction enzymes SalI and AflII. The obtained DNA fragment was inserted into pFAST-BacHTa (Invitrogen) digested with restriction enzymes SalI and AflII to give expression plasmid ACC2mito7/pFAST-BacHTa.
Using the expression plasmid and BAC-TO-BAC Baculovirus Expression System (Invitrogen), virus stock BAC-ACC2 (N terminal deleted (hereinafter Nd)) of recombinant Baculovirus was prepared.

(2) Preparation of ACC2 (Nd) protein

SF-9 cells (Invitrogen) were inoculated to a medium (2 L) for insecT cells (Sf-900IISFM medium (Invitrogen) containing 10% fetal bovine serum (Trace), 50 mg/L Gentamicin (Invitrogen), 0.1% Pluronic F-68 (Invitrogen)) at $0.5\times10^6$ cells/mL, and cultured with shaking in Wave Bioreactor (Wave) at 27°C, 20 rpm, rocking angle 6°, oxygen concentration 30%.
On day 4 of the culture, 3 L of the medium for insecT cells was added, the rocking angle was set to 8°, and the cells were cultured. On day 5 of the culture, 100 mL of recombinant Baculovirus BAC-ACC2 (Nd) was added, 5 L of the medium for insecT cells was further added, the rocking angle was set to 11°, and the cells were cultured for 3 days. The culture medium was centrifuged at 1000xg for 10 min to give virus-infected cells. The cells were washed with phosphate buffered saline (Invitrogen) and centrifuged under the same conditions. The obtained cells were cryopreserved at -80°C. The cryopreserved cells were thawed in ice and suspended in 900 mL of 25 mM HEPES buffer (pH 7.5) containing 10% Glycerol, 0.13 M NaCl, 1 mM EDTA, 25 mM Sodium β-Glycerophosphate and 1 mM Sodium Orthovanadate, and

supplemented with Complete Protease Inhibitor (Nippon Boehringer Ingelheim Co., Ltd.). The obtained suspension was homogenized 3 times in a polytron homogenizer (Kinematica) at 20,000 rpm for 30 sec. The obtained cell disruption solution was clarified by centrifugation at 31000xg for 60 min, and filtered through a 0.45 μm filter. The filtrate was passed through a column packed with 60 mL of Ni-NTA Super Flow Gel (QUIAGEN) at a flow rate of about 5 mL/min. The column was washed with buffer A (50 mM HEPES (pH 7.5) containing 0.3 M NaCl), further washed with buffer A containing 20 mM Imidazole, and eluted with buffer A containing 100 mM Imidazole. The eluate was concentrated with Vivaspin 20 (Vivascience) with a molecular weight cut off of 30K. The obtained concentrate was dialyzed against 50 mM HEPES (pH 7.5) containing 10 mM $MgCl_2$, 2 mM Dithiothreitol, 10 mM Tripotassium Citrate and 0.3 M NaCl. The inner dialysate was filtered through a 0.22 μm filter to give ACC2 (Nd). The obtained ACC2 (Nd) was cryopreserved at - 80°C.

(3) Measurement of ACC2 inhibitory activity

ACC2 (Nd) (1.1 mg/ml) obtained in the above-mentioned (2) was diluted with an enzyme reaction buffer (50 mM HEPES (pH 7.5), 10 mM $MgCl_2$, 10 mM Tripottasium Citrate, 2 mM Dithiothreitol, 0.75 mg/ml Fatty acid free BSA) to a concentration of 6.4 μg/ml, and the mixture was added to each well of a 384 well assay plate (Nunc 265196) by 10 μl. A test compound was dissolved in dimethyl sulfoxide (DMSO) and the mixture was diluted with an enzyme reaction buffer and the resulting solution (5 μl) was added to each well. The mixture was incubated at 30°C for 60 min. Then, a substrate solution (50 mM $KHCO_3$, 200 μM ATP, 200 μM Acetyl-CoA, 5 μl) was added to each well, and the mixture was reacted at 30°C for 20 min (test compound addition group).
In addition, a reaction was performed in the same manner as above and without adding the test compound (test compound non-addition group).
Furthermore, a reaction was performed in the same manner as above and without adding the test compound and Acetyl-CoA (control group).
The reaction was quenched by adding a malachite green solution to each of the obtained reaction mixtures by 5 μl and stirring the mixtures. The obtained reaction mixture was left standing at room temperature for 20 min, and absorbance (620 nm) was measured using wallac1420 (PerkinElmer Japan Co., Ltd.). The above-mentioned malachite green solution was prepared by mixing Solution A (0.12% malachite green solution, prepared with 5N $H_2SO_4$, preserved at 4°C in shading), Solution B (7.5% aqueous ammonium molybdate solution, prepared when in use) and Solution C (11% aqueous Tween 20 solution, preserved at room temperature) at a ratio of Solution A: Solution B: Solution C=100:25:2 (volume ratio).
Then, ACC1 inhibitory rate (%) was obtained from the formula:

(1-(absorbance of test compound addition group - absorbance of control group)÷(absorbance of test compound non-addition group - absorbance of control group))×100,

and $IC_{50}$ value was calculated. As a result, the compounds of Examples 2, 5, 8, 12 - 16, 18 - 21, 24, 27, 30, 37, 42, 43, 68, 69, 74, 79, 82, 83, 87, 91 - 94, 97, 112, 116, 122, 124 - 126, 129, 131, 132, 134 - 137, 140, 147, 150 to 152, 154, 1156, 158, 160, 165, 166, 168 - 170, 173, 174, 177, 180, 181, 183, 186, 188, 191, 195, 196, 198, 200, 203, 214, 215, 218, 219, 223, 225, 229, 231, 234, 236, 238, 240 and 242 showed $IC_{50}$ values of 1 to 100 nM.
As shown above, the compound of the present invention has a superior ACC2 inhibitory action.

Formulation Example 1 (production of capsules)

| | |
|---|---|
| 1) compound of Example 1 | 30 mg |
| 2) finely divided powder cellulose | 10 mg |
| 3) lactose | 19 mg |
| 4) magnesium stearate | 1 mg |
| | total 60 mg |

1), 2), 3) and 4) are mixed and filled in a gelatin capsule.

| Formulation Example 2 (production of tablets) | |
|---|---|
| 1) compound of Example 1 | 30 g |
| 2) lactose | 50 g |
| 3) cornstarch | 15 g |
| 4) calcium carboxymethylcellulose | 44 g |
| 5) magnesium stearate | 1 g |
| 1000 tablets | total 140 g |

The total amount of 1), 2) and 3) and 4) (30 g) is kneaded with water, vacuum dried, and sieved.
The sieved powder is mixed with 4) (14 g) and 5) (1 g), and punched by a tableting machine, whereby 1000 tablets containing 30 mg of the compound of Example 1 per tablet are obtained.

**Industrial Applicability**

The compound of the present invention has ACC (acetyl-CoA carboxylase) inhibitory action, and is useful for the prophylaxis or treatment of obesity, diabetes, hypertension, hyperlipidemia, cardiac failure, diabetic complications, metabolic syndrome, sarcopenia, cancer and the like.
This application is based on patent application No. 39946/2007 filed in Japan, the contents of which are hereby incorporated by reference.

## Claims

1. A compound represented by the formula (I):

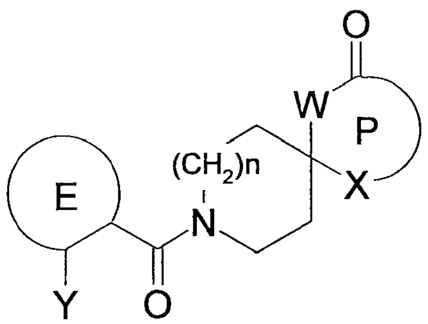

(I)

wherein

ring E is an optionally further substituted 6-membered aromatic ring, or an optionally fused 5-membered aromatic heterocycle which is optionally further substituted;
ring P is an optionally fused non-aromatic ring which is optionally further substituted;
W is O, S, a $C_{1-4}$ alkylene or $NR^{3a}$ wherein $R^{3a}$ is a hydrogen atom or a substituent;
X is O, S, SO, $SO_2$, CO, $CR^1R^2$ or $NR^{3b}$ wherein $R^1$, $R^2$ and $R^{3b}$ are the same or different and each is a hydrogen atom or a substituent;
Y is an optionally substituted amino group; and
n is 0, 1, 2 or 3,
or a salt thereof.

2. The compound of claim 1, wherein the optionally fused 5-membered aromatic heterocycle of the optionally fused 5-membered aromatic heterocycle which is optionally further substituted for ring E is an optionally fused thiophene ring.

3. The compound of claim 1, wherein the 6-membered aromatic ring of the optionally further substituted 6-membered

aromatic ring for ring E, or the optionally fused 5-membered aromatic heterocycle of the optionally fused 5-membered aromatic heterocycle which is optionally further substituted for ring E is a benzene ring, a pyridine ring, a thiophene ring, a benzothiophene ring or a thienopyridine ring.

**4.** The compound of claim 1, wherein the optionally fused non-aromatic ring of the optionally fused non-aromatic ring which is optionally further substituted for ring P is a dihydrochromene ring, an optionally oxidized dihydrothiochromene ring or a piperidine ring, each of which is substituted by oxo group(s).

**5.** A compound represented by the formula (IIa):

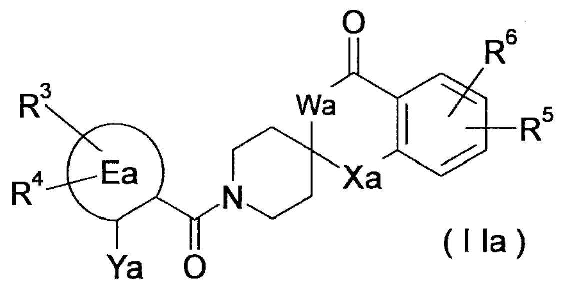

(I Ia)

wherein

ring Ea is a thiophene ring optionally condensed with a benzene ring or a pyridine ring, or a pyridine ring;
Wa is O, S, $CH_2$ or NH;
Xa is O, S, SO, $SO_2$, CO, $CH_2$ or NH;
Ya is $-NH_2$, -NHCONHR, -NHCOOR or -NHCOR;
$R^3$ to $R^6$ are the same or different and each is a hydrogen atom, a halogen atom, a $C_{1-6}$ alkyl group, a $C_{1-6}$ alkoxy group, a cyano group, a nitro group, an amino group, a carboxy group, a hydroxy group, -COR, $-NHSO_2R$, -NHCONHR, $-C_{1-6}$ alkylene-COOH, $-C_{1-6}$
alkylene-COOR, $-0-C_{1-6}$ alkylene-$C_{6-14}$ arene, -CONH-(5- or 6-membered heterocycle), a 5- or 6-membered heterocyclic group or a $C_{6-14}$ aryl group; and
R is a hydrogen atom or a $C_{1-6}$ alkyl group,
or a salt thereof.

**6.** The compound of claim 5, wherein ring Ea is a thiophene ring optionally condensed with a benzene ring or a pyridine ring.

**7.** The compound of claim 5, wherein ring Ea is a thiophene ring, a benzothiophene ring or a thienopyridine ring.

**8.** 1'-({7-Methoxy-2-[(methylcarbamoyl) amino]-1-benzothiophen-3-yl}carbonyl)-4-oxo-3,4-dihydrospiro[chromene-2,4'-piperidine]-6-carboxylic acid,
1-ethyl-3-(3-{[4'-oxo-6'-(5-oxo-4,5-dihydro-1,2,4-oxadiazol-3-yl)-3',4'-dihydro-1H-spiro[piperidine-4,2'-thiochromen]-1-yl]carbonyl}-1-benzothiophen-2-yl)urea,
4-{5-[(ethylcarbamoyl) amino]-4-[(4'-oxo-3',4'-dihydro-1H-spiro[piperidine-4,2'-thiochromen]-1-yl) carbonyl] thiophen-2-yl}benzoic acid,
N-{3-[(6-cyano-4-oxo-3,4-dihydro-1'H-spiro[chromene-2,4'-piperidin]-1'-yl)carbonyl]-6-methoxy-1-benzothiophen-2-yl}acetamide,
N-[({3-[(6-cyano-4-oxo-3,4-dihydro-1'H-spiro[chromene-2,4'-piperidin]-1'-yl) carbonyl]-7-methoxy-1-benzothiophen-2-yl}amino)carbonyl]methanesulfonamide,
1-{3-[(6'-cyano-4'-oxo-3',4'-dihydro-1H-spiro[piperidine-4,2'-thiochromen]-1-yl)carbonyl]-6-(trifluoromethyl)thieno[2,3-b]pyridin-2-yl}-3-methylurea,
1-{3-[(6-methyl-4-oxo-3,4-dihydro-1'H-spiro[chromene-2,4'-piperidin]-1'-yl)carbonyl]-6-(trifluoromethyl)thieno[2,3-b]pyridin-2-yl}-3-methylurea,
1-{3-[(6-cyano-4-oxo-3,4-dihydro-1'H-spiro[chromene-2,4'-piperidin]-1'-yl)carbonyl]-6-(trifluoromethyl)thieno[2,3-b]pyridin-2-yl}-3-methylurea,
1-{3-[(6-cyano-4-oxo-3,4-dihydro-1'H-spiro[chromene-2,4'-piperidin]-1'-yl)carbonyl]-6-(trifluoromethyl)thieno[2,3-b]pyridin-2-yl}-3-ethylurea,

1-ethyl-3-{3-[(6-methyl-4-oxo-3,4-dihydro-1'H-spiro[chromene-2,4'-piperidin]-1'-yl)carbonyl]-7-oxo-4,5,6,7-tetrahydro-1-benzothiophen-2-yl}urea, or
1- ethyl- 3-(7- oxo- 3- { [4- oxo- 6-(trifluoromethyl)- 3,4- dihydro- 1'H- spiro [chromene- 2,4'- piperidin]- 1'- yl] carbonyl}-4,5,6,7-tetrahydro-1-benzothiophen-2-yl)urea
or a salt thereof.

**9.** A prodrug of the compound of claim 1 or 5.

**10.** A pharmaceutical agent comprising the compound of claim 1 or 5 or a prodrug thereof.

**11.** The pharmaceutical agent of claim 10, which is an acetyl-CoA carboxylase inhibitor.

**12.** The pharmaceutical agent of claim 10, which is an agent for the prophylaxis or treatment of obesity, diabetes, hypertension, hyperlipidemia, cardiac failure, diabetic complications, metabolic syndrome, sarcopenia or cancer.

**13.** Use of the compound of claim 1 or 5 or a prodrug thereof, for the production of an acetyl-CoA carboxylase inhibitor.

**14.** use of the compound of claim 1 or 5 or a prodrug thereof, for the production of an agent for the prophylaxis or treatment of obesity, diabetes, hypertension, hyperlipidemia, cardiac failure, diabetic complications, metabolic syndrome, sarcopenia or cancer.

**15.** A method of inhibiting acetyl-CoA carboxylase in a mammal, which comprises administering the compound of claim 1 or 5 or a prodrug thereof to the mammal.

**16.** A method for the prophylaxis or treatment of obesity, diabetes, hypertension, hyperlipidemia, cardiac failure, diabetic complications, metabolic syndrome, sarcopenia or cancer in a mammal, which comprises administering the compound of claim 1 or 5 or a prodrug thereof to the mammal.

## INTERNATIONAL SEARCH REPORT

International application No. PCT/JP2008/052708

A. CLASSIFICATION OF SUBJECT MATTER
*C07D471/10*(2006.01)i, *A61K31/438*(2006.01)i, *A61K31/537*(2006.01)i, *A61P1/00* (2006.01)i, *A61P1/12*(2006.01)i, *A61P1/16*(2006.01)i, *A61P3/00*(2006.01)i, *A61P3/04*(2006.01)i, *A61P3/06*(2006.01)i, *A61P3/10*(2006.01)i,

According to International Patent Classification (IPC) or to both national classification and IPC

B. FIELDS SEARCHED

Minimum documentation searched (classification system followed by classification symbols)
C07D471/10, A61K31/438, A61K31/537, A61P1/00, A61P1/12, A61P1/16, A61P3/00, A61P3/04, A61P3/06, A61P3/10, A61P5/48, A61P7/00, A61P9/04, A61P9/10, A61P9/12, A61P13/12, A61P15/00, A61P19/10, A61P21/04, A61P25/16, A61P25/22,

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2008 |
| Kokai Jitsuyo Shinan Koho | 1971-2008 | Toroku Jitsuyo Shinan Koho | 1994-2008 |

Electronic data base consulted during the international search (name of data base and, where practicable, search terms used)
CAplus(STN), CAOLD(STN), REGISTRY(STN), MEDLINE(STN), BIOSIS(STN), EMBASE(STN), WPIDS(STN)

C. DOCUMENTS CONSIDERED TO BE RELEVANT

| Category* | Citation of document, with indication, where appropriate, of the relevant passages | Relevant to claim No. |
|---|---|---|
| X<br>Y<br>A | JP 49-25673 B1 (Yoshitomi Pharmaceutical Industries, Ltd.), 02 July, 1974 (02.07.74), Full text; particularly, page 1, general formula [II]; column 1, line 36 to column 2, line 1; column 2, lines 15 to 17; column 3, lines 7 to 9 (Family: none) | 1-3,9,10,12, 14<br>11,13<br>4-8 |
| X<br>Y<br>A | JP 48-31114 B1 (Yoshitomi Pharmaceutical Industries, Ltd.), 26 September, 1973 (26.09.73), Full text; particularly, page 1, column 1, general formula [II]; column 2, lines 1 to 4; column 2, lines 18 to 20; column 3, lines 14 to 16 (Family: none) | 1-3,9,10,12, 14<br>11,13<br>4-8 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>19 March, 2008 (19.03.08) | Date of mailing of the international search report<br>01 April, 2008 (01.04.08) |
| Name and mailing address of the ISA/<br>Japanese Patent Office<br>Facsimile No. | Authorized officer<br><br>Telephone No. |

Form PCT/ISA/210 (second sheet) (April 2007)

**INTERNATIONAL SEARCH REPORT**

International application No. PCT/JP2008/052708

C (Continuation). DOCUMENTS CONSIDERED TO BE RELEVANT

| Category* | Citation of document, with indication, where appropriate, of the relevant passages | Relevant to claim No. |
|---|---|---|
| X<br><br>Y<br>A | WO 2004/022554 A1 (CELLTECH R & D LTD.),<br>18 March, 2004 (18.03.04),<br>Full text; particularly, pages 21 to 22;<br>example 30; Claims<br>& AU 2003263323 A | 1-3,9,10,12,<br>14<br>11,13<br>4-8 |
| Y<br>A | WO 2007/011809 A1 (MERCK & CO., INC.),<br>25 January, 2007 (25.01.07),<br>Full text<br>& US 2007/21453 A1 & AU 2006270145 A | 11,13<br>1-10,12,14 |
| Y<br>A | WO 2007/011811 A1 (MERCK & CO., INC.),<br>25 January, 2007 (25.01.07),<br>Full text<br>& US 2007/21453 A1 | 11,13<br>1-10,12,14 |
| Y<br>A | JP 2005-119987 A (Ajinomoto Co., Inc.),<br>12 May, 2005 (12.05.05),<br>Full text<br>(Family: none) | 11,13<br>1-10,12,14 |
| Y<br>A | WO 2007/013691 A1 (Takeda Chemical Industries,<br>Ltd.),<br>01 February, 2007 (01.02.07),<br>Full text<br>(Family: none) | 11,13<br>1-10,12,14 |

Form PCT/ISA/210 (continuation of second sheet) (April 2007)

**INTERNATIONAL SEARCH REPORT**

International application No.
PCT/JP2008/052708

**Box No. II Observations where certain claims were found unsearchable (Continuation of item 2 of first sheet)**

This international search report has not been established in respect of certain claims under Article 17(2)(a) for the following reasons:

1. [x] Claims Nos.: 15, 16
because they relate to subject matter not required to be searched by this Authority, namely:
Claims 15 and 16 involve embodiments concerning methods for treatment of the human body by therapy and thus relate to a subject matter which this International Searching Authority is not required, under the provisions of Article 17(2)(a)(i) of the PCT (continued to extra sheet)

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.
2. [ ] As all searchable claims could be searched without effort justifying additional fees, this Authority did not invite payment of additional fees.
3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** the
[ ] The additional search fees were accompanied by the applicant's protest and, where applicable, payment of a protest fee.
[ ] The additional search fees were accompanied by the applicant's protest but the applicable protest fee was not paid within the time limit specified in the invitation.
[ ] No protest accompanied the payment of additional search fees.

Form PCT/ISA/210 (continuation of first sheet (2)) (April 2007)

**INTERNATIONAL SEARCH REPORT**

International application No.
PCT/JP2008/052708

Continuation of Box No.II-1 of continuation of first sheet(2)

and Rule 39.1(iv) of the Regulations under the PCT, to search.

Form PCT/ISA/210 (extra sheet) (April 2007)

INTERNATIONAL SEARCH REPORT

International application No.
PCT/JP2008/052708

Continuation of A. CLASSIFICATION OF SUBJECT MATTER
(International Patent Classification (IPC))

*A61P5/48*(2006.01)i, *A61P7/00*(2006.01)i, *A61P9/04*(2006.01)i, *A61P9/10*(2006.01)i, *A61P9/12*(2006.01)i, *A61P13/12*(2006.01)i, *A61P15/00*(2006.01)i, *A61P19/10*(2006.01)i, *A61P21/04*(2006.01)i, *A61P25/16*(2006.01)i, *A61P25/22*(2006.01)i, *A61P25/28*(2006.01)i, *A61P29/00*(2006.01)i, *A61P35/00*(2006.01)i, *A61P43/00*(2006.01)i, *C07D491/107*(2006.01)i, *C07D495/10*(2006.01)i, *C07D498/10*(2006.01)i, *C07D519/00*(2006.01)i

(According to International Patent Classification (IPC) or to both national classification and IPC)

Continuation of B. FIELDS SEARCHED
Minimum documentation searched (International Patent Classification (IPC))

A61P25/28, A61P29/00, A61P35/00, A61P43/00, C07D491/107, C07D495/10, C07D498/10, C07D519/00

Minimum documentation searched (classification system followed by classification symbols)

Form PCT/ISA/210 (extra sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2007011809 A
- WO 2007011811 A
- JP 2005119987 A
- WO 2007013691 A
- WO 0114372 A
- WO 9710224 A
- WO 0182925 A
- WO 0187834 A
- WO 2006053024 A2
- WO 2004000846 A
- WO 07013691 A
- WO 07119833 A
- JP 2007039946 A


### Non-patent literature cited in the description


- IYAKUHIN no KAIHATSU. Design of Molecules. HIROKAWA SHOTEN, 1990, vol. 7, 163-198
- *Journal of Medicinal Chemistry,* 1998, 2439-2441
- Protective Groups in Organic Synthesis. John Wiley and Sons, 1980
- *Journal of Medicinal Chemistry,* 1998, 4118-4129
- *Journal of Medicinal Chemistry,* 1988, 486-491
- *Journal of Organic Chemistry,* 2004, 2441-2450
- Jikken Kagaku Kouza. vol. 20, 304, 477-479
- *Heterocycles,* 1992, 2263-2267
- *Tetrahedron Letters,* 2001, 8345-8349